# EUROPEAN PATENT SPECIFICATION

(11) **EP 2 082 235 B1**
(45) Date of publication and mention of the grant of the patent: **03.10.2018**
(21) Application number: 07815354.1
(22) Date of filing: 11.10.2007
(51) Int. Cl.: G01N 33/574, C12Q 1/68, G01N 33/68

(54) **A METHOD OF DIAGNOSIS AND AGENTS USEFUL FOR SAME**
DIAGNOSEVERFAHREN UND DAFÜR GEEIGNETE AGENTIEN
PROCEDE DE DIAGNOSTIC ET AGENTS CONVENANT A CET EFFET

(30) Priority: 11.10.2006 US 851277 P
(43) Date of publication of application: 29.07.2009
(73) Proprietor: MEDVET SCIENCE PTY. LTD., Underdale SA 5032 (AU)
(72) Inventor: BROWN, Michael, Paul, St. Georges, South Australia 5064 (AU); AL-EJEH, Fares, Brisbane Queensland 4006 (AU); DARBY, Jocelyn Margaret, Ferntree Tasmania 7054 (AU)
(74) Representative: Dehns
(86) International application number: PCT/AU2007/001549
(87) International publication number: WO 2008/043153

(56) References cited:
- WO-A1-2004/073739
- DE-A1- 19 546 339
- TROTTA ROSSANA ET AL: "BCR/ABL activates mdm2 mRNA translation via the La antigen" CANCER CELL, CELL PRESS, US, vol. 3, no. 2, 1 February 2003 (2003-02-01), pages 145-160, XP002397382 ISSN: 1535-6108
- AL-EJEH F. ET AL.: 'In vivo Targeting of Dead Tumor Cells in a Murine Tumor Model Using a Monoclonal Antibody Specific for the La Autoantigen' CLIN. CANCER RES. vol. 13, no. 18, SUPPL., 15 September 2007, pages 5519S - 5527S, XP008105259
- AL-EJEH F. ET AL.: 'The La Autoantigen Is a Malignancy-Associated Cell Death Target That is Induced by DNA-Damaging Drugs' CLIN. CANCER RES. vol. 13, no. 18, SUPPL., 15 September 2007, pages 5509S - 5518S, XP008105261
- AL-EJEH FARES ET AL: "Postchemotherapy and Tumor-Selective Targeting with the La-Specific DAB4 Monoclonal Antibody Relates to Apoptotic Cell Clearance", JOURNAL OF NUCLEAR MEDICINE, vol. 55, no. 5, May 2014 (2014-05), pages 772-779, ISSN: 0161-5505(print)
- "Oral presentations", CANCER BIOTHERAPY & RADIOPHARMACEUTICALS, vol. 21, no. 4, 1 August 2006 (2006-08-01) , pages 382-398, XP55151072, Retrieved from the Internet: URL:http://online.liebertpub.com/doi/pdfpl us/10.1089/cbr.2006.21.382>

## Description

### FIELD OF THE INVENTION

The present invention relates generally to screening for a neoplastic cell in a subject and provider methods and uses therefor. More particularly, the present invention relates to screening for both viable neoplastic cells and, still further, cytotoxin induced neoplastic cell death by detecting the level of expression of La protein and/or gene by a cellular population in said subject or in a biological sample derived from said subject. The method and uses of the present invention are useful in a range of applications including, but not limited to, diagnosing, prognosing or assessing a neoplastic condition, monitoring the progression of such a condition, assessing the effectiveness of a therapeutic agent or therapeutic regime and predicting the likelihood of a subject either progressing to a more advanced disease state or entering a remissive state.

### BACKGROUND OF THE INVENTION

Bibliographic details of the publications referred to by author in this specification are collected alphabetically at the end of the description.

Malignant tumours, or cancers, grow in an uncontrolled manner, invade normal tissues, and often metastasize and grow at sites distant from the tissue of origin. In general, cancers are derived from one or only a few normal cells that have undergone a poorly understood process called malignant transformation. Cancers can arise from almost any tissue in the body. Those derived from epithelial cells, called carcinomas, are the most common kinds of cancers. Sarcomas are malignant tumours of mesenchymal tissues, arising from cells such as fibroblasts, muscle cells, and fat cells. Solid malignant tumours of lymphoid tissues are called lymphomas, and marrow and blood-borne malignant tumours of lymphocytes and other hematopoietic cells are called leukemias.

Cancer is one of the three leading causes of death in industrialized nations. As treatments for infectious diseases and the prevention of cardiovascular disease continues to improve, and the average life expectancy increases, cancer is likely to become the most common fatal disease in these countries. Therefore, successfully treating cancer requires that all the malignant cells be removed or destroyed without killing the patient. An ideal way to achieve this would be to induce an immune response against the tumour that would discriminate between the cells of the tumour and their normal cellular counterparts. However, immunological approaches to the treatment of cancer have been attempted for over a century with unsustainable results.

Accordingly, current methods of treating cancer continue to follow the long used protocol of surgical excision (if possible) followed by radiotherapy and/or chemotherapy, if necessary. The success rate of this rather crude form of treatment is extremely variable but generally decreases significantly as the tumour becomes more advanced and metastasises. Further, these treatments are associated with severe side effects including disfigurement and scarring from surgery (e.g. mastectomy or limb amputation), severe nausea and vomiting from chemotherapy, and most significantly, the damage to normal tissues such as the hair follicles, gut and bone marrow which is induced as a result of the relatively non-specific targeting mechanism of the toxic drugs which form part of most cancer treatments.

Further, most anti-cancer treatments, which include cytotoxic chemotherapeutic agents, signal transduction inhibitors, radiotherapy, monoclonal antibodies and cytotoxic lymphocytes, kill cancer cells by apoptosis. Although tumours may contain a proportion of apoptotic cells and even areas of necrosis before anti-cancer treatment is given, an increased number of apoptotic cells and larger areas of necrosis are anticipated in tumours that respond to the anti-cancer treatment. However, when cytotoxic chemotherapeutic agents are used for the treatment of advanced cancer, the degree of cell kill and thus the response of the tumour to the first treatment is frequently difficult to assess soon after administration. Conventionally, patients receive a minimum of three cycles of chemotherapy before a clinical and radiological assessment of tumour response is made. Usually, only a minority of patients with advanced cancer responds to cytotoxic drugs and so patients may experience the side effects of treatment without obtaining benefit. Hence, there is an unmet medical need for a diagnostic method that would enable rapid, convenient and reliable detection of tumour cell kill after the first cycle of treatment that would predict treatment response, which in turn often predicts survival. For example, the use of positron emission tomography with fluoro-deoxyglucose (FDG-PET) in patients with oesophageal adenocarcinoma, who received chemoradiotherapy before surgery, differentiated treatment responders from non-responders with >90% sensitivity and specificity and tended to predict those who would subsequently undergo a curative resection of their tumours. Knowing whether the tumour is responding early would spare the majority of patients from ineffective and potentially toxic treatment. Then, non-responding patients can be offered second line treatments or clinical trials of investigational agents.

In work leading up to the present invention it has been surprisingly determined that La expression is upregulated in cancer cells and upregulated, still further, in cancer cells which have undergone cell death as a result of treatment which induces DNA damage, as opposed to cell death caused by other means. This finding is quite distinct from the prior art, where La has been used as a general indicator of the presence of apoptotic cells, since the presence of apoptotic cells is not conclusively diagnostic of the neoplastic nature of that cell. Still further, since apoptosis can be induced by a wide variety of factors, of which DNA damage is merely one type of factor, the findings detailed herein have enabled the development of a means of differentiating between neoplastic cell death resulting from non-DNA damage related means versus that induced by a DNA damaging agent. This distinction is significant when one bears in mind that even in an untreated patient, at any given point in time there exist a proportion of dead and dying neoplastic cells, the apoptosis of which has been induced by factors other than treatment which induces DNA damage. Accordingly, this determination has facilitated the development of a means for identifying neoplastic cells based on screening for increased levels, relative to normal levels, of La expression and still further, screening for DNA damage induced neoplastic cell death based on screening for a yet further increase in La expression levels. This is particularly important in the context of monitoring the progress of a therapeutic treatment regime which is directed to killing neoplastic cells.

WO 2004/073739 discloses methods for detecting the translocation of nuclear antigents, including La, from the nucleus to the cytoplasm as a marker of apoptosis of the cell. Particularly, the non-nuclear localisation of La is detected in late apoptopic cells which have lost membrane integrity, and may be of use in the detection of neoplastic cells.

Abstract 12 in "Oral Presentations", Cancer Biotherapy & Radiopharmaceuticals, Vol. 21, No. 4, 1 August 2006 at pages 382 - 398 discloses that a La-specific monocloral antibody preferentially binds dead tumour cells *in vitro* and *in vivo.* The antibody is used to coat nanostructures, which are proposed for use in cancer diagnosis and treatment. Abstract 24 in this document discloses a preliminary investigation of the use of an anti-La antibody to evaluate tumour chemoresponsiveness, but does not disclosre the increases in La levels upon which the present invention is based.

### SUMMARY OF THE INVENTION

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

As used herein, the term "derived from" shall be taken to indicate that a particular integer or group of integers has originated from the species specified, but has not necessarily been obtained directly from the specified source. Further, as used herein the singular forms of "a", "and" and "the" include plural referents unless the context clearly dictates otherwise.

Unless otherwise defined, all technical and scientific terms used herein have the same meaning as commonly understood by one of ordinary skill in the art to which this invention belongs.

One aspect of the present invention is directed to an *in vitro* method for detecting a neoplastic cell in a subject, assessing or monitoring a neoplastic condition in a subject, or assessing and/or monitoring the effectiveness of a neoplastic therapeutic treatment regime in a subject, said method comprising screening for a change in the level of La protein and/or gene expression in cells in a biological sample derived from said subject, wherein
(a) to detect a neoplastic cell or to assess or monitor a neoplastic condition said method comprises correlating the levels of La protein and/or gene expression in cells in said sample to a normal level of La protein and/or gene expression in a biological sample from a subject who has not developed a neoplastic condition, wherein an increase in the level of said La expression in cells of the subject relative to normal La expression levels is indicative of a neoplastic cell,
(b) to detect a dead neoplastic cell, which cell death has been induced by a DNA damaging agent, or
(c) to assess and/or monitor the effectiveness of a neoplastic therapeutic treatment regime in a subject,
said method comprises screening for the level of La protein and/or gene expression in dead cells in said biological sample and correlating said level to the level of La expression in viable neoplastic cells, wherein an increase in the level of La expression in said dead cells relative to viable neoplastic cell levels is indicative of DNA damage induced neoplastic cell death, and hence of the presence of a neoplastic cell rendered dead by a DNA-damaging agent or the effectiveness of said therapeutic treatment regime.

In another aspect of the present invention there is provided an immunointeractive molecule capable of disclosing altered expression levels of La protein expression product for *in vivo* use in a diagnostic or prognostic method for
(a) the diagnosis or prognosis of a neoplastic condition in a subject, wherein said diagnosis or prognosis comprises
   (i) detecting a neoplastic cell in said subject, by screening for the level of La protein expression in dead cells in said subject and correlating this to a normal level of La protein expression in a subject who has not developed a neoplastic condition wherein an increase in the level of said La expression relative to normal La expression levels is indicative of a neoplastic cell; or
   (ii) detecting a dead neoplastic cell in said subject, which cell death has been induced by a DNA damaging agent, by screening for the level of La protein expression in dead cells in said subject and correlating said level to the level of La expression in viable neoplastic cells, wherein an increase in the level of La expression relative to viable neoplastic cell La expression levels is indicative of DNA damage induced neoplastic cell death; or
(b) a method for assessing and/or monitoring a neoplastic condition or the effectiveness of a neoplastic therapeutic treatment regime in a subject, said method comprising screening for the level of La protein expression in dead cells in said subject, and correlating said level to the level of La expression to a normal level of La expression or to the level of La expression in viable neoplastic cells, wherein an increase in the level of La in said cells relative to normal levels is indicative of a neoplastic cell and an increase in the level of La in dead cells relative to viable neoplastic cell levels is indicative of the presence of DNA damage induced neoplastic cell non-viability;
wherein said immunointeractive molecule is an antibody or a fragment thereof, or a T-cell associated antigen-binding molecule, and
wherein said immunointeractive molecule is coupled to an imaging agent to enable detection.

In an embodiment, the neoplastic cell is a malignant neoplastic cell.

In an embodiment, a dead malignant neoplastic cell is detected, wherein death has been induced by a cytotoxic agent.

A diagnostic kit for a biological sample useful in the methods and uses of the present invention may comprise an agent for detecting La or a nucleic acid molecule encoding La and reagents useful for facilitating the detection by said agent.

An interactive molecule directed to La may be used in the manufacture of a quantitative or semi-quantitative diagnostic kit to detect dead neoplastic cells in a biological sample from a patient.

### BRIEF DESCRIPTION OF THE DRAWINGS

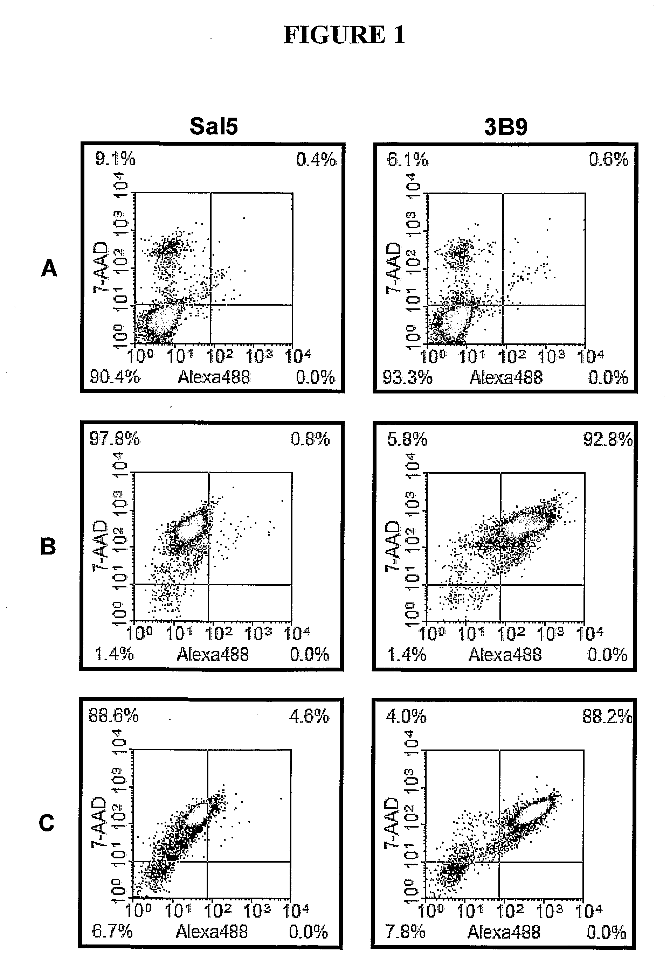

**Figure 1** depicts antigen-specific *in vitro* binding of anti-La mAb to EL4 thymic lymphoma cells after treatment with cytotoxic drugs. EL4 cells were cultured for 48 hours in the absence (**A** and **B**) or presence (**C**) of 20 µg/mL etoposide and 20 µg/mL cyclophosphamide. Untreated cells were incubated for 10 min. at room temperature (RT) in PBS (**A**) or 2 % paraformaldehyde (**B**) then diluted 1:10 in ice-cold PBS or ice-cold 100% methanol, respectively. Control cells (**A**), fixed and permeabilised cells (**B**) and cytotoxic drug-treated cells (**C**) were stained first with 5 µg/mL 3B9 or 5 µg/mL Sal5 (as the isotype control mAb) then with 2 µg/mL anti-mouse IgG Alexa₄₈₈-conjugated secondary antibody. 7-AAD 2 µg/mL was added to evaluate viability before cytofluographic analysis. Density plots show fluorescence from Alexa₄₈₈ (X-axis) and 7-AAD (Y-axis). 7AAD⁺ cells were defined as dead and were located in the upper outer quadrant if they specifically accumulated anti-La mAb (3B9).

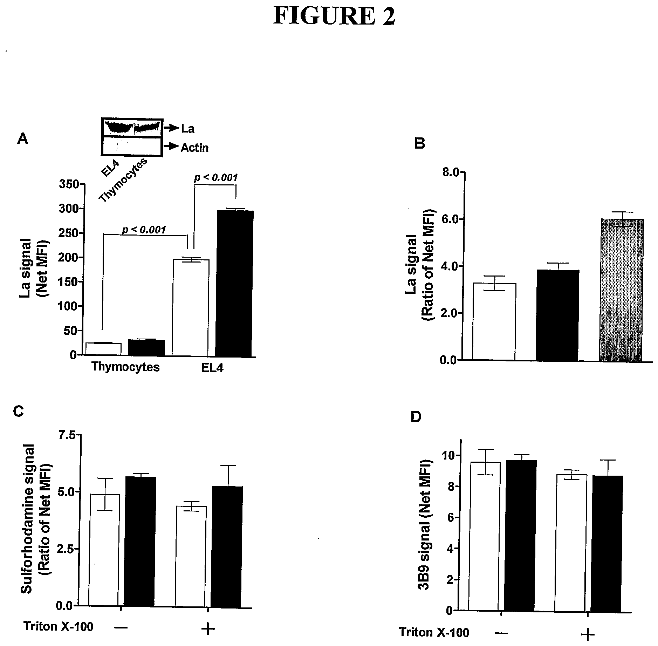

**Figure 2** depicts that La is overexpressed in malignant EL4 cells, and that La expression is further upregulated by *in vitro* treatment with DNA damaging drugs. Its expression in apoptotic EL4 cells is detergent resistant and also mediates selective detergent-resistant binding of La-specific mAb to apoptotic EL4 cells. (**A**) Cultured EL4 cells and normal mouse thymocytes were fixed using 2% paraformaldehyde at RT for 10 min. then resuspended 1:10 in ice-cold methanol (clear columns). Cells were also cultured for 48 hr in the presence of 20 µg/mL etoposide and 20 µg/mL cyclophosphamide PI⁺ and 3B9⁺ events (filled columns). Cells were stained with mAb then with anti-mouse IgG Alexa₄₈₈-conjugated secondary antibody and 7-AAD and analysed by flow cytometry using a gate restricted to 7-AAD⁺ events. Data are expressed as net mean fluorescence intensity (MFI) ± standard error of the mean (SEM) (*n*=3) of 7-AAD⁺ events. Net MFI for La-specific staining was obtained after subtraction of individual MFI values for Sal5 staining. **Inset**: Lysates of cultured cells were analysed by Western blotting for La protein expression using 3B9 together with an actin expression control for equivalence of loading. (**B**) EL4 cells and thymocytes were cultured for 48 hours in media containing 1 µM of the pan-kinase inhibitor staurosporine (STS) (clear columns), 20 µg/mL etoposide (filled columns), or 20 µg/mL cyclophosphamide and 20 µg/mL etoposide (grey columns). After incubation with 1% Triton X-100 for 5-10 min. at RT, cells were analysed by flow cytometry as described in A. Data are expressed as the ratio of the net MFI ± SEM (*n*=3) for La staining (after subtraction of individual MFI values for Sal5 staining) in EL4 cells to that in thymocytes. (**C**) EL4 cells and thymocytes were cultured in media containing 20 µg/mL etoposide (clear columns) or 10 µg/mL cisplatin (filled columns) for 96 hours. Cells were washed, stained with 1 µg/mL sulforhodamine B, washed again and incubated in the presence or absence of 1% Triton X-100 for 5 min. at RT and analysed by flow cytometry. Data are expressed as the ratio of the MFI ± SEM (*n*=4) for sulforhodamine staining in EL4 cells to that in thymocytes. (**D**) During 96 hours treatment with 20 µg/mL etoposide (clear columns) or 10 µg/mL cisplatin (filled columns), EL4 cells and thymocytes were incubated with either 50 µg/mL 3B9 or Sal5. Cells were washed and incubated with 1% Triton X-100 for 5 min at RT then washed again before staining with anti-mouse IgG Alexa₄₈₈-conjugated secondary antibody and 2 µg/mL 7-AAD. Data are expressed as the ratio of the net MFI ± SEM (*n*=3) for detergent-resistant 3B9 binding (after subtraction of individual MFI values for Sal5 binding) obtained in EL4 cells to that obtained in thymocytes.

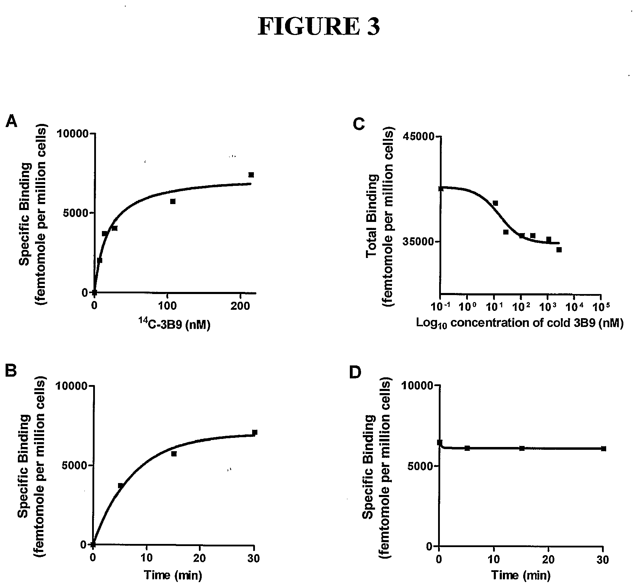

**Figure 3** depicts that La-specific mAb binds with high affinity to apoptotic EL4 cells in a specific, rapid, saturable and irreversible manner. Apoptosis was induced in EL4 cells after 48 hours treatment with 20 µg/mL etoposide and 20 µg/mL cyclophosphamide. (**A**) Treated cells were incubated at RT with increasing concentrations of ¹⁴C-3B9 in the presence or absence of 50-fold molar excess of unlabelled 3B9. Cells were washed and the radioactivity measured. Specific binding was calculated in units of femtomole bound per million cells and plotted as a function of ¹⁴C-3B9 concentration. The concentration required to reach half-maximal saturation of 10⁶ apoptotic cells was ∼18nM (*n*=3). (**B**) Cells were incubated with ¹⁴C-3B9 and samples were removed at specified time points. Specific binding in units of femtomole bound per million cells was plotted as a function of time. The time required to reach half-maximal saturation of 10⁶ apoptotic cells was 5 min. (*n*=3). (**C**) Treated cells were incubated with a set concentration (100 nM) of ¹⁴C-3B9 in the presence of increasing concentrations of unlabelled 3B9. Radioactivity was measured and converted to units of femtomole bound per million cells and plotted as a function of the log-concentration of 3B9. The concentration of unlabelled 3B9 required to inhibit half-maximal binding of ¹⁴C-3B9 was ∼28nM (*n*=5). (**D**) Cells were incubated with ¹⁴C-3B9 for 30 min. Cells were washed then incubated in binding buffer alone and samples were removed at specified time points. Samples were washed and specific binding in units of femtomole bound per million cells was plotted as a function of time. Bound antibody did not dissociate after cells were incubated in binding buffer for 30 min. at 37°C (*n*=3).

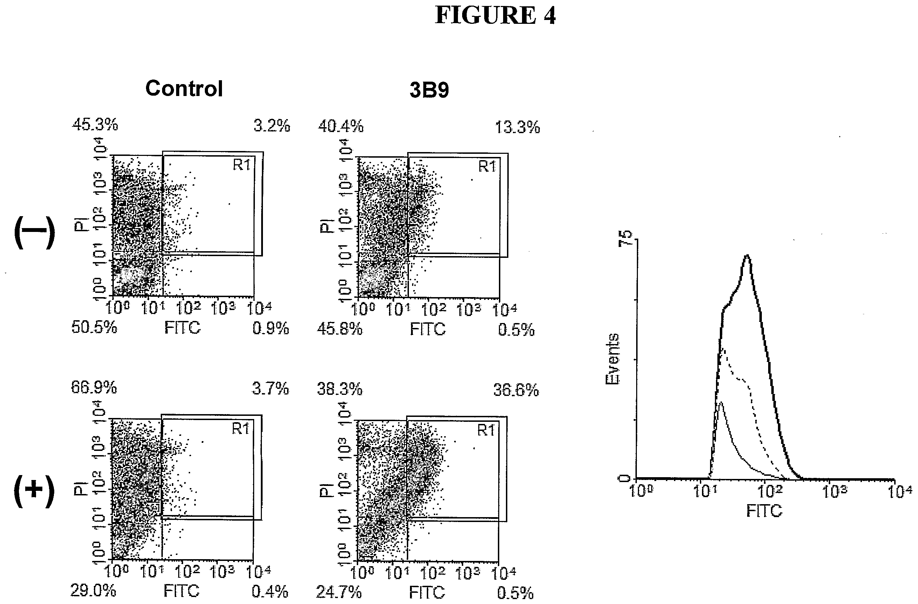

**Figure 4** depicts that anti-La mAb binds specifically to dead cells isolated from EL4 tumour explants and both proportionate and per cell binding increases after cytotoxic drug treatment. EL4 tumour-bearing mice (*n*=5) were treated or not with etoposide 67 mg/kg and cyclophosphamide 100 mg/kg by intraperitoneal injection on two occasions 24 hours apart. At 48 hours after the commencement of treatment, tumours were excised and single cell suspensions prepared. Cells were washed with PBS and incubated with 5 µg/mL FITC-conjugates of 3B9 or Sal5 isotype control. PI 0.5 µg/mL was added to evaluate cell viability and analysis performed using flow cytometry. Data shown are representative density plots and histogram overlays of FITC and PI emissions from stained cells that were isolated from EL4 tumour explants of mice treated (+; upper row of panels) or not (-; lower row of panels) with cytotoxic chemotherapy. Data depicted in the histogram overlays originated from region 1 (R1), which represents the FITC⁺ subset of PI⁺ events, and shows Sal5-FITC staining of tumour cells from chemotherapy-treated mice (grey fill), 3B9-FITC staining of tumour cells from untreated mice (dashed line) and chemotherapy-treated mice (thick line). After chemotherapy, the fraction of PI⁺ cells in tumor explants increased significantly from a mean (± SE) of 50 ± 2% to 70 ± 1% (P < 0.001). Similarly, the 3B9⁺ subset of PI⁺ cells increased significantly from 15 ± 1% to 38 ± 2% (*P* < 0.01) after chemotherapy, whereas isotype control staining was unaltered. Only the PI⁺ tumour subpopulation bound 3B9, which indicated that La was recognized specifically in dead tumour cells. Histogram analysis indicated that specific per cell binding of 3B9-FITC to PI⁺ cells was significantly augmented in tumors exposed *in vivo* to cytotoxic chemotherapy (net median fluorescence intensity ± SE of 18 ± 3 with chemotherapy and 1 ± 3 without chemotherapy, *P* < 0.05).

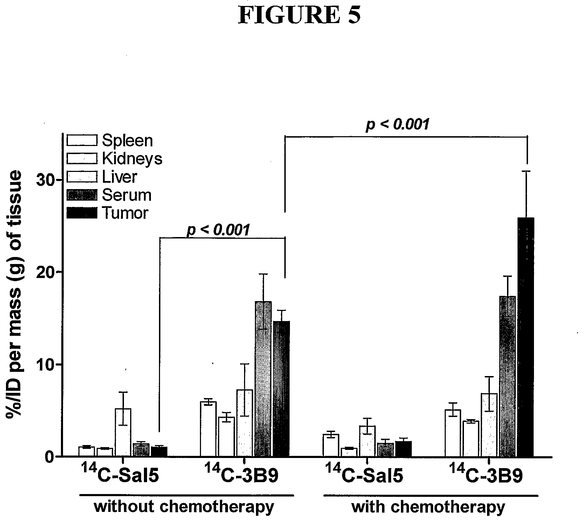

**Figure 5** depicts that biosynthetically labelled La-specific ¹⁴C-3B9 mAb is taken up preferentially by tumours, particularly after cytotoxic chemotherapy. Intrinsic labelling of the La-specific mAb indicates that tumour targetting results from antigen-binding activity of the antibody rather than from non-specific localisation of the radiolabel. EL4 tumour-bearing mice (*n*=4) were given intravenous injections of 100 µg of either ¹⁴C-3B9 or ¹⁴C-Sal5 at a dose of approximately 5 mg/kg. Then, mice were treated or not with etoposide 67 mg/kg and cyclophosphamide 100 mg/kg, which were given as two intraperitoneal injections 24 hours apart. Forty-eight hours after the commencement of treatment, the mice were killed and their organs collected for radioactivity measurement. Radioactivity was normalised to the mass of tissue counted (dpm/g of tissue) and the percentage accumulation over the injected dose (%/ID per mass of tissue) was calculated based on the specific radioactivity of the injected agents. Data are presented as %/ID ± SEM. Statistical comparisons were made using two-way analysis of variance, which was performed as a Bonferroni post-test comparison between all groups (*p <* 0.001; *n*=4).

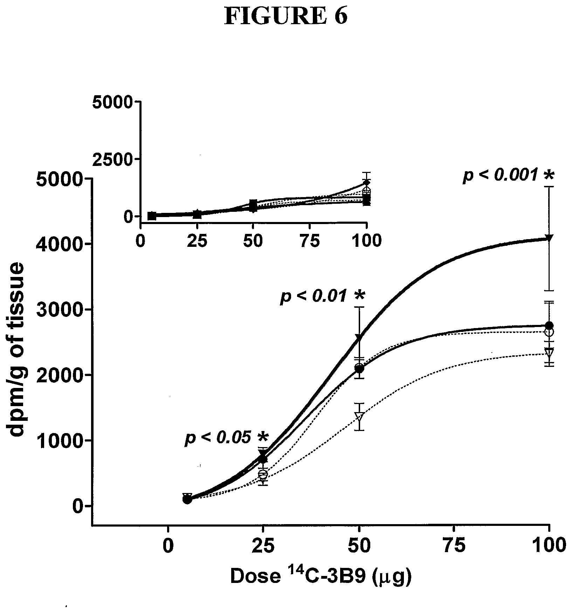

**Figure 6** depicts that the intra-tumoral accumulation of biosynthetically labelled La-specific ¹⁴C-3B9 mAb is dose-dependent and is augmented by cytotoxic chemotherapy. The biodistribution of different doses of ¹⁴C-labelled 3B9 was studied in EL4 tumour bearing mice with or without chemotherapy. ¹⁴C-3B9 accumulated significantly more only in the tumour with chemotherapy when its uptake approximately doubled at ¹⁴C-3B9 doses of 25 µg, 50 µg and 100 µg. Mice were given intravenous injections of 5, 25, 50 or 100 µg of ¹⁴C-3B9 or ¹⁴C-Sal5 and then treated or not with etoposide 67 mg/kg and cyclophosphamide 100 mg/kg, which were given as two intraperitoneal injections 24 hours apart. Forty-eight hours after commencement of treatment, the mice were killed and organs collected for radioactivity measurement. Radioactivity was normalised to the mass of tissue counted (dpm/g of tissue) and tissues included (◆) liver, (■) spleen, (▲) kidneys, (●) serum and (▼) tumour. Data are presented as dpm/g ± SEM. Closed symbols, with chemotherapy; open symbols, without chemotherapy. Statistical comparisons were made using two-way analysis of variance, which was performed as a Bonferroni post-test comparison between all groups (*n*>*4*).

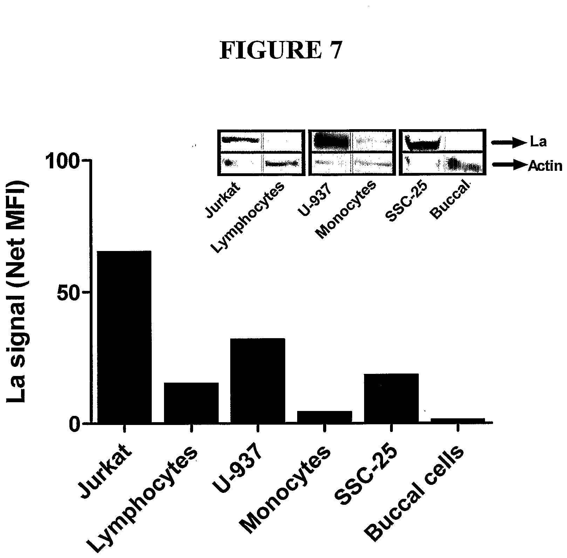

**Figure 7** depicts that La is overexpressed in human cancer cell lines. Cultured cells from cancer cell lines and cells of their normal counterpart cell type were fixed and permeabilised using paraformaldehyde and methanol. Cells were stained with 3B9 or Sal5 then with anti-mouse IgG Alexa₄₈₈ secondary antibody conjugate and 7-AAD before cytofluographic analysis was performed. Data are presented as the net MFI for 3B9 staining after subtraction of individual MFI values for Sal5 staining after first gating on 7-AAD⁺ events, which comprised >98 % of cells. **Inset:** Lysates of cultured cells were analysed by Western blotting for La protein expression using 3B9 together with an actin expression control for equivalence of loading.

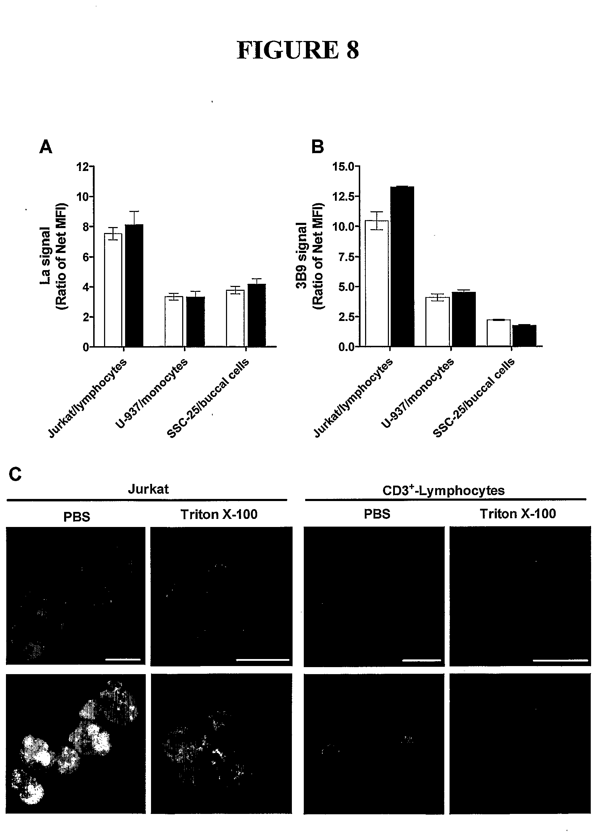

**Figure 8** depicts that both La and its binding by 3B9 in apoptotic human malignant cells is resistant to detergent treatment. (**A**) Cells were treated first with STS (clear columns) or etoposide (filled columns) for 48 h and then with Triton X-100. Cells were stained with 3B9 or Sal5 then with anti-mouse IgG Alexa₄₈₈ secondary antibody conjugate and 7-AAD before cytofluographic analysis was performed. Data are expressed as the ratio of the net MFI ± SEM (*n*≥4) for La-specific staining in the malignant cells to that in the corresponding normal cells. **(B)** During 96 hours treatment with 10 µg/mL cisplatin (clear columns) or 20 µg/mL etoposide (filled columns), cells were incubated with either 50 µg/mL 3B9 or Sal5. Cells were washed and incubated with 1% Triton X-100 for 5 min. at RT. Then cells were washed again before staining with anti-mouse IgG Alexa₄₈₈-conjugated secondary antibody and 2 µg/mL 7-AAD and analysed by flow cytometry. Data are expressed as the ratio of the net MFI ± SEM (*n*≥4) for detergent-resistant 3B9 binding (after subtraction of individual MFI values for Sal5 binding) obtained in the malignant cells to that obtained in the corresponding normal cells. (**C**) Cells were prepared as described in B, treated or not with Triton X-100, then stained with anti-mouse IgG Alexa₄₈₈ secondary antibody conjugate or sulforhodamine B before laser scanning confocal microscopy analysis. 3B9, green; sulforhodamine B, red; co-localised images, orange/yellow. Scale bar represents 20 µm.

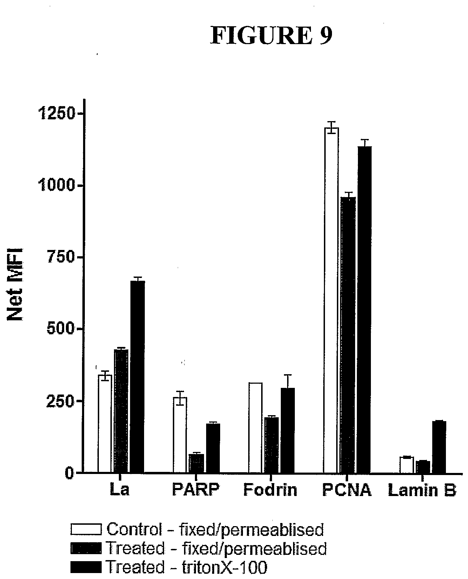

**Figure 9** depicts that in comparison with other nuclear antigens, La is preferentially retained in cisplatin-treated apoptotic Jurkat cells. Jurkat cells were cultured in the absence (control) or presence (treated) of 20 µg/mL cisplatin for 48 h. Then the cells were incubated as follows: (i) 15 min in 500 µL PBS (control - fixed and permeabilised), (ii) 10 min in 50 µL of 2 % paraformaldehyde then in 450 µL of ice-cold absolute methanol (treated - fixed and permeabilised), (iii) 10 min in 500 µL Triton X-100 then vortexing cells for 30 secs (treated - Triton X-100). Cells were washed with PBS and incubated for 30 min at room temperature (RT) in 5 µg/mL Sal5 isotype control mAb or 5 µg/mL of monoclonal antibodies specific for the antigens shown. Cells were washed and incubated (30 min, RT) with 2 µg/mL of anti-mouse IgG-Alexa₄₈₈. Finally, cells were washed, incubated with 2 mg/mL 7-AAD and analysed by flow cytometry. Data were obtained after gating on 7-AAD⁺ events and are presented as Net Mean Fluorescence Intensity (MFI) ± SEM (*n*=2) after subtraction of the MFI for isotype control staining. The upper and lower panels depict results from different experiments. PARP, Poly(ADP-ribose) polymerase; PCNA, proliferating cell nuclear antigen.

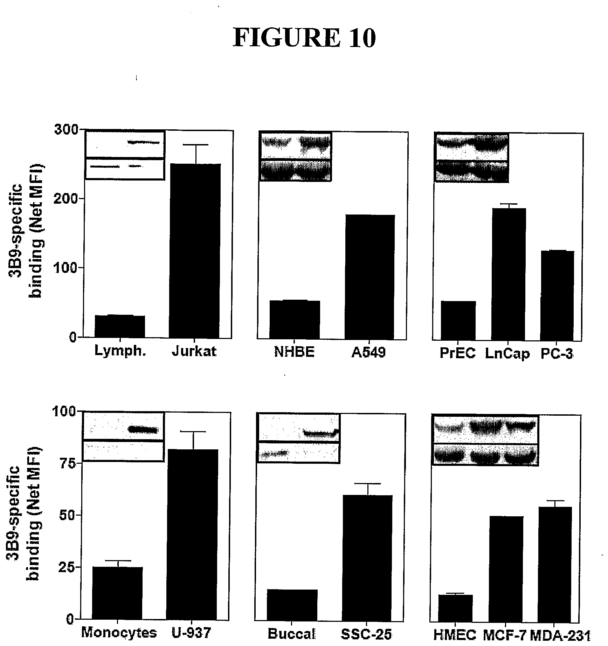

**Figure 10** depicts that La is overexpressed in human malignant cell lines. Panels show expression of La in malignant cell lines compared with its expression in the corresponding primary cell types using indirect immunofluorescence staining of fixed and permeabilized cells. 3B9-specific binding is shown as Net MFI ± SEM (*n*=3). **Insets:** Immunoblots of cell lysates from cells probed for expression of La with 3B9 (upper row of bands) or actin as a loading control (lower row of bands). Three independent experiments were performed and representative data are shown. **Key:** CD3-enriched peripheral blood lymphocytes (Lymph.) *vs*. Jurkat adult T-leukemia cells; Normal Human Bronchial Epithelium (NHBE) *vs*. A549 bronchogenic carcinoma cells; Prostate Epithelial Cells (PrEC) *vs*. PC-3 and LNCaP prostate cancer cells (PrEC and PC-3 lysates are shown in the inset); CD14-enriched monocytes *vs*. U-937 myeloid leukaemia cells; Buccal cavity epithelial cells (Buccal) *vs*. SSC-25 oropharyngeal squamous cell carcinoma cells; Human Mammary Epithelial Cells (HMEC) *vs*. MCF-7 and MDA-MB-231, breast cancer cell lines.

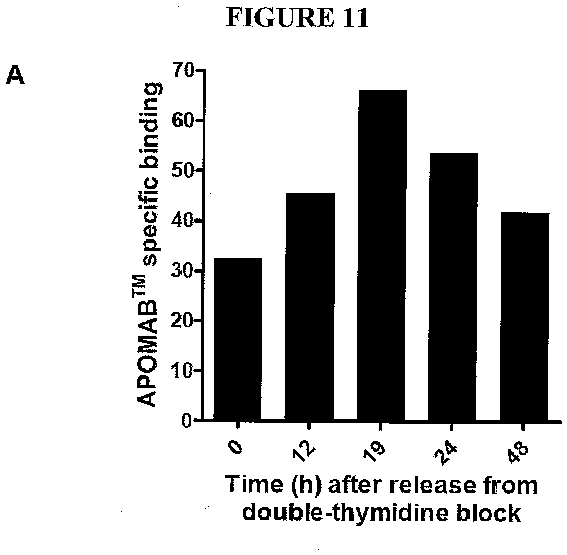

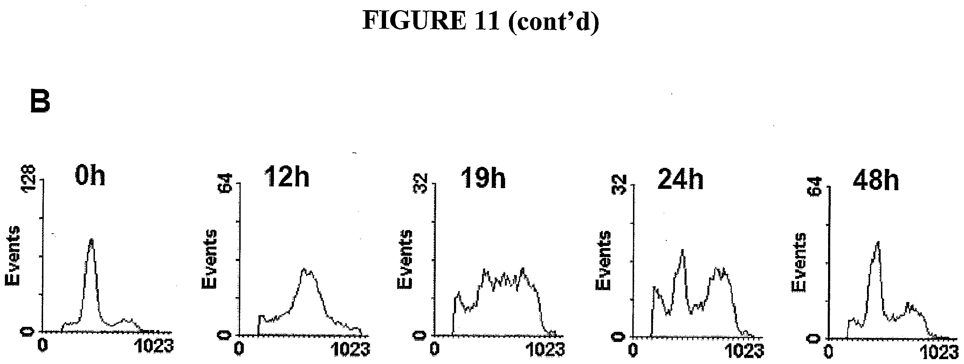

**Figure 11** is a graphical representation demonstrating that La expression is cell cycle dependent. Jurkat cells are incubated with 100µM thymidine for 18h then washed and incubated without thymidine for 16h. Cells are collected and re-incubated with 100µM thymidine for 18h for a double-thymidine block to synchronise cells at G1/S phase. At the time points indicated, cells are washed and (**A**) subjected to indirect immunofluorescence staining with Apomab or Sal5 isotype control mAb or (**B**) resuspended in 70% ethanol for cell cycle analysis by PI staining (50ng/mL). Apomab-specific binding is calculated from the net mean fluorescence intensity (MFI) value after subtraction of individual MFI values for staining with the Sal5 isotype control.

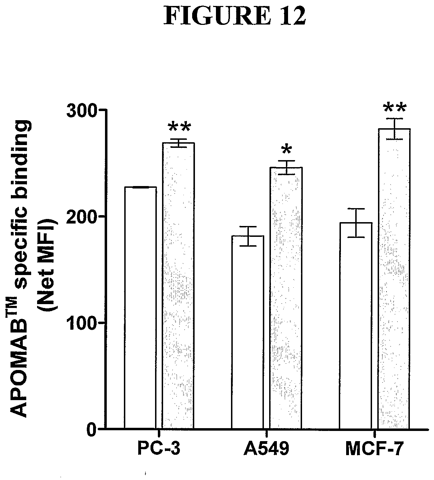

**Figure 12** is a graphical representation demonstrating that La expression increases in malignant cells after mitogenic stimulation. Cells are incubated with (filled bars) or without (clear bars) 200ng/mL phorbol 12-myristate 13-acetate (PMA) for 48h. Cells are fixed and permeabilised by incubation in 2% paraformaldehyde for 10min. followed by 1:10 dilution in -20°C methanol for 2-3min. Cells are washed extensively with PBS then subjected to indirect immunofluorescence staining with Apomab or Sal5 isotype control mAb. Apomab-specific binding is calculated from the net mean fluorescence intensity (MFI) value after subtraction of individual MFI values for staining with the Sal5 isotype control. Statistical analysis of Apomab-specific binding is done using two-way ANOVA and a Bonferroni post-test comparison (*, *P*<0.05; **, P<0.01).

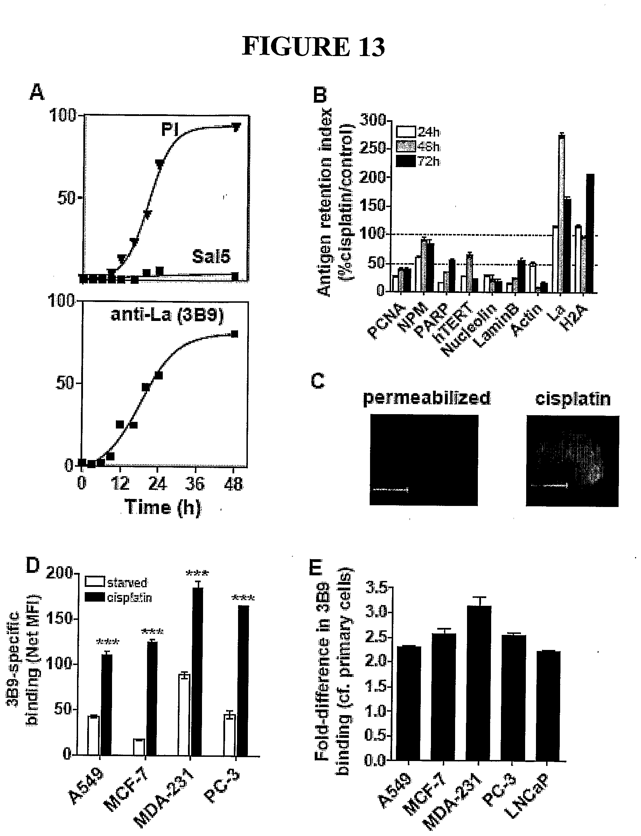

**Figure 13** depicts that La-specific 3B9 mAb preferentially binds apoptotic malignant cells *in vitro.* (A) Etoposide-treated (20µg/mL) Jurkat cells were stained with PI (▼), 3B9 or its control Sal5 mAb (■), and percentages of positively stained cells determined by cytofluography (Y-axis) are plotted as a function of time in hours (X-axis). (B) Jurkat cells were incubated in the presence or absence of 20µg/mL cisplatin. At specified times, cells were fixed, permeabilized and stained. Specific staining of different antigens was calculated as net MFI ± SEM and data are presented as an antigen retention index, which was calculated using the following formula: % retention = MFI from cisplatin-treated cells/MFI from control cells X 100%). The antigen retention index was calculated in two independent experiments each comprising triplicate samples. Data shown are representative of one of these experiments.(C) Permeabilized Jurkat cells and cisplatin-treated Jurkat cells were stained with 7-AAD (red) and 3B9 (green) and viewed by confocal microscopy. Scale bars represent 5µm of actual length. (D) Cell death was induced by 72h treatment with 20µg/mL cisplatin or by serum deprivation. Cells were stained with 3B9 and 7-AAD and data are shown as Net MFI ± SEM (*n* =2) for the 7-AAD⁺ population. Data shown are representative of identical assays performed on three separate occasions. (E) Cell death was induced in cultured cells after 48h treatment with 20µg/mL cisplatin. For each cell type, 3B9-specific binding was calculated as net MFI and the fold-difference in 3B9 binding was calculated as the ratio of specific binding in the malignant cells to that in the corresponding primary cell type: MCF-7 and MDA-MB-231 cells *vs.* HMEC; A549 cells *vs.* NHBE cells and LNCaP and PC-3 cells *vs* PrEC. Data are presented as the fold difference ± SEM (*n*=2).

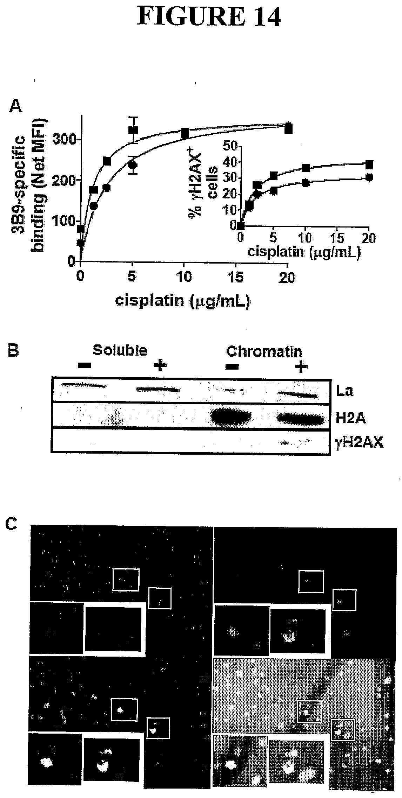

**Figure 14** depicts that 3B9 binding is augmented by DNA damaging agents. (**A**) Jurkat cells were incubated with increasing concentrations of cisplatin in the absence (●) or presence (■) of 100 ng/mL trichostatin A (TSA). At 48h, cells were analyzed for 3B9 binding. Inset: Samples were removed at 3h from incubation with cisplatin in the absence (●) or presence (■) of 100ng/mL TSA, fixed and permeabilized and stained for γH2AX. (**B**) Immunoblots of soluble and chromatin fractions from a chromatin-binding assay after Jurkat cells were treated with 20 µg/mL cisplatin for 3 h (+) or not (-). We inferred that La redistributed to DNA double-stranded breaks after observing increased chromatin-associated La and γH2AX. (**C**) Adherent cultures of PANC-1 cells were incubated in slide chambers for 3h with 20µg/mL cisplatin. Cells were fixed, permeabilized and stained with 3B9 and anti-γH2AX. Images were acquired for 3B9 binding (green) and γH2AX staining (red) and overlayed to investigate co-localization (orange-yellow). 3B9 and γH2AX overlays were superimposed on transmission images of cells to ensure nuclear localization of detected antigens. Shown are representative data of four independent experiments, which produced similar results.

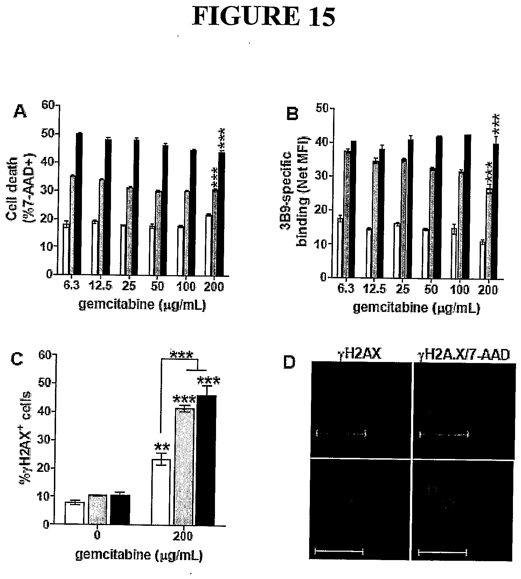

**Figure 15** depicts that 3B9 binding to dead PANC-1 cells is augmented after combination treatment with gemcitabine and TSA produced additive cytotoxicity and DNA-damaging effects. PANC-1 cells were incubated with increasing concentrations of gemcitabine in the absence (clear columns) or presence of 100ng/mL (hatched columns) or 200ng/mL (filled columns) of TSA. Analysis was done for viability using 7-AAD **(A),** 3B9-specific binding **(B)** and DNA damage using γH2AX staining (C). Data are presented as mean ± SEM from three independent experiments. Photomicrographs show PANC-1 cells treated with gemcitabine and TSA (+) or not (-), which were stained with γH2AX-specific mAb and 7-AAD (**D**).

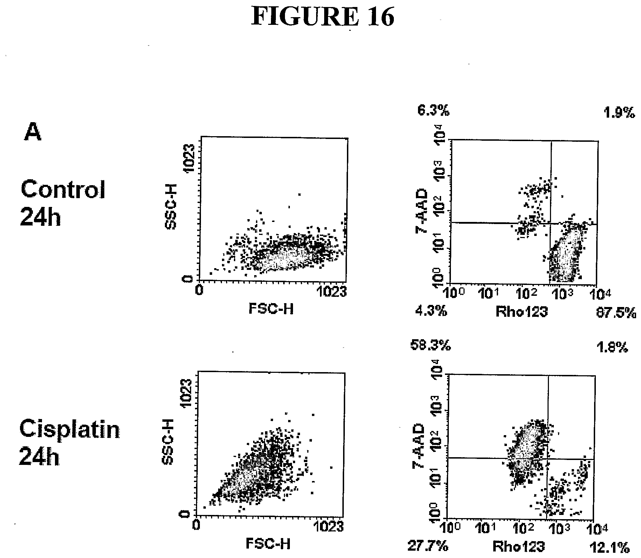

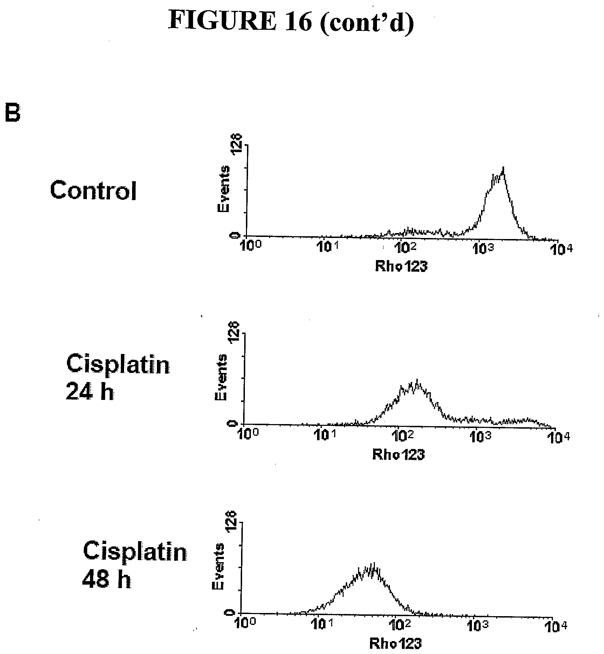

**Figure 16** is a graphical representation depicting that cisplatin-induced cell death is demonstrated to be apoptotic. Jurkat cells were incubated in the presence or absence of 20µg/mL cisplatin. Cell death was assessed using the DNA-binding dye, 7-AAD, and the mitochondrial membrane potential dye, Rhodamine 123. **A.** Dotplots are shown of untreated control cells and cells treated with cisplatin for 24h; left-hand panels, forward scatter (size) *vs*. side scatter (internal complexity); right hand panels, 7-AAD *vs*. rhodamine-123. **B.** Histograms are shown of rhodamine-123 staining for untreated control cells and cells treated with cisplatin for 24h and 48h.

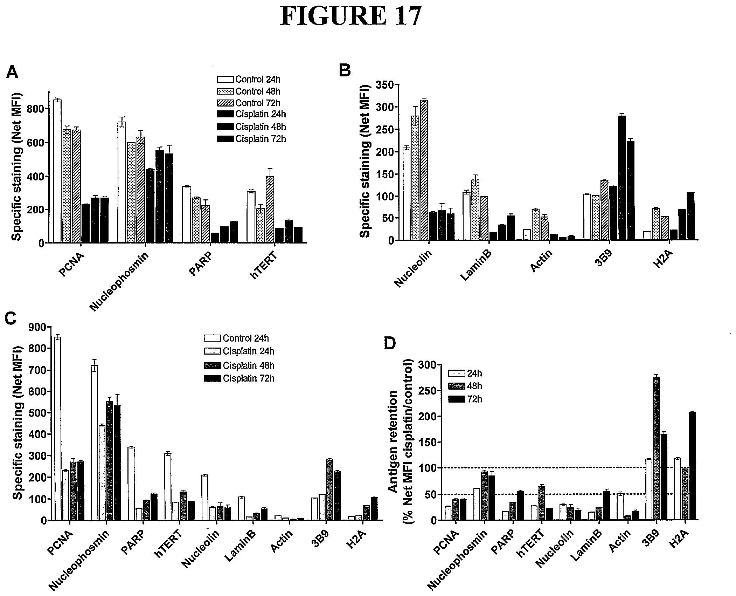

**Figure 17** is a graphical representation depicting that the La antigen is preferentiallly retained in dead Jurkat cells 48h after cisplatin-induced apoptosis. Jurkat cells were incubated in the presence or absence of 20µg/mL cisplatin. At the specified time points cells were removed and cell pellets were resuspended in 2% paraformaldehyde and incubated for 10 min. at RT then diluted 1:10 in ice-cold 100% methanol for another 2min. Pellets were collected and washed in PBS then incubated (30min. at RT) with 10µg/mL of antigen-specific murine mAb (PCNA, Nucleolin, Nucleophosmin, PARP, 3B9 (Apomab), Sal5 and Lamin B), 125µL of rat anti-hTERT or 20µg/mL rat IgG₂ₐₖ matched isotype mAb or with rabbit anti-actin, rabbit anti-H2A or rabbit IgG control Ab at 10 µg/mL. Cells were collected and washed with PBS then incubated for 30 min. at RT with 2µg/mL of relevant secondary antibodies conjugated to AleXa₄₈₈. Cells were washed and samples were analysed by flow cytometry. (**A** and **B**) specific staining for the different antigens is presented as the net MFI (after substraction of the corresponding isotoype Ab) ± SEM. (**C**) Simplified form of panels A and B with control cells from 24h only shown. (**D**) antigen retention index was calculated from A and B using the following formula: % retention = specific signal from cisplatin treated cells/specific signal from control cells X 100%.

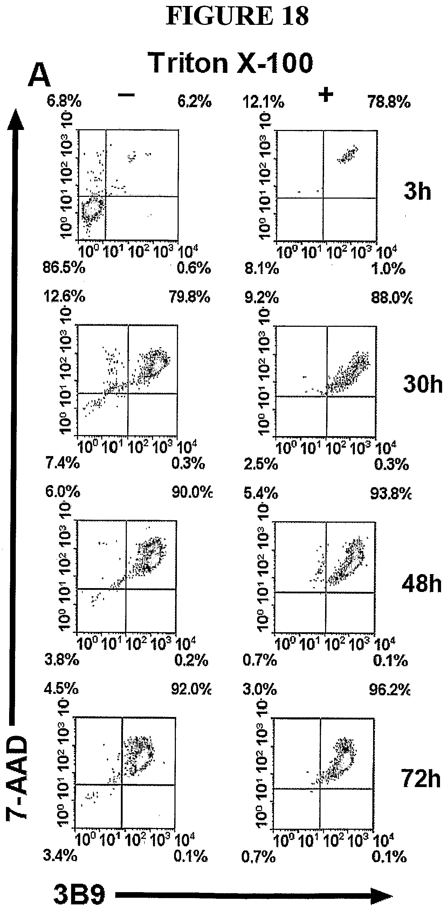

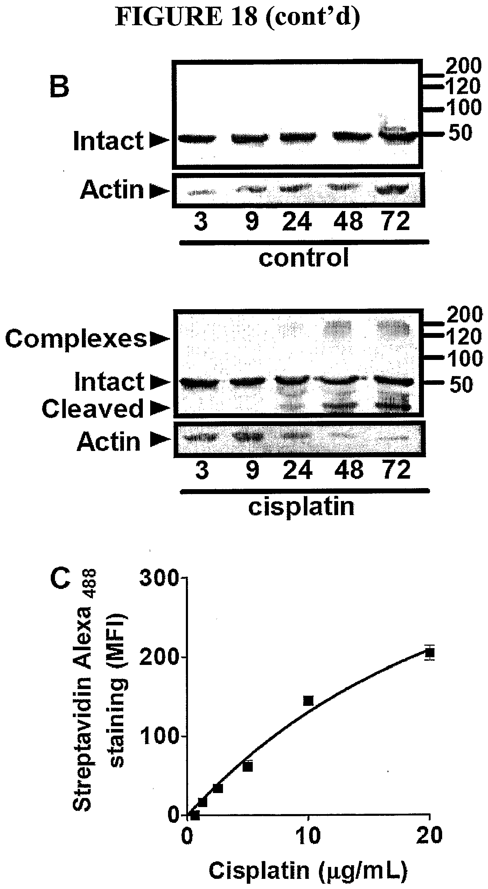

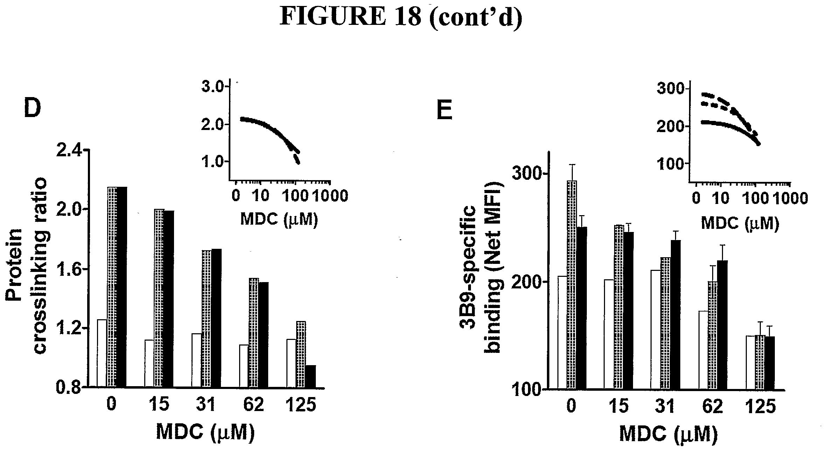

**Figure 18** depicts that La antigen is crosslinked during apoptosis. (**A**) Jurkat cells incubated in 20µg/mL cisplatin were removed at specified time points and incubated for 10min. in 1% Triton X-100 solution before 3B9 binding analysis. Data are presented as density plots of staining with 3B9 vs. 7-AAD. (**B**) Immunoblots of cell lysates of control and cisplatin-treated Jurkat cells were probed with 3B9 for expression of La (top panels) or actin as a loading control (bottom panels) at specified times. (**C**) Jurkat cells incubated in the absence or presence of increasing concentrations of cisplatin were labeled with cadaverine-biotin added at 100µM for 48h. Cells were stained with strepatavidin-Alexa₄₈₈ and analyzed by flow cytomtery. Data shown are MFI ± SEM (n=3). (**D**) Jurkat cells incubated in the absence or presence of 20µg/mL cisplatin were incubated with increasing concentrations of MDC. Cells were analyzed at specified times (24 □, 48 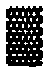 and 72h ■) for sulforhodamine 101 staining. **Inset** summarizes the dependence of the protein cross-linking ratio on MDC concentration at 48h (-) and 72h (▪▪▪) after cisplatin treatment of Jurkat cells. (E) Jurkat cells incubated with cisplatin in the absence or presence of increasing concentrations of MDC were stained with 3B9. 3B9-specific binding is presented as Net MFI ± SEM (*n*=3). Inset summarizes the dependence of 3B9-specific binding on MDC concentration at 24h (-), 48h (--) and 72h (▪▪▪) after cisplatin treatment of Jurkat cells. Data are representative of three identical and independent assays, which produced similar results.

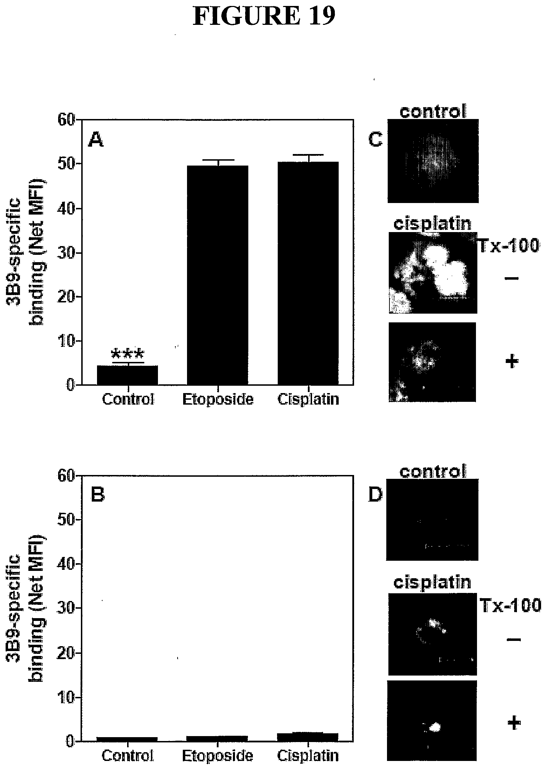

**Figure 19** depicts that the preferential binding of 3B9 to dead malignant cells after cytotoxic drug treatment is detergent-resistant and co-localizes with other intracellular proteins. Cultures of Jurkat cells (A) and their counterpart primary CD3-enriched peripheral blood lymphocytes (**B**) were either left untreated (control) or treated with etoposide or cisplatin for 48h in the presence of 50µg/mL of 3B9 or its Sal5 control mAb. After 48h, cells were treated or not with 1% Triton X-100 before fluorocytometric analysis. Data shown are the net MFI ± SEM of 7-AAD⁺ events from four independent assays. Statistical comparisons were made between control and treated cells. Photomicrographs show representative Jurkat cells (C) and CD3-enriched peripheral blood lymphocytes (**D**), which had been treated with 20µg/mL cisplatin in the presence of 50µg/mL 3B9, treated or not with 1% Triton X-100 and stained with sulforhodamine (red) and Alexa₄₈₈-conjugated anti-mouse IgG (green). Control: non-permeabilized and untreated Jurkat cells. Scale bars represent either 40µm (PBS) or 20µm (Triton X-100) of actual length.

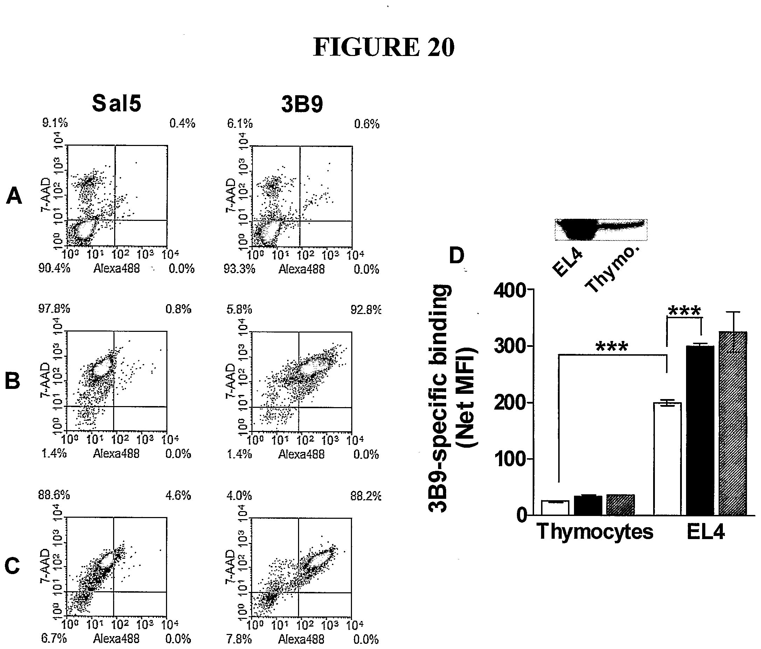

**Figure 20** depicts that La-specific mAb binds specifically to malignant EL4 cells after *in vitro* treatment with cytotoxic drugs. EL4 cells were cultured for 48h in the absence (**A** and **B**) or presence (**C**) of cyclophosphamide and etoposide. Untreated control cells (**A**), fixed and permeabilized cells (**B**) and cytotoxic drug-treated cells (**C**) were stained with 7-AAD and Sal5 isotype control or La-specific 3B9 mAb. Density plots show fluorescence from Alexa₄₈₈ (X-axis) and 7-AAD (Y-axis). (**D**) EL4 cells and syngeneic murine thymocytes were fixed and permeabilized (clear columns) or cultured for 48h with cyclophosphamide and etoposide and subsequently treated (striped columns) or not (filled columns) with Triton X-100. 3B9-specific binding is presented as net MFI ± SEM (*n*=3) of 7-AAD⁺ events, ***, *P*<0.001. **Inset:** Immunoblots of cell lysates were probed for expression of La using 3B9.

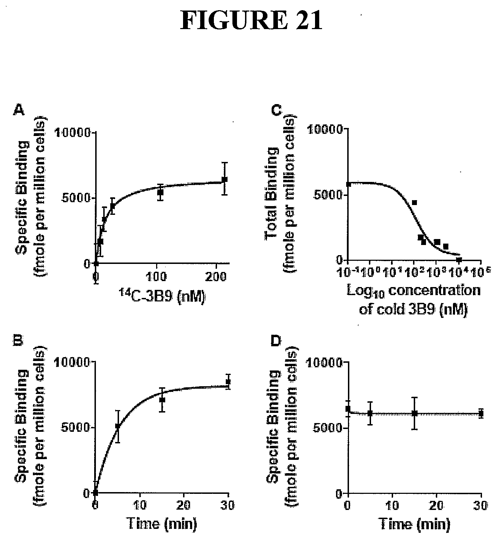

**Figure 21****:** depicts that biosynthetically labelled La-specific ¹⁴C-3B9 mAb is taken up preferentially by tumours, particularly after cytotoxic chemotherapy. Intrinsic labelling of the La-specific mAb indicates that tumour targetting results from antigen-binding activity of the antibody rather than from non-specific localisation of the radiolabel. EL4 tumour-bearing mice (*n*=4) were given intravenous injections of 100 µg of either ¹⁴C-3B9 or ¹⁴C-Sal5 at a dose of approximately 5 mg/kg. Then, mice were treated or not with etoposide 67 mg/kg and cyclophosphamide 100 mg/kg, which were given as two intraperitoneal injections 24 hours apart. Forty-eight hours after the commencement of treatment, the mice were killed and their organs collected for radioactivity measurement. Radioactivity was normalised to the mass of tissue counted (dpm/g of tissue) and the percentage accumulation over the injected dose (%/ID per mass of tissue) was calculated based on the specific radioactivity of the injected agents. Data are presented as %/ID ± SEM. Statistical comparisons were made using two-way analysis of variance, which was performed as a Bonferroni post-test comparison between all groups (*p* < 0.001; *n*=4)*.*

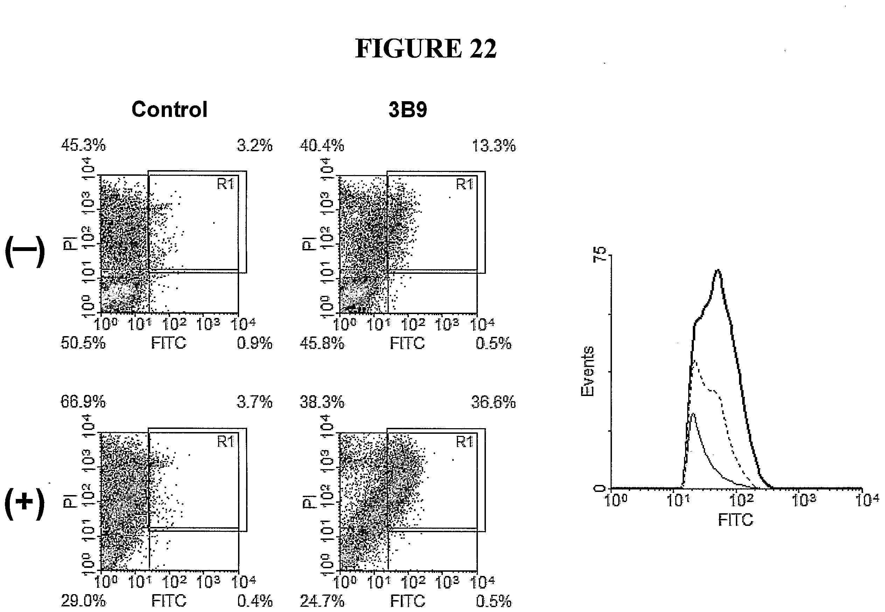

**Figure 22****:** depicts that anti-La mAb binds specifically to dead cells isolated from EL4 tumour explants and both proportionate and per cell binding increases after cytotoxic drug treatment. EL4 tumour-bearing mice (*n*=5) were treated or not with etoposide 67 mg/kg and cyclophosphamide 100 mg/kg by intraperitoneal injection on two occasions 24 hours apart. At 48 hours after the commencement of treatment, tumours were excised and single cell suspensions prepared. Cells were washed with PBS and incubated with 5 µg/mL FITC-conjugates of 3B9 or Sal5 isotype control. PI 0.5 µg/mL was added to evaluate cell viability and analysis performed using flow cytometry. Data shown are representative density plots and histogram overlays of FITC and PI emissions from stained cells that were isolated from EL4 tumour explants of mice treated (+; upper row of panels) or not (-; lower row of panels) with cytotoxic chemotherapy. Data depicted in the histogram overlays originated from region 1 (R1), which represents the FITC⁺ subset of PI⁺ events, and shows Sal5-FITC staining of tumour cells from chemotherapy-treated mice (grey fill), 3B9-FITC staining of tumour cells from untreated mice (dashed line) and chemotherapy-treated mice (thick line). After chemotherapy, the fraction of PI⁺ cells in tumor explants increased significantly from a mean (± SE) of 50 ± 2% to 70 ± 1% (*P* < 0.001). Similarly, the 3B9⁺ subset of PI⁺ cells increased significantly from 15 ± 1% to 38 ± 2% (*P* < 0.01) after chemotherapy, whereas isotype control staining was unaltered. Only the PI⁺ tumour subpopulation bound 3B9, which indicated that La was recognized specifically in dead tumour cells. Histogram analysis indicated that specific per cell binding of 3B9-FITC to PI⁺ cells was significantly augmented in tumors exposed *in vivo* to cytotoxic chemotherapy (net median fluorescence intensity ± SE of 18 ± 3 with chemotherapy and 1 ± 3 without chemotherapy, *P* < 0.05).

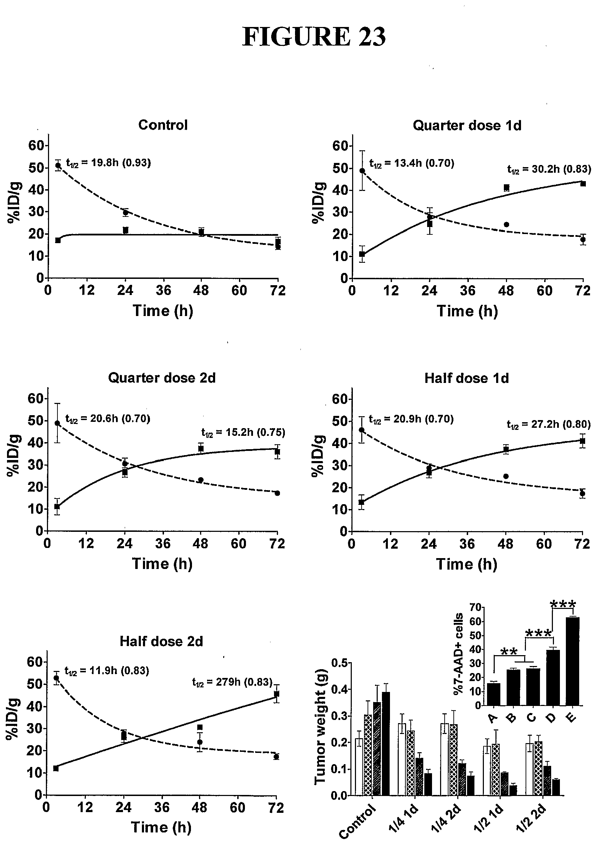

**Figure 23** depicts that time-activity curves of ¹¹¹In-DOTA-3B9 in EL-4 tumour-bearing mice treated or not with cytotoxic chemotherapy. EL4 tumour-bearing mice (*n*=5) were left untreated (control) or treated with cyclophosphamide and etoposide in four different regimens of escalating dose intensity. For each analysis time point after ¹¹¹In-DOTA-3B9 administration, data are expressed as percentage of organ radioactivity normalized to organ weight and divided by radioactivity of injected dose (%ID/g). Data are presented as mean ± SEM and time activity curves were fitted for blood (--) and tumour (-). Half-life (t_{1/2}) values for blood clearance and tumour accumulation are displayed next to each fitted curve and regression values are shown in brackets. Lower right-hand panel: column graph showing tumour weights (g) from control and treated mice, which are expressed as mean ± SEM measured at 3h (clear), 24h (light hatched), 48h (dark hatched) and 72h (filled) post-injection. **Inset:** In separate experiments, single cell suspensions prepared from EL4 tumours (*n*=4) were analyzed by flow cytometry for 7-AAD binding at 48h post-injection for control mice (A) or mice treated with the following chemotherapy regimens: quarter dose 2d (B), half dose 1d (C), half dose 2d (D), and half dose 2d analyzed at 72h post-injection (E). Data are shown as mean ± SEM of percentage 7-AAD⁺ tumour cells. Statistical comparisons were made with control tumours.

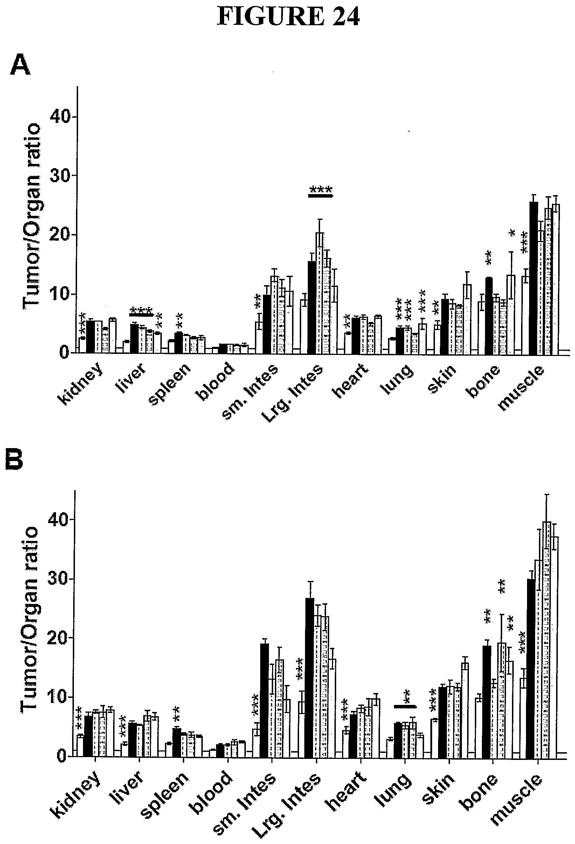

**Figure 24** depicts that tumour targeting efficiency of ¹¹¹In-DOTA-3B9 is enhanced by cytotoxic chemotherapy. The accumulation of ¹¹¹In-DOTA-3B9 in the tumour (%ID/g) measured at (A) 48h and (B) 72h was divided on that of other organs at these time points. The ratio is presented as the mean ± SEM from control mice (clear bars), mice treated with quarter dose 1d (black bars), quarter dose 2d (streaked bars), half dose 1d (dark filled bars) or half dose 2d (light filled bars). Asterisks (* *P*<*0.05,* ** *P<0.01* or *** *P<0.001*) denote significant differences in comparison to control mice. Solid line represent ratio of 1. Only tumour/muscle ratio was significantly different in chemotherapy treated mice at 24h after administration (*P<0.001*) (data not shown).

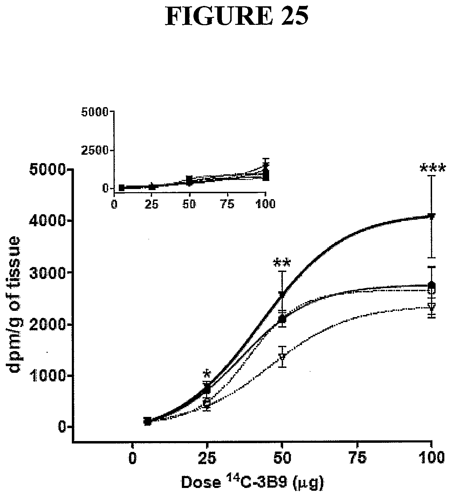

**Figure 25** depicts that the intra-tumoral accumulation of biosynthetically labelled La-specific ¹⁴C-3B9 mAb is dose-dependent and is augmented by cytotoxic chemotherapy. The biodistribution of different doses of ¹⁴C-labelled 3B9 was studied in EL4 tumour bearing mice with or without chemotherapy. ¹⁴C-3B9 accumulated significantly more only in the tumour with chemotherapy when its uptake approximately doubled at ¹⁴C-3B9 doses of 25 µg, 50 µg and 100 µg. Mice were given intravenous injections of 5, 25, 50 or 100 µg of ¹⁴C-3B9 or ¹⁴C-Sal5 and then treated or not with etoposide 67 mg/kg and cyclophosphamide 100 mg/kg, which were given as two intraperitoneal injections 24 hours apart. Forty-eight hours after commencement of treatment, the mice were killed and organs collected for radioactivity measurement. Radioactivity was normalised to the mass of tissue counted (dpm/g of tissue) and tissues included (◆) liver, (■) spleen, (▲) kidneys, (●) serum and (▼) tumour. Data are presented as dpm/g ± SEM. Closed symbols, with chemotherapy; open symbols, without chemotherapy. Statistical comparisons were made using two-way analysis of variance, which was performed as a Bonferroni post-test comparison between all groups (*n*>4).

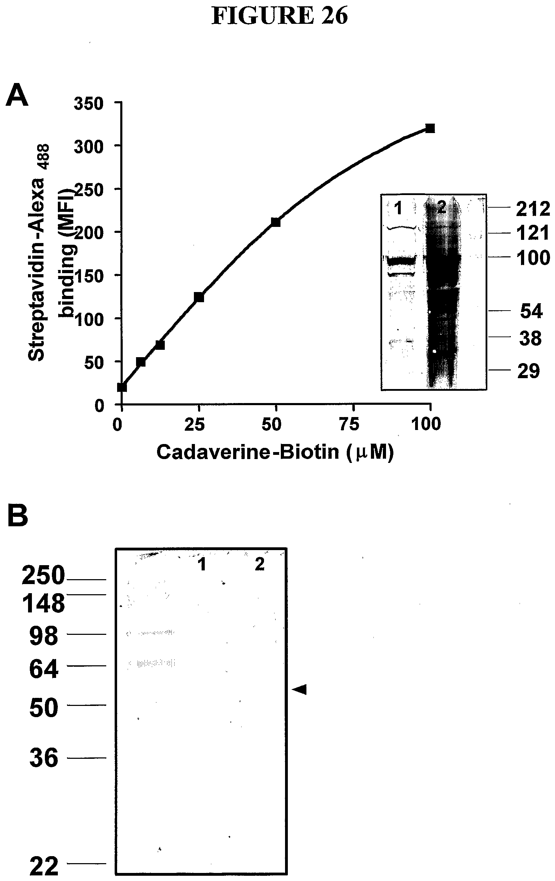

**Figure 26** depicts that incorporation of cadaverine-biotin into the target for Apomab during apoptosis. (**A**) Jurkat cells are induced to undergo apoptosis using 20µg/mL cisplatin for 48h with or without increasing concentrations of cadaverine-biotin. Cells are incubated with 2µg/mL of streptavidin-Alexa₄₈₈ for 20 min., washed, stained with 2µg/mL 7-AAD to assess viability and then analysed by flow cytometry. Data are presented as the MFI ± SEM from two independent assays. > 90% Jurkat cells are 7-AAD⁺ and Alexa₄₈₈ fluorescence emitted from 7-AAD⁺ cells is plotted as a function of cadaverine-biotin concentration. **Inset:** lysates (12 µg) of cells incubated in the absence (lane 1) or the presence (lane 2) of 100µM cadaverine-biotin are fractionated on 12% SDS-PAGE. The gel is transferred to PVDF membrane and probed using streptavidin-AP. (**B**) Lysates of cells incubated in the absence (lane 1) or presence of 100µM cadaverine-biotin (lane 2) are first immunoprecipitated using Apomab before probing the immunoblots with streptavidin-AP. Arrow head, position of putative La band in lane 2.

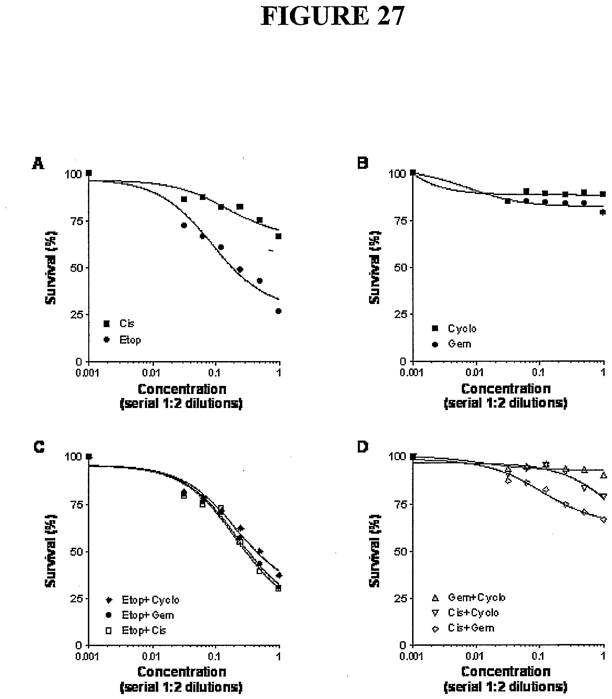

**Figure 27** depicts that different cytotoxic drugs differentially impair survival of primary ALL blasts. ALL blasts were cultured in the presence or absence of serial 1:2 dilutions of A. 40 µg/mL etoposide, or 20 µg/mL cisplatin, or **B.** 200 µg/mL gemcitabine, or 20 µg/mL cyclophosphamide, or **C.** and **D.** combinations of these drugs at the same concentrations also in serial dilution. The percentage of viable ALL blasts (7-AAD-negative) in medium without incubation of drug was 66.3%, which was normalised to 100% survival for comparing the effects of drug treatment on survival of ALL blasts. Sigmoidal dose-response curves were fitted to the data using GraphPad Prism v.4.0 software.

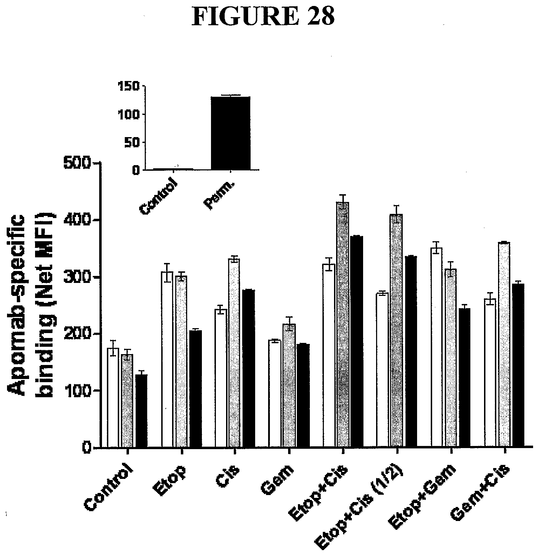

**Figure 28** depicts that Apomab binds specifically to 7-AAD⁺ primary ALL blasts after treatment with cytotoxic drugs *in vitro.* Cells were incubated in the absence (control) or presence of 40 µg/mL etoposide (Etop), 20 µg/mL cisplatin (Cis), 200 µg/mL gemcitabine (Gem), 40 µg/mL etoposide and 20 µg/mL cisplatin (Etop+Cis), 20 µg/mL etoposide and 10 µg/mL cisplatin (Etop+Cis [1/2]), 40 µg/mL etoposide and 200 µg/mL gemcitabine (Etop+Gem), and 200 µg/mL gemcitabine and 20 µg/mL cisplatin (Gem+Cis). 7-AAD⁺ events were gated and specific Apomab binding was determined as the net MFI after subtraction of the net MFI value for the Sal5 isotype control mAb. Columns, net MFI; bars, SE; (*n*=2). Apomab-specific binding was analysed at 24h (clear columns), 48h (grey columns) and 72h (black columns) after cells were incubated with cytotoxic drugs. **Inset,** Apomab-specific binding to viable ALL blasts, which were left untreated (control) or permeabilised.

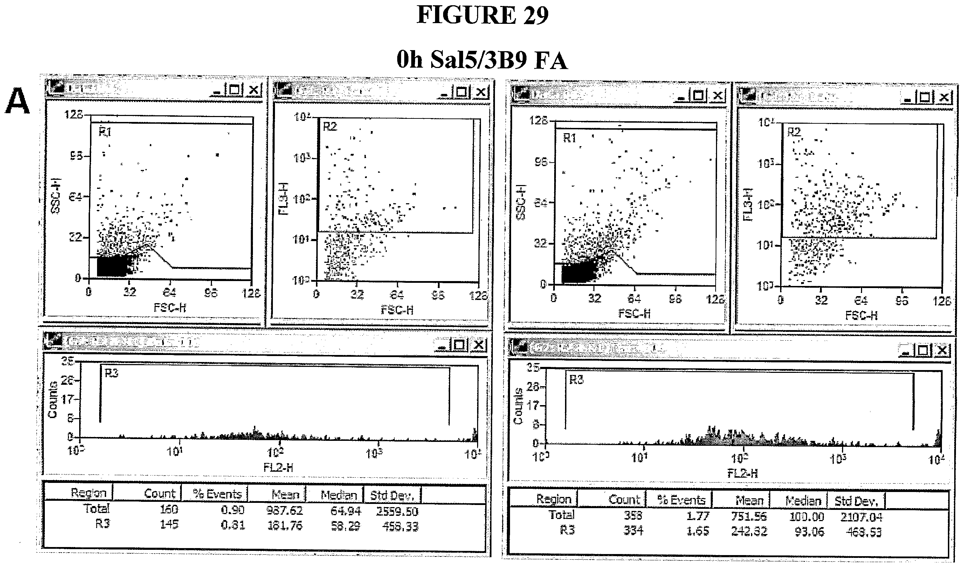

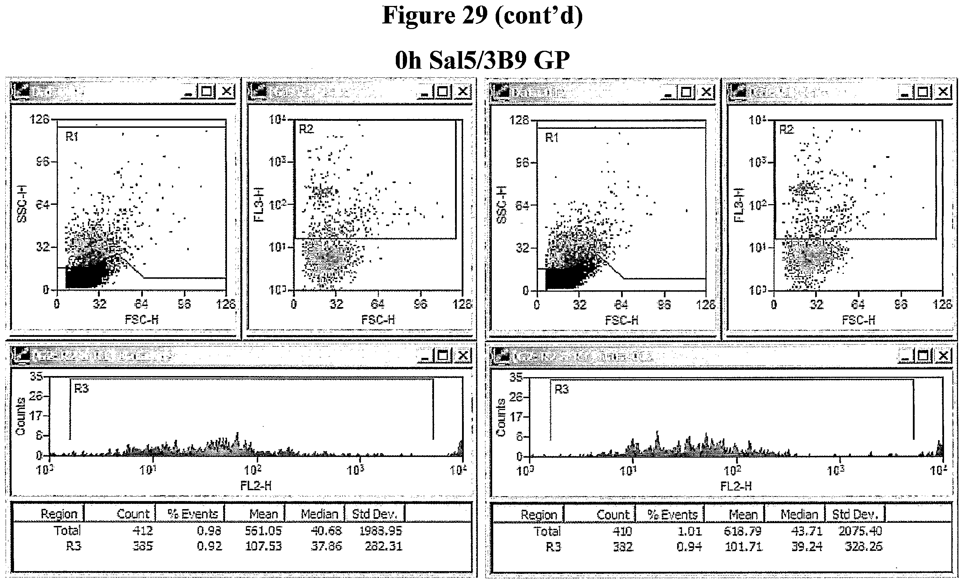

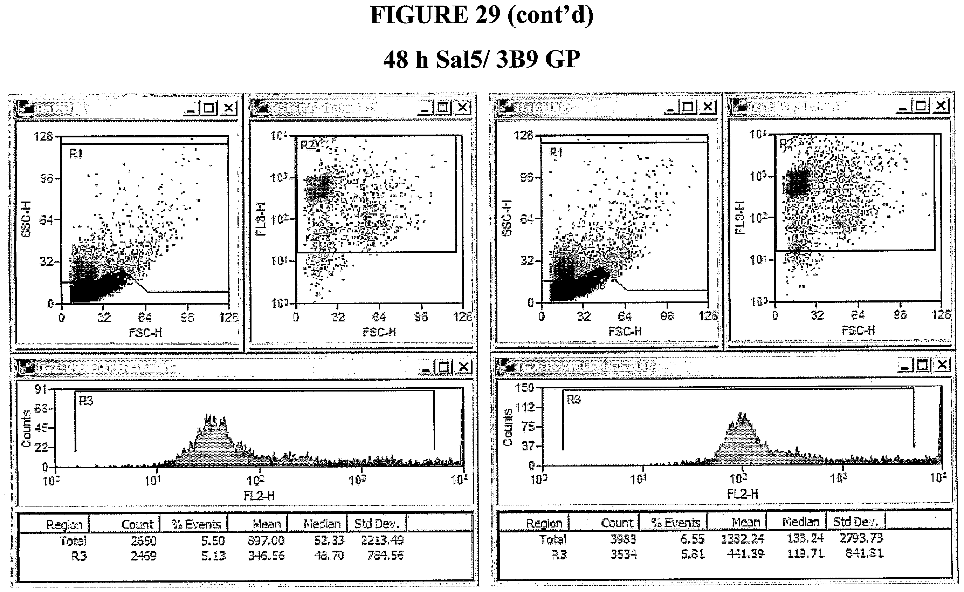

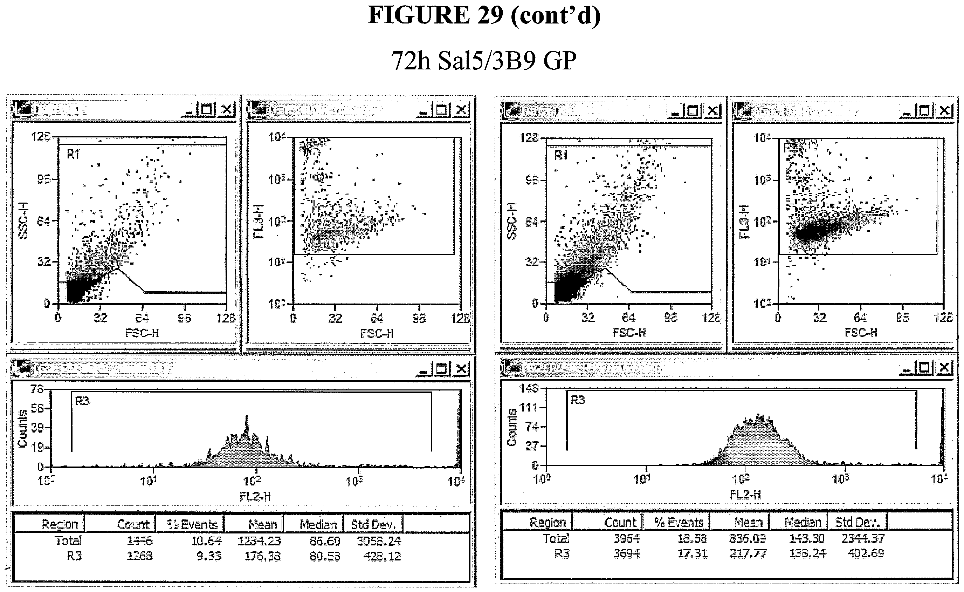

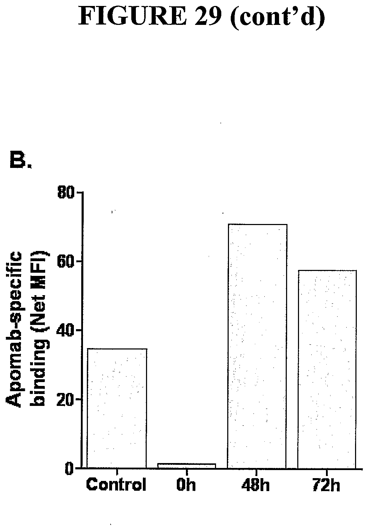

**Figure 29** depicts that Apomab binds to BerEP4-enriched cells from an SCLC patient particularly after cytotoxic chemotherapy was administered to the patient. Blood samples (2.5 mL) collected in heparinised tubes were enriched for circulating epithelial cells using the BerEP4 epithelial marker using the CELLection™ Epithelial Enrich kit as per manufacturer instructions (Dynal® Biotech, Invitrogen, USA). Briefly, 250 µL of BerEP4-beads was mixed with the blood sample for 30 min at RT. Beads were bound to Dynal® magnet and washed 5 times with PBS. Bead-bound cells were released using PBS containing 0.1 % BSA and DNase I as described by the manufacturer. Beads were bound to Dynal® magnet and released cells were removed to a fresh tube, centrifuged and washed with PBS. Samples were incubated (30 min at RT) with anti-mouse IgG (2 µg/mL) to block the mouse BerEP4 antibody remaining on the isolated cells. Samples were washed with PBS and aliquots were stained with Apomab or matching irrelevant isotype (Sal5). Finally, cells were washed with PBS and analysed by flow cytometry after incubation (15 min) with 2 µg/mL 7-AAD at RT. A. FSC vs.SSC density plots were constructed for all samples. A region (R1) was drawn based on control blood to exclude events related to normal blood cells. Density plots of FSC *vs.* FL-3 (7-AAD) were constructed from gate R1. Viable cells are shown as red and dead cells as green. A second region (R2) was constructed to select dead (7-AAD⁺) cells. FL-2 histograms of Sal5 (left) and 3B9 (right) staining are shown in green and are gated on 7-AAD⁺ cells. **B.** A summary column graph shows Apomab-specific binding as net median fluorescence intensity (MFI) (after subtraction of MFI values for Sal5 staining). *Notes:* (i) A 24h time point is not shown because bead separation failed. (ii) In the case of 48 and 72 h samples, a population with very high fluorescence was observed and was not different between control and positive staining. Therefore, an R3 region was drawn to exclude these populations from the histograms.

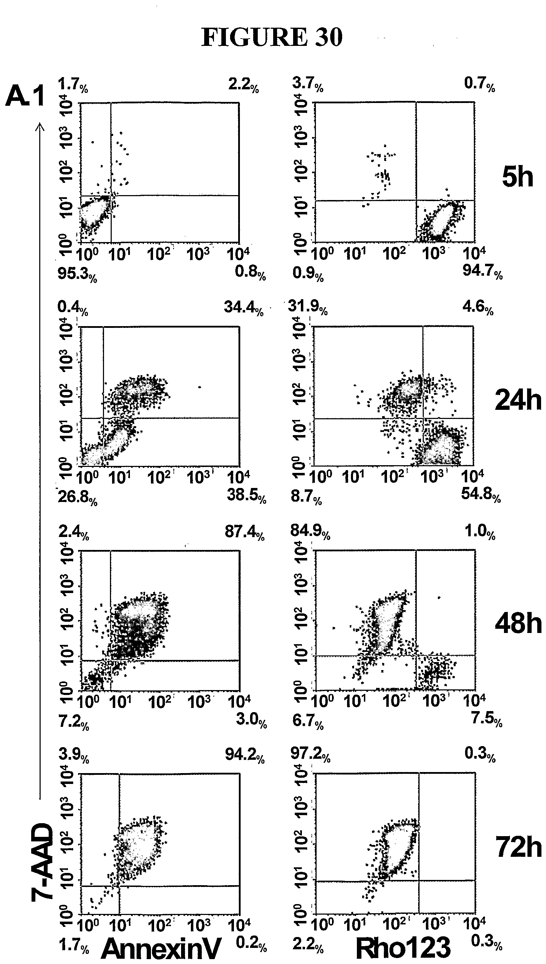

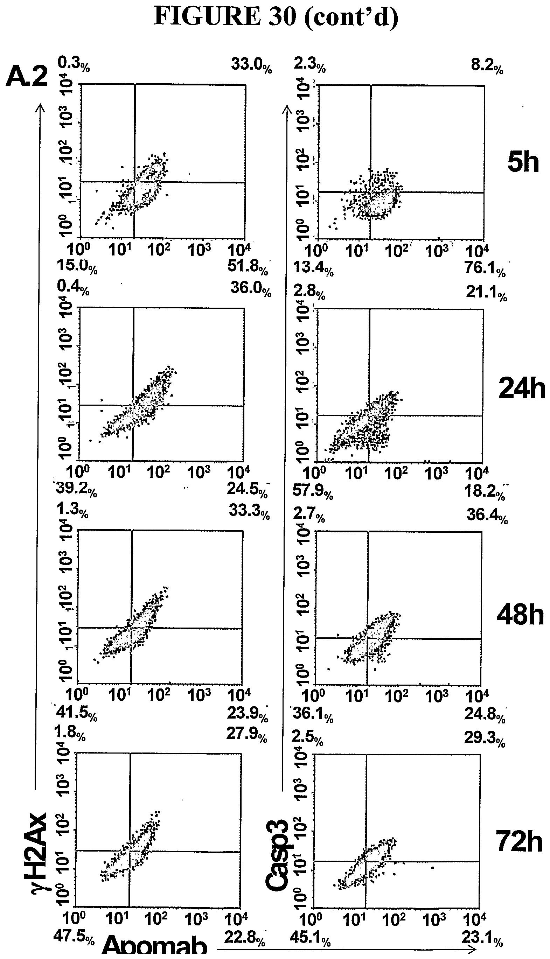

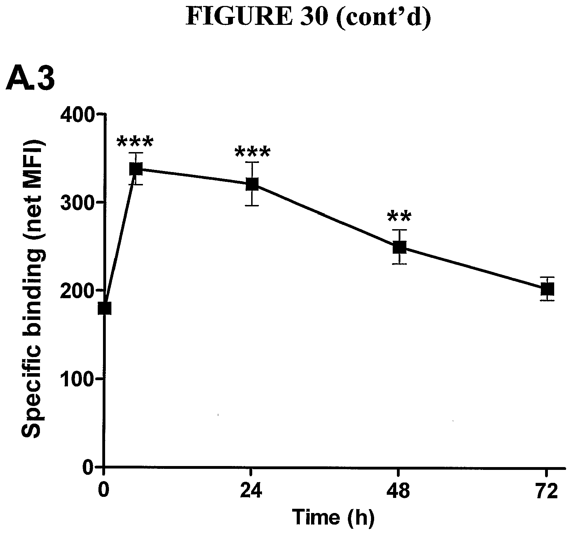

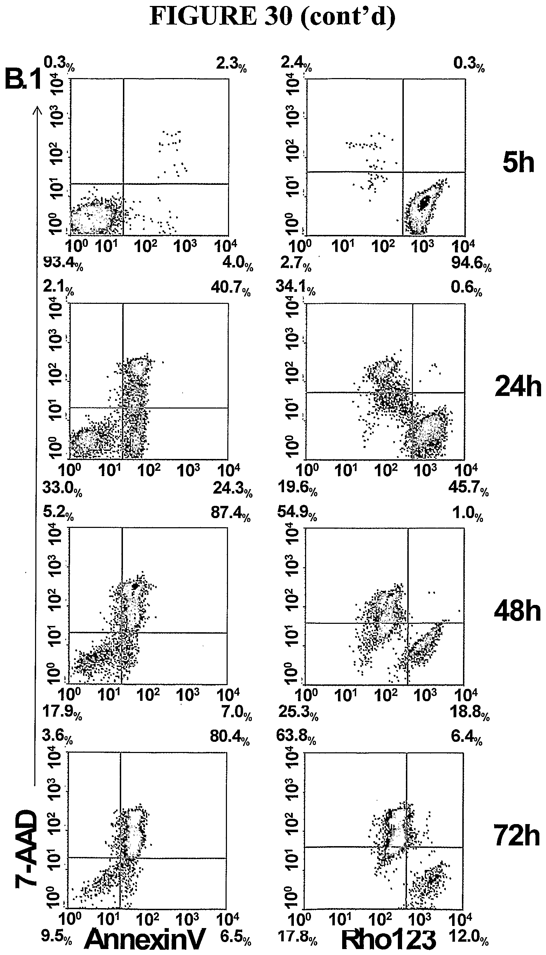

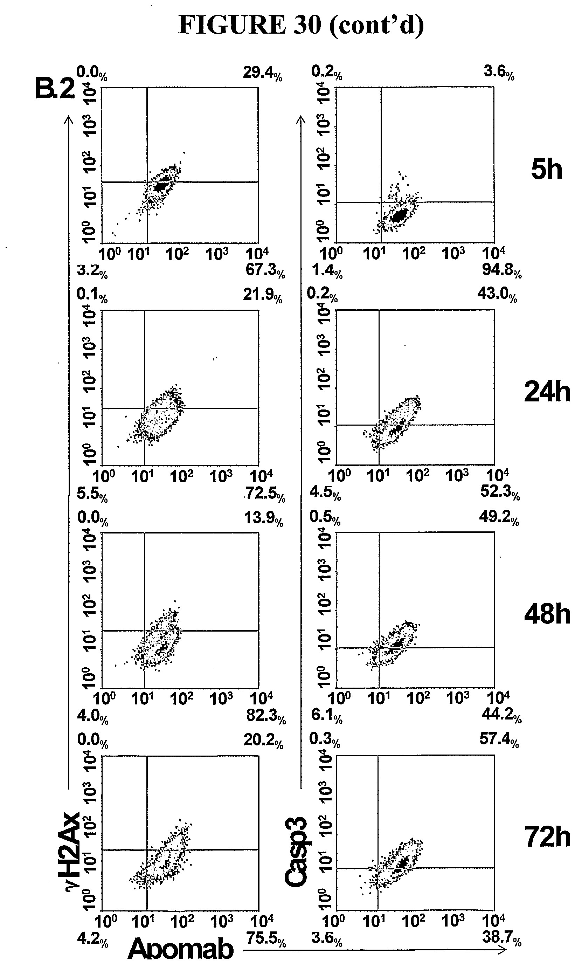

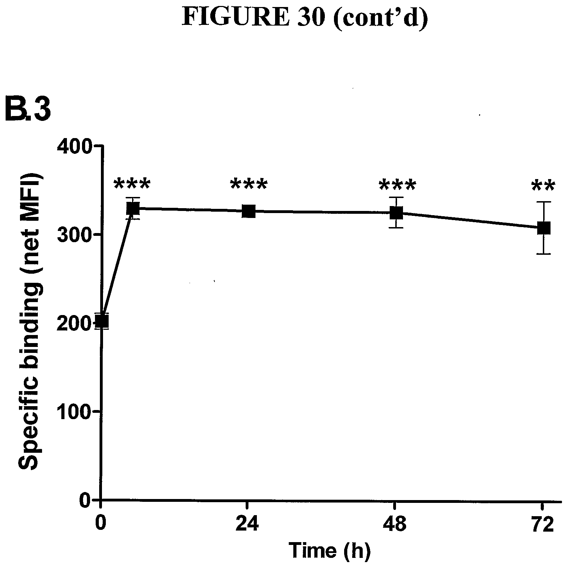

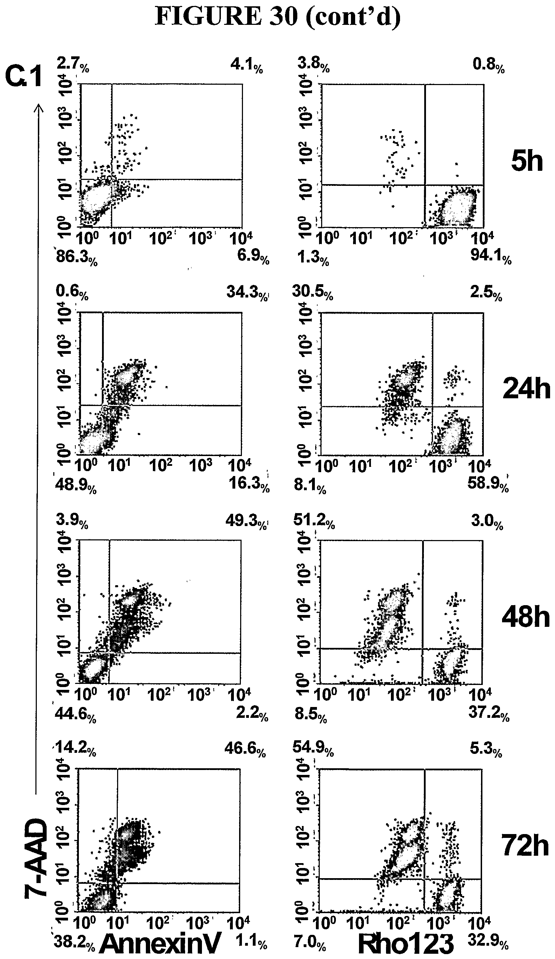

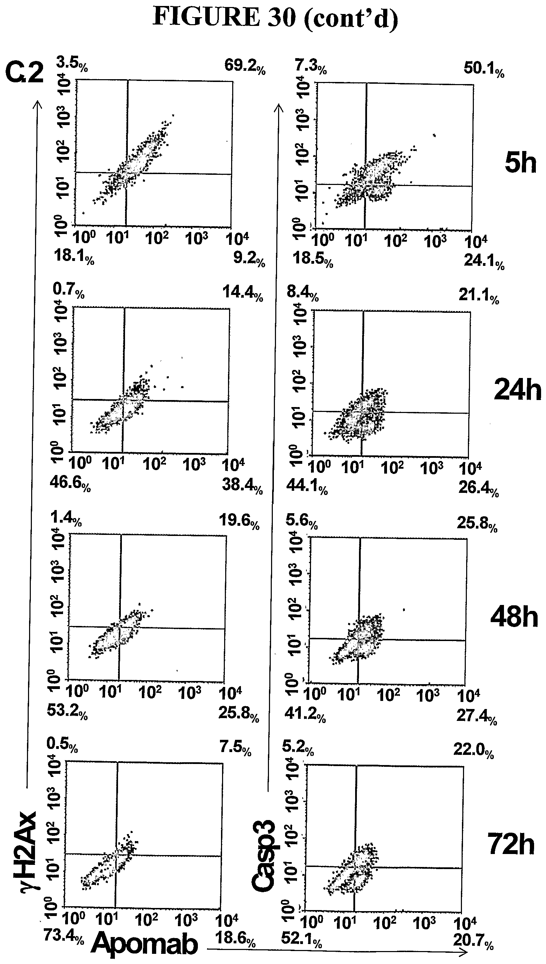

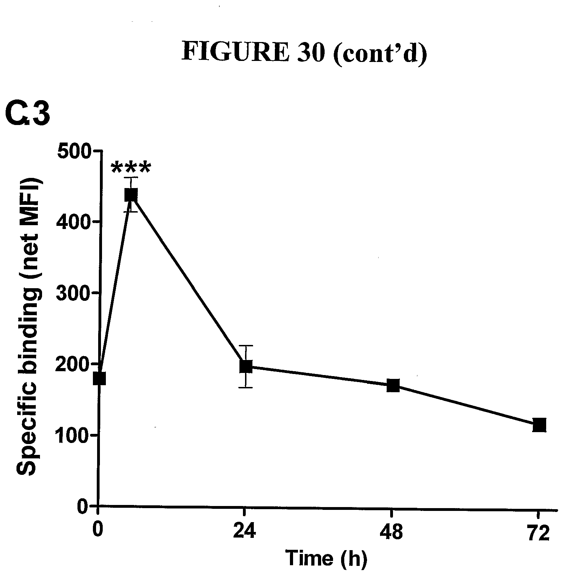

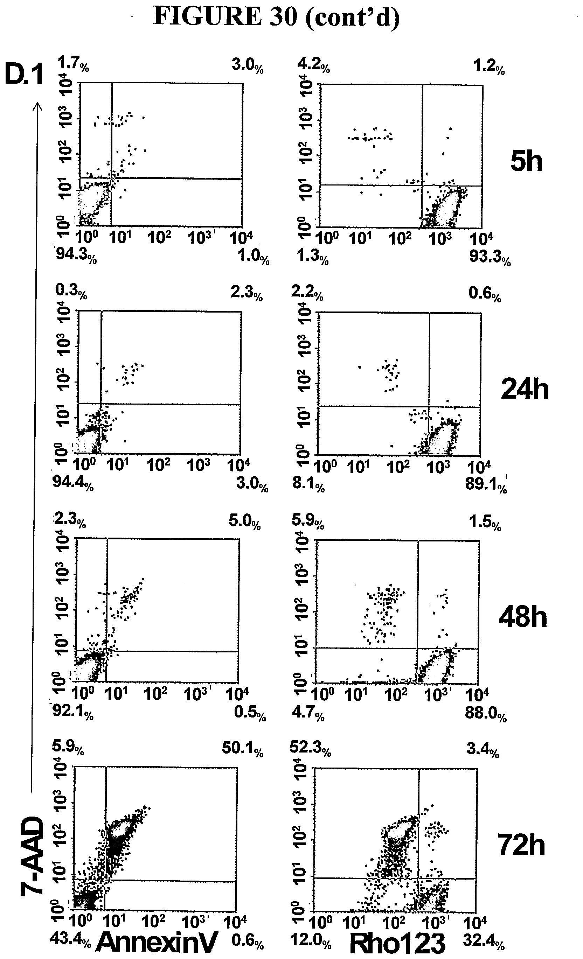

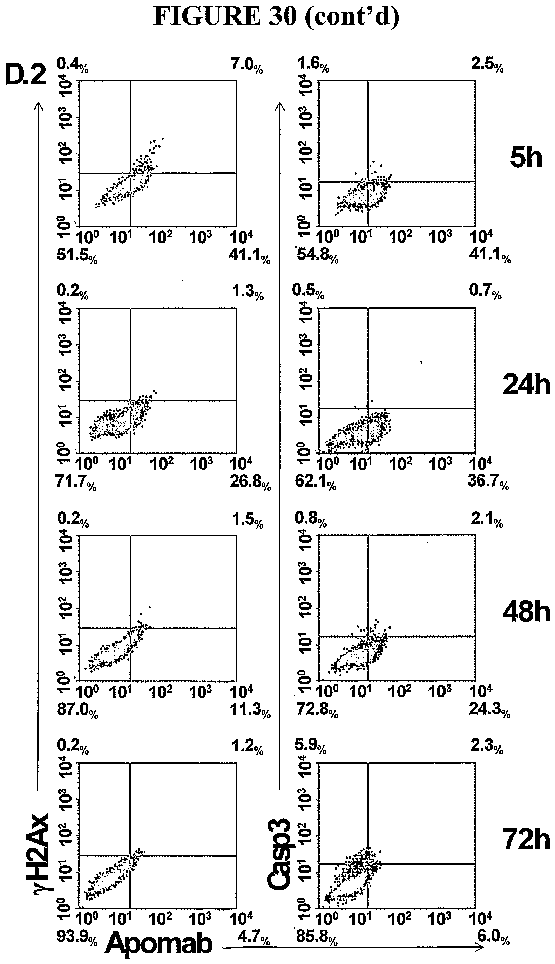

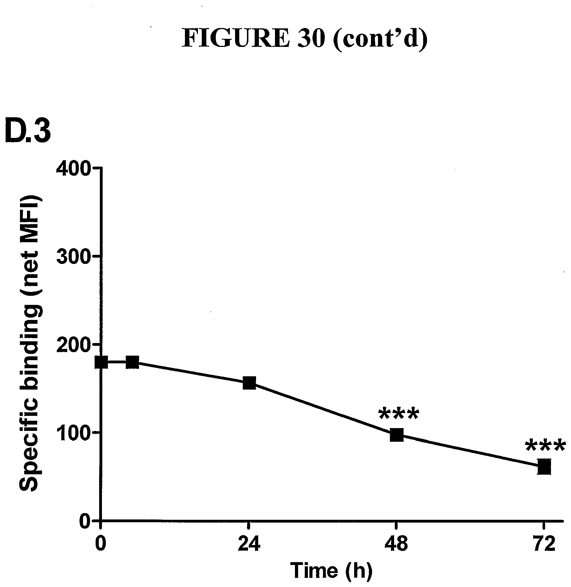

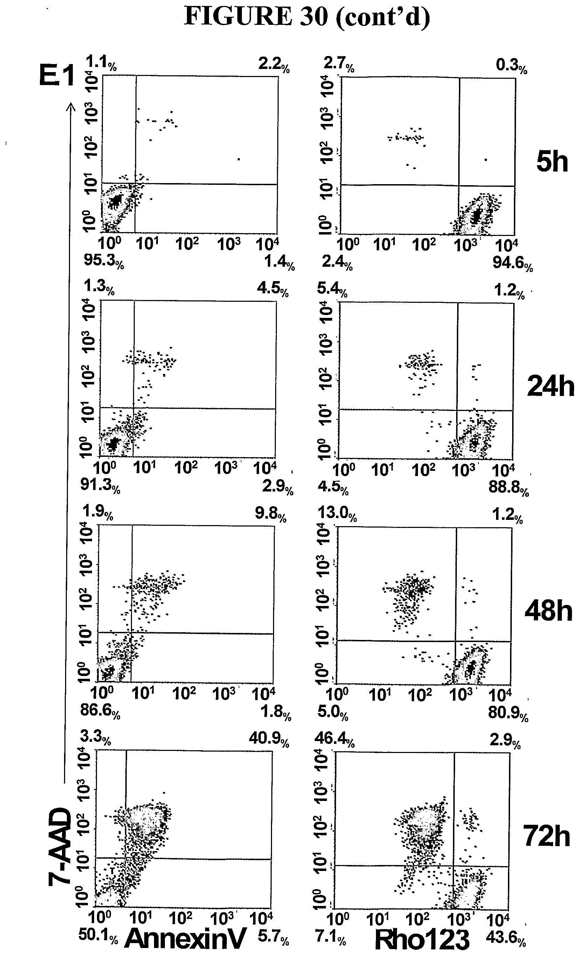

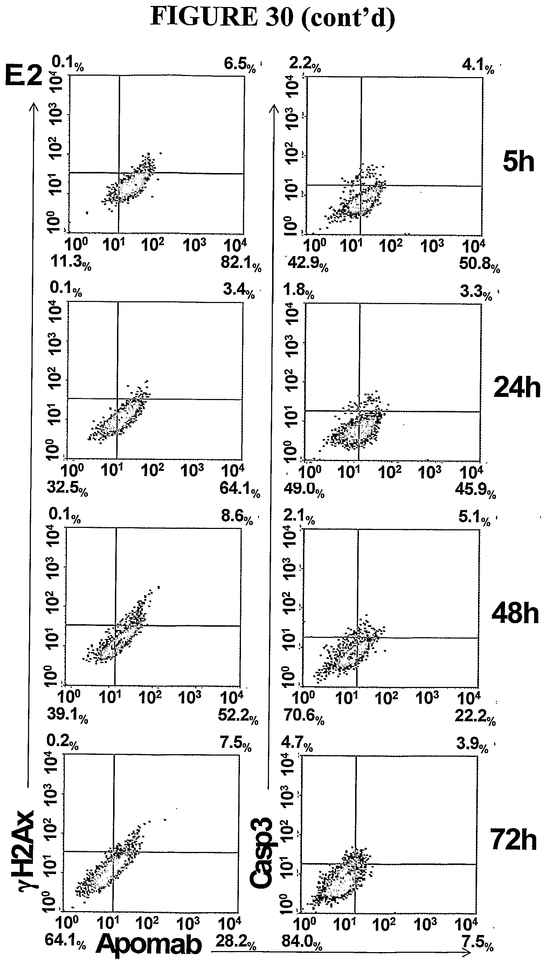

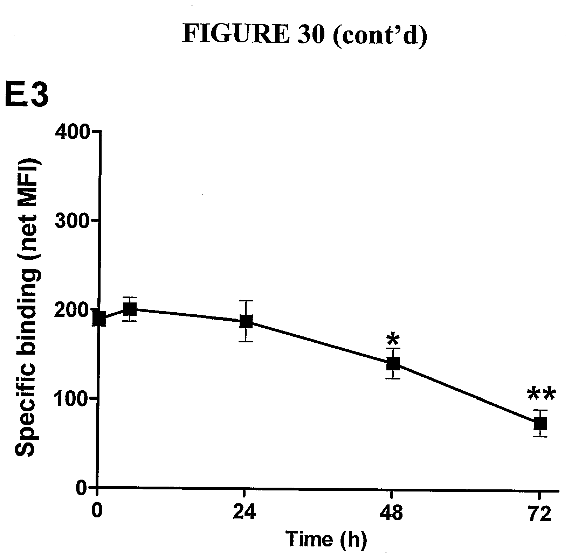

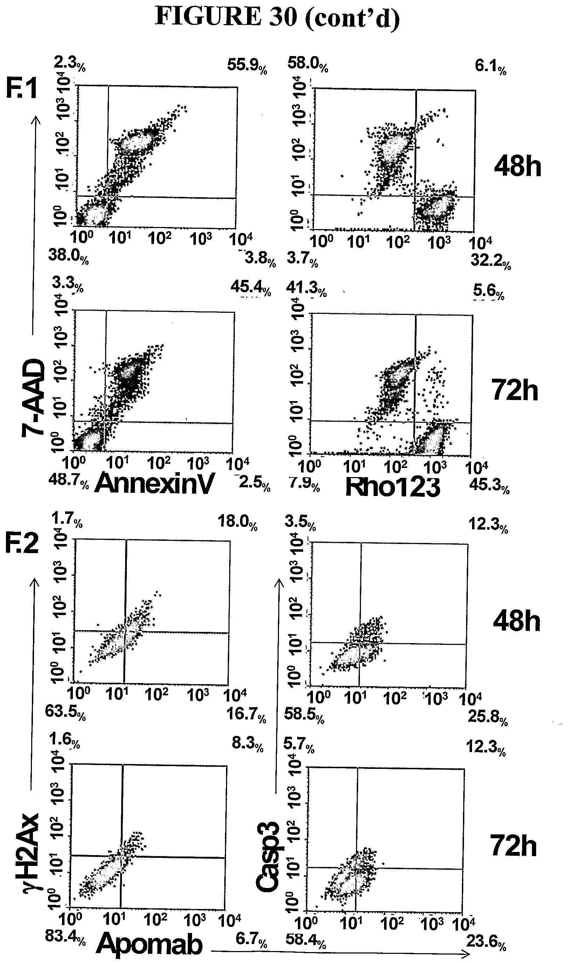

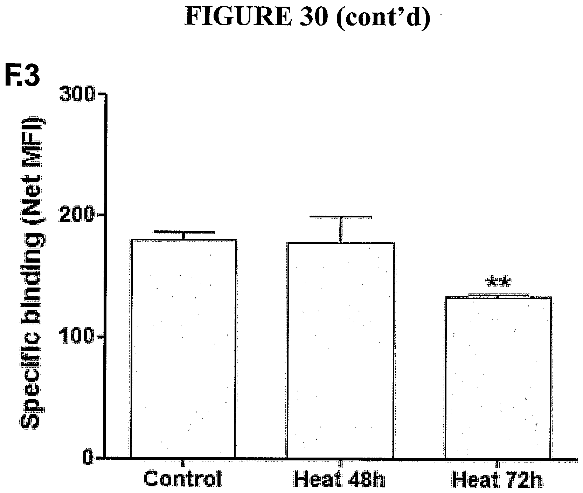

**Figure 30** depicts that activators of the intrinsic apoptotic pathway extends the kinetics of Apomab binding compared with extrinsic apoptotic pathway activators. Jurkat cells were treated with stimuli that induced apoptosis *via* mainly either the intrinsic pathway using **A.** cisplatin 20 µg/mL or **B.** γ -radiation 15 Gy, or the extrinsic pathway using **C.** anti-Fas mAb 250 ng/mL. In addition, Jurkat cells were stressed by either **D.** allowing overgrowth in the culture medium or **E.** serum withdrawal. **F.** Necrosis was induced by heat treatment. Aliquots of the Jurkat cell cultures were removed at the indicated time points and stained with annexin V, rhodamine 123 or 7-AAD without fixation or permeabilisation, or were fixed and permeabilised and then stained with either Sal5 or 3B9 and γH2AX, or Sal5 or 3B9 and anti-activated caspase-3 mAb. **, *P*<0.01; ***, *P*<0.001;

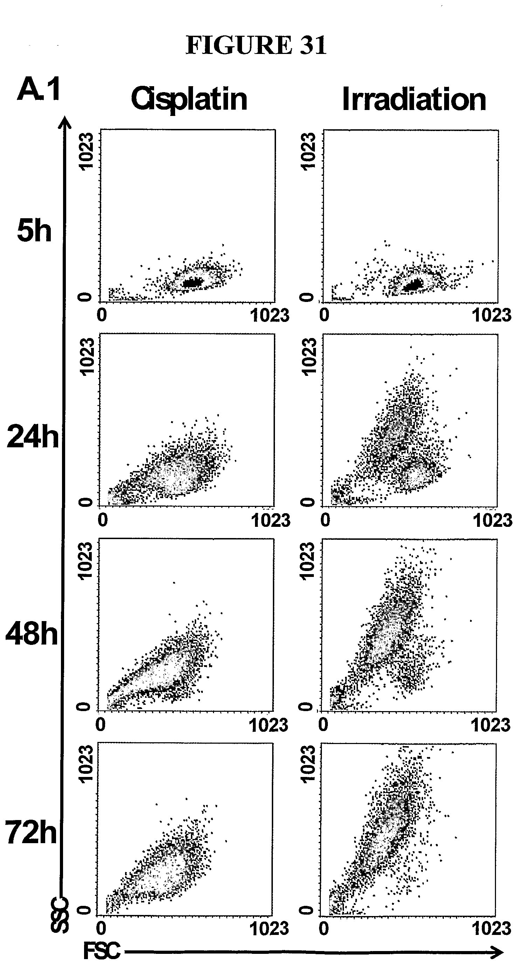

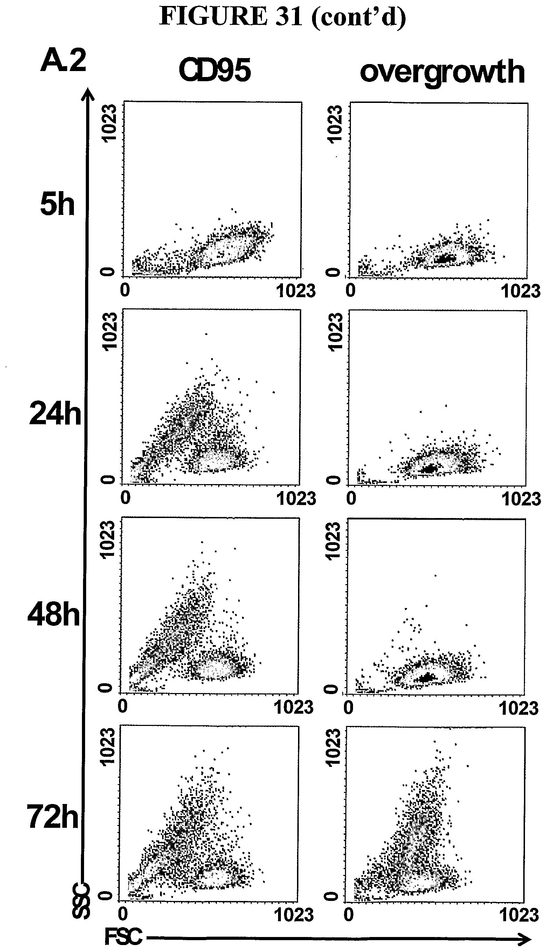

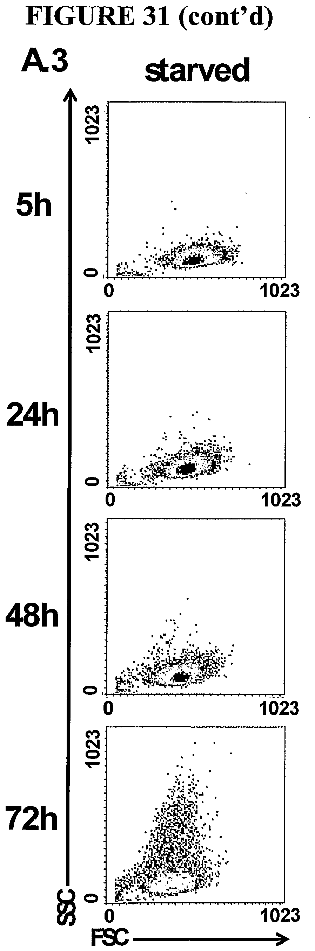

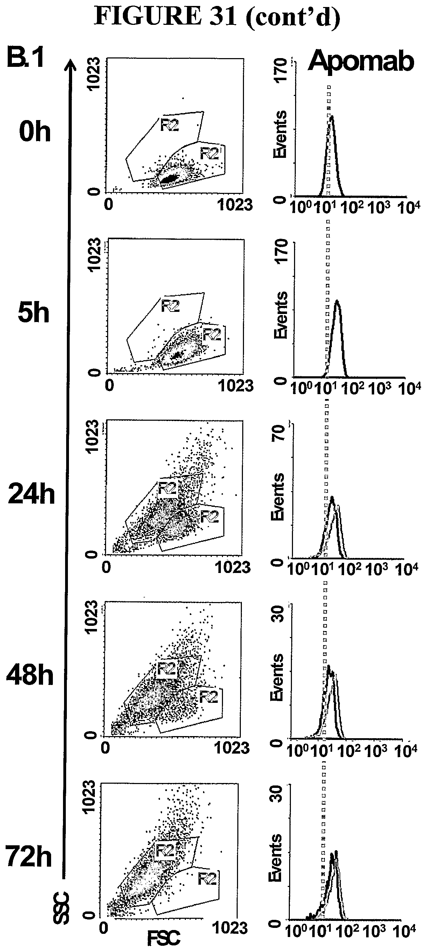

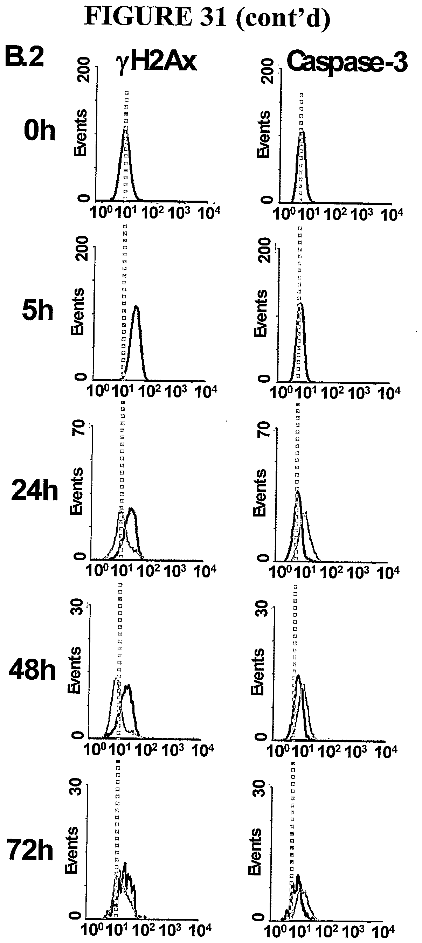

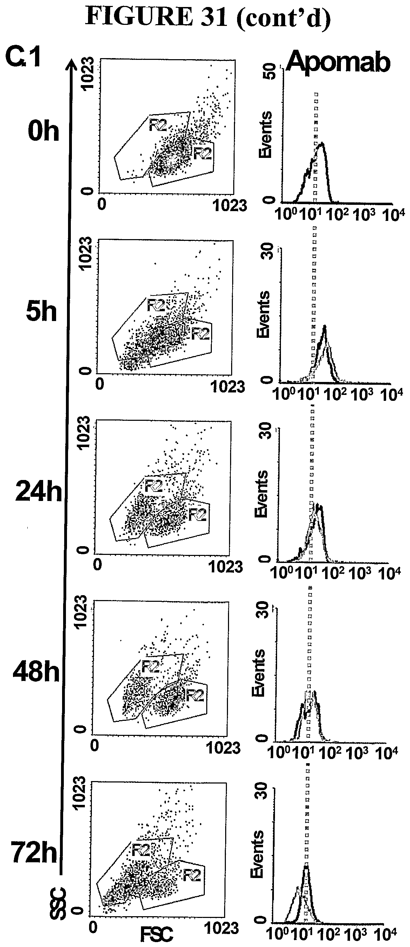

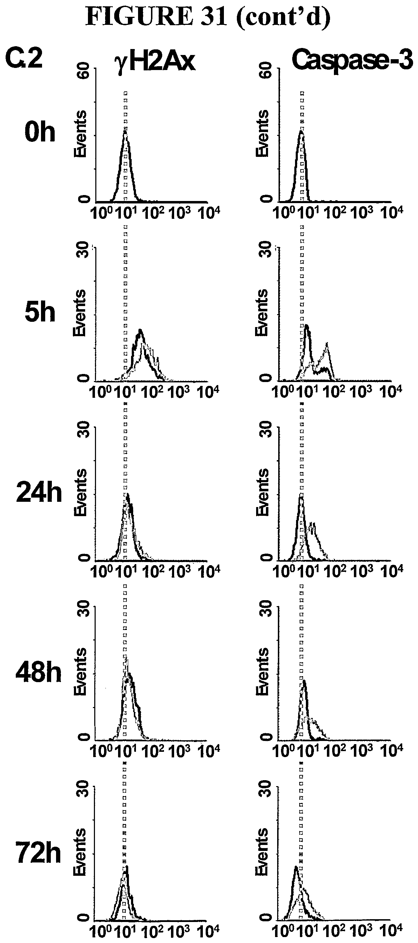

**Figure 31** depicts that γ-radiation and Fas ligation induce apobody formation with different kinetics and different patterns of γH2AX and La induction. **A.** Scatter plots are shown for each of the experimental conditions indicated in Fig. 4. **B.** Jurkat cells were γ-irradiated to a dose of 15 Gy, or treated with 250 ng/mL anti-Fas (CD95) mAb. At the specified time points, cells were fixed and permeabilised and stained with either Apomab and anti-γH2AX or Apomab and anti-activated caspase-3. Gates R1 and R2 were drawn from FSC *vs.* SSC plots and Apomab, γH2AX or activated caspase-3⁺ staining are shown for the different gates (Rl, black; R2, red)

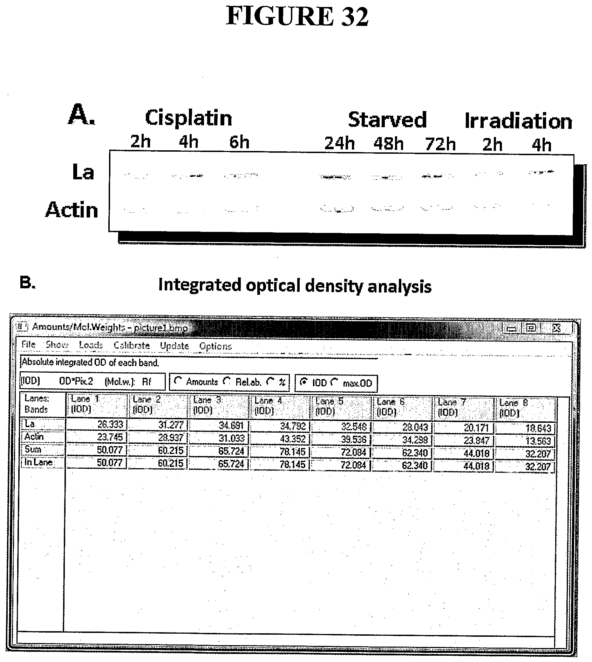

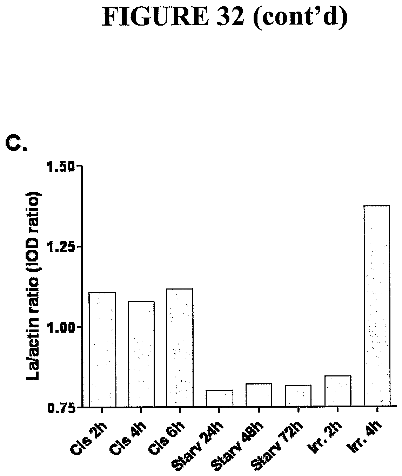

**Figure 32** depicts that DNA-damaging treatment induces early expression of La protein in Jurkat cells *in vitro.* **A.** Lysates of cultured Jurkat cells were prepared at the indicated time points after treatment or not and electrophoresed by SDS-PAGE. After transfer, the blots were probed with Apomab or anti-actin antibodies, and **B.** integrated optical density analysis performed. **C.** The results of this analysis are shown in a column graph.

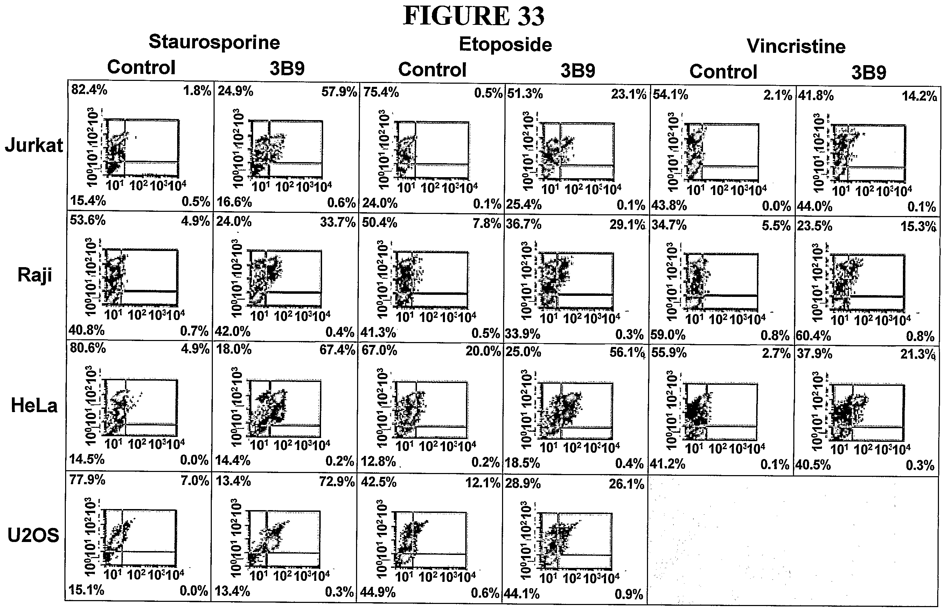

**Figure 33** depicts that cytotoxic drugs induce binding of 3B9 (Apomab) to malignant human cell lines after cell death *in vitro.* Staining with propidium iodide (PI) is indicated on the y-axis. Jurkat, T cell leukemia; Raji, B cell leukemia; HeLa, cervical carcinoma; U2OS, osteosarcoma.

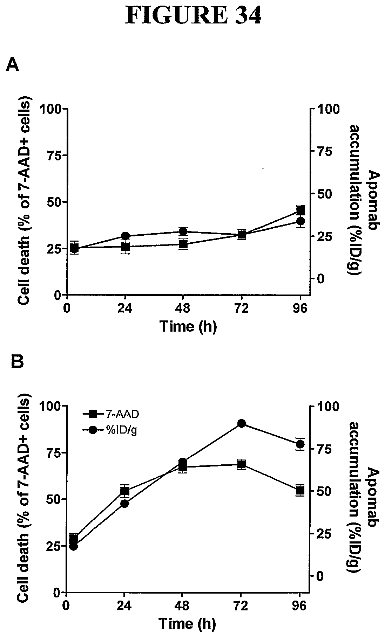

**Figure 34** depicts that after CE chemotherapy, tumour Apomab accumulation *in vivo* is disproportionately higher than the tumour cell death rate, which suggests that the La target antigen is induced by DNA-damaging chemotherapy. EL4 tumour cell death and Apomab accumulation were analysed at the indicated time points in A. untreated control and B. treated mice (*n*=3). Apomab accumulation was calculated from ¹¹¹In-Apomab accumulation after accounting for radioactive decay of ¹¹¹Indium. Data points, mean %7-AAD⁺ cells (left Y-axis, squares) and mean %ID/g Apomab (right Y-axis, circles); bars, SE.

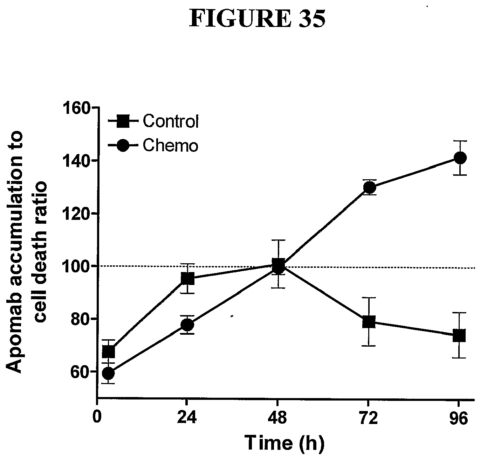

**Figure 35** depicts that after CE chemotherapy, the ratio of tumour Apomab accumulation to tumour cell death declines in control tumours and is enhanced in treated tumours, which suggests that the La target antigen is induced by DNA-damaging chemotherapy. Tumour Apomab accumulation (%ID/g) was directly related to tumour cell death (%7-AAD⁺ cells) at each of the indicated time points for EL4 tumours derived from control or treated mice (*n*=3). Data points, mean ratio; bars, SE; control (squares); chemotherapy (circles); dotted line, 100%.

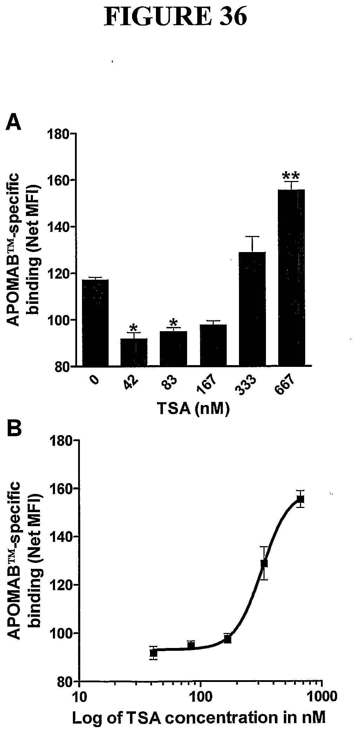

**Figure 36** depicts that the histone deacetylase inhibitor (HDACi) trichostatin A (TSA) exerts dose-dependent synergy with cisplatin to augment Apomab-specific binding to dead Jurkat cells. Jurkat cells were incubated for 48h with 10µg/mL cisplatin together with increasing concentrations of TSA. Cells were subjected to indirect immunofluorescence staining using Apomab or its Sal5 isotype control mAb and viability was assessed using 7-AAD. Apomab-specific binding was calculated as the Net MFI after gating on 7-AAD⁺ events. Data are presented as the mean ± SEM from two separate experiments. (**A**) Apomab-specific binding to cisplatin-treated Jurkat cells is plotted as a function of TSA concentration. Binding in the presence of TAS was compared with binding in the absence of TSA using two-way ANOVA and a Bonferroni post-test comparison (*, *P*<0.05; **, *P*<0.01). (**B**) TSA concentration data were log-transformed to suggest a dose-response effect of TSA dose on Apomab-specific binding. GraphPad Prism software was used to find a sigmoidal-dose response with variable slope as the best-fit model for the data (*r*² = 0.98).

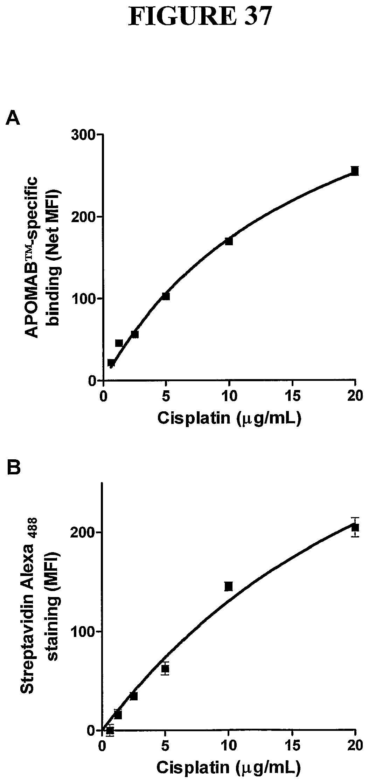

**Figure 37** depicts that the extent of Apomab binding to apoptotic Jurkat cells *in vitro* correlates directly with both cisplatin dose and TG2 protein cross linking activity. (**A**) Jurkat cells were incubated for 48h in increasing concentrations of cisplatin and then subjected to indirect immunofluorescence staining using 5µg/mL Apomab followed by 2µg/mL anti-mouse IgG Alexa₄₈₈ antibody. (**B**) Jurkat cells were incubated with 100µM cadaverine-biotin and increasing concentrations of cisplatin. After 48h, cells were incubated at RT for 15min. with 2µg/mL streptavidin-Alexa₄₈₈ then washed and analysed by flow cytometry. Data (*n*=3) shown were gated on 7-AAD⁺ events and are presented as (A) net MFI (±SEM) for Apomab-specific binding or as (B) MFI (±SEM) for cadaverine-biotin incorporation.

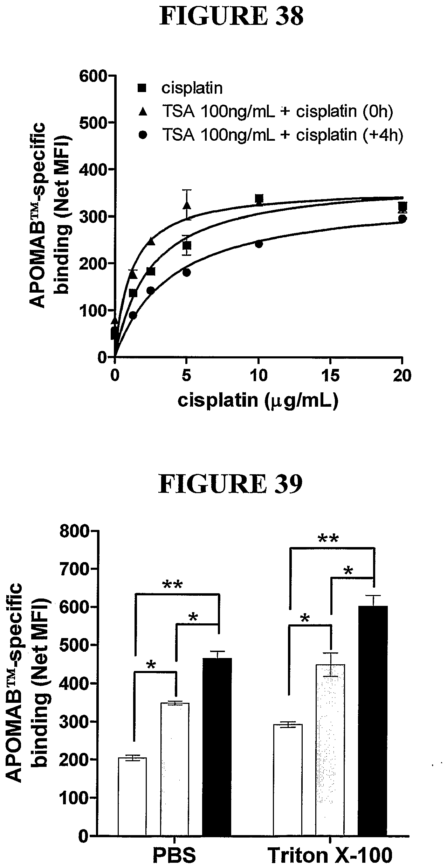

**Figure 38** depicts that the synergistic effect of TSA on Apomab-specific binding to cisplatin-treated Jurkat cells is schedule-dependent. Jurkat cells were treated with increasing concentrations of cisplatin alone (■), increasing concentrations of cisplatin in the presence of 100ng/mL TSA (▲), or increasing concentrations of cisplatin added 4h from the time of addition of 100ng/mL TSA (●). After 48h, cells were subjected to indirect immunofluorescence staining using Apomab or its Sal5 isotype control mAb and viability was assessed using 7-AAD. Apomab-specific binding was calculated as the Net MFI after gating on 7-AAD⁺ events. Data are shown as the Net MFI ± SEM (*n*=3)*.*
**Figure 39** depicts that the histone deacetylase inhibitor (HDACi) trichostatin A (TSA) synergises with cisplatin to augment detergent-resistant Apomab-specific binding to dead Jurkat cells. Jurkat cells were incubated in 200ng/mL TSA (clear bars) or 20µg/mL cisplatin (grey bars) singly or in combination (black bars). After 48h, cells were subjected to indirect immunofluorescence staining using Apomab or its Sal5 isotype control mAb and viability was assessed using 7-AAD. Apomab-specific binding was calculated as the Net MFI after gating on 7-AAD⁺ events. Data are shown as the Net MFI ± SEM (*n*=3). Statistical analysis was performed using two-way ANOVA and a Bonferroni post-test comparison (*, *P*<0.01; **, *P*<0.001).

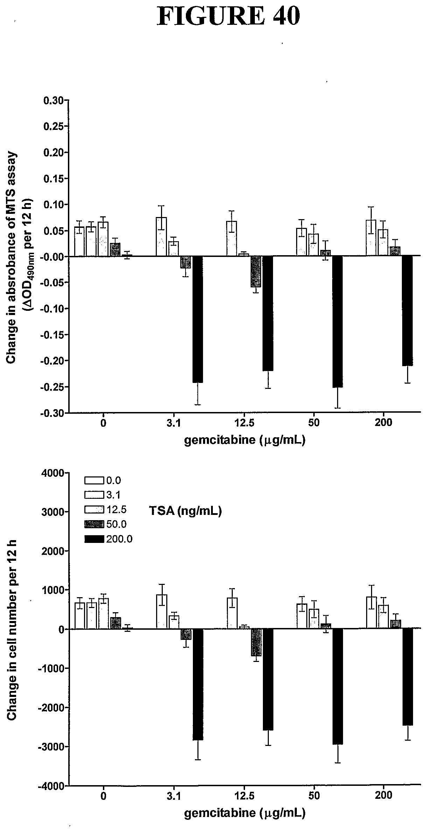

**Figure 40** depicts that gemcitabine and TSA synergise to reduce proliferation rates among PANC-1 cells *in vitro.* PANC-1 cells (2 x 10⁴ cells) were seeded in flat bottom 96-well plates overnight using phenol-free RPMI-1640 medium containing 10 % FCS (medium). A serial dilution of 2 x 10⁴ cells was used to construct a standard curve for the MTS assay. Triplicate wells were incubated with increasing concentrations of TSA (nM) with or without the addition of increasing concentrations of gemcitabine. Three identical plates were incubated for 24, 48 or 72h then medium was decanted and fresh medium (100µL) containing 20µL MTS/PMS substrate was added to all wells. Assays were performed according to the manufacturer's instructions. The change in the absorbance reading over time was calculated for each condition in units of change per 12 h (upper panel), which was converted to units of cell number change per 12h (lower panel) using a standard curve of the absorbance reading as a function of time (r² = 0.99).

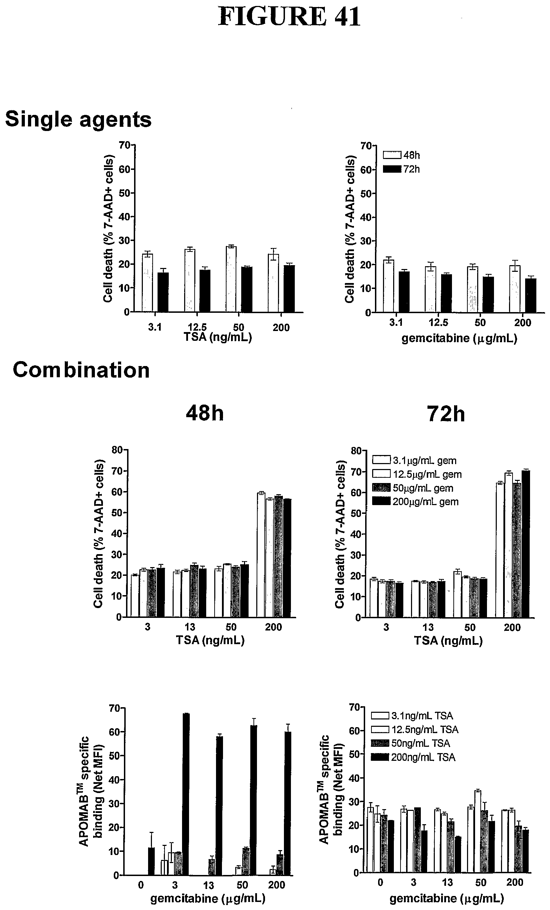

**Figure 41** depicts that gemcitabine and TSA synergise to increase cell death among PANC-1 cells, which subsequently demonstrate peak Apomab-specific binding 48h after induction of cell death. PANC-1 cells (2 x 10⁴ cells) were seeded in flat bottom 96-well plates overnight using phenol-free RPMI-1640 medium containing 10 % FCS (medium). Cells were incubated with the specified concentrations of gemcitabine or TSA singly or in combination. Cells were collected (from media and EDTA detached adherent cells) and subjected to indirect immunofluorescence staining with Apomab or its Sal5 isotype control mAb and assessed for viability with 7-AAD staining. Data are shown as the percentage of cells staining with 7-AAD (upper four panels) or as net MFI ± SEM (*n*=3) for Apomab-specific binding to 7-AAD⁺ cells (lower two panels).

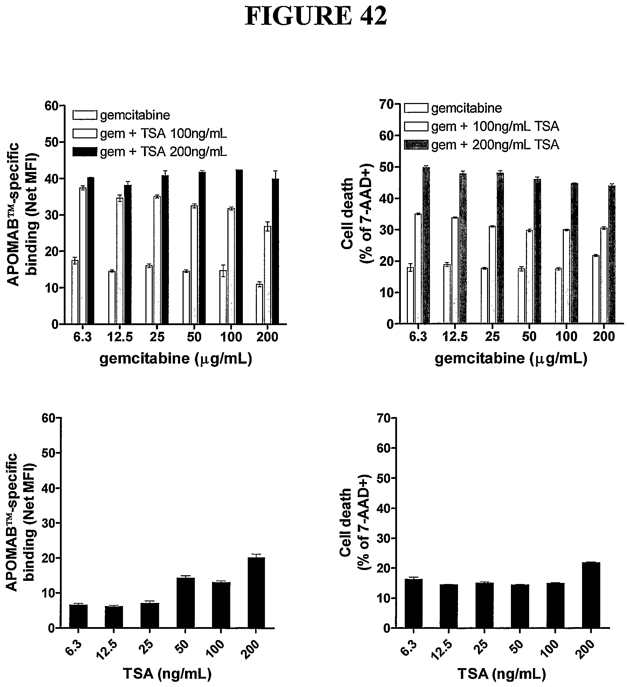

**Figure 42** depicts that gemcitabine and TSA synergise to increase cell death among PANC-1 cells, which subsequently demonstrate augmented Apomab-specific binding. PANC-1 cells were incubated with increasing concentrations of gemcitabine alone (clear bars), increasing concentrations of TSA alone (black bars), increasing concentrations of gemcitabine and 100ng/mL TSA (light grey bars) or increasing concentrations of gemcitabine and 200ng/mL TSA (dark grey bars). After 48h, cells were collected (from media and EDTA detached adherent cells) and subjected to indirect immunofluorescence staining with Apomab or its Sal5 isotype control mAb and assessed for viability with 7-AAD staining. Data are shown as net MFI ± SEM (*n*=3) for Apomab-specific binding (left-hand two panels) or as the percentage of cells staining with 7-AAD (right-hand two panels).

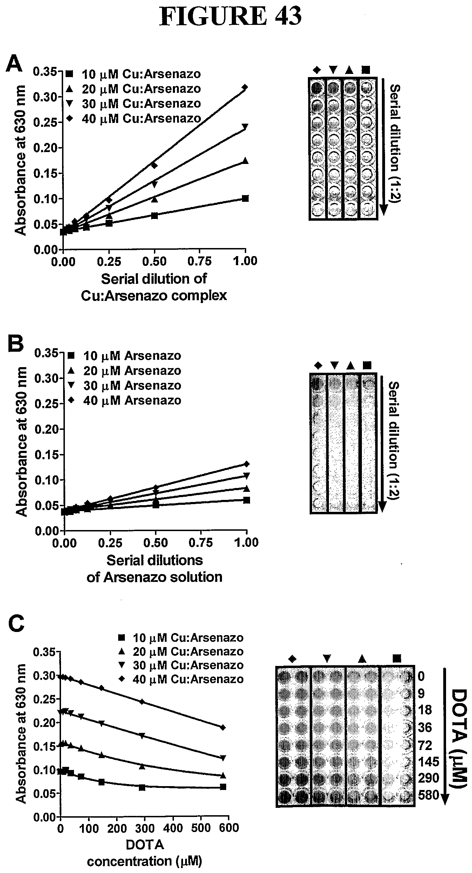

**Figure 43** depicts that modification of Cu:Arsenazo(III) assay. (A) Cu:Arsenazo(III) reagent prepared at different concentrations from stock solution was serially diluted 1:2 using milliQ water in 96-wells titre plate. Absorbance was measured at 630nm and was plotted as a function of the serial dilution performed. (**B**) Arsenazo(III) solutions prepared identically as described in A however without the addition of Cu were serially diluted 1:2 using milliQ water. Absorbance was measured at 630nm and plotted as a function of the dilutions performed. (**C**) Aliquots (10µL) of DOTA standard solutions were added in duplicates to 96-wells plate. Cu:Arsenazo(III) reagent prepared at four different concentration was added to these wells (190µL). Plate was incubated at 37°C for 20 min, absorbance was measured at 630nm and plotted as a function of DOTA concentration. Subsequent Cu:Arsenazo(III) assays were performed as described in this panel.

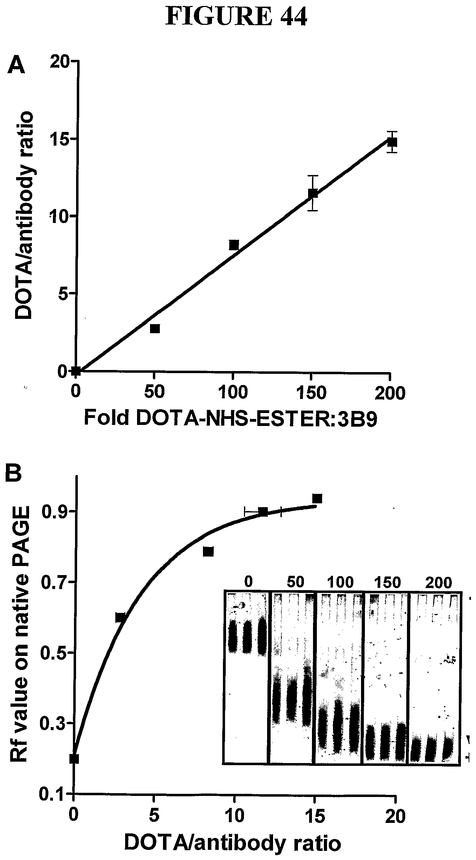

**Figure 44** depicts that conjugation condition affects DOTA/antibody ratio and net charge of the immunoconjugates. 3B9 was conjugated, at three separate occasions, with increasing concentrations of DOTA-NHS-ESTER calculated to be at 0-, 50-, 100-, 150- and 200-fold molar excess to the concentration of antibody. Conjugates were purified as described in Methods section. (**A**) DOTA concentration in the purified conjugates was measured using 10mL aliquots in modified Cu:Arsenzo(III) assay as described in Figure 1C. The protein concentration was measured using the BCA protein assay kit as described in methods. The DOTA/antibody ratio was calculated as the concentration of DOTA (µM) to that of antibody (µM) and the mean ratio ± standard error of the mean (SEM) (*n*=3) was plotted as a function of the concentration of DOTA-NHS-ESTER added during conjugation. (**B**) Samples of 3B9 incubated in the absence or presence of increasing concentrations of DOTA-NHS-ESTER (0-200 fold molar excess) as described above (triplicate reactions) were fractionated in native PAGE. Gel was stained with BBR250 and documented (inset). The (-) and (+) symbols denote the cathode and anode with the migration direction during electrophoresis is indicated by the arrow. The Rf value for the detected bands was calculated using GelPro Analyzer and was plotted as a function of the DOTA/antibody ratio calculated from panel A.

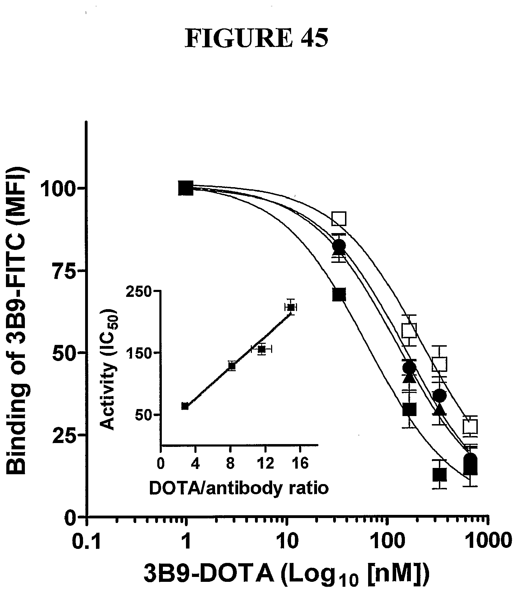

**Figure 45** depicts that increased DOTA/antibody ratio affects antibody avidity. Fixed and permeabilized Jurkat cells were incubated with 33.3nM of 3B9-FITC in the absence or presence of increasing 3B9-DOTA concentrations which were prepared at (□) 50-fold, (▲) 100-fold, (●) 150-fold and (■) 200-fold molar excess of DOTA-NHS-ESTER to antibody. Cells were washed and analyzed by flow cytometry and the mean fluorescent intensity (MFI±SEM, n=3) for 3B9-FITC binding was plotted as a function of the log10 concentration of 3B9-DOTA (nM) added to incubation. Competition binding curve was fitted to the data using GraphPad which found that data did not significantly deviate from the fitted model and reproduced the concentration of 3B9-DOTA required to inhibit half of the binding of 3B9-FITC (IC₅₀, in nM). **Inset:** The IC₅₀ (nM±SEM) of the different conjugates which is indicative of avidity was plotted as a function of the DOTA/antibody ratio. Linear correlation (*r²*=0.99) was fitted by GraphPad and described the relationship between avidity and the extent of conjugation taking place on the antibody.

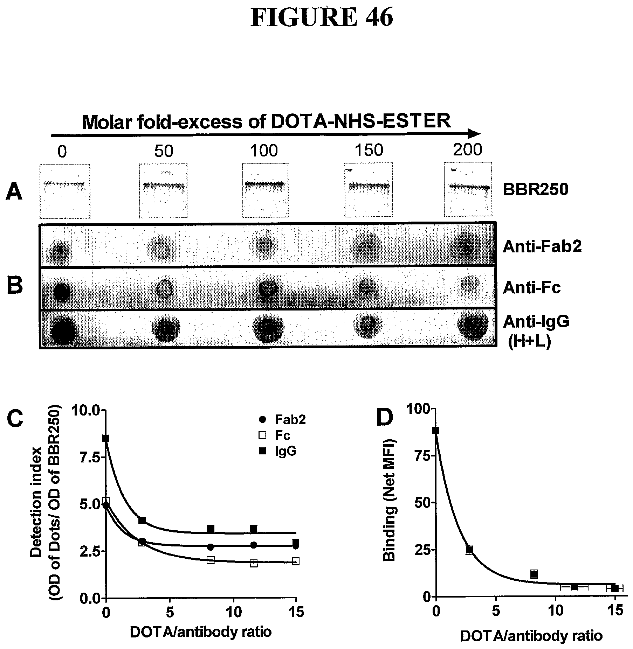

**Figure 46** depicts that conjugation modifies Fc and Fab regions of monoclonal antibody. Samples of 3B9 incubated in the absence or presence of increasing concentration of DOTA-NHS-ESTER (0-200 fold molar excess) were used for (**A**) non-reducing SDS-PAGE and BBR250 staining or (**B**) dot blot for staining with anti-mouse (Fab)₂, anti-mouse Fc or anti-mouse whole IgG. (C) The optical density of dots detected using the anti-mouse antibodies was normalized to the optical density of BBR250 reflecting the amount of IgG present in sample. This ratio was plotted as a function of the DOTA/antibody ratio achieved by the corresponding conjugation condition. A one-phase exponential decay curve was fitted to the data using GraphPad. This assay was performed at three separate occasions and similar data was obtained. (**D**) Fixed and permeabilized Jurkat cells were subjected to indirect immunofluorescent staining using 3B9 or 3B9-DOTA followed by anti-mouse IgG Alexa₄₈₈ conjugated antibody as described in Methods. Cells were analyzed by flow cytometry and data shown is the Net MFI (signal from 3B9 or 3B9-DOTA after subtraction of the signal from Sal5 or corresponding Sal5-DOTA). Error bars are the SEM from triplicate incubations and the drawn curve is a one-phase exponential decay model fitted using GraphPad.

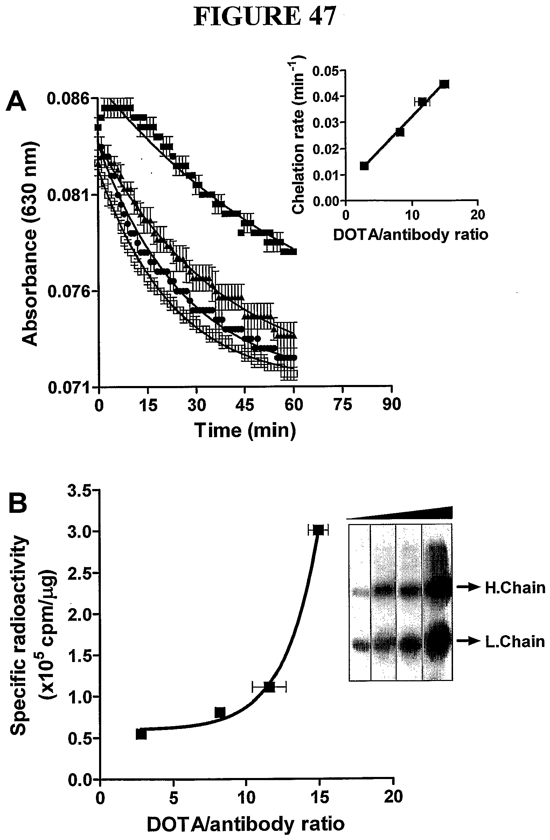

**Figure 47** depicts that DOTA/antibody ratio affects metal chelation rate and radionuclide loading capacity. (**A**) Identical amounts of 3B9-DOTA prepared at increasing concentrations of DOTA-NHS-ESTER were incubated with Terbium:Arsenazo(III) reagent which was assayed kinetically for 1h at 37°C measuring absorbance at 630nm. Absorbance was plotted as a function of time for 3B9-DOTA prepared at (■) 50-fold, (▲) 100-fold, (●) 150-fold or (□) 200-fold molar excess of DOTA-NHS-ESTER. Data presented is the mean ± SEM and was fitted using GraphPad to a one-phase exponential decay model which provided a measurement of chelation rate (min⁻¹) ± SEM. **Inset:** chelation rate was plotted as a function of DOTA/antibody ratio which showed a direct correlation. (**B**) Identical amounts of 3B9-DOTA prepared at increasing concentrations of DOTA-NHS-ESTER were labelled with ¹¹¹In. After purification, the concentrations of antibody and radioactivity in the samples were measured as described in Methods and specific radioactivity expressed as cpm/µg was plotted as a function of DOTA/antibody ratio. **Inset:** Identical amounts of reduced samples of ¹¹¹In-labelled DOTA-3B9 prepared at increasing DOTA-NHS-ESTER concentrations were fractionated by SDS-PAGE. Gel was exposed to x-ray film and documented. The heavy and light chains of the IgG are marked by arrows. Note the increase in the intensity of bands with increasing DOTA concentrations (◢) and the labelling of both heavy and light chains.

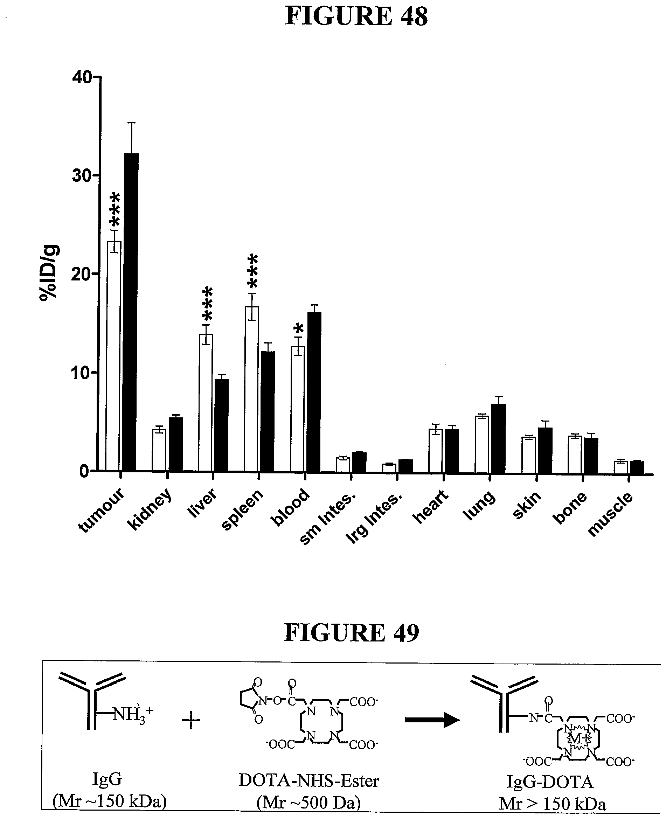

**Figure 48** depicts that modification of monoclonal antibody by conjugation is reflected *in vivo.* C57BL/6 mice bearing EL4 tumors and treated with cytotoxic chemotherapy as described in Methods received an intravenous injection at time 0 containing 100µg of 111In-DOTA-3B9 prepared at 200-fold molar excess of DOTA-NHS-ESTER (clear bars) or 100µg of ¹¹¹In-DOTA-3B9 prepared at 50-fold molar excess of DOTA-NHS-ESTER (filled bars). Mice were euthanized at 48h and blood, tumors and other organs were collected, weighed and counted using gamma counter. Data presented is the accumulation in the organs calculated as the percentage of radioactivity in the organs per mass or organ to the radioactivity of the injected dose at time (%ID/g). Error bars are the SEM from 5 mice at each treatment and asterisks denote statistically different data (* *P*<*0.05,* ** *P<0.001*)*.*
**Figure 49** is a schematic representation of the conjugation of DOTA to the lysine residues of IgG.

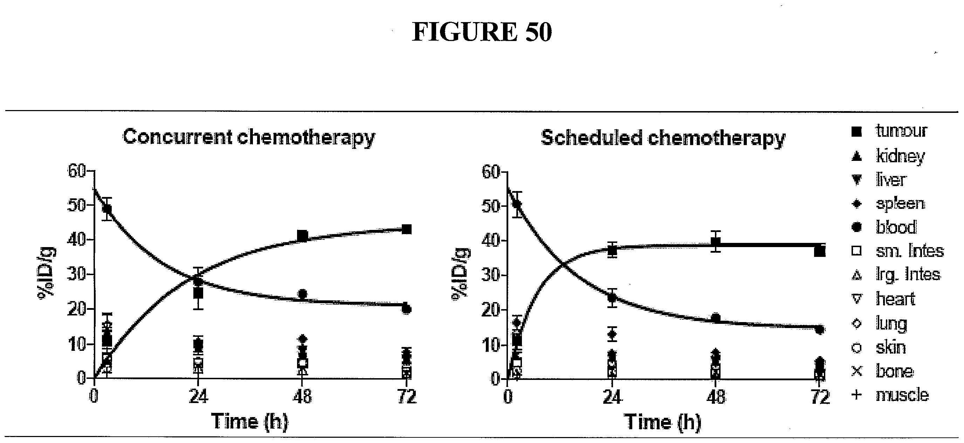

**Figure 50** depicts that time activity curves of ¹¹¹In-DOTA-3B9 injected with concurrent or 24h proceeding chemotherapy. Groups of 5 mice each were injected with 19mg/kg etoposide and 25mg/kg cyclophosphamide i.p at -24h or time 0. All mice were also injected with ¹¹¹In-DOTA-3B9 i.v. in the tail vein at time 0. Mice were sacrificed at 3, 24, 48 and 72h after antibody administration and organ radioactivity was measured and divided on the mass of organ counted which was normalised as the percentage to the injected dose (%ID/g). Data presented is the mean %ID/g ± SEM. Blood clearance was fitted to one-phase exponential decay and tumour accumulation was fitted to one-phase exponential association (*r2* > 0.9).

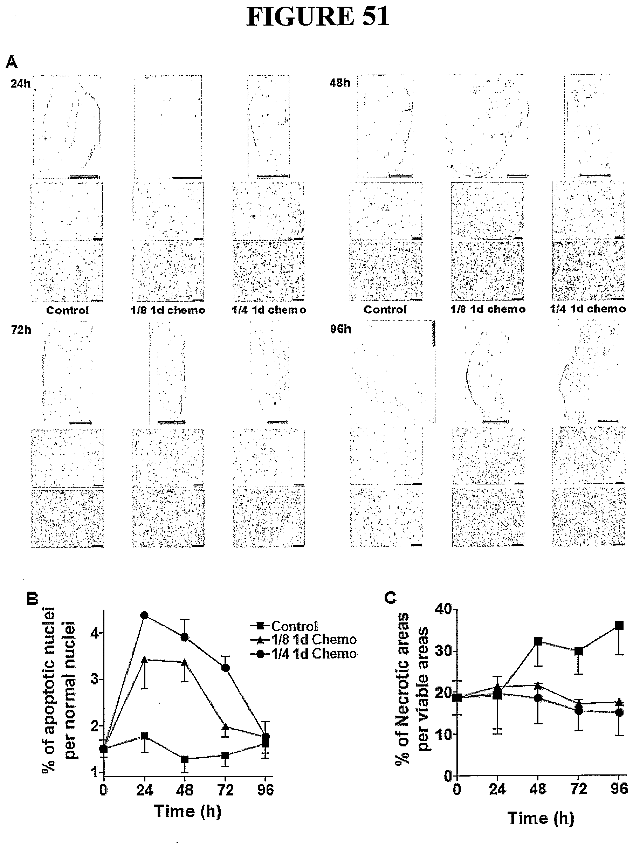

**Figure 51** depicts that cell death in control and CE-treated EL4 tumours. B6 mice bearing EL-4 tumours (125mm³) were left untreated or treated with 1/8 or 1/4 dose chemotherapy at time 0. Mice were killed at specified time points (*n*=3) and tumours were fixed in 10% formalin in PBS. Each tumour was cut symmetrically and each half embedded in paraffin and sectioned. Sections were stained with H&E (data not shown) or with anti-caspase-3 (**A**). The entire sections were scanned using DotSlide acquisition program (Soft Imaging System, Olympus, Tokyo, Japan) on DotSlide BX51 Olympus light microscope (Olympus) at 20X magnification. Scanned sections were visualised using OlyVIA software (Olympus Viewer for Imaging Applications) where images of the entire tumours or 6 random regions at 10X or 20X were obtained for analysis using analySIS® software (Soft Imaging System, Olympus). Phase colour analysis was performed for all images using pixels to define the different phases: viable nuclei were defined as blue-counterstained nuclei, apoptotic nuclei were defined as brown-stained nuclei from DAB deposition and necrotic areas were defined as faint blue areas, which lack appropriate nuclear morphology. Phase analysis produced the percentage of viable, apoptotic and necrotic phases to the total area of the analysed tumour. Scale bars on entire tumour images (first row) represent 2mm of actual length except for tumours treated with 1/4 dose 1d chemotherapy at 72 and 96h where it was 1mm. Scale bars on images from the 10X magnification (second row) and the 20X magnification (third row) were 200µm and 100µm, respectively. Shown are representative images from one half of an entire tumour of a group of three tumours at each time point for each treatment group. Magnified images are 1 of 6 randomly selected images at each magnification from that selected half. (**B**) Apoptotic index was calculated as the percentage of apoptotic cells in the total area to the percentage of viable cells in the total area. Each tumour was analysed first using all magnifications and all randomly selected areas and an average value calculated for that tumour. Data shown are the mean±SEM from 3 mice at each time point for each treatment group calculated from the representative average for each tumour analysed as above. (C) The percentage of necrotic areas to the percentage of viable areas was calculated to represent percentage of necrosis at each time point using the different treatments as described above. Data shown are the mean±SEM from three mice for each treatment group. Similar values were obtained from analysis of H&E sections (data not shown) however, for simplicity only analysis of necrosis from immunohistochemistry (IHC) sections is shown.

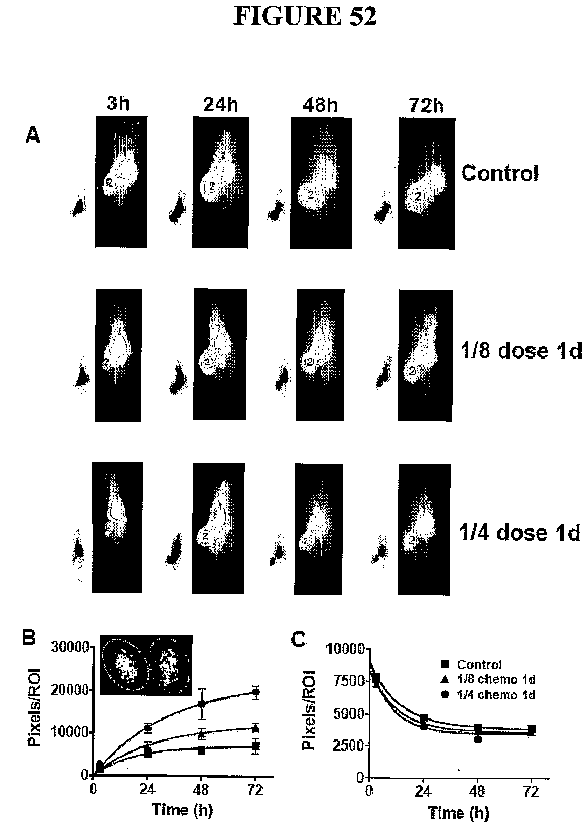

**Figure 52** depicts that radioimaging of B6 mice bearing EL-4 tumour. B6 mice were injected with EL4 cells in the right hindquarter and tumours were grown for 7 days when the mice were left untreated (control) or treated with 1/8 dose 1d or 1/4 dose 1d chemotherapy. Chemotherapy was administered 24h before 111In-Apomab was injected i.v. to all mice at time 0h. Mice were killed by inhalation of isofluorene at 3h, 24h, 48h and 72h (*n*=3) after radioimmunoconjugate injection and imaged using a GE Millennium clinical gamma camera. (**A**) Representative images are shown for the three treatment groups at the 4 time points investigated. Gray scale images are shown to the left of colour map images, which were prepared using ImageJ® image analysis software (v. 1.37, NIH, USA). Regions of interest (ROI) 1 and 2 were drawn based on colour map images and correspond to the contours of mouse body and tumour, respectively. The average of pixels in the ROI and the area of the ROI were produced by the image analysis software and Apomab accumulation was calculated as the average pixels in the ROI standardised to area of the ROI. (**B**) accumulation (pixels/ROI) for region 2, and (**C**) accumulation (pixels/ROI) for region 1. **Inset** (panel B), control tumour at 72h after injection of 111In-labelled Apomab. Tumour was cut in half and the two halves imaged using the gamma imager. Dotted lines represent the outline of each tumour half to show that reactioactivity accumulated in the centre of the tumour only.

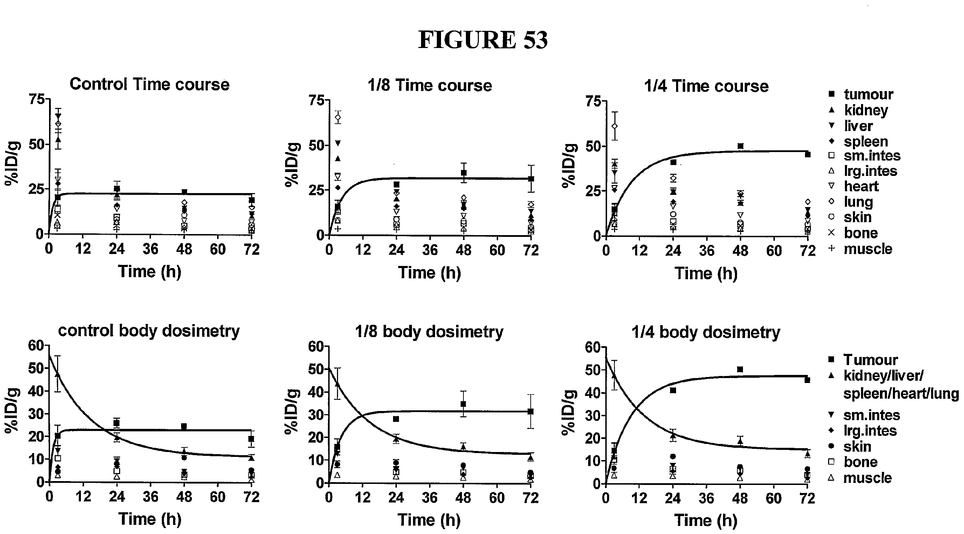

**Figure 53** depicts that biodistribution of Apomab in mice used for radioimmunoimaging. After image acquisition using the GE Millennium gamma camera, mice were dissected to collect organs for radioactivity counting on a gamma counter. Data in the upper row of panels are shown as %ID/g±SEM (*n*=3) at the specified time points for control untreated mice, and mice treated with 1/81d and 1/41d chemotherapy regimens. Tumour accumulation was fitted to a one-phase exponential association model by GraphPad Prism®. Mice were killed by isofluorene inhalation, rather than cardiac bleeding and cervical dislocation, which explains the unusually high blood pool accumulation of Apomab in the kidneys, liver, spleen, heart and lungs. The average accumulation in these organs was calculated and the total is presented in the lower row of panels for each treatment regimen. Again, the accumulation in these organs fitted a one-phase exponential decay model, which was identical to the blood clearance rate reported for Apomab above.

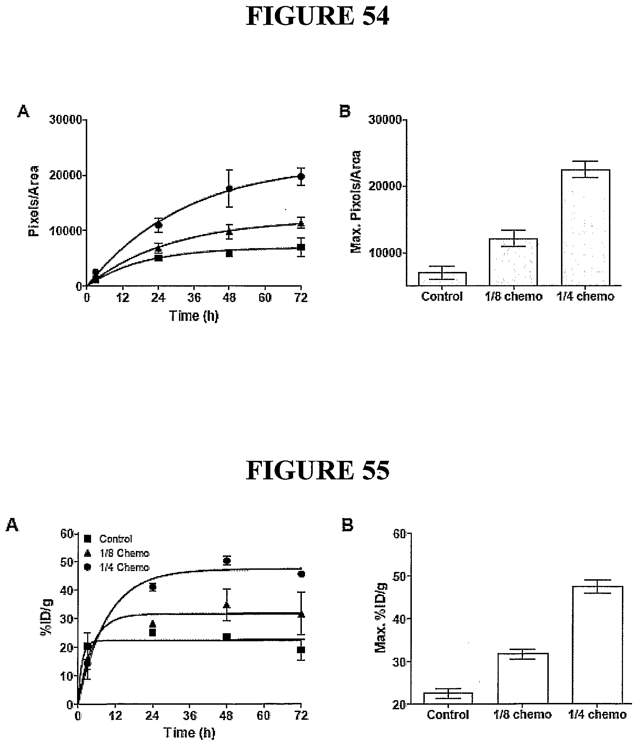

**Figure 54** depicts that Apomab radio-immunoimaging in control and CE-treated EL4-tumour bearing mice. B6 mice were injected with EL4 cells in the right hindquarter and tumours were grown for 7 days when the mice were left untreated (control) or treated with 1/8 dose 1d or 1/4 dose 1d chemotherapy. Chemotherapy was administered 24h before 111In-Apomab was injected i.v. to all mice at time 0h. Mice were killed by inhalation of isofluorene at 3h, 24h, 48h and 72h (*n*=3) after injection of radioimmunoconjugate and imaged using a GE Millennium clinical gamma camera. The average number of pixels in the ROI and the area of the ROI were produced by the image analysis software (see Figure 52) and Apomab accumulation was calculated as the average number of pixels in the ROI standardised to area of the ROI. (**A**) Accumulation (pixels/ROI) for region 2 over 72h after Apomab injection (i.e. 96h after chemotherapy injection). (**B**) Maximum accumulation was obtained from the fitted one-phase exponential association curve (A) using GraphPad Prism™ software and plotted for each treatment as Max. pixels/area.
**Figure 55** depicts that Apomab biodistribution in control and CE-treated EL4-tumour bearing mice. After image acquisition using the GE Millennium gamma camera, mice were dissected to collect organs for radioactivity counting on a gamma counter. (**A**) Tumour accumulation is shown as %ID/g±SEM (*n*=3) at the specified time points for control untreated mice, and mice treated with 1/8 1d and 1/4 1d chemotherapy regimens. Tumour accumulation was fitted to a one-phase exponential association model by GraphPad Prism® to produced the maximum accumulation values shown in (**B**) as Max. %ID/g+SEM.

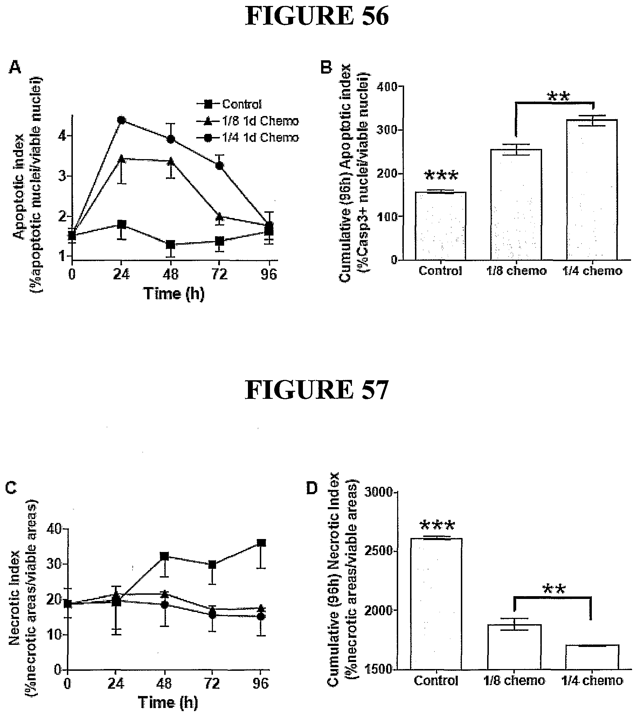

**Figure 56** depicts that apoptosis in control and CE-treated EL4 tumours. B6 mice bearing EL-4 tumours (125mm³) were left untreated or treated with 1/8 or 1/4 dose chemotherapy at time 0. Mice were killed at specified time points (*n*=3) and tumours were fixed in 10% formalin in PBS. Each tumour was cut symmetrically and each half embedded in paraffin and sectioned. Sections were stained with H&E (data not shown) or for caspase-3 activation as a marker of apoptosis (Figure 51). Tissue sections were scanned in their entirety using the DotSlide acquisition program (Soft Imaging System, Olympus, Tokyo, Japan) on DotSlide BX51 Olympus light microscope (Olympus) at 20X magnification. Scanned sections were visualised using OlyVIA software (Olympus Viewer for Imaging Applications) where images of entire tumours or 6 random regions at 10X or 20X were obtained for analysis using analySIS® software (Soft Imaging System, Olympus). Phase colour analysis was performed for all images using pixels to define the different phases: viable nuclei were defined as blue-counterstained nuclei and apoptotic nuclei were defined as brown-stained nuclei from DAB deposition. (A) The Apoptotic Index was calculated as the percentage of apoptotic cells in the total area to the percentage of viable cells in the total area. Each tumour was analysed first using all magnifications and all randomly selected areas and an average value calculated for that tumour. Data shown are the mean±SEM from 3 mice at each time point for each treatment group calculated from the representative average for each tumour analysed as above. (B) A Cumulative (96h) Apoptotic Index (±SEM) was created by using GraphPad Prism® software to measure the area under the curve.
**Figure 57** depicts that necrosis in control and CE-treated EL4 tumours. As in Figure 56, phase colour analysis was performed for all images using pixels to define the viable nuclei as blue-counterstained nuclei and necrotic areas as faint blue areas that lack appropriate nuclear morphology. The Necrotic Index was calculated as the percentage of necrotic areas to the percentage of viable areas at each time point for each treatment. (A) Data shown are the mean±SEM from three mice for each treatment group. Similar values were obtained from analysis of H&E sections (data not shown) however, for simplicity only analysis of necrosis from immunohistochemistry (IHC) sections is shown. (B) A Cumulative (96h) Necrotic Index (±SEM) was created by using GraphPad Prism® software to measure the area under the curve (AUC) (A).

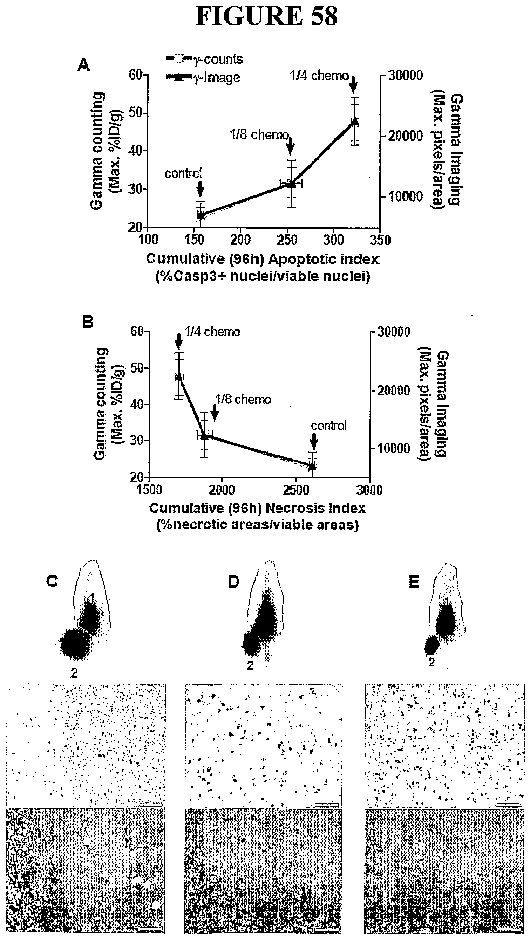

**Figure 58** depicts that correlation of Apomab accumulation to apoptotic cell death. Accumulation measured as the maximum %ID/g from gamma counting (Figure 54) or the maximum pixels/area from gamma radioimaging (Figure 55) were plotted as a function of cumulative apoptosis index (A) calculated from Figure 56 or as a function of cumulative necrosis index (B)calculated from Figure 57. (C-E) are representative gamma camera images of control, 1/8 Id and 1/41d CE-treated mice, respectively. Representative IHC sections are shown below where activated caspase-3 was stained using DAB(brown) and viable nuclei were conterstained (blue). Necrotic areas were characterised as areas without proper nuclear or cellular morphology. The IHC sections were subjected to colour phase analysis using analySIS software(lower panels) to calculate the percentage of apoptotic nuclei (green) to viable nuclei (red)and the percentage or necrotic areas (black) to viable areas (red).

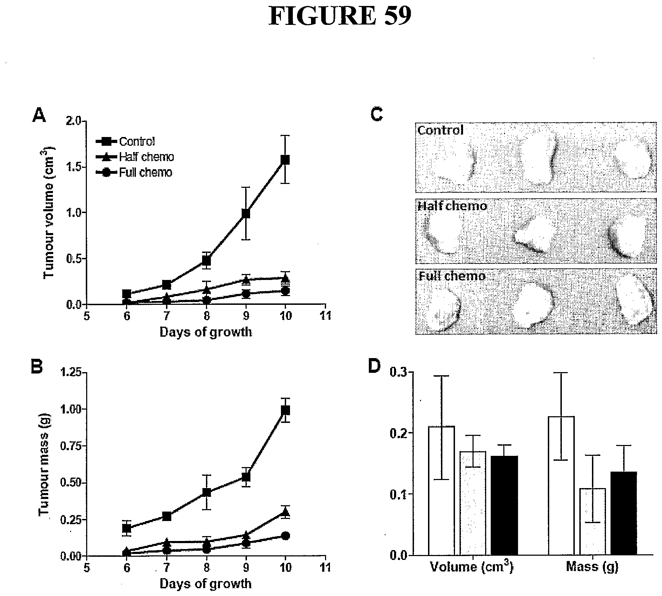

**Figure 59** depicts that tumour mass and volume for control and chemotherapy-treated EL4 tumours. C57 mice were injected with EL4 cells on day 1, 2, 3, 4 or 5 and on day 7 mice were left untreated or treated with half dose (1/8 Id) or full dose (1/4 Id) CE chemotherapy. All mice were killed on day 10 and tumour volume (A) and mass (B) were determined and plotted as a function of days since tumour implantation (days of growth, e.g. for tumours injected on day 1 and killed on day 10 the days of growth are 10 days and ones injected on day 2 and killed on day 10 the days of growth are 9 days). Control mice with tumours grown for 6 days, half chemo-treated mice with tumours grown for 8 days and full chemo-treated mice with tumours grown for 10 days were selected as size-matched tumours for comparison. (C) Photograph of selected tumours (D) volume and mass of selected control mice (clear bars), half chemo-treated mice (grey bars) and full chemo-treated mice (black bars). Data is the mean±SEM (n=3).

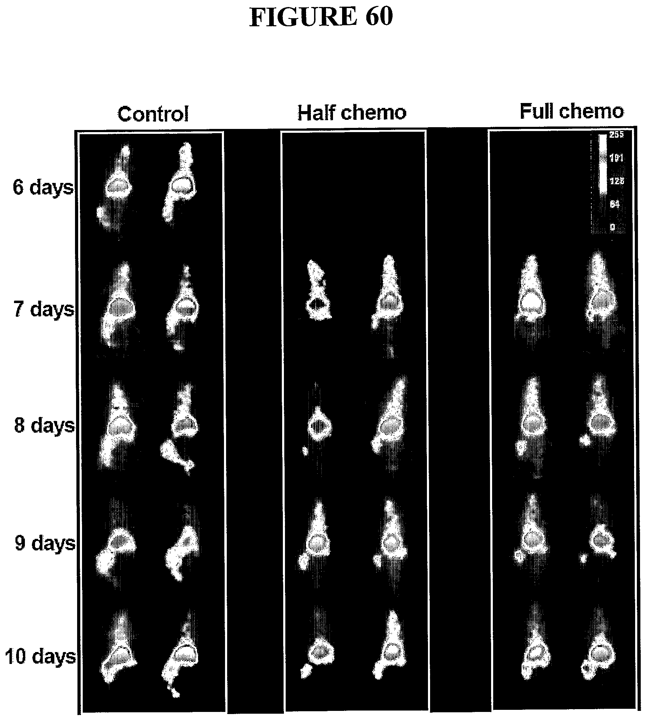

**Figure 60** depicts that radioimaging of 111In-Apomab in EL4 tumour-bearing mice. Gamma camera images are shown for control and chemotherapy treated mice taken 48h after injection of Apomab. Chemotherapy was administered 24h before Apomab injection. Number of days on the left denote the time between EL4 cell injection and time when images were acquired.

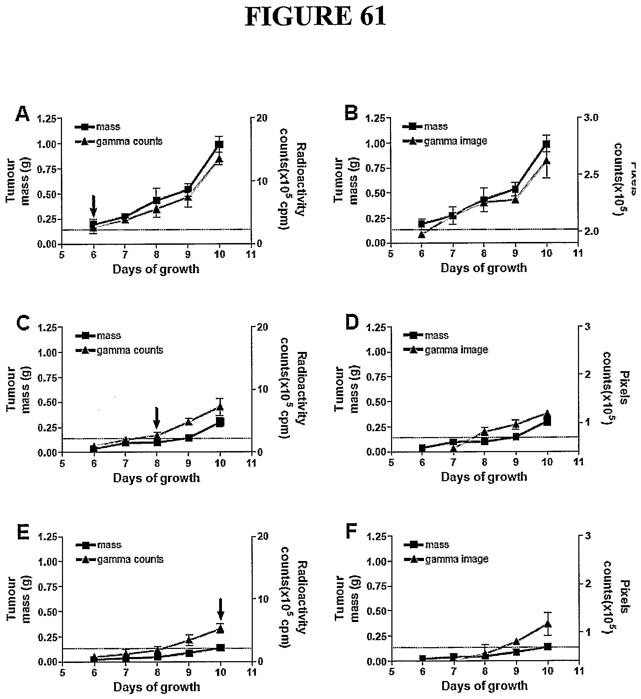

**Figure 61** depicts that relationship between tumour mass and Apomab accumulation in control and chemotherapy treated EL4 tumour-bearing mice. After radioimaging (Figure 60), mice were dissected and tumours were weight and radioactivity was counted using Cobra gamma counter. Panels A, C and E show tumour mass (in black) and radioactivity counts (in red) from control mice and mice treated with half and full dose chemotherapy, respectively. Panels B, D and F show tumour mass and pixels counts obtained for tumour regions in the gamma camera images (Figure 60) for control mice and mice treated with half and full dose chemotherapy, respectively. Dotted lines on Y axes represent the mass of tumours in mice treated with full dose chemotherapy where tumours were grown for a total period of 10 days. Arrows in panels A and C point tumours in control mice and mice treated with half dose chemotherapy where tumours were matched in size to those in mice treated with full dose of chemotherapy.

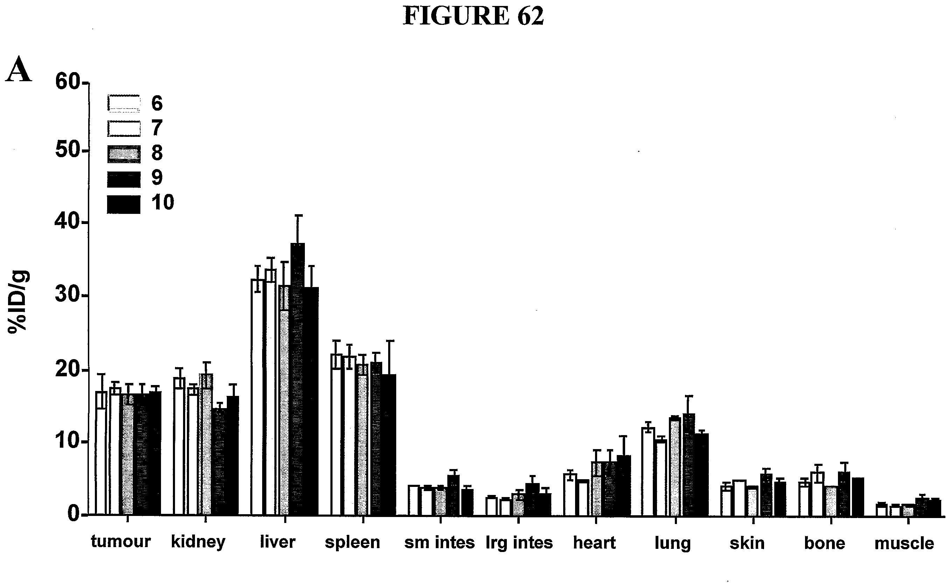

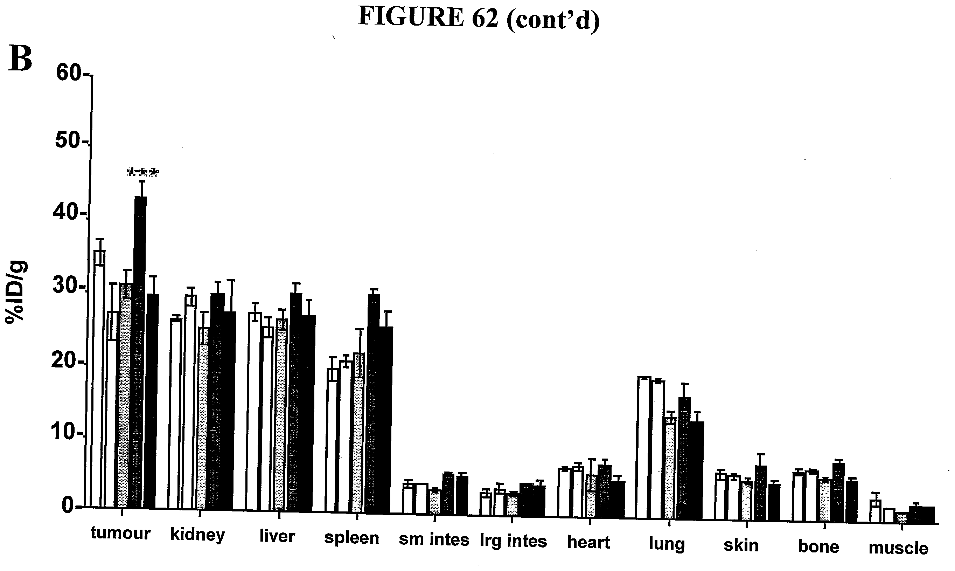

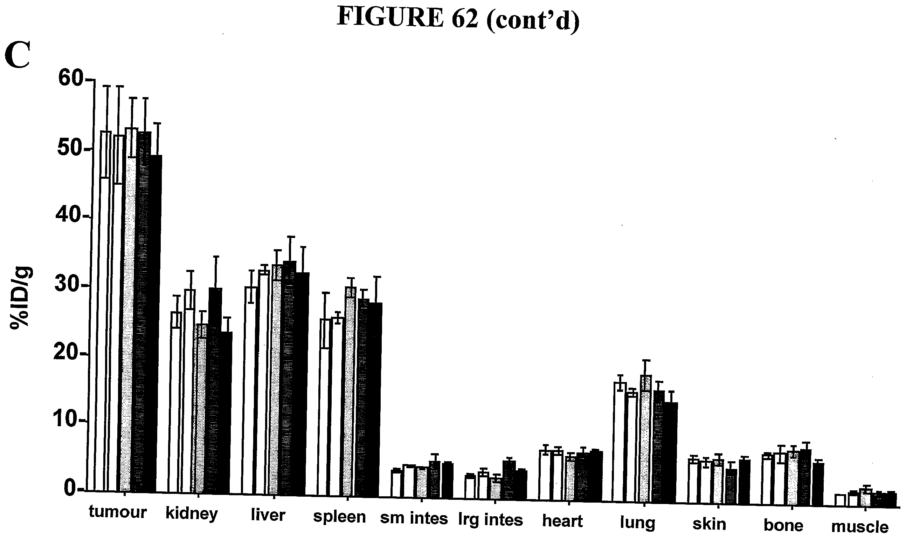

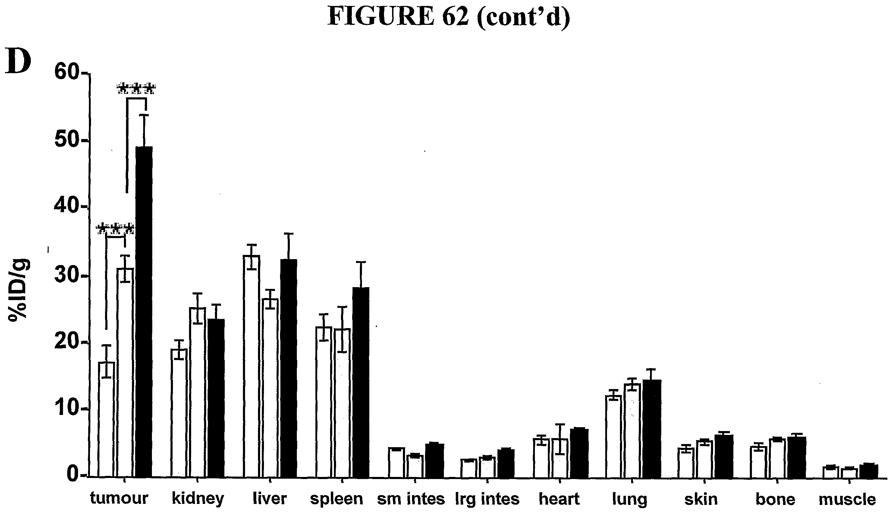

**Figure 62** depicts that biodistribution of Apomab in control and chemotherapy treated EL4 tumour-bearing mice. Mice bearing different size tumours were left untreated (**A**) or treated with half chemo (**B**) or full chemo (**C**) 24h before ¹¹¹In-Apomab injection and killed 48h after radioimmunoconjugate injection. After radioimaging (Figure 60) mice were dissected and biodistribution was measured as previously described. Data shown is the mean %ID/g±SEM (n=3) for tumours grown for different number of days (6-10 days). (**D**) Based on Figure 59, size-matched tumours were selected for comparison of biodistribution in control mice (6 days of growth, clear bars) or mice treated with half (8 days of growth, light grey bars) or full (10 days of growth, dark grey bars) chemotherapy. Asterisks denote significant difference (*P<0.001*) between half chemotherapy treated mice and control mice and significant difference (*P<0.001*) between full and half chemotherapy treated mice.

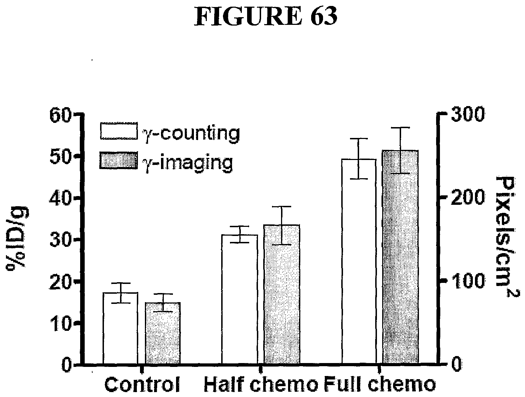

**Figure 63** depicts that correlation of gamma radio-counting and gamma radio-imaging in EL4 tumour-bearing mice. Accumulation of ¹¹¹In-Apomab measured in tumours from selected control mice and mice treated with half and full doses of chemotherapy is shown as %ID/g±SEM (clear bars) calculated from gamma counting and as pixels/cm² (grey bars) as measured from gamma-imaging.

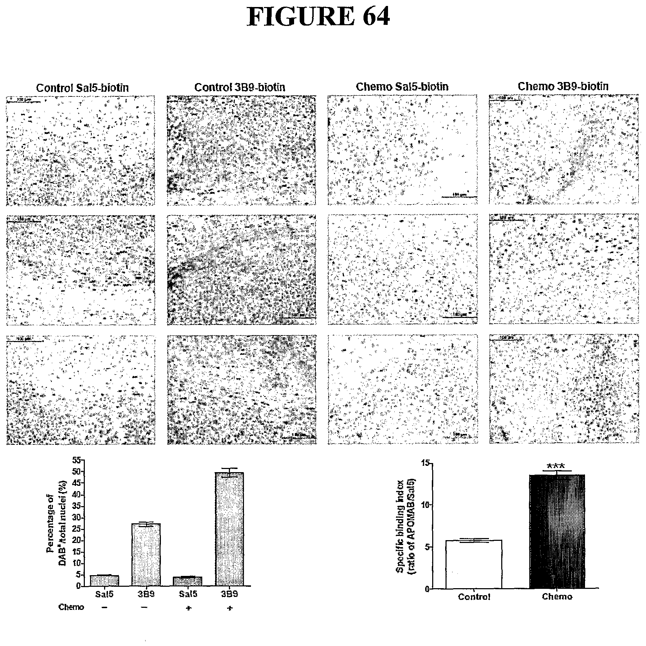

**Figure 64** depicts that localisation of bound Apomab in control and chemotherapy-treated EL4-tumour bearing mice. Initial optimisation showed specific detected of Apomab-biotin in tumours compared to Sal5-biotin which was augmented after chemotherapy. Further work will aim to characterise the type of cells (late necrotic due to apoptotic cell death or primary necrotic cells).

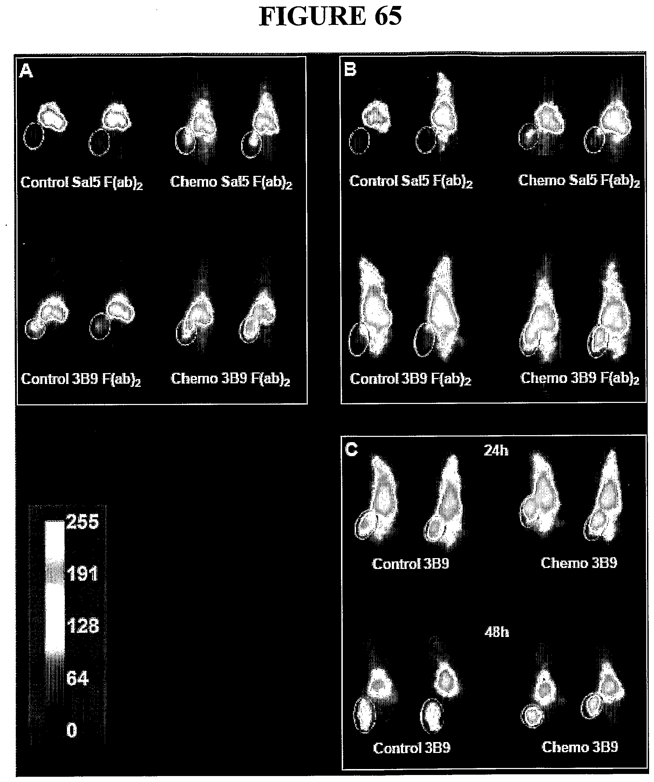

**Figure 65** depicts that gamma camera imaging of EL4-tumour bearing C57 mice using 111In-Apomab. Control and chemotherapy-treated mice bearing size-matched EL4 tumour were injected with F(ab)2 fragments of control antibody (Sal5) or Apomab (3B9) 24h before imaging in the absence **(A)** or presence **(B)** of D-lysine injections. **(C)** Control and chemotherapy-treated EL4-tumour bearing mice were injected with 111In-labelled whole IgG of Apomab and imaged 24 and 48h after injection. Two mice (of 3) for each group is presented and tumour outline was drawn (in red).

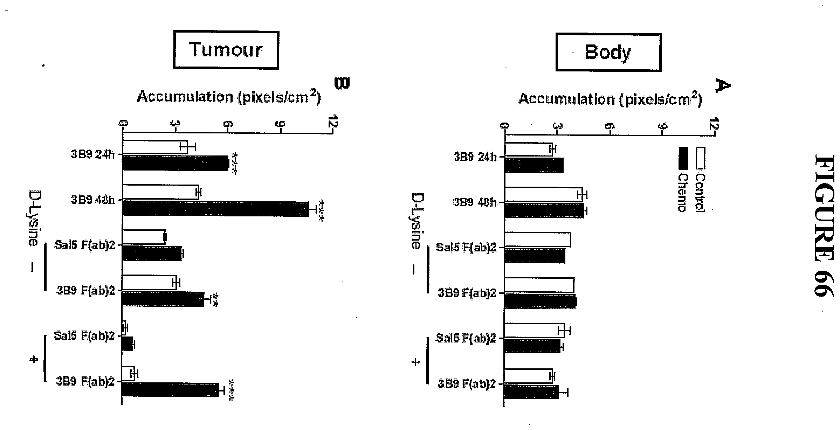

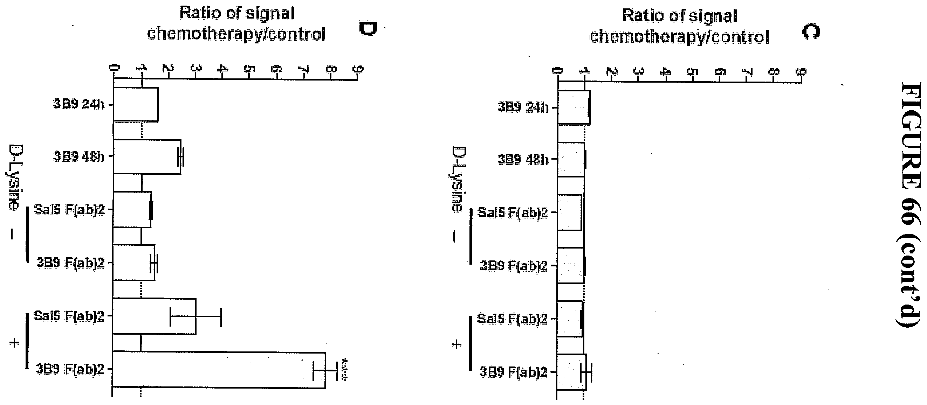

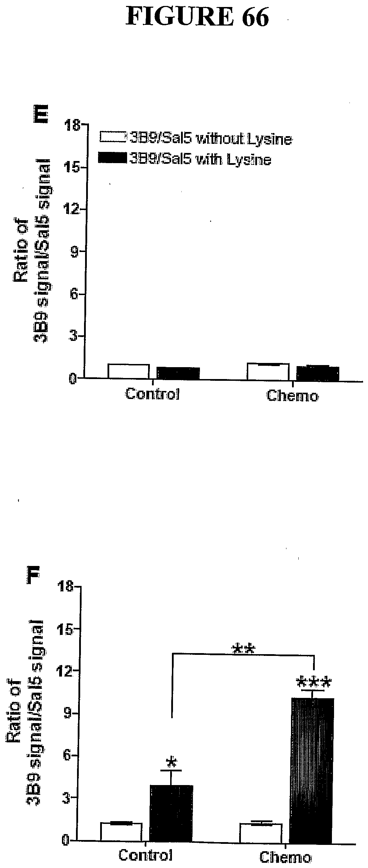

**Figure 66** depicts that analysis of gamma camera images from Apomab radioimmunoimaging. Image analysis software (ImageJ, v1.37, NIH, USA) was used to quantify signals (pixels/cm2) for regions of interests (Body and tumours) in Figure 65. (A and B) show the accumulation of RIC injected in the body and tumours and significant differences between control and chemotherapy mice were only seen using Apomab (IgG or F(ab)2 fragment) (** *P*<*0.01,* *** *P<0.001*)*.* (C and D) is the ratio of signal for each RIC from mice treated with chemotherapy to that from control mice. Chemotherapy showed some increase in accumulation of F(ab)2 fragment of Sal5 in the tumour however only the tumour accumulation of 3B9 F(ab)2 with the lysine injection was significantly different (***, *P<0.001*)*.* (E and F) show the ratio of signal from F(ab)2 fragment of 3B9 to that from Sal5 in control and chemotherapy-treated mice in the absence (clear bars) or presence (filled bars) of D-lysine injections. The specific tumour binding of 3B9 F(ab)2 in control mice and chemotherapy-treated mice was enhanced by D-lysine injection (*, *P*<*0.05,* ***, *P<0.001*)*.*

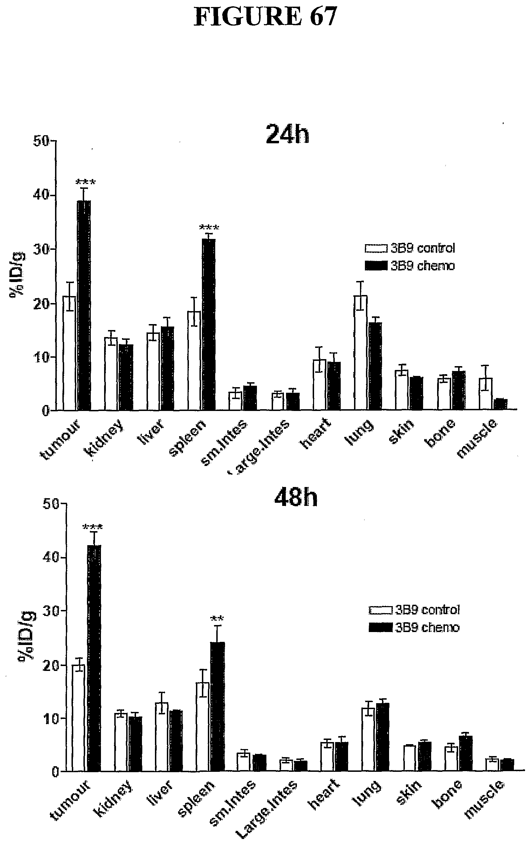

**Figure 67** depicts that biodistribution of IgG form of Apomab in control and chemotherapy-treated EL4-tumour bearing mice. 111In-labelled IgG Apomab was injected 24h after chemotherapy to treated and control mice. Mice were killed for imaging (overdose of anaesthetic) and after imaging organs were dissected, weighed and radioactivity counted using gamma-counter. Radioactivity counts was measured as the percentage to the injected dose and normalised to the weight of the organ counted. Two-way ANOVA was used to measure statistical differences (***, P<0.001, **<0.01).

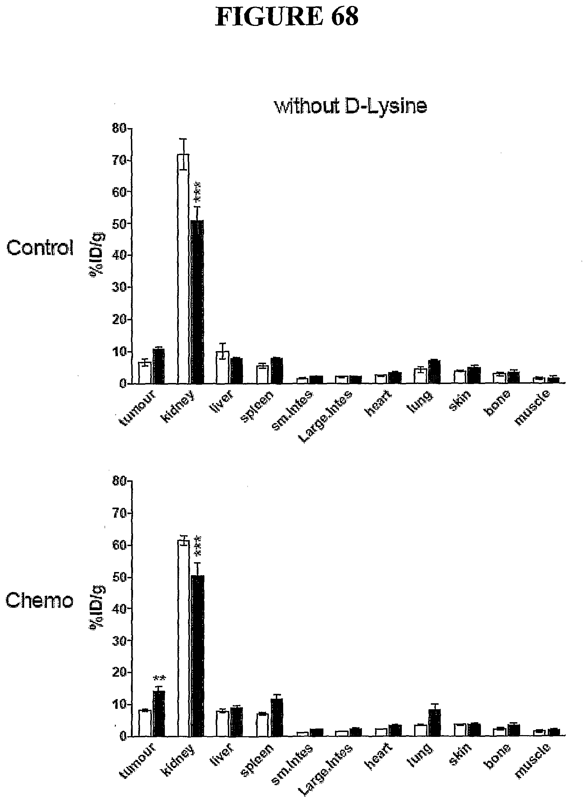

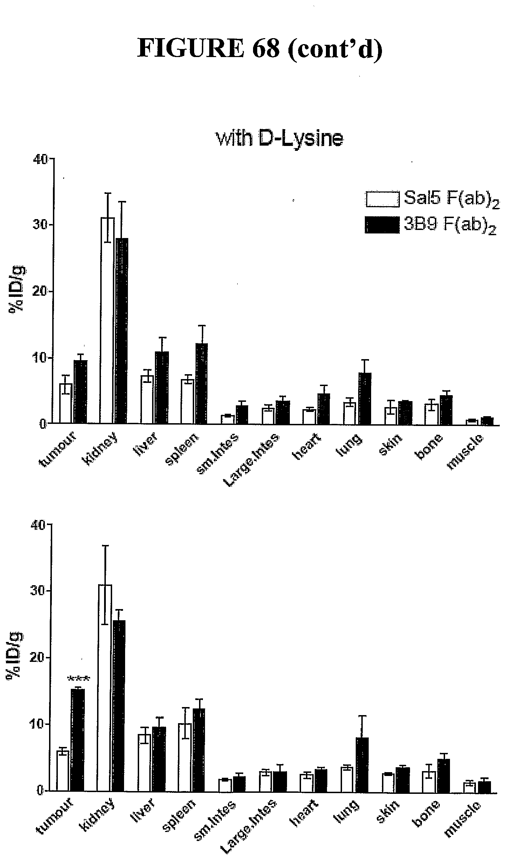

**Figure 68** depicts that biodistribution of F(ab)₂ form of Apomab and control antibody in EL4-tumour bearing mice. Control and chemotherapy-treated mice injected with F(ab)₂ of Sla5 or 3B9 in the absence or presence of D-lysine injections were dissected after radioimaging and accumulation (%ID/g) was calculated as described above.

### DETAILED DESCRIPTION OF THE INVENTION

The present invention is predicated, in part, on the surprising determination that La expression is increased upon transformation of a normal cell to a neoplastic state and, further, that the induction of neoplastic cell death by a DNA damaging agent, such as a cytotoxic agent, results in a still further increase in the expression of La. Accordingly, the detection of increased levels of La expression by neoplastic cells, relative to normal levels, provides a convenient and precise mechanism for qualitatively and/or quantitatively assessing neoplastic cell levels while levels of La in dead neoplastic cells which are higher than that of the live cells is specifically indicative of a DNA damage induced cell death. These findings therefore provide a highly sensitive and accurate means for assessing a neoplastic condition in a mammal, in particular in the context of monitoring the progression of such a condition or assessing the effectiveness of a therapeutic agent or therapeutic regime.

Accordingly, the methods and uses of the present invention are based on detecting a neoplastic cell in a subject, by screening for the level of La protein and/or gene expression in cells in said subject or in a biological sample derived from said subject wherein an increase in the level of cellular La expression relative to normal La expression levels is indicative of a neoplastic cell.

In a related aspect, a dead neoplastic cell in a subject may be detected, wherein death has been induced by a DNA damaging agent, by screening for the level of La protein and/or gene expression by dead cells in said subject or in a biological sample derived from said subject wherein an increase in the level of La expression relative to viable neoplastic cell La expression levels is indicative of cytotoxicity induced neoplastic cell death.

Reference to a cell being "non-viable" should be understood as a reference to the subject cell being dead or dying. In relation to the latter, some killing mechanisms result in a series of stages leading to complete cell death. For example, apoptosis is marked by a series of cellular events which occur subsequently to the onset of the apoptotic signal but prior to final cell death. Reference to "dying" is intended to encompass reference to any cell which has received a signal or other stimulus which has resulted in commitment to the cell death events. Without limiting the present invention to any one theory or mode of action, neoplastic cells are generally characterised by both unlimited replicative potential and evasion of apoptosis that would otherwise be induced by DNA damage and failure of checkpoint controls. Nevertheless, even in the absence of a therapeutic treatment regime, a tumour mass will usually be characterised by a percentage of dead or dying cells which may result, for example, from the known heterogeneity of tumour blood supply. More commonly, however, neoplastic cell death is induced via exogenous means such as therapeutic radiotherapy or chemotherapy. In terms of monitoring the progress of a patient who is suffering from a neoplastic condition, distinguishing between neoplastic cell death induced by a cytotoxic treatment regime and other forms of unrelated cell death is crucial to optimising both patient care and the treatment provided.

Without limiting the present invention to any one theory or mode of action, the mechanisms by which neoplastic treatment regimes achieve neoplastic cell death are variable and depend on the specific nature of the treatment regime which has been selected for use. Although DNA damage would normally induce apoptosis of the affected cell, an intrinsic feature of malignant cells is the disabling of apoptosis pathways. Therefore, it would be expected that malignant cells would be resistant to the pro-apoptotic effects of DNA damaging agents. Nonetheless, cell death often still occurs in response to treatment with ionising radiation and/or cytotoxic drugs because alternative mechanisms of cell growth inhibition or death are activated such as necrosis, mitotic catastrophe, autophagy and premature senescence. Hence, the induction of neoplastic cell apoptosis is not the only immediate outcome of these widely used treatment regimes but may manifest after a period of days has elapsed. Recent data indicate that if the malignant cell does not die an early death from apoptosis then there will be time for DNA repair. However, if there is lack of repair or misrepair of DNA that is sensed during mitosis then post-mitotic cell death will occur (Brown and Attardi, 2004, Nature Reviews Cancer 5:231-237). Nevertheless, the induction of neoplastic cell apoptosis is still the most commonly observed outcome in the context of any of the more widely used treatment regimes.

Unfortunately, due to the relatively non-specific effects of neoplastic treatment regimens (in particular systemically administered chemotherapy) all rapidly dividing cellular populations (normal and malignant) are affected. The patient may therefore experience severe treatment related toxicities. Accordingly, the design of means for both more accurately and rapidly monitoring the effectiveness of treatment regimes is of significant value in that it provides a more effective means of assessing and/or tailoring one of the most commonly administered treatment regimes. In the context of the present invention, it should therefore be understood that a "non-viable" neoplastic cell is one which has been rendered non-viable due to the actions of a DNA damaging agent, as opposed to other forms of cell death induction. Specifically, said non-viable neoplastic cell is a dead neoplastic cell.

Accordingly, for detecting a dead neoplastic cell in a subject, which cell death was induced by a DNA damaging agent, the methods and uses of the present invention are based on screening for the level of La protein and/or gene expression by dead cells in said subject or in a biological sample derived from said subject wherein an increase in the level of cellular La expression relative to viable neoplastic cell La expression levels is indicative of DNA damage induced neoplastic cell death.

Reference to a "DNA damaging agent" should be understood as a reference to any proteinaceous or non-proteinaceous agent which acts to damage cellular DNA. The agent may be a cytotoxic agent or a non-cytotoxic agent. Without limiting the present invention to any one theory or mode of action, many such agents function via the induction of apoptotic processes. However, this is not the only mechanism by which such agents function and it is conceivable that the subject DNA damage may be induced by some other mechanism. Examples of DNA damaging agents include, but are not limited to, the traditionally understood chemotherapy agents such as Actinomycin D, Arsenic Trioxide, Asparaginase, Bleomycin, Busulfan, Carboplatin, Carmustine, Chlorambucil, Cisplatin, Corticosteroids, Cyclophosphamide, Daunorubicin, Docetaxel, Doxorubicin, Epirubicin, Etoposide, Fludarabine, Fluorouracil, Gemcitabine, Hydroxyurea, Idarubicin, Ifosfamide, Irinotecan, Lomustine, Melphalan, Mercaptopurine, Methotrexate, Mitomycin, Mitoxantrone, Oxaliplatin, Paclitaxel, Procarbizine, Raltitrexed, Streptozocin, Thioguanine, Thiotepa, Topotecan, Treosulfan, Vinblastine, Vincristine, Vindesine, Vinorelbine. Other means of inducing DNA damage include ionising radiation as well as the use of molecules such as inhibitors of poly-(ADP ribosyl) transferase (PARP) or agents which induce DNA damage as part of a synergistic process with another agent, for example e.g. Gemcitabine or Irinotecan and CHK 1/2 inhibitors such as CBP-501 or AZD7762. In addition, new classes of antineoplastic agents such as histone deacetylase inhibitors (HDACi) e.g. vorinostat, BH3 mimetics e.g. ABT737, and Tumor Necrosis Factor-Related Apoptotis-Inducing Ligand (TRAIL), are pro-apoptotic particularly when administered in conjunction with conventional cytotoxic agents. Hence, singly or in combination, these pro-apoptotic compounds will likely increase the amount of La-specific signal detectable in the malignant neoplasm.

Reference to a "neoplasm" should be understood as a reference to an encapsulated or unencapsulated growth of neoplastic cells. Reference to a "neoplastic cell" should be understood as a reference to a cell exhibiting abnormal growth. The term "growth" should be understood in its broadest sense and includes reference to enlargement of neoplastic cell size as well as proliferation.

The phrase "abnormal growth" in this context is intended as a reference to cell growth which, relative to normal cell growth, exhibits one or more of an increase in individual cell size and nuclear/cytoplasmic ratio, an increase in the rate of cell division, an increase in the number of cell divisions, a decrease in the length of the period of cell division, an increase in the frequency of periods of cell division or uncontrolled proliferation and evasion of apoptosis. Without limiting the present invention in any way, the common medical meaning of the term "neoplasia" refers to "new cell growth" that results as a loss of responsiveness to normal growth controls, eg. to neoplastic cell growth. Neoplasias include "tumours" which may be either benign, pre-malignant or malignant. The term "neoplasm" should be understood as a reference to a lesion, tumour or other encapsulated or unencapsulated mass or other form of growth which comprises neoplastic cells.

The term "neoplasm", in the context of the present invention should be understood to include reference to all types of cancerous growths or oncogenic processes, metastatic tissues or malignantly transformed cells, tissues or organs irrespective of histopathologic type or state of invasiveness.

The term "carcinoma" is recognised by those skilled in the art and refers to malignancies of epithelial or endocrine tissues including respiratory system carcinomas, gastrointestinal system carcinomas, genitourinary system carcinomas, testicular carcinomas, breast carcinomas, prostate carcinomas, endocrine system carcinomas and melanomas. Exemplary carcinomas include those forming from tissue of the breast. The term also includes carcinosarcomas, e.g. which include malignant tumours composed of carcinomatous and sarcomatous tissues. An "adenocarcinoma" refers to a carcinoma derived from glandular tissue or in which the tumour cells form recognisable glandular structures.

The neoplastic cells comprising the neoplasm may be any cell type, derived from any tissue, such as an epithelial or non-epithelial cell. Reference to the terms "malignant neoplasm" and "cancer" and "carcinoma" herein should be understood as interchangeable.

The term "neoplasm" should be understood as a reference to a lesion, tumour or other encapsulated or unencapsulated mass or other form of growth which comprises neoplastic cells. The neoplastic cells comprising the neoplasm may be any cell type, derived from any tissue, such as an epithelial or non-epithelial cell. Examples of neoplasms and neoplastic cells encompassed by the present invention include, but are not limited to central nervous system tumours, retinoblastoma, neuroblastoma paediatric tumours, head and neck cancers (e.g. squamous cell cancers), breast and prostate cancers, lung cancer (both small and non-small cell lung cancer), kidney cancers (e.g. renal cell adenocarcinoma), oesophagogastric cancers, hepatocellular carcinoma, pancreaticobiliary neoplasias (e.g. adenocarcinomas and islet cell tumours), colorectal cancer, cervical and anal cancers, uterine and other reproductive tract cancers, urinary tract cancers (e.g. of ureter and bladder), germ cell tumours (e.g. testicular germ cell tumours or ovarian germ cell tumours), ovarian cancer (e.g. ovarian epithelial cancers), carcinomas of unknown primary, human immunodeficiency associated malignancies (e.g. Kaposi's sarcoma), lymphomas, leukemias, malignant melanomas, sarcomas, endocrine tumours (e.g. of thyroid gland), mesothelioma and other pleural or peritoneal tumours, neuroendocrine tumours and carcinoid tumours.

Preferably, said neoplastic cell is a malignant neoplastic cell.

Accordingly, a preferred embodiment of the present invention is directed to detecting a malignant neoplastic cell in a subject by screening for the level of La protein and/or gene expression by a cellular population in said subject or in a biological sample derived from said subject wherein an increase in the level of cellular La expression relative to normal La expression levels is indicative of a malignant neoplastic cell.

For detecting a dead malignant neoplastic cell in a subject, which death has been induced by a DNA damaging agent, the methods and uses of the present invention are based on screening for the level of La protein and/or gene expression by dead cells in said subject or in a biological sample derived from said subject wherein an increase in the level of La expression relative to viable malignant neoplastic cell La expression levels is indicative of DNA damage induced neoplastic cell death.

In one embodiment, said DNA damaging agent is a cytotoxic agent.

Reference herein to "La" includes reference to all forms of La or their homologues, or orthologs or derivatives. Reference to "La" should be understood to include reference to any isoforms which arise from alternative splicing of La mRNA or mutants or polymorphic variants of La. It should also be understood that "La" is a molecule which is alternatively term SS-B.

Reference herein to a "subject" should be understood to encompass humans, primates, livestock animals (e.g. sheep, pigs, cattle, horses, donkeys), laboratory rest animals (e.g. mice, rabbits, rats, guinea pigs), companion animals (e.g. dogs, cats) and captive wild animals (e.g. foxes, kangaroos, deer). Preferably, the subject is a mammal, more preferably the mammal is a human.

Reference to a "biological sample" should be understood as a reference to any sample of biological material derived from an animal such as, but not limited to, cellular material, biofluids (eg. blood, urine, sputum), cerebrospinal fluid, faeces, tissue biopsy specimens, surgical specimens or fluid which has been introduced into the body of an animal and subsequently removed (such as, for example, the solution retrieved from lung lavage or an enema wash). The biological sample which is tested according to the method of the present invention may be tested directly or may require some form of treatment prior to testing. For example, a biopsy or surgical sample may require homogenisation prior to testing or it may require sectioning for in situ testing. Alternatively, the dead cell sample may require permeabilisation prior to testing. Further, to the extent that the biological sample is not in liquid form, (if such form is required for testing) it may require the addition of a reagent, such as a buffer, to mobilise the sample.

To the extent that the target molecule is present in a biological sample, the biological sample may be directly tested or else all or some of the nucleic acid material present in the biological sample may be isolated prior to testing. In yet another example, the sample may be partially purified or otherwise enriched prior to analysis. For example, to the extent that a biological sample comprises a very diverse cell population, it may be desirable to select out a subpopulation of particular interest, such as enriching for dead cells or enriching for the cell population of which the neoplastic cell forms part. It is within the scope of the present invention for the target cell population or molecules derived therefrom to be pretreated prior to testing, for example, inactivation of live virus or being run on a gel. It should also be understood that the biological sample may be freshly harvested or it may have been stored (for example by freezing) prior to testing or otherwise treated prior to testing (such as by undergoing culturing).

The choice of what type of sample is most suitable for testing in accordance with the method disclosed herein will be dependent on the nature of the situation, such as the nature of the condition being monitored. Preferably, said sample is of blood, urine, cerebrospinal fluid, pleural or peritoneal effusions and ascites, washings and brushings form oropharynx, lung, biliary tree, colon or bladder, biliary, pancreatic and mammary aspirates, and biopsies and surgical resections.

The present invention is predicated on the unexpected finding that neoplastic cells exhibit upregulated levels of La expression and, still further, that DNA damage induced neoplastic cell death is identifiable by virtue of a further increase in the expression levels of La. Accordingly, this finding now provides a means of both diagnosing neoplasias and thereafter monitoring the responsiveness of a neoplastic condition to a DNA damage-based treatment regime. This provides a highly sensitive and rapid means for facilitating the optimisation of a treatment regime. In this regard, the person of skill in the art will understand that one may screen for changes to the levels of La at either the protein or the encoding nucleic acid molecule level. To the extent that it is not always specified, reference herein to screening for the level of "La" should be understood to include reference to screening for either the relevant protein or its encoding primary RNA transcript or mRNA.

As detailed hereinbefore, the present invention is directed to the correlation of the level of La relative to control levels of this molecule. "Control" levels may be either "normal" levels (eg. in the context of neoplastic cell diagnosis) or the levels obtained from the same patient but at a previous point in time (eg. in the context of monitoring the effectiveness of a cytotoxic treatment regime. The "normal" level is the level of La protein or encoding nucleic acid molecule in a biological sample corresponding to the sample being analysed of any individual who has not developed a neoplastic condition. This result therefore provides the background levels, if any, of La which are not due to neoplastic cell transformation but merely correspond to normal intracellular levels. Without limiting the present invention to any one theory or mode of action, La is an abundant, ubiquitous nuclear phosphoprotein that has high binding affinity for the 3' oligo-U motif of all polymerase (pol) III-catalysed transcripts together with certain small RNAs synthesized by other RNA polymerases. La contains two RNA-binding domains and is almost exclusively located in the nucleoplasm because of the nuclear localisation signal (NLS) located at its carboxy-terminus (J Maraia. J Cell Biol 153, F13-18, 2001; Wolin SL and Tommy Cedervall T. Annu Rev Biochem 71, 375-403, 2002). The most conserved region of the La protein is the La motif, which is a domain found in several other RNA-binding proteins. A high-resolution X-ray crystallographic structure shows that La motif adopts a winged helix-turn-helix architecture associated with a highly conserved patch of mainly aromatic surface residues. Mutagenesis experiments demonstrate that this patch partly determines the binding specificity of the La protein for RNAs ending in 3' hydroxyl group, which is one of its defining characteristics (Dong G et al. EMBO J 23, 1000-1007, 2004). Major functions of La include stabilizing newly synthesized small RNAs to protect them from exonuclease digestion and acting as a molecular chaperone for small RNA biogenesis. Thus La facilitates pre-tRNA maturation and assembly of small RNAs into functional ribonucleoproteins (RNPs) and may also enable retention of certain nascent small RNAs in the nucleus. Finally, a still controversial hypothesis posits that La binding of specific mRNAs facilitates initiation of translation (Wolin SL and Tommy Cedervall T. Annu Rev Biochem 71, 375-403, 2002).

The method of the present invention should be understood to encompass all suitable forms of analysis such as the analysis of test results relative to a standard result which reflects individual or collective results obtained from healthy individuals. In a preferred embodiment, said normal reference level is the level determined from one or more subjects of a relevant cohort to that of the subject being screened by the method of the invention. By "relevant cohort" is meant a cohort characterised by one or more features which are also characteristic of the subject who is the subject of screening. These features include, but are not limited to, age, gender, ethnicity, smoker/non-smoker status or other health status parameter. As detailed hereinbefore, said test result may also be analysed relative to a control level which corresponds to an earlier La level result determined from the body fluid of said subject. This is a form of relative analysis (which may nevertheless also be assessed relative to "normal" levels) which provides information in relation to the rate and extent of neoplastic cell death over a period of time, such as during the course of a treatment regime.

Said "normal level" or "control level" may be a discrete level or a range of levels. Individuals exhibiting La levels higher than the normal range are generally regarded as having undergone neoplastic transformation. Those patients exhibiting non-viable cells expressing a level of La higher than that of their viable neoplastic cells are regarded as having undergoing DNA damaging agent induced neoplastic cell death. This corresponds to an encouraging prognosis since it may indicate treatment responsiveness and, potentially, the move to a remissive state. In this regard, it should be understood that La levels may be assessed or monitored by either quantitative or qualitative readouts.

Accordingly, the present invention provides means for assessing both the existence and extent of a population of viable neoplastic cells and/or neoplastic cells which have responded to a treatment regime. As detailed hereinbefore, this has extremely important implications in terms of assessing the effectiveness of a therapeutic treatment regime. To this end, although a one-off analysis of neoplastic dead cell levels in a biological sample provides information in relation to whether treatment induced neoplastic cell death has occurred, the present invention is also useful, and particularly valuable, as an ongoing monitor. This can be essential in the context of identifying and monitoring a therapeutic treatment regime where an initial event of neoplastic cell responsiveness to a chemotherapy drug which is being utilised ultimately shifts to neoplastic cell resistance to the chemotherapy drug which is being utilised. The results observed utilising the screening regime herein described may correspond to screening for the existence of La levels as a one-off test, thereby providing information in relation to relative proportions of viable neoplastic cells versus cells which have undergone DNA damaging agent induced cell death. Alternatively, where a patient is subject to ongoing monitoring and where each successive test result is related to previous results, one may observe a series of increases and decreases in La expression which map out the on going actions and effectiveness of the treatment regime which has been selected for use. Accordingly, increased dead cell La levels relative to viable neoplastic cell levels is indicative the effectiveness of a treatment regime. Increased levels of dead cell La expression relative to a previously analysed sample from that patient may indicate on going effectiveness of the treatment regime while a loss of effectiveness of the treatment regime would be characterised by a loss of non-viable cells exhibiting La expression levels which are higher than that of viable neoplastic cells.

Alternatively, since overexpression of La in malignant neoplastic cells appears to be closely linked to the increased protein synthesis characteristic of malignant cells, successful treatment with antineoplastic agents that inhibit cellular protein synthesis such as the class of mammalian Target Of Rapamycin (mTOR) inhibitors, e.g. temsirolimus or everolimus, may result in the diminution of the La-specific signal in malignant neoplastic cells compared with normal cells.

Accordingly, the methods and uses of the present invention for assessing and/or monitoring a neoplastic condition in a subject are based on screening for the level of La protein and/or gene expression by viable and/or dead cells in said subject or in a biological sample derived from said subject wherein an increase in the level of La in viable cells relative to normal levels is indicative of a neoplastic cell and an increase in the level of La in dead cells relative to viable neoplastic cell levels is indicative of the presence of DNA damage-induced neoplastic cell death.

More preferably, said neoplastic condition is characterised by central nervous system tumours, retinoblastoma, neuroblastoma and other paediatric tumours, head and neck cancers (e.g. squamous cell cancers), breast and prostate cancers, lung cancer (both small and non-small cell lung cancer), kidney cancers (e.g. renal cell adenocarcinoma), oesophagogastric cancers, hepatocellular carcinoma, pancreaticobiliary neoplasias (e.g. adenocarcinomas and islet cell tumours), colorectal cancer, cervical and anal cancers, uterine and other reproductive tract cancers, urinary tract cancers (e.g. of ureter and bladder), germ cell tumours (e.g. testicular germ cell tumours or ovarian germ cell tumours), ovarian cancer (e.g. ovarian epithelial cancers), carcinomas of unknown primary,
human immunodeficiency associated malignancies (e.g. Kaposi's sarcoma), lymphomas, leukemias, malignant melanomas, sarcomas, endocrine tumours (e.g. of thyroid gland), mesothelioma and other pleural or peritoneal tumours, neuroendocrine tumours and carcinoid tumours.

For assessing and/or monitoring the effectiveness of a neoplastic therapeutic treatment regime in a subject, the methods and uses of the present inventions are based on screening for the level of La protein and/or gene expression by viable and/or dead cells in said subject or in a biological sample derived from said subject wherein an increase in the level of La in viable cells relative to normal levels is indicative of a neoplastic cell and an increase in the level of La in dead cells relative to viable neoplastic cell levels is indicative of the presence of DNA damage induced neoplastic cell death.

The DNA damaging agent may be a cytotoxic agent.

As detailed hereinbefore, one may screen for La at either the protein or mRNA level. To the extent that it is not otherwise specified, reference herein to screening for "La" should be understood to include reference to screening for either the La protein or its encoding primary RNA transcript or mRNA.

Means of screening for changes in La levels in a subject, or biological sample derived therefrom, can be achieved by any suitable method, which would be well known to the person of skill in the art. Briefly, one may seek to detect La protein in a biological sample. La interacting molecules may be used to identify La protein directly. Examples of such molecules include, but are not limited to, antibodies or fragments thereof, affibodies, phylomers, aptamers, single chain antibodies, deimmunized antibodies, humanized antibodies and T cell associated antigen binding molecules. In terms of in vivo analyses, these molecules could be coupled to medical imaging agents in order to visualise specific binding to neoplastic cells, in particular, following the administration of cytotoxic anti-cancer treatments. More specifically, these methods include, but are not limited to:
(i) *In vivo* detection of La. Molecular Imaging may be used following administration of imaging probes or reagents capable of disclosing altered expression levels of the La RNA, mRNA or protein expression product in the biological sample.
   Molecular imaging (Moore et al, BBA, 1402:239-249, 1988; Weissleder et al, Nature Medicine, 6:351-355, 2000) is the *in vivo* imaging of molecular expression that correlates with the macro-features currently visualized using "classical" diagnostic imaging techniques such as X-Ray, computed tomography (CT), MRI, Positron Emission Tomography (PET) or SPECT. In one embodiment, the interactive molecule is coupled to a nuclear medicine imaging agent such as Indium-111 or Technetium-99 or to PET imaging agents to MRI imaging agents such as nanoparticles. In another example, one might include the coupling of enzymes as detection agents, for example ADEPT-like where the analyte is generated *in vivo* after binding of the La-interactive molecule to the target and after injection of the enzyme substrate.
(ii) Analysis of RNA expression in the cells by Fluorescent *In Situ* Hybridization (FISH), or in extracts from the cells by technologies such as Quantitative Reverse Transcriptase Polymerase Chain Reaction (QRTPCR) or Flow cytometric qualification of competitive RT-PCR products (Wedemeyer et al., Clinical Chemistry 48:9 1398-1405, 2002) or array technologies.
   For example, a labelled polynucleotide encoding La may be utilized as a probe in a Northern blot of an RNA extract obtained from a biological sample. Preferably, a nucleic acid extract from the subject is utilized in concert with oligonucleotide primers corresponding to sense and antisense sequences of a polynucleotide encoding La, or flanking sequences thereof, in a nucleic acid amplification reaction such as RT PCR, real time PCR or SAGE. A variety of automated solid-phase detection techniques are also appropriate. For example, very large scale immobilized primer arrays (VLSIPS™) are used for the detection of nucleic acids as, for example, described by Fodor et al., 1991 (Science 251(4995):767-73) and Kazal *et al.,* 1996. The above genetic techniques are well known to persons skilled in the art.
   For example, to detect La encoding RNA transcripts, RNA is isolated from a cellular sample suspected of containing neoplastic cells. RNA can be isolated by methods known in the art, e.g. using TRIZOL™ reagent (GIBCO-BRL/Life Technologies, Gaithersburg, Md.). Oligo-dT, or random-sequence oligonucleotides, as well as sequence-specific oligonucleotides can be employed as a primer in a reverse transcriptase reaction to prepare first-strand cDNAs from the isolated RNA. Resultant first-strand cDNAs are then amplified with sequence-specific oligonucleotides in PCR reactions to yield an amplified product.
   "Polymerase chain reaction" or "PCR" refers to a procedure or technique in which amounts of a preselected fragment of nucleic acid, RNA and/or DNA, are amplified as described in U.S. Patent No. 4,683,195. Generally, sequence information from the ends of the region of interest or beyond is employed to design oligonucleotide primers. These primers will be identical or similar in sequence to opposite strands of the template to be amplified. PCR can be used to amplify specific RNA sequences and cDNA transcribed from total cellular RNA. See generally Mullis et al., 1987; (Methods Enzymol 155:335-50) and Erlich, 1989 (J Clin Immunol 9(6):437-47). Thus, amplification of specific nucleic acid sequences by PCR relies upon oligonucleotides or "primers" having conserved nucleotide sequences wherein the conserved sequences are deduced from alignments of related gene or protein sequences. For example, one primer is prepared which is predicted to anneal to the antisense strand and another primer prepared which is predicted to anneal to the sense strand of a cDNA molecule which encodes La.
   To detect the amplified product, the reaction mixture is typically subjected to agarose gel electrophoresis or other convenient separation technique and the relative presence of La specific amplified nucleic acid detected. For example, the La amplified nucleic acid may be detected using Southern hybridization with a specific oligonucleotide probe or comparing its electrophoretic mobility with nucleic acid standards of known molecular weight. Isolation, purification and characterization of the amplified La nucleic acid may be accomplished by excising or eluting the fragment from the gel (for example, see references Lawn *et al.,* 1981; Goeddel *et al.,* 1980), cloning the amplified product into a cloning site of a suitable vector, such as the pCRII vector (Invitrogen), sequencing the cloned insert and comparing the sequence to the known sequence of La. The relative amounts of La mRNA and cDNA can then be determined.
(iii) Measurement of altered La protein levels in cell extracts or blood or other suitable biological sample, either qualitatively or quantitatively, for example by immunoassay, utilising immunointeractive molecules such as monoclonal antibodies.

In one example, one may seek to detect La -immunointeractive molecule complex formation. For example, an antibody according to the invention, having a reporter molecule associated therewith, may be utilized in immunoassays. Such immunoassays include but are not limited to radioimmunoassays (RIAs), enzyme-linked immunosorbent assays (ELISAs) and immunochromatographic techniques (ICTs), Western blotting which are well known to those of skill in the art. These assays may be performed as direct assays or indirect assays. For example, reference may be made to "Current Protocols in Immunology", 1994 which discloses a variety of immunoassays which may be used in accordance with the present invention. Immunoassays may include competitive assays. It will be understood that the present invention encompasses qualitative and quantitative immunoassays.

Suitable immunoassay techniques are described, for example, in U.S. Patent Nos. 4,016,043, 4,424,279 and 4,018,653. These include both single-site and two-site assays of the non-competitive types, as well as the traditional competitive binding assays. These assays also include direct binding of a labelled antigen-binding molecule to a target antigen. The antigen in this case is La or a fragment thereof.

Two-site assays are one example of an assay which may be used in the present invention. A number of variations of these assays exist, all of which are intended to be encompassed by the present invention. Briefly, in a typical forward assay, an unlabelled antigen-binding molecule such as an unlabelled antibody is immobilized on a solid substrate and the sample to be tested brought into contact with the bound molecule. After a suitable period of incubation, for a period of time sufficient to allow formation of an antibody-antigen complex, another antigen-binding molecule, suitably a second antibody specific to the antigen, labelled with a reporter molecule capable of producing a detectable signal is then added and incubated, allowing time sufficient for the formation of another complex of antibody-antigen-labelled antibody. Any unreacted material is washed away and the presence of the antigen is determined by observation of a signal produced by the reporter molecule. The results may be either qualitative, by simple observation of the visible signal, or may be quantitated by comparing with a control sample containing known amounts of antigen. Variations on the forward assay include a simultaneous assay, in which both sample and labelled antibody are added simultaneously to the bound antibody. These techniques are well known to those skilled in the art, including minor variations as will be readily apparent.

In the typical forward assay, a first antibody having specificity for the antigen or antigenic parts thereof is either covalently or passively bound to a solid surface. The solid surface is typically glass or a polymer, the most commonly used polymers being cellulose, polyacrylamide, nylon, polystyrene, polyvinyl chloride or polypropylene. The solid supports may be in the form of tubes, beads, discs of microplates, or any other surface suitable for conducting an immunoassay. The binding processes are well known in the art and generally consist of cross-linking covalently binding or physically adsorbing, the polymer-antibody complex is washed in preparation for the test sample. An aliquot of the sample to be tested is then added to the solid phase complex and incubated for a period of time sufficient and under suitable conditions to allow binding of any antigen present to the antibody. Following the incubation period, the antigen-antibody complex is washed and dried and incubated with a second antibody specific for a portion of the antigen. The second antibody has generally a reporter molecule associated therewith that is used to indicate the binding of the second antibody to the antigen. The amount of labelled antibody that binds, as determined by the associated reporter molecule, is proportional to the amount of antigen bound to the immobilized first antibody.

An alternative method involves immobilizing the antigen in the biological sample and then exposing the immobilized antigen to specific antibody that may or may not be labelled with a reporter molecule. Depending on the amount of target and the strength of the reporter molecule signal, a bound antigen may be detectable by direct labelling with the antibody. Alternatively, a second labelled antibody, specific to the first antibody is exposed to the target-first antibody complex to form a target-first antibody-second antibody tertiary complex. The complex is detected by the signal emitted by the reporter molecule.

From the foregoing, it will be appreciated that the reporter molecule associated with the antigen-binding molecule may include the following:-
(a) direct attachment of the reporter molecule to the antibody;
(b) indirect attachment of the reporter molecule to the antibody; i.e., attachment of the reporter molecule to another assay reagent which subsequently binds to the antibody; and
(c) attachment to a subsequent reaction product of the antibody.

The reporter molecule may be selected from a group including a chromogen, a catalyst, an enzyme, a fluorochrome, a chemiluminescent molecule, a paramagnetic ion, a lanthanide ion such as Europium (Eu³⁴), a radioisotope including other nuclear tags and a direct visual label.

In the case of a direct visual label, use may be made of a colloidal metallic or non-metallic particle, a dye particle, an enzyme or a substrate, an organic polymer, a latex particle, a liposome, or other vesicle containing a signal producing substance and the like.

A large number of enzymes suitable for use as reporter molecules is disclosed in U.S. Patent Nos. U.S. 4,366,241, U.S. 4,843,000, and U.S. 4,849,338. Suitable enzymes useful in the present invention include alkaline phosphatase, horseradish peroxidase, luciferase, β-galactosidase, glucose oxidase, lysozyme, malate dehydrogenase and the like. The enzymes may be used alone or in combination with a second enzyme that is in solution.

Suitable fluorochromes include, but are not limited to, fluorescein isothiocyanate (FITC), tetramethylrhodamine isothiocyanate (TRITC), R-Phycoerythrin (RPE), and Texas Red. Other exemplary fluorochromes include those discussed by Dower et al., International Publication No. WO 93/06121. Reference also may be made to the fluorochromes described in U.S. Patent Nos. 5,573,909 (Singer et al)*,* 5,326,692 (Brinkley et al)*.* Alternatively, reference may be made to the fluorochromes described in U.S. Patent Nos. 5,227,487, 5,274,113, 5,405,975, 5,433,896, 5,442,045, 5,451,663, 5,453,517, 5,459,276, 5,516,864, 5,648,270 and 5,723,218.

In the case of an enzyme immunoassay, an enzyme is conjugated to the second antibody, generally by means of glutaraldehyde or periodate. As will be readily recognised, however, a wide variety of different conjugation techniques exist which are readily available to the skilled artisan. The substrates to be used with the specific enzymes are generally chosen for the production of, upon hydrolysis by the corresponding enzyme, a detectable colour change. Examples of suitable enzymes include those described *supra.* It is also possible to employ fluorogenic substrates, which yield a fluorescent product rather than the chromogenic substrates noted above. In all cases, the enzyme-labelled antibody is added to the first antibody-antigen complex, allowed to bind, and then the excess reagent washed away. A solution containing the appropriate substrate is then added to the complex of antibody-antigen-antibody. The substrate will react with the enzyme linked to the second antibody, giving a qualitative visual signal, which may be further quantitated, usually spectrophotometrically, to give an indication of the amount of antigen which was present in the sample.

Alternately, fluorescent compounds, such as fluorescein, rhodamine and the lanthanide, europium (EU), may be chemically coupled to antibodies without altering their binding capacity. When activated by illumination with light of a particular wavelength, the fluorochrome-labelled antibody adsorbs the light energy, inducing a state to excitability in the molecule, followed by emission of the light at a characteristic colour visually detectable with a light microscope. The fluorescent-labelled antibody is allowed to bind to the first antibody-antigen complex. After washing off the unbound reagent, the remaining tertiary complex is then exposed to light of an appropriate wavelength. The fluorescence observed indicates the presence of the antigen of interest. Immunofluorometric assays (IFMA) are well established in the art and are particularly useful for the present method. However, other reporter molecules, such as radioisotope, chemiluminescent or bioluminescent molecules may also be employed.
(iv) Determining altered protein expression based on any suitable functional test, enzymatic test or immunological test in addition to those detailed in point (iii) above.
(v) Nanotechnology-related techniques such as those outlined in Ferrari (Nature Reviews Cancer 5:161-171, 2005) and Duncan (Nature Reviews Drug Discovery, 2:347-360, 2003)

The use of antibodies, in particular monoclonal antibodies to detect La is a preferred method of the present invention. Antibodies may be prepared by any of a number of means. For the detection of human La, for example, human-human monoclonal antibody hybridomas may be derived from B cells, which have been obtained from patients who make anti-La autoantibodies because they have systemic autoimmune diseases such as systemic lupus erythematosis (SLE) or Sjorgren's syndrome (Ravirajan et al. Lupus 1(3):157-165, 1992). Antibodies are generally but not necessarily derived from non-human animals such as primates, livestock animals (e.g. sheep, cows, pigs, goats, horses), laboratory test animals (e.g. mice, rats, guinea pigs, rabbits) and companion animals (e.g. dogs, cats). Generally, antibody based assays are conducted *in vitro* on cell or tissue biopsies. However, if an antibody is suitably deimmunized or, in the case of human use, humanized, then the antibody can be labelled with, for example, a nuclear tag, administered to a patient and the site of nuclear label accumulation determined by radiological techniques. The La antibody is regarded, therefore, as a cellular apoptosis targeting agent. Accordingly, the present invention extends to deimmunized forms of the antibodies for use in cellular apoptosis imaging in human and non-human patients. This is described further below.

Currently available antibodies include SW3 and 3B9.

For the generation of antibodies to La, this molecule is required to be extracted from a biological sample whether this be from animal including human tissue or from cell culture if produced by recombinant means. The La can be separated from the biological sample by any suitable means. For example, the separation may take advantage of any one or more of La's surface charge properties, size, density, biological activity and its affinity for another entity (e.g. another protein or chemical compound to which it binds or otherwise associates). Thus, for example, separation of La from the biological fluid may be achieved by any one or more of ultra-centrifugation, ion-exchange chromatography (e.g. anion exchange chromatography, cation exchange chromatography), electrophoresis (e.g. polyacrylamide gel electrophoresis, isoelectric focussing), size separation (e.g., gel filtration, ultra-filtration) and affinity-mediated separation (e.g. immunoaffinity separation including, but not limited to, magnetic bead separation such as Dynabead™ separation, immunochromatography, immuno-precipitation). Choice of the separation technique(s) employed may depend on the biological activity or physical properties of the La sought or from which tissues it is obtained.

Preferably, the separation of La from the biological fluid preserves conformational epitopes present on the protein and, thus, suitably avoids techniques that cause denaturation of the enzyme. Persons of skill in the art will recognize the importance of maintaining or mimicking as close as possible physiological conditions peculiar to La (e.g. the biological fluid from which it is obtained) to ensure that the antigenic determinants or active sites on La, which are exposed to the animal, are structurally identical to that of the native protein. This ensures the raising of appropriate antibodies in the immunised animal that would recognize the native protein. In a preferred embodiment, La is separated from the biological fluid using any one or more of affinity separation, gel filtration and ultra-filtration.

Immunization and subsequent production of monoclonal antibodies can be carried out using standard protocols as for example described by Kohler and Milstein, Nature 256: 495-499, 1975; Kohler and Milstein, Eur. J. Immunol. 6(7): 511-519, 1976; Coligan et al., Current Protocols in Immunology, John Wiley & Sons, Inc., 1991-1997, or Toyama et al., "Monoclonal Antibody, Experiment Manual", published by Kodansha Scientific, 1987. Essentially, an animal is immunized with a La-containing biological fluid or fraction thereof by standard methods to produce antibody-producing cells, particularly antibody-producing somatic cells (e.g. B lymphocytes). These cells can then be removed from the immunized animal for immortalization.

Where a fragment of La is used to generate antibodies, it may need to first be associated with a carrier. By "carrier" is meant any substance of typically high molecular weight to which a non- or poorly immunogenic substance (e.g. a hapten) is naturally or artificially linked to enhance its immunogenicity.

Immortalization of antibody-producing cells may be carried out using methods which are well-known in the art. For example, the immortalization may be achieved by the transformation method using Epstein-Barr virus (EBV) (Kozbor et al., Methods in Enzymology 121: 140, 1986). In a preferred embodiment, antibody-producing cells are immortalized using the cell fusion method (described in Coligan *et al.,* 1991-1997, *supra),* which is widely employed for the production of monoclonal antibodies. In this method, somatic antibody-producing cells with the potential to produce antibodies, particularly B cells, are fused with a myeloma cell line. These somatic cells may be derived from the lymph nodes, spleens and peripheral blood of humans with circulating Lareactive antibodies, and primed animals, preferably rodent animals such as mice and rats. Mice spleen cells are particularly useful. It would be possible, however, to use rat, rabbit, sheep or goat cells, or cells from other animal species instead.

Specialized myeloma cell lines have been developed from lymphocytic tumours for use in hybridoma-producing fusion procedures (Kohler and Milstein, 1976, *supra;* Shulman et al., Nature 276: 269-270, 1978; Volk et al., J. Virol. 42(1): 220-227, 1982). These cell lines have been developed for at least three reasons. The first is to facilitate the selection of fused hybridomas from unfused and similarly indefinitely self-propagating myeloma cells. Usually, this is accomplished by using myelomas with enzyme deficiencies that render them incapable of growing in certain selective media that support the growth of hybridomas. The second reason arises from the inherent ability of lymphocytic tumour cells to produce their own antibodies. To eliminate the production of tumour cell antibodies by the hybridomas, myeloma cell lines incapable of producing endogenous light or heavy immunoglobulin chains are used. A third reason for selection of these cell lines is for their suitability and efficiency for fusion.

Many myeloma cell lines may be used for the production of fused cell hybrids, including, e.g. P3X63-Ag8, P3X63-AG8.653, P3/NS1-Ag4-1 (NS-1), Sp2/0-Ag14 and S194/5.XXO.Bu.1. The P3X63-Ag8 and NS-1 cell lines have been described by Köhler and Milstein (1976, *supra).* Shulman *et al.* (1978, *supra)* developed the Sp2/0-Ag14 myeloma line. The S194/5.XXO.Bu.1 line was reported by Trowbridge, J. Exp. Med. 148(1): 313-323, 1978.

Methods for generating hybrids of antibody-producing spleen or lymph node cells and myeloma cells usually involve mixing somatic cells with myeloma cells in a 10:1 proportion (although the proportion may vary from about 20:1 to about 1:1), respectively, in the presence of an agent or agents (chemical, viral or electrical) that promotes the fusion of cell membranes. Fusion methods have been described (Kohler and Milstein, 1975, *supra;* 1976, *supra;* Gefter et al., Somatic Cell Genet. 3: 231-236, 1977; Volk *et al.,* 1982, *supra).* The fusion-promoting agents used by those investigators were Sendai virus and polyethylene glycol (PEG).

Because fusion procedures produce viable hybrids at very low frequency (e.g. when spleens are used as a source of somatic cells, only one hybrid is obtained for roughly every 1x10⁵ spleen cells), it is preferable to have a means of selecting the fused cell hybrids from the remaining unfused cells, particularly the unfused myeloma cells. A means of detecting the desired antibody-producing hybridomas among other resulting fused cell hybrids is also necessary. Generally, the selection of fused cell hybrids is accomplished by culturing the cells in media that support the growth of hybridomas but prevent the growth of the unfused myeloma cells, which normally would go on dividing indefinitely. The somatic cells used in the fusion do not maintain long-term viability in *in vitro* culture and hence do not pose a problem. In the example of the present invention, myeloma cells lacking hypoxanthine phosphoribosyl transferase (HPRT-negative) were used. Selection against these cells is made in hypoxanthine/aminopterin/thymidine (HAT) medium, a medium in which the fused cell hybrids survive due to the HPRT-positive genotype of the spleen cells. The use of myeloma cells with different genetic deficiencies (drug sensitivities, etc.) that can be selected against in media supporting the growth of genotypically competent hybrids is also possible.

Several weeks are required to selectively culture the fused cell hybrids. Early in this time period, it is necessary to identify those hybrids which produce the desired antibody, so that they may subsequently be cloned and propagated. Generally, around 10% of the hybrids obtained produce the desired antibody, although a range of from about 1 to about 30% is not uncommon. The detection of antibody-producing hybrids can be achieved by any one of several standard assay methods, including enzyme-linked immunoassay and radioimmunoassay techniques as, for example, described in Kennet et al. (eds) Monoclonal Antibodies and Hybridomas: A New Dimension in Biological Analyses, pp. 376-384, Plenum Press, New York, 1980 and by FACS analysis.

Once the desired fused cell hybrids have been selected and cloned into individual antibody-producing cell lines, each cell line may be propagated in either of two standard ways. A suspension of the hybridoma cells can be injected into a histocompatible animal. The injected animal will then develop tumours that secrete the specific monoclonal antibody produced by the fused cell hybrid. The body fluids of the animal, such as serum or ascites fluid, can be tapped to provide monoclonal antibodies in high concentration. Alternatively, the individual cell lines may be propagated *in vitro* in laboratory culture vessels. The culture medium containing high concentrations of a single specific monoclonal antibody can be harvested by decantation, filtration or centrifugation, and subsequently purified.

The cell lines are tested for their specificity to detect the La by any suitable immunodetection means. For example, cell lines can be aliquoted into a number of wells and incubated and the supernatant from each well is analyzed by enzyme-linked immunosorbent assay (ELISA), indirect fluorescent antibody technique, or the like. The cell line(s) producing a monoclonal antibody capable of recognizing the target La but which does not recognize non-target epitopes are identified and then directly cultured *in vitro* or injected into a histocompatible animal to form tumours and to produce, collect and purify the required antibodies.

These antibodies are La specific. This means that the antibodies are capable of distinguishing La from other molecules. More broad spectrum antibodies may be used provided that they do not cross react with molecules in a normal cell.

In one embodiment, the subject antibody is anti-human La monoclonal antibodies, 8G3 and 9A5 (Bachmann et al. Proc Natl Acad Sci USA 83 (20):7770-7774, 1986), anti-human La monoclonal antibody (mAb), La1B5 (Mamula et al. J Immunol 143(9):2923-2928, 1989), anti-human La monoclonal antibodies (Carmo-Fonseca et al. ExpCell Res 185(1):73-85, 1989), anti-human and anti-bovine La monoclonal antibodies, SW1, SW3 and SW5 (Pruijn et al. Eur J Biochem 232(2):611-619, 1995), anti-human and anti-rodent La mAb, La4B6 (Troster et al. J Autoimmunity 8(6):825-842, 1995) or anti-human and anti-murine La mAb, 3B9 (Tran et al. Arthritis Rheum 46(1):202-208, 2002) or derivative, homologue, analogue, chemical equivalent, mutant or mimetic thereof.

Since the monoclonal antibody may be destined for *in vivo* use, it may be desirable to deimmunise the antibody. The deimmunization process may take any of a number of forms including the preparation of chimeric antibodies which have the same or similar specificity as the monoclonal antibodies prepared according to the present invention. Chimeric antibodies are antibodies whose light and heavy chain genes have been constructed, typically by genetic engineering, from immunoglobulin variable and constant region genes belonging to different species. Thus, in accordance with the present invention, once a hybridoma producing the desired monoclonal antibody is obtained, techniques are used to produce interspecific monoclonal antibodies wherein the binding region of one species is combined with a non-binding region of the antibody of another species (Liu et al., Proc. Natl. Acad. Sci. USA 84: 3439-3443, 1987). For example, complementary determining regions (CDRs) from a non-human (e.g. murine) monoclonal antibody can be grafted onto a human antibody, thereby "humanizing" the murine antibody (European Patent Publication No. 0 239 400; Jones et al., Nature 321: 522-525, 1986; Verhoeyen et al., Science 239: 1534-1536, 1988; Richmann et al., Nature 332: 323-327, 1988). In this case, the deimmunizing process is specific for humans. More particularly, the CDRs can be grafted onto a human antibody variable region with or without human constant regions. The non-human antibody providing the CDRs is typically referred to as the "donor" and the human antibody providing the framework is typically referred to as the "acceptor". Constant regions need not be present, but if they are, they must be substantially identical to human immunoglobulin constant regions, i.e. at least about 85-90%, preferably about 95% or more identical. Hence, all parts of a humanized antibody, except possibly the CDRs, are substantially identical to corresponding parts of natural human immunoglobulin sequences. Thus, a "humanized antibody" is an antibody comprising a humanized light chain and a humanized heavy chain immunoglobulin. A donor antibody is said to be "humanized", by the process of "humanization", because the resultant humanized antibody is expected to bind to the same antigen as the donor antibody that provides the CDRs. Reference herein to "humanized" includes reference to an antibody deimmunized to a particular host, in this case, a human host.

It will be understood that the deimmunized antibodies may have additional conservative amino acid substitutions which have substantially no effect on antigen binding or other immunoglobulin functions. Exemplary conservative substitutions may be made according to Table 1.

**TABLE 1**

| **ORIGINAL RESIDUE** | **EXEMPLARY SUBSTITUTIONS** |
|---|---|
| Ala | Ser |
| Arg | Lys |
| Asn | Gln, His |
| Asp | Glu |
| Cys | Ser |
| Gln | Asn |
| Glu | Asp |
| Gly | Pro |
| His | Asn, Gln |
| Ile | Leu, Val |
| Leu | Ile, Val |
| Lys | Arg, Gln, Glu |
| Met | Leu, Ile |
| Phe | Met, Leu, Tyr |
| Ser | Thr |
| Thr | Ser |
| Trp | Tyr |
| Tyr | Trp, Phe |
| Val | Ile, Leu |

Exemplary methods which may be employed to produce deimmunized antibodies according to the present invention are described, for example, in references Richmann *et al.,* 1988, *supra;* European Patent Publication No. 0 239 400; Chou et al. (U.S. Patent No. 6,056,957); Queen et al. (U.S. Patent No. 6,180,370); Morgan et al. (U.S. Patent No. 6,180,377).

Thus, in one embodiment, the present invention contemplates the use of a deimmunized antibody molecule having specificity for an epitope recognized by a monoclonal antibody to La wherein at least one of the CDRs of the variable domain of said deimmunized antibody is derived from the said monoclonal antibody to La and the remaining immunoglobulin-derived parts of the deimmunized antibody molecule are derived from an immunoglobulin or an analogue thereof from the host for which the antibody is to be deimmunized.

This aspect of the present invention involves manipulation of the framework region of a non-human antibody.

The present invention extends to the use of mutants, analogues and derivatives of the subject antibodies but which still retain specificity for La.

The terms "mutant" or "derivatives" includes one or more amino acid substitutions, additions and/or deletions.

As used herein, the term "CDR" includes CDR structural loops which covers the three light chain and the three heavy chain regions in the variable portion of an antibody framework region which bridge β strands on the binding portion of the molecule. These loops have characteristic canonical structures (Chothia et al., J. Mol. Biol. 196: 901, 1987; Chothia et al., J. Mol. Biol. 227: 799, 1992).

By "framework region" is meant region of an immunoglobulin light or heavy chain variable region, which is interrupted by three hypervariable regions, also called CDRs. The extent of the framework region and CDRs have been precisely defined (see, for example, Kabat et al., "Sequences of Proteins of Immunological Interest", U.S. Department of Health and Human Services, 1983). The sequences of the framework regions of different light or heavy chains are relatively conserved within a species. As used herein, a "human framework region" is a framework region that is substantially identical (about 85% or more, usually 90-95% or more) to the framework region of a naturally occurring human immunoglobulin. The framework region of an antibody, that is the combined framework regions of the constituent light and heavy chains, serves to position and align the CDRs. The CDRs are primarily responsible for binding to an epitope of La.

As used herein, the term "heavy chain variable region" means a polypeptide which is from about 110 to 125 amino acid residues in length, the amino acid sequence of which corresponds to that of a heavy chain of a monoclonal antibody of the invention, starting from the amino-terminal (N-terminal) amino acid residue of the heavy chain. Likewise, the term "light chain variable region" means a polypeptide which is from about 95 to 130 amino acid residues in length, the amino acid sequence of which corresponds to that of a light chain of a monoclonal antibody of the invention, starting from the N-terminal amino acid residue of the light chain. Full-length immunoglobulin "light chains" (about 25 Kd or 214 amino acids) are encoded by a variable region gene at the NH₂-terminus (about 110 amino acids) and a κ or λ constant region gene at the COOH-terminus. Full-length immunoglobulin "heavy chains" (about 50 Kd or 446 amino acids), are similarly encoded by a variable region gene (about 116 amino acids) and one of the other aforementioned constant region genes, e.g. γ (encoding about 330 amino acids).

The term "immunoglobulin" or "antibody" is used herein to refer to a protein consisting of one or more polypeptides substantially encoded by immunoglobulin genes. The recognized immunoglobulin genes include the κ, λ, α, γ (IgG₁, IgG₂, IgG₃, IgG₄), δ, ε and µ constant region genes, as well as the myriad immunoglobulin variable region genes. One form of immunoglobulin constitutes the basic structural unit of an antibody. This form is a tetramer and consists of two identical pairs of immunoglobulin chains, each pair having one light and one heavy chain. In each pair, the light and heavy chain variable regions are together responsible for binding to an antigen, and the constant regions are responsible for the antibody effector functions. In addition to antibodies, immunoglobulins may exist in a variety of other forms including, for example, Fv, Fab, Fab' and (Fab')₂.

The invention also contemplates the use and generation of fragments of monoclonal antibodies produced by the method of the present invention including, for example, Fv, Fab, Fab' and F(ab')₂ fragments. Such fragments may be prepared by standard methods as for example described by Coligan *et al.* (1991-1997, *supra).*

The present invention also contemplates synthetic or recombinant antigen-binding molecules with the same or similar specificity as the monoclonal antibodies of the invention. Antigen-binding molecules of this type may comprise a synthetic stabilised Fv fragment. Exemplary fragments of this type include single chain Fv fragments (sFv, frequently termed scFv) in which a peptide linker is used to bridge the N terminus or C terminus of a V*_{H}* domain with the C terminus or N-terminus, respectively, of a V*_{L}* domain. ScFv lack all constant parts of whole antibodies and are not able to activate complement. Suitable peptide linkers for joining the V*_{H}* and V*_{L}* domains are those which allow the V*_{H}* and V*_{L}* domains to fold into a single polypeptide chain having an antigen binding site with a three dimensional structure similar to that of the antigen binding site of a whole antibody from which the Fv fragment is derived. Linkers having the desired properties may be obtained by the method disclosed in U.S. Patent No 4,946,778. However, in some cases a linker is absent. ScFvs may be prepared, for example, in accordance with methods outlined in Krebber *et al.* (Krebber et al., J. Immunol. Methods 201(1): 35-55, 1997). Alternatively, they may be prepared by methods described in U.S. Patent No 5,091,513, European Patent No 239,400 or the articles by Winter and Milstein (Winter and Milstein, Nature 349: 293, 1991) and Plückthun *et al.* (Plückthun et al., In Antibody engineering: A practical approach 203-252, 1996).

Alternatively, the synthetic stabilized Fv fragment comprises a disulphide stabilized Fv (dsFv) in which cysteine residues are introduced into the V*_{H}* and V*_{L}* domains such that in the fully folded Fv molecule the two residues will form a disulphide bond therebetween. Suitable methods of producing dsFv are described, for example, in (Glockshuber et al., Biochem. 29: 1363-1367, 1990; Reiter et al., Biochem. 33: 5451-5459, 1994; Reiter et al., Cancer Res. 54: 2714-2718, 1994; Reiter et al., J. Biol. Chem. 269: 18327-18331, 1994; Webber et al., Mol. Immunol. 32: 249-258, 1995).

Also contemplated as synthetic or recombinant antigen-binding molecules are single variable region domains (termed dAbs) as, for example, disclosed in (Ward et al., Nature 341: 544-546, 1989; Hamers-Casterman et al., Nature 363: 446-448, 1993; Davies & Riechmann, FEBS Lett. 339: 285-290, 1994).

Alternatively, the synthetic or recombinant antigen-binding molecule may comprise a "minibody". In this regard, minibodies are small versions of whole antibodies, which encode in a single chain the essential elements of a whole antibody. Suitably, the minibody is comprised of the V*_{H}* and V*_{L}* domains of a native antibody fused to the hinge region and CH3 domain of the immunoglobulin molecule as, for example, disclosed in U.S. Patent No 5,837,821.

In an alternate embodiment, the synthetic or recombinant antigen binding molecule may comprise non-immunoglobulin derived, protein frameworks. For example, reference may be made to (Ku & Schutz, Proc. Natl. Acad. Sci. USA 92: 6552-6556, 1995) which discloses a four-helix bundle protein cytochrome b562 having two loops randomized to create CDRs, which have been selected for antigen binding.

The synthetic or recombinant antigen-binding molecule may be multivalent (i.e. having more than one antigen binding site). Such multivalent molecules may be specific for one or more antigens. Multivalent molecules of this type may be prepared by dimerization of two antibody fragments through a cysteinyl-containing peptide as, for example disclosed by (Adams et al., Cancer Res. 53: 4026-4034, 1993; Cumber et al., J. Immunol. 149: 120-126, 1992). Alternatively, dimerization may be facilitated by fusion of the antibody fragments to amphiphilic helices that naturally dimerize (Plünckthun, Biochem. 31: 1579-1584, 1992) or by use of domains (such as leucine zippers *jun* and *fos*) that preferentially heterodimerize (Kostelny et al., J. Immunol. 148: 1547-1553, 1992).

The present invention further encompasses chemical analogues of amino acids in the subject antibodies. The use of chemical analogues of amino acids is useful *inter alia* to stabilize the molecules such as if required to be administered to a subject. The analogues of the amino acids contemplated herein include, but are not limited to, modifications of side chains, incorporation of unnatural amino acids and/or their derivatives during peptide, polypeptide or protein synthesis and the use of crosslinkers and other methods which impose conformational constraints on the proteinaceous molecule or their analogues.

Examples of side chain modifications contemplated by the present invention include modifications of amino groups such as by reductive alkylation by reaction with an aldehyde followed by reduction with NaBH₄; amidination with methylacetimidate; acylation with acetic anhydride; carbamoylation of amino groups with cyanate; trinitrobenzylation of amino groups with 2, 4, 6-trinitrobenzene sulphonic acid (TNBS); acylation of amino groups with succinic anhydride and tetrahydrophthalic anhydride; and pyridoxylation of lysine with pyridoxal-5-phosphate followed by reduction with NaBH₄.

The guanidine group of arginine residues may be modified by the formation of heterocyclic condensation products with reagents such as 2,3-butanedione, phenylglyoxal and glyoxal.

The carboxyl group may be modified by carbodiimide activation *via* O-acylisourea formation followed by subsequent derivatisation, for example, to a corresponding amide.
Sulphydryl groups may be modified by methods such as carboxymethylation with iodoacetic acid or iodoacetamide; performic acid oxidation to cysteic acid; formation of a mixed disulphides with other thiol compounds; reaction with maleimide, maleic anhydride or other substituted maleimide; formation of mercurial derivatives using 4-chloromercuribenzoate, 4-chloromercuriphenylsulphonic acid, phenylmercury chloride, 2-chloromercuri-4-nitrophenol and other mercurials; carbamoylation with cyanate at alkaline pH.

Tryptophan residues may be modified by, for example, oxidation with N-bromosuccinimide or alkylation of the indole ring with 2-hydroxy-5-nitrobenzyl bromide or sulphenyl halides. Tyrosine residues on the other hand, may be altered by nitration with tetranitromethane to form a 3-nitrotyrosine derivative.

Modification of the imidazole ring of a histidine residue may be accomplished by alkylation with iodoacetic acid derivatives or N-carbethoxylation with diethylpyrocarbonate.

Examples of incorporating unnatural amino acids and derivatives during peptide synthesis include, but are not limited to, use of norleucine, 4-amino butyric acid, 4-amino-3-hydroxy-5-phenylpentanoic acid, 6-aminohexanoic acid, t-butylglycine, norvaline, phenylglycine, ornithine, sarcosine, 4-amino-3-hydroxy-6-methylheptanoic acid, 2-thienyl alanine and/or D-isomers of amino acids. A list of unnatural amino acid, contemplated herein is shown in Table 2.

**TABLE 2**

| Non-conventional amino acid | Code | Non-conventional amino acid | Code |
|---|---|---|---|
| α-aminobutyric acid | Abu | L-N-methylalanine | Nmala |
| α-amino-α-methylbutyrate | Mgabu | L-N-methylarginine | Nmarg |
| aminocyclopropane- | Cpro | L-N-methylasparagine | Nmasn |
| carboxylate | | L-N-methylaspartic acid | Nmasp |
| aminoisobutyric acid | Aib | L-N-methylcysteine | Nmcys |
| aminonorbornyl- | Norb | L-N-methylglutamine | Nmgln |
| carboxylate | | L-N-methylglutamic acid | Nmglu |
| cyclohexylalanine | Chexa | L-Nmethylhistidine | Nmhis |
| cyclopentylalanine | Cpen | L-N-methylisolleucine | Nmile |
| D-alanine | Dal | L-N-methylleucine | Nmleu |
| D-arginine | Darg | L-N-methyllysine | Nmlys |
| D-aspartic acid | Dasp | L-N-methylmethionine | Nmmet |
| D-cysteine | Dcys | L-N-methylnorleucine | Nmnle |
| D-glutamine | Dgln | L-N-methylnorvaline | Nmnva |
| D-glutamic acid | Dglu | L-N-methylornithine | Nmorn |
| D-histidine | Dhis | L-N-methylphenylalanine | Nmphe |
| D-isoleucine | Dile | L-N-methylproline | Nmpro |
| D-leucine | Dleu | L-N-methylserine | Nmser |
| D-lysine | Dlys | L-N-methylthreonine | Nmthr |
| D-methionine | Dmet | L-N-methyltryptophan | Nmtrp |
| D-ornithine | Dorn | L-N-methyltyrosine | Nmtyr |
| D-phenylalanine | Dphe | L-N-methylvaline | Nmval |
| D-proline | Dpro | L-N-methylethylglycine | Nmetg |
| D-serine | Dser | L-N-methyl-t-butylglycine | Nmtbug |
| D-threonine | Dthr | L-norleucine | Nle |
| D-tryptophan | Dtrp | L-norvaline | Nva |
| D-tyrosine | Dtyr | α-methyl-aminoisobutyrate | Maib |
| D-valine | Dval | α-methyl-γ-aminobutyrate | Mgabu |
| D-α-methylalanine | Dmala | α-methylcyclohexylalanine | Mchexa |
| D-α-methylarginine | Dmarg | α-methylcylcopentylalanine | Mcpen |
| D-α-methylasparagine | Dmasn | α-methyl-α-napthylalanine | Manap |
| D-α-methylaspartate | Dmasp | α-methylpenicillamine | Mpen |
| D-α-methylcysteine | Dmcys | N-(4-aminobutyl)glycine | Nglu |
| D-α-methylglutamine | Dmgln | N-(2-aminoethyl)glycine | Naeg |
| D-α-methylhistidine | Dmhis | N-(3-aminopropyl)glycine | Norn |
| D-α-methylisoleucine | Dmile | N-amino-α-methylbutyrate | Nmaabu |
| D-α-methylleucine | Dmleu | α-napthylalanine | Anap |
| D-α-methyllysine | Dmlys | N-benzylglycine | Nphe |
| D-α-methylmethionine | Dmmet | N-(2-carbamylethyl)glycine | Ngln |
| D-α-methylornithine | Dmorn | N-(carbamylmethyl)glycine | Nasn |
| D-α-methylphenylalanine | Dmphe | N-(2-carboxyethyl)glycine | Nglu |
| D-α-methylproline | Dmpro | N-(carboxymethyl)glycine | Nasp |
| D-α-methylserine | Dmser | N-cyclobutylglycine | Ncbut |
| D-α-methylthreonine | Dmthr | N-cycloheptylglycine | Nchep |
| D-α-methyltryptophan | Dmtrp | N-cyclohexylglycine | Nchex |
| D-α-methyltyrosine | Dmty | N-cyclodecylglycine | Ncdec |
| D-α-methylvaline | Dmval | N-cylcododecylglycine | Ncdod |
| D-N-methylalanine | Dnmala | N-cyclooctylglycine | Ncoct |
| D-N-methylarginine | Dnmarg | N-cyclopropylglycine | Ncpro |
| D-N-methylasparagine | Dnmasn | N-cycloundecylglycine | Ncund |
| D-N-methylaspartate | Dnmasp | N-(2,2-diphenylethyl)glycine | Nbhm |
| D-N-methylcysteine | Dnmcys | N-(3,3-diphenylpropyl)glycine | Nbhe |
| D-N-methylglutamine | Dnmgln | N-(3-guanidinopropyl)glycine | Narg |
| D-N-methylglutamate | Dnmglu | N-(1-hydroxyethyl)glycine | Nthr |
| D-N-methylhistidine | Dnmhis | N-(hydroxyethyl))glycine | Nser |
| D-N-methylisoleucine | Dnmile | N-(imidazolylethyl))glycine | Nhis |
| D-N-methylleucine | Dnmleu | N-(3-indolylyethyl)glycine | Nhtrp |
| D-N-methyllysine | Dnmlys | N-methyl-y-aminobutyrate | Nmgabu |
| N-methylcyclohexylalanine | Nmchexa | D-N-methylmethionine | Dnmmet |
| D-N-methylornithine | Dnmorn | N-methylcyclopentylalanine | Nmcpen |
| N-methylglycine | Nala | D-N-methylphenylalanine | Dnmphe |
| N-methylaminoisobutyrate | Nmaib | D-N-methylproline | Dnmpro |
| N-(1-methylpropyl)glycine | Nile | D-N-methylserine | Dnmser |
| N-(2-methylpropyl)glycine | Nleu | D-N-methylthreonine | Dnmthr |
| D-N-methyltryptophan | Dnmtrp | N-(1-methylethyl)glycine | Nval |
| D-N-methyltyrosine | Dnmtyr | N-methyla-napthylalanine | Nmanap |
| D-N-methylvaline | Dnmval | N-methylpenicillamine | Nmpen |
| γ-aminobutyric acid | Gabu | N-(p-hydroxyphenyl)glycine | Nhtyr |
| L-*t*-butylglycine | Tbug | N-(thiomethyl)glycine | Ncys |
| L-ethylglycine | Etg | penicillamine | Pen |
| L-homophenylalanine | Hphe | L-α-methylalanine | Mala |
| L-α-methylarginine | Marg | L-α-methylasparagine | Masn |
| L-α-methylaspartate | Masp | L-α-methyl-*t*-butylglycine | Mtbug |
| L-α-methylcysteine | Mcys | L-methylethylglycine | Metg |
| L-α-methylglutamine | Mgln | L-α-methylglutamate | Mglu |
| L-α-methylhistidine | Mhis | L-α-methylhomophenylalanine | Mhphe |
| L-α-methylisoleucine | Mile | N-(2-methylthioethyl)glycine | Nmet |
| L-α-methylleucine | Mleu | L-α-methyllysine | Mlys |
| L-α-methylmethionine | Mmet | L-α-methylnorleucine | Mnle |
| L-α-methylnorvaline | Mnva | L-α-methylomithine | Morn |
| L-α-methylphenylalanine | Mphe | L-α-methylproline | Mpro |
| L-α-methylserine | Mser | L-α-methylthreonine | Mthr |
| L-α-methyltryptophan | Mtrp | L-α-methyltyrosine | Mtyr |
| L-α-methylvaline | Mval | L-N-methylhomophenylalanine | Nmhphe |
| N-(N-(2,2-diphenylethyl) carbamylmelhyl)glycine | Nnbhm | N-(N-(3,3-diphenylpropyl) carbamylmethyl)glycine | Nnbhe |
| 1-carboxy-1-(2,2-diphenyl-ethylamino)cyclopropane | Nmbc | | |

Crosslinkers can be used, for example, to stabilize 3D conformations, using homo-bifunctional crosslinkers such as the bifunctional imido esters having (CH₂)ₙ spacer groups with n=1 to n=6, glutaraldehyde, N-hydroxysuccinimide esters and hetero-bifunctional reagents which usually contain an amino-reactive moiety such as N-hydroxysuccinimide and another group specific-reactive moiety such as maleimido or dithio moiety (SH) or carbodiimide (COOH).

It should also be understood that the method and use of the present invention can be performed as an isolated test or it can be combined with any other suitable diagnostic test which may provide additional diagnostic or prognostic information. For example, and without limiting the application of the present invention in any way, the method and use of the present invention may be performed together with a technology such as CellSearch®, which efficiently and robustly identifies low frequencies of circulating tumour cells in peripheral blood. The method and use may also be applied as part of a treatment regime.

Without limiting the present invention to any one theory or mode of action, it has been observed that the screening of a neoplastic cell sample for La results in fixation of the antibody or other interactive molecule which is used. This provides a robust screening system. It should also be understood that one may seek to screen for La which has been localised to the cellular cytoplasm and/or which is associated with apoptotic bodies.

An interactive molecule directed to La may be used in the manufacture of a quantitative or semi-quantitative diagnostic kit to detect non-viable neoplastic cells in a biological sample. The kit may come with instructions for use and may be automated or semi-automated or in a form which is compatible with an automated machine or software.

The present invention is further described by reference to the following non-limiting examples.

### EXAMPLE 1

### IN VIVO TARGETTING OF THE RIBONUCLEOPROTEIN La IN A MOUSE TUMOUR MODEL

### Materials and Methods

### Materials

Cell culture media, RPMI-1640, DMEM and Ham's F12, and fetal calf serum (FCS) were all purchased from JRH Biosciences Inc. (KS, USA). Trypsin-EDTA solution, trypan blue, propidium iodide (PI), bovine serum albumin (BSA), hydrocortisone and staurosporine (STS) were obtained from Sigma-Aldrich Co. (MO, USA). Hybond-P membrane (PVDF), ECF™ substrate, L-[U-¹⁴C]Leucine, D-[U-¹⁴C]Glucose and Protein G purification columns were purchased from Amersham Biosciences, (NJ, USA). The miniPERM bioreactor was obtained from Vivascience (Hannover, Germany) and the BCA Protein Reagent Assay from Pierce Biotechnology Inc. (IL, USA). Solvable™ and UltimaGold™ were purchased from PerkinElmer Inc. (MA, USA). H₂O₂. The anti-poly(ADP-ribose) polymerase (PARP) monoclonal antibody (IgG1 mAb) clone C-2-10 was obtained from Oncogene™ Research Products (EMD Biosciences Inc., CA, USA). The anti-actin (N-20) affinity purified goat polyclonal antibody was purchased from Santa Cruz Biotechnology Inc. (CA, USA). Goat anti-mouse IgG alkaline phosphatase (AP)-conjugated antibody and rabbit anti-goat IgG AP-conjugated antibody were purchased from Johnson Laboratories (USA). The anti-La/SS-B IgG mAb 3B9 cell line (Tran *et al.,* 2002), prepared by Dr M. Bachmann (Oklahoma Medical Research Foundation, OK, USA), was a generous gift from Dr T.P. Gordon (Department of Immunology, Allergy, and Arthritis, Flinders Medical Centre, SA, Australia). The irrelevant 1D4.5 mAb Sal5 cell line, prepared by Dr L.K. Ashman (Medical Science Building, University of Newcastle, NSW, Australia), was a kind gift from Dr S. McColl (School of Molecular Biosciences, University of Adelaide, SA, Australia). These mAbs were affinity-purified on Protein G purification columns. Purified 3B9 and Sal5 mAbs were conjugated to fluorescein isothiocyanate (FITC) as described by manufacturer's instructions Sigma-Aldrich Co. (MO, USA). Anti-mouse IgG antibody conjugated to Alexa₄₈₈ and 7-AAD were purchased from Molecular Probes® (Invitrogen, USA). Lymphoprep™ was purchased from Axis-Shield PoC AS (Oslo, Norway). Etoposide and vincristine for injection (Pfizer Inc., NY, USA) and cyclophosphamide, cisplatin were purchased.

### Cell lines and culture

The tumour cell lines, Jurkat (ATCC# TIB-152, acute T cell leukemia), EL4 (ATCC# TIB-39, mouse T-lymphocyte lymphoma), U-937 (ATCC# CRL-1593.2, monocytic leukemia) and Raji (ATCC# CCL-86, Burkitt's lymphoma) were routinely grown as suspension cultures in RPMI-1640 containing 5 % FCS and passaged every 48-72 h at 1:4 dilution. The U2OS osteosarcoma cell line (ATCC# HTB-96), SAOS-2 osteosarcoma cell line (ATCC# HTB-85) and HeLa cervical adenocarcinoma (ATCC# CCL-2) were routinely cultured in DMEM containing 5 % FCS and passaged every 48 - 72 h after detachment using trypsin-EDTA solution. The squamous cell carcinoma cell line, SCC-25 (ATCC# CRL-1628), was cultured in a 1:1 mixture of DMEM and Ham's F12 medium containing supplemented with 400 ng/mL hydrocortisone and 10 % FCS and passaged after detachment using trypsin-EDTA solution. Fresh blood from normal volunteers was subjected to Lymphoprep® separation to isolate peripheral blood monocytic cells (PBMC). PBMC were cultured overnight in RPMI-1640 and 5 % FCS to separate suspension and adherent cells. The cells in suspension were defined as lymphocytes because more than 70% were CD3⁺ whereas the adherent cells were defined as monocytes because more than 70% were CD14⁺. Mouse thymocytes were obtained from mice. Finally, buccal cells were isolated from the gum lining of healthy volunteers as described.

Apoptosis in all cultures was induced by incubation of these cells in culture media described above and in the presence of specified concentrations of cytotoxic chemotherapy drugs (see Figure legends).

### Flow cytometry

Direct immunofluorescence staining was performed using 1-2 x 10⁵ cells at 10⁶ cells/mL for 30 min at room temperature in PBS containing 0.1% BSA and 5 µg/mL of FITC-conjugated mAb. Cells were thoroughly washed using PBS and centrifugation at 450xg. Cells were resuspended in PBS containing 0.5 µg/mL PI and acquired immediately by a Becton-Dickinson FACScan™ flow cytometry system (BD Biosciences, CA, USA). Positive staining using the mAb-FITC conjugates was determined in comparison to FITC-conjugated isotype control mAb detected using the FL-1 channel (530-nm filter). Indirect immunofluorescent staining was performed using purified mouse antibodies followed by anti-mouse IgG conjugated to Alexa₄₈₈ detected using the FL-1 channel (530-nm filter). Cell viability was assessed by the exclusion of PI detected using the FL-2 (585-nm filter) or the exclusion of 7-AAD detected using the FL-3 (> 650 nm filter). Flow cytometry data was analysed using WinMDI v 2.8 (Scripps Research Institute, CA, USA). Unless otherwise specified, no gating was performed in any of the analysis shown in this paper.

### SDS-polyacrylamide gel electrophoresis (SDS-PAGE) and Western blotting

SDS-PAGE was performed as per manufacturer instructions using the Hoefer® Mighty Small II SE 250 electrophoresis system (Amersham Biosciences, NJ, USA) under reducing condition using 12 % resolving polyacrylamide gel as per Laemmli (Nature 227: 680-685, 1970). Transfer of polyacrylamide gel onto Hybond-P membrane was carried out as per manufacturer instruction using the TE 22 Mini Tank Transfer Unit (Amersham Biosciences, NJ, USA). Blotting was performed as per standard procedure using 3B9 or anti-PARP mAbs followed by AP-conjugated anti-mouse IgG mAb or anti-actin polyclonal antibody (pAb) followed by AP-conjugated anti-goat IgG mAb. All blots were developed using the ECF™ substrate and scanned using the Fluorlmager™ 595 (Molecular Dynamics, Amersham Biosciences, NJ, USA) with 488 nm excitation laser and emission collected using 570 nm filter.

### Radioligand binding study with anti-La mAb

Sal5 isotype and 3B9 mAb were labelled with ¹⁴C by incubating the hybridoma cells (35 million cells) in 35 ml RPMI-1640 containing 10 % FCS in the production module of miniPERM bioreactor and 400 ml of RPMI-1640 containing 10 % FCS and 250 µCi of D-[U-¹⁴C]glucose and 250 µCi of L-[U-¹⁴C]Leucine in the nutrient module. The bioreactor was incubated in 5 % CO₂ humidified air at 37°C on a bottle-rotating device for 5 days. The medium in the production chamber was collected for antibody purification using protein G purification columns as per manufacturer instructions. Radioactivity of purified antibodies (10 µl sample) was counted in UltimaGold™ scintillation liquid (1 ml) for 20 min. using Tri-Carb 3100 β-counter (Packard, regularly calibrated using supplied ¹⁴C standards). Protein concentration was determined using BCA Protein Reagent Assay as per manufacturer instructions. The specific radioactivity of ¹⁴C-Sal5 and ¹⁴C-3B9 was 120.3 and 130.8 dpm/µg, respectively.

Saturation binding study was performed by incubating apoptotic EL4 cells (5 x 10⁵ cells) at 24 h after treatment with etoposide and cyclophosphamide with increasing concentration of ¹⁴C-3B9 in the absence (total) or presence (non specific) of 50-fold molar excess of unlabelled 3B9. After 30 min, cells were washed thoroughly using PBS and radioactivity was measured using the β-counter as described above. Specific binding was calculated as the difference between total and nonspecific binding and plotted as a function of concentration of ¹⁴C-3B9. Competition binding curve was constructed by incubating apoptotic EL4 cells with ¹⁴C-3B9 in the presence of increasing concentrations of unlabelled 3B9. Radioactivity was measured as described earlier and plotted as a function of unlabelled 3B9 concentration. Association time course was performed by incubation of apoptotic EL4 cells with ¹⁴C-3B9 in the absence or presence of 50-fold molar excess of unlabelled 3B9 for the specified times. Samples were washed and radioactivity was measure and specific binding was plotted as function of time.

### EL4 tumour model in C57BL/6 mice

EL4 cells, established from a lymphoma induced in a C57BL/6 mouse (Gorer, British Journal of Cancer 4: 372-379, 1950), were used to establish subcutaneous tumour implants in 6 - 8 weeks old C57BL/6 mice. Mice were housed and treated as per protocols approved by the Animal Ethics Committee at The University of Adelaide. Briefly, 10⁵ EL4 cells were injected subcutaneously in the right flank of each mouse. Once the tumour reached 1 cm diameter, mice were randomly divided into two groups one of which received intraperitoneal injection of etoposide and cyclophosphamide to achieve a dose of 76 mg/kg and 100 mg/kg, respectively (time 0). These two groups (untreated or treated) were used for the studied described below.

Treated mice received a second injection of etoposide and cyclophosphamide at 24 h after the first injection while untreated mice were left untreated. After 24 h (i.e. time point 48 h), all mice were euthanised, whole blood was obtained by cardiac puncture and EL4 tumours were excised from these sacrificed mice. Excised tumours tissue was disrupted to produce a single cell suspension, washed with PBS and used for immunofluorescent staining with 3B9 and PI and flow cytometry analysis as described earlier.

In other studies, treated mice that received the first chemotherapy injection at time 0, received a second injection of etoposide and cyclophosphamide at 24 h and an intravenous injection of specified amount of ¹⁴C-3B9 or ¹⁴C-Sal5. Untreated mice only received the intravenous injection of ¹⁴C-3B9 or ¹⁴C-Sal5. All mice were euthanased, whole blood was obtained by cardiac puncture and EL4 tumours as well as other organs were collected for radioactivity measurement. Serum and organs were solubilised using 1 ml of Solvable™ for 2 h at 50°C, decolourised using 100 µl of H₂O₂ (30 %). UltimaGold™ scintillation liquid (1 ml) was added and samples were counted for 10 min. using the β-counter.

### Results

### Anti-La mAb binds to apoptotic malignant EL4 thymic lymphoma cells in vitro

Cytofluographic analysis of cultured EL4 thymic lymphoblastic lymphoma cells, which were stained with the DNA binding dye 7-AAD as a test of membrane integrity, indicated that fewer than 10 % of the cultured cells were spontaneously apoptotic and did not bind La-specific 3B9 mAb (Figure 1A). In contrast, after EL4 cells were fixed and permeabilised, significantly more 3B9 bound than the isotype-matched control mAb, Sal5 (Figure 1B). This result indicated that specific binding of 3B9 to intracellular La depended on the loss of cell membrane integrity. After treatment of the cultured EL4 cells with the cytotoxic and DNA-damaging drugs, cyclophosphamide and etoposide, the majority of EL4 cells bound 7-AAD and, in contrast to the Sal5 control, virtually all 7-AAD⁺ cells bound 3B9 (upper outer quadrant, Figure 1C).

After cell permeabilisation, it was found that significantly higher levels of 3B9 bound to the malignant EL4 lymphoma cell line than to its normal murine counterpart cell type of the thymocyte (Figure 2A), which suggested that La was overexpressed in the malignant cells. This observation was confirmed by Western blot analysis using the 3B9 mAb to probe lysates of EL4 cells and thymocytes (Figure 2A - inset). Similarly, after cytotoxic drug treatment, which induced apoptosis of both EL4 cells and thymocytes, significantly higher levels of 3B9 bound to EL4 cells than to thymocytes (Figure 2A). Moreover, significantly more 3B9 bound EL4 cells after cytotoxic drug treatment than after cell permeabilisation, which suggested that cytotoxic drug treatment either induced higher levels of La expression and/or increased the availability of the 3B9 epitope on the La antigen (Figure 2A). In addition, treatment of the apoptotic EL4 and thymocytes with the nonionic detergent, Triton X-100, did not alter the fluorescence intensity of 3B9-mediated detection of the La antigen in EL4 cells (Figure 2B and data not shown). Detergent resistance of the La signal suggested that the La antigen may be covalently cross linked during apoptosis induction. Further support for this contention is given by the result shown in Figure 2C. Sulforhodamine B stains proteins indiscriminately and sulforhodamine staining of malignant EL4 cells, which were rendered apoptotic by cytotoxic drug treatment, was also resistant to detergent treatment (Figure 2C). Surprisingly, Figure 2D shows that protein-protein cross linking in apoptotic cells was not confined to the La antigen but also included the 3B9 mAb itself. EL4 cells and thymocytes that were induced to undergo apoptosis in the presence of 3B9 mAb showed significantly higher binding of the detection reagent, anti-mouse IgG Alexa₄₈₈-conjugated antibody, than those cells undergoing apoptosis in the presence of the Sal5 isotype mAb (data not shown). This result further supports the notion that 3B9 binds specifically to apoptotic cells and preferentially to malignant apoptotic cells. Treatment of the apoptotic cells with Triton X-100 after antibody incubation indicated that binding of the 3B9 mAb was detergent resistant and suggested that 3B9 was cross linked to other proteins within the apoptotic malignant cells (Figure 2D).

### Anti-La mAb binds specifically, efficiently and irreversibly to apoptotic EL4 cells in vitro

La-specific mAb, which was biosynthetically labelled with the radioisotope carbon-14 (¹⁴C-3B9), bound to apoptotic EL4 cells after they were treated with cyclophosphamide and etoposide in a specific and saturable manner. The concentration of agent required to reach half-maximal saturation of one million apoptotic cells was 18 nM and maximal binding was -7500 femtomole/million apoptotic cells (Figure 3A). Half-maximal saturation of ¹⁴C-3B9 binding was found to occur within 5 minutes at room temperature (Figure 3B). Specific binding was largely irreversible as bound antibody did not dissociate when cells were incubated in binding buffer for 30 minutes at 37°C (Figure 3C). The concentration of unlabelled 3B9 required to inhibit half-maximal binding of ¹⁴C-3B9 (IC₅₀) was estimated to be 28 nM (Figure 3D). Altogether, these data describe specific, rapid, irreversible and high affinity binding of ¹⁴C-3B9 to apoptotic tumour cells. Binding was not detected when EL4 cells were tested in the absence of cytotoxic drugs (data not shown), which indicates that binding depends on apoptosis induction. It should be noted that these binding parameters relate to the binding of ¹⁴C-3B9 to apoptotic cells rather than the binding of antibody to the La antigen itself.

### Cytotoxic chemotherapy increases the target for 3B9 in the tumour mass

Single cell suspensions were prepared from tumour explants of EL4 tumour-bearing mice, which had been treated or not with cytotoxic chemotherapy. As illustrated in Figure 4, only the PI⁺ subpopulation of tumour cells displayed binding with the 3B9-FITC conjugate, which indicated that the La antigen had been recognised specifically in dead tumour cells. After the use of cytotoxic chemotherapy, the fraction of PI⁺ cells in the tumour explants increased significantly from 50 ± 2% to 70 ± 1% (*P*<0.0001) and, similarly, the 3B9⁺ subset of PI⁺ cells increased significantly from 15 ± 1 % to 28 ± 2% (*P*<0.01). In order to describe the effect of chemotherapy on 3B9 targeting to La, we analysed the frequency distributions of PI⁺ cells, which bound FITC conjugates and which are defined in the upper right quadrant of each density plot. As illustrated in the representative histograms in Figure 4, the specific binding of 3B9-FITC to PI⁺ cells was significantly augmented in tumours exposed *in vivo* to cytotoxic chemotherapy. For all tumour explant samples, the net MFI for La-specific staining (after subtraction of individual MFI values for Sal5 staining) was 18 ± 3 with chemotherapy and 1 ± 3 without chemotherapy (*P*<0.05). These results provide *in vivo* evidence of increased La expression and/or increased availability of the 3B9 epitope in dead cells obtained from tumours that were exposed to cytotoxic chemotherapy.

### La-specific mAb targets EL4 tumours in vivo especially after cytotoxic chemotherapy

EL4 tumour-bearing mice were used to demonstrate that 3B9 La-specific mAb targeted a tumour mass *in vivo* and that the 3B9 targeting was enhanced 48 hours after the mice were treated with cytotoxic chemotherapy. As illustrated graphically in Figure 5, 100µg ¹⁴C-labelled isotype control mAb (Sal5) did not accumulate significantly in any organ or tissue including the tumour. In contrast and compared with all other organs, 100µg ¹⁴C-3B9 accumulated significantly in serum and the tumour both before and after chemotherapy (*P*<0.001). Moreover, after the mice were treated with cytotoxic chemotherapy, only tumours accumulated significantly more ¹⁴C-3B9 (*P*<0.001) (Figure 5).

The biodistribution studies of ¹⁴C-3B9 in EL4 tumour-bearing mice were extended to include lower doses of the radiolabelled agent. Again, as shown in Figure 6, there was no significant accumulation of ¹⁴C-3B9 in any tissue except the tumour and only after administration of cytotoxic chemotherapy. The intra-tumoural accumulation of ¹⁴C-3B9 was dose-dependent and the sigmoidal dose-response relationship suggested that binding of the target was specific and saturable. In comparison to untreated mice, tumour uptake of ¹⁴C-3B9 with chemotherapy was significantly higher and demonstrated fold increases of 1.9, 1.9 and 1.8 at ¹⁴C-3B9 doses of 25 µg, 50 µg and 100 µg, respectively. Irrespective of the use of chemotherapy, no significant difference in the tumour uptake of ¹⁴C-3B9 was observed at a 5 µg dose. Although a 25µg dose of ¹⁴C-3B9 produced significant intra-tumoural accumulation 48 hours after administration of cytotoxic chemotherapy at an average 20 ± 3% of injected dose, the same dose of ¹⁴C-3B9 did not accumulate significantly in bone marrow, which received less than 3 ± 2% of the injected dose. These data further support the concept that La-specific 3B9 mAb selectively targets malignant tissues rather than critical normal tissues such as bone marrow, which is particularly susceptible to apoptosis after cytotoxic drug administration. Together with the previous data showing enhanced expression of La in EL4 tumour cells after *in vivo* use of cytotoxic chemotherapy (Figure 4), these data strengthen the contention that enhanced *in vivo* tumour targeting of 3B9 results from increased levels of cell death caused by cytotoxic chemotherapy.

### Anti-La as α potential tumour targeting agent for human malignancies

Using a murine tumour model, La-specific *in vitro* labelling of dead malignant cells and La-specific *in vivo* targeting of malignant tumours, which were both significantly augmented by the use of cytotoxic chemotherapy, have been described. Hence, it was sought to determine if similar findings applied to human malignant cells *in vitro.* First, it was found that in comparison to the counterpart normal cell type, La was overexpressed in the Jurkat T cell leukemia line (*cf.* CD3⁺ lymphocytes), the U-937 monocytic leukemia line (*cf* CD14⁺ monocytes) and the oropharyngeal squamous cell carcinoma line SSC-25 (*cf* normal buccal mucosal cells) (Figure 7). La overexpression was also found in the osteosarcoma lines, U2OS and SAOS-2 (*cf* normal bone marrow stromal cells) and in the A549 bronchogenic carcinoma line (*cf.* normal human bronchial epithelium) (data not shown). Second, as shown earlier for EL4 tumour cells, cytofluographic analysis of human cancer cell lines showed that 3B9-FITC binding was evident only after treatment of these cells with cytotoxic drugs (Table 3). Third, La overexpression in malignant cells, which were rendered apoptotic by cytotoxic drug treatment, was resistant to detergent treatment (Figure 8A). Finally, as shown previously for EL4 cells and in contrast to normal primary cell counterparts, the detergent-resistant binding of 3B9 mAb to apoptotic human malignant cell lines was significantly higher as demonstrated by flow cytometry (Figure 8B) and laser scanning confocal microscopy (Figure 8C). These data suggested that 3B9 may be suitable for the *in vivo* targeting of dead cells within human tumours after cytotoxic chemotherapy and/or radiotherapy. For diagnostic purposes, 3B9 could be conjugated to radio-imaging agents and conjugation of 3B9 to cytotoxic radionuclides may provide a therapeutically useful means for delivery of bystander killing to surrounding viable malignant and supporting tissues.

**TABLE 3**

| **Cell line** | **Staurosporine** | **Etoposide** | **Vincristine** |
|---|---|---|---|
| **Jurkat** | 56.1% | 22.6% | 12.1% |
| **Raji** | 28.8% | 21.3% | 9.8% |
| **HeLa** | 62.5% | 36.1% | 18.6% |
| **U2OS** | 65.9% | 28.0% | ND |

**Table 3: Binding of La-specific mAb to apoptotic human cancer cell lines.** Cell lines were cultured in the presence of 0.5 µM staurosporine (STS) (2 µM for Raji cell line), 20 µg/mL etoposide or 0.1 µg/mL vincristine. Cells were stained with Sal5-FITC or 3B9-FITC and analysed by dual-colour flow cytometry using PI for viability determination. After apoptosis induction, data shown are the percentages of PI⁺ and 3B9⁺ events after subtraction of the corresponding percentage of control (PI⁺ and Sal5⁺) events. ND, not determined.

### EXAMPLE 2

### ANALYSIS OF GENE EXPRESSION PROFILING DATA TO IDENTIFY SUITABLE TARGETS FOR imaging and TCS

### Introduction

Oncomine™ is a cancer-specific database containing microarray data from 962 studies of which 209 were analysed. The database contains 14,177 microarrays from 35 cancer types (information publicly available at the website www.oncomine.org). Several cancer signatures have been deduced from large scale analysis of data held in the database (Hampton, Jama 292(17): 2073, 2004; Rhodes et al., Proc Natl Acad Sci USA 101(25): 9309-14, 2004a; Rhodes et al., Neoplasia 6(1): 1-6, 2004b; Rhodes and Chinnaiyan, Nat Genet 37 Suppl: S31-7, 2005; Rhodes et al., Nat Genet 37(6): 579-83, 2005). 209 studies in the database as described below were analysed in order to investigate certain malignancy signatures, which may provide useful targets for the present invention

### Method

In the catalogue of the database Oncomine™ on www.oncomine.org, the tissue of interest was selected and only analysed data are shown. Studies were viewed using the Advanced Analysis module only for overexpressed genes and enrichment for these genes was achieved using the following options: (1) InterPro for analysis of motifs, (2) Gene Ontogeny (GO) molecular function for the analysis of function and (3) GO cellular component for cellular compartmentation analysis. Two parameters were used to describe the gene sets deduced from the above analysis: (a) Odds Ratio and (b) P-value. Only the groups of interest and genes with a P-value lower than 1E-4 were considered significant for reporting herein.

### Results

As shown in Table 4 and Table 5, the ribonucleoprotein (RNP-1) motif was identified , which comprises the RNA recognition motif (RRM), to be at higher odds of being associated with a cancer signature compared to the nucleus as a cellular component. Similarly, the RNA binding gene set, which was enriched as a cellular function, was also at high odds of being associated with a cancer signature. Finally and more importantly, nucleolar and heteronuclear ribonucleoprotein (hnRNP) components appeared to have very high odds as gene sets in association with a cancer signature.

### Conclusion

Consequently, components with the RNP-1 motif generally and hnRNP complex specifically are suitable targets for the method of the present invention. This recommendation is justified by (1) association of hnRNP with cancer at the gene expression level (mRNA data reported herein), (2) the relative abundance of this protein and its correlation with cancer and cancer progression (see review Carpenter et al., Biochimica et Biophysica Acta 1765(2): 85-100, 2006), and (3) the reorganisation of the hnRNP network during apoptosis into the HERDS (Biggiogera et al., Biologie Cellulaire 96(8): 603-15. 2004) and the accessibility of the hnRNP network for detection with antibodies. Nucleolar proteins generally and specifically nucleophosmin represent suitable targets for this strategy. This is justified by the data shown in the analysis of the Oncomine™ data base as well as the literature regarding nucleolar function in cancer (Maggi and Weber, Cancer Investigation 23(7): 599-608, 2005) and, in particular, nucleophosmin as putative-proto-oncogene (Grisendi et al., Nature Reviews. Cancer 6(7): 493-505, 2006).

**Table 4: mRNA expression in clinical tumors. La mRNA is upregulated in malignancy**

| **Tissue type** | **No. genes** | **Comparison** | **% of overexpressed genes** | **Enrichment type** | **Group** | **Odds Ratio** | **P-value** |
|---|---|---|---|---|---|---|---|
| Adrenal gland (Giordano *et al.,* 2003) | 12625 | Adrenocortical carcinoma *vs*. normal adrenal cortex, adrenocortical adenoma and macronodular hyperplasia | 2.3% | InterPro motif | **RNP-1 motif** | **2.98** | **7.8E-4** |
| | | | | | e.g. hnRNP A1/B2 | | 9E-4 |
| | | | | Cellular function | **RNA binding** | **2.06** | **4.4E-5** |
| | | | | Cellular component | **Nucleus** | **1.93** | **7.8E-23** |
| | | | | | **hnRNP complex** | **11.72** | **3.9E-4** |
| Brain (Phillips *et al.,* 2006) | 44792 | grade IV vs. grade III astrocytoma | 9.6% | InterPro motif | **RNP-1 motif** | **2.25** | **1.7E-5** |
| | | | | | e.g. La/SS-B | | 1.5E-10 |
| | | | | | hnRNP H1 | | 1.0E-8 |
| | | | | Cellular function | **RNA binding** | **1.78** | **9.6E-8** |
| | | | | Cellular component | **Nucleus** | **1.53** | **7.4E-17** |
| | | | | | **hnRNP complex** | **11.38** | **2.5E-5** |
| | | | | | **Nucleolus** | **4.38** | **6E-4** |
| | | | | | e.g. nucleolin | | 5.7E-5 |
| Brain (Phillips *et al.,* 2006, *supra*) | 44792 | dead vs. alive in astrocytoma 5-year survival | 1.0% | InterPro motif | **RNP-1 motif** | **1.75** | **3.8E-4** |
| | | | | | e.g. La/SS-B | | 3.3E-5 |
| | | | | | hnRNP H1 | | 3.7E-4 |
| | | | | Cellular function | **RNA binding** | **1.81** | **5E-8** |
| | | | | Cellular component | **Nucleus** | **1.75** | **1E-27** |
| | | | | | **hnRNP complex** | **7.36** | **3.4E-4** |
| Brain (Khatua *et al*., 2003) | 12625 | High grade *vs*. low grade astrocytoma | 1.3% | InterPro motif | **RNP-1 motif** | **2.59** | **1.6E-6** |
| | | | | | e.g. hnRNP A/B | | 2.3E-4 |
| | | | | Cellular function | **RNA binding** | **2.8** | **1.7E-14** |
| | | | | Cellular component | **Nucleus** | **1.88** | **2.2E-21** |
| | | | | | **hnRNP complex** | **17.47** | **2.4E-7** |
| | | | | | **Nucleolus** | **4.03** | **2.2E-4** |
| Brain (Shai *et al.,* 2003) | 12625 | Glioblastoma Multiforme vs. Normal white matter | 17% | InterPro motif | **RNP-1 motif** | **2.29** | **3.3E-5** |
| | | | | | e.g. hnRNP A0 | | 6.6E- 10 |
| | | | | Cellular function | **RNA binding** | **2.43** | **7E-11** |
| | | | | Cellular component | **Nucleus** | **1.47** | **7.5E-9** |
| | | | | | e.g. PCNA | | 8.9E-7 |
| | | | | | **hnRNP complex** | **6.75** | **3.6E-4** |
| | | | | | **Nucleolus** | **4.38** | **6E-4** |
| | | | | | e.g. nucleolin | | 5.7E-5 |
| Brain (Liang *et al.,* 2005) | 23079 | Dead vs. alive glioblastoma multiforme 1 year survival | 0.2% | InterPro motif | **RNP-1 motif** | **2.70** | **1.2E-8** |
| | | | | | e.g. hnRNP A3 | | 2.5E-4 |
| | | | | Cellular function | **RNA binding** | **2.38** | **5.7E-12** |
| | | | | Cellular component | **Nucleus** | **1.75** | **4.8E-12** |
| | | | | | **Nucleolus** | **3.3** | **9.6E-4** |
| Brain (Rickman *et al.,* 2001) | 6668 | Glioma vs. normal neocortex of temporal lobe | 7% | InterPro motif | **RNP-1 motif** | **2.64** | **9.9E-5** |
| | | | | | e.g. hnRNP H1 | | 1.2E-4 |
| | | | | Cellular function | **RNA binding** | **3.13** | **1.1E-12** |
| | | | | Cellular component | **hnRNP complex** | **19.64** | **5.6E-6** |
| Breast (Wang *et at.,* 2005) | 22283 | Relapse vs. no disease in breast carcinoma - 5-year disease free survival | 1.1% | InterPro motif | **RNP-1 motif** | **2.08** | **2E-5** |
| | | | | | e.g. hnRNP A1 | | 3.7E-4 |
| | | | | Cellular function | **RNA binding** | **1.66** | **3E-5** |
| | | | | Cellular component | **Nucleus** | **1.44** | **1.1E-10** |
| Breast (Wang *et al.,* 2005, *supra*) | 22283 | Relapse vs. no disease in ER⁺ breast carcinoma; 5-year disease free survival | 0.8% | InterPro motif | **RNP-1 motif** | **2.51** | **4.7E-8** |
| | | | | Cellular function | **RNA binding** | **1.86** | **2.4E-7** |
| | | | | Cellular component | **Nucleus** | 1.5 | **6.4E-13** |
| Breast (Dairkee *et al.,* 2004) | 10761 | Breast cancer vs. Breast Cancer Culture and Immortalized Breast Cell-Line | 8.7% | InterPro motif | **RNP-1 motif** | **3.54** | **3.2E-8** |
| | | | | | e.g. hnRNP H1 | | 5.7E-6 |
| | | | | | hnRNPD | | 2.2E-5 |
| | | | | Cellular function | **RNA binding** | **2.46** | **2.4E-7** |
| | | | | Cellular component | **Nucleus** | **1.66** | **4.8E-11** |
| Mammary epithelial cells - oncogene trans fected (Bild *et al.,* 2006) | 54675 | Activated H-Ras vs. GFP-transfected primary cells | 16.1% | InterPro motif | **RNP-1 motif** | **2.21** | **1.5E-7** |
| | | | | | e.g.hnRNPA/B | | 2.6E-11 |
| | | | | | Nucleolin | | 3.2E-9 |
| | | | | | La/SS-B | | 5.1 E-5 |
| | | | | Cellular function | **RNA binding** | **2.08** | **3E-12** |
| | | | | Cellular component | **Nucleolus** | **4.2** | **3.4E-5** |
| | | | | | e.g. nucleophosmin | | 6.5E-7 |
| Mammary epithelial cells - oncogene trans fected (Bild *et al.,* 2006, *supra*) | 54675 | c-Myc *vs*. GFP transfected primary cells | 3.7% | InterPro motif | **RNP-1 motif** | **2.5** | **7.6E-7** |
| | | | | | e.g. Nucleolin | | 2.8E-11 |
| | | | | | **hnRNP** A/B | | 9.8E-7 |
| | | | | | La/SS-B | | 8.5E-6 |
| | | | | Cellular function | **RNA binding** | **3.13** | **1.2E-20** |
| | | | | Cellular component | **Nucleolus** | **12.57** | **9.3E-13** |
| | | | | | e.g. nucleophosmin | | 7.2E-6 |
| Colon (Notterman *et al.,* 2001) | 6745 | Colon adenocarcinoma *vs*. normal colon | 7.2% | InterPro motif | **RNP-1 motif** | **4.11** | **1.2E-6** |
| | | | | | e.g. hnRNP F | | 2.2E-8 |
| | | | | | **hnRNP** D | | 5.7E-5 |
| | | | | | La/SS-B | | 6.7E-5 |
| | | | | Cellular function | **RNA binding** | **3.92** | **2E-16** |
| | | | | Cellular component | **Nucleus** | **1.66** | **4.6E-8** |
| | | | | | e.g. PCNA | | 1.1E-9 |
| | | | | | **hnRNP complex** | **20.15** | **4.8E-6** |
| | | | | | **Nucleolus** | **7.62** | **3.5E-5** |
| | | | | | e.g. nucleophosmin | | 9.2E-12 |
| Colon (Alon *et al.,* 1999) | 1988 | Colon adenocarcinoma *vs*. normal colon | 9.7% | InterPro motif | **RNP-1 motif** | **4.99** | **1.3E-5** |
| | | | | | e.g. hnRNP F | | 2.9E-4 |
| | | | | Cellular function | **RNA binding** | **5.79** | **2.6E-13** |
| | | | | Cellular component | **Nucleus** | **1.73** | **2.4E-4** |
| Liver (Chen *et al.,* 2002) | 22033 | Metastasis to liver vs. hepatocellular carcinoma | 6.2% | InterPro motif | **RNP-1 motif** | **2.33** | **1.2E-6** |
| | | | | | e.g. hnRNP A3 | | 1.2E-5 |
| | | | | Cellular function | **RNA binding** | **1.91** | **2.5E-7** |
| | | | | Cellular component | **Nucleus** | **1.38** | **1.7E-7** |
| Lung (Bhattacharjee *et al.,* 2001) | 11158 | Lung adenocarcinoma *vs*. normal lung | 31.1% | InterPro motif | **RNP-1 motif** | **2.72** | **6.3E-7** |
| | | | | | e.g. hnRNP C1/C2 | | 9.5E-7 |
| | | | | | **hnRNP** A3 | | 1.6E-6 |
| | | | | | La/SS-B | | 1.3E-5 |
| | | | | Cellular function | **RNA binding** | **3.1** | **7.4E-17** |
| | | | | Cellular component | **Nucleus** | **1.31** | **6.8E-5** |
| | | | | | e.g. PARP-1 | | 3.5E-9 |
| Lung (Bhattacharjee *et al.,* 2001, *supra*) | 10881 | Small cell lung cancer vs. normal lung | 26.6% | InterPro motif | **RNP-1 motif** | **3.82** | **8.2E-12** |
| | | | | | e.g. hnRNP D | | 1E-4 |
| | | | | Cellular function | **RNA binding** | **3.04** | **8.3E-16** |
| | | | | Cellular component | **Nucleus** | **2.19** | **1.3E-29** |
| | | | | | e.g. PCNA | | 3.6E-6 |
| | | | | | PARP-1 | | 4.4E-6 |
| Lung (Bhattacharjee *et al.,* 2001, *supra*) | 10030 | Squamous cell lung carcinoma vs. normal lung | 7.1% | InterPro motif | **RNP-1 motif** | **2.39** | **2.7E-5** |
| | | | | | e.g. hnRNP A3 | | 8.5E-7 |
| | | | | Cellular function | **RNA binding** | **2.72** | **9.2E-13** |
| | | | | Cellular component | **Nucleus** | **1.48** | **3.4E-8** |
| | | | | | e.g. PARP-1 | | 5.7E-8 |
| | | | | | PCNA | | 7.7E-8 |
| | | | | | **hnRNP complex** | **6.27** | **8.1E-4** |
| Lymph (Hummel *et al.,* 2006) | 22215 | Ig-Myc fusion vs. translocation negative lymphoma | 27.9% | InterPro motif | **RNP-1 motif** | **3.94** | **2.2E-17** |
| | | | | | e.g. hnRNP A3 | | 4.4E-20 |
| | | | | | La/SS-B | | 5.6E-13 |
| | | | | | hnRNP A2/B1 | | 6.3E-11 |
| | | | | Cellular function | **RNA binding** | **4.11** | **5.9E-37** |
| | | | | Cellular component | **Nucleus** | **1.71** | **2.1E-21** |
| | | | | | **hnRNP complex** | **15.22** | **3.1E-6** |
| | | | | | **Nucleolus** | **8.68** | **4.9E-11** |
| | | | | | e.g. nucleophosmin | | 4.7E-28 |
| Lymph (Hummel *et al.,* 2006, *supra*) | 22215 | Definite Burkitt's lymphoma *vs*. not definite | 27.9% | InterPro motif | **RNP-1 motif** | **3.47** | **2.7E-14** |
| | | | | | e.g. La/SS-B | | 1.7E-9 |
| | | | | | **hnRNP** A3 | | 1.3E-6 |
| | | | | | Nucleolin | | 1.9E-4 |
| | | | | Cellular function | **RNA binding** | **4.31** | **5.6E-29** |
| | | | | Cellular component | **Nucleus** | **1.71** | **2.1E-21** |
| | | | | | **hnRNP complex** | **9.22** | **1.6E-4** |
| | | | | | **Nucleolus** | **3.43** | **2E-4** |
| | | | | | e.g. nucleophosmin | | 2.1E-5 |
| Mesothelioma (Gordon *et al.,* 2005) | 22215 | Malignant mesothelioma *vs*. pleura | 2.6% | InterPro motif | **RNP-1 motif** | **2.02** | **3.4E-5** |
| | | | | | e.g. hnRNP A/B | | 1E-5 |
| | | | | | **hnRNP** H1 | | 5.1E-4 |
| | | | | Cellular function | **RNA binding** | **1.81** | **5.3E-7** |
| | | | | Cellular component | **Nucleus** | **1.47** | **1.3E-7** |
| | | | | | **hnRNP complex** | **10.16** | **4.2E-5** |
| Ovary (Schaner *et al.,* 2003) | 21057 | Undifferentiated vs. clear cell, endometrioid and Serous Papillary histological subtypes of ovarian carcinoma | 1.3% | InterPro motif | **RNP-1 motif** | **3.16** | **3.6E-8** |
| | | | | Cellular function | **RNA binding** | **2.07** | **3.8E-6** |
| | | | | Cellular component | **hnRNP complex** | **9.12** | **4.1E-4** |
| Prostate (Schaner *et al.,* 2003, *supra*) | 54675 | Prostate carcinoma vs. normal prostate | 1.3% | InterPro motif | **RNP-1 motif** | **2.06** | **2.2E-6** |
| | | | | | e.g. hnRNP C | | 1.5E-4 |
| | | | | Cellular function | **RNA binding** | **1.66** | **2.7E-6** |
| | | | | Cellular component | **Nucleolus** | **4.03** | **5.9E-5** |
| | | | | | e.g. nucleophosmin | | 2.4E-4 |
| | | | | | **hnRNP complex** | **9.61** | **6.3E-5** |
| Prostate (Vanaja *et al*., 2006) | 44928 | Metastatic *vs*. primary vs. benign prostate | 2.4% | InterPro motif | **RNP-1 motif** | **2.22** | **2.2E-5** |
| | | | | | e.g. hnRNP C | | 5.6E-4 |
| | | | | Cellular function | **RNA binding** | **3.4** | **5.8E-15** |
| | | | | Cellular component | **Nucleus** | **1.39** | **2.6E-9** |
| | | | | | **hnRNP complex** | **11.51** | **2.3E-5** |
| | | | | | **Nucleolus** | **7.84** | **7.2E-8** |
| | | | | | e.g. nucleophosmin | | 1.2E-5 |
| Prostate (Singh *et al.,* 2002) | 9892 | Prostate tumour vs. non-tumour prostate | 4.9% | InterPro motif | **RNP-1 motif** | **3.08** | **1.9E-8** |
| | | | | | e.g. hnRNP A0 | | 3.2E-4 |
| | | | | | La/SS-B | | 9.5E-4 |
| | | | | Cellular function | **RNA binding** | **3.43** | **1.5E-19** |
| | | | | Cellular coponent | **hnRNP complex** | **10.52** | **1.2E-5** |
| Prostate (Vanaja *et al*., 2006, *supra*) | 44928 | Metastatic vs. primary *vs*. benign prostate | 2.4% | InterPro motif | **RNP-1 motif** | **2.22** | **2.2E-5** |
| | | | | | e.g. hnRNP C | | 5.6E-4 |
| | | | | Cellular function | **RNA binding** | **3.4** | **5.8E-15** |
| | | | | Cellular component | **Nucleus** | **1.39** | **2.6E-9** |
| | | | | | **hnRNP complex** | **11.51** | **2.3E-5** |
| | | | | | **Nucleolus** | **7.84** | **7.2E-8** |
| | | | | | e.g. nucleophosmin | | 1.2E-5 |
| Salivary gland (Frierson *et al,* 2002) | 10142 | Adenoid Cystic Carcinoma of Salivary Gland vs. Normal Salivary Gland | 13.5% | InterPro motif | **RNP-1 motif** | **4.96** | **9.7E-17** |
| | | | | | e.g. hnRNP H3 | | 6.6E-9 |
| | | | | | hnRNP A2/B1 | | 2.0E-4 |
| | | | | | ......La/SS-B | | 6.4E-4 |
| | | | | Cellular function | **RNA binding** | **3.01** | **3.7E-15** |
| | | | | Cellular component | **Nucleus** | **2.05** | **3.4E-24** |
| | | | | | **hnRNP complex** | **8.76** | **5.1E-4** |

**TABLE 5**

| **Tissue type (reference)** | **No. genes in study** | **Comparison** | **% of over-expressed genes** | **P-value for La mRNA over-expression** |
|---|---|---|---|---|
| Lymphoma (23) | 22215 | IgG-Myc fusion *vs*. fusion-negative lymphoma | 27.9% | **1.1E-12** |
| Brain (11) | 44792 | grade IV vs. grade III astrocytoma | 9.6% | **2.9E-10** |
| Lymphoma (23) | 22215 | Definite Burkitts Lymphoma *vs*. not definite | 27.9% | **3.4E-9** |
| Seminoma (29) | 44760 | Adult male germ cell tumor *vs*. normal testis | 40.1% | **4.7E-7** |
| Mammary epithelial cells - oncogene transfected (18) | 54675 | Activated H-ras vs GFP transfected primary cells | 16.1% | **2E-6** |
| Brain (11) | 44792 | dead vs. alive in astrocytoma 5 year survival | 1.0% | **6.5E-5** |
| Lung (22) | 11158 | Lung adenocarcinoma *vs*. normal lung | 31.1% | **2.6E-5** |
| Mammary epithelial cells - oncogene transfected (18) | 54675 | c-Myc vs GFP transfected primary cells | 3.7% | **2E-5** |
| Multiple cancers (30) | 15708 | Progression of primary cancer *vs*. normal tissue | 11.1% | **5E-4** |
| Lung (31) | 22646 | Lung adenocarcinoma *vs*. normal Lung | 8.6% | **1.6E-4** |
| Colon (19) | 6745 | Colon adenocarcinoma *vs*. normal colon | 7.2% | **1.3E-4** |
| Tongue (32) | 12558 | Tongue squamous cell carcinoma *vs*. normal tongue | 23.5% | **1.3E-4** |
| Multiple cancers (30) | 14252 | Progression of metastatic cancer vs. primary cancer | 4.8% | **1.2E-4** |
| Oral (33) | 14119 | Oral squamous cell carcinoma *vs*. oral squamous epithelium | 4.2% | **1.2E-4** |
| Brain (34) | 44692 | Grade V *vs*. Grade III glioma | 13.4% | **0.001** |
| Head-Neck (35) | 22215 | Head and neck squamous cell carcinoma vs. normal oral Mucosa | 21.4% | **0.001** |
| Melanoma | 22283 | Melanoma vs. normal skin and nevus | 30.5% | **0.001** |
| Ovary (36) | 4995 | Ovarian adenocarcinoma *vs*. normal ovary | 11.3% | **0.001** |
| Lung (31) | 22646 | squamous cell carcinoma *vs*. normal lung | 8.6% | **0.002** |
| Prostate (27) | 9892 | Prostate tumor vs. non-tumor prostate | 4.9% | **0.002** |
| Lung (22) | 12241 | Metastatic *vs*. primary adenocarcinoma | 48.8% | **0.003** |
| Ovarian (37) | 63174 | Serous ovarian carcinoma *vs*. ovarian surface epithelium | 2.3% | **0.003** |
| Brain (11) | 44792 | Grade IV necrosis positive vs. negative astrocytoma | 0.6% | **0.004** |
| Brain (13) | 12625 | Glioblastoma multiforme *vs*. normal white matter | 17% | **0.006** |
| Lung (22) | 10030 | squamous cell lung carcinoma vs. normal lung | 7.1% | **0.006** |
| Liver (21) | 22033 | Metastasis to liver vs. hepatocellular carcinoma | 6.2% | **0.007** |
| Lung (22) | 10881 | Small cell lung cancer vs. normal lung | 26.6% | **0.014** |
| Breast (16) | 22283 | Relapse vs. no disease in 5-year disease free survival study in breast carcinoma | 1.1% | **0.016** |
| Cell line (38) | 22215 | Camptothecin-treated HeLa cells vs. non-treated | 0.9% | **0.024** |
| Brain (34) | 44692 | Dead vs. alive in 3-year survival study of glioma | 5.9% | **0.025** |
| Breast (39) | 44611 | Alive *vs*. Dead in 5-year survival study of breast carcinoma | 0.6% | **0.036** |
| Breast (40) | 7937 | Breast carcinoma vs. benign breast | 4.6% | **0.037** |
| Prostate (41) | 19111 | Lymph node metastasis vs. prostate cancer | 3.9% | **0.049** |

### EXAMPLE 3

### IN VITRO RATIONALE FOR TARGETTING OF THE RIBONUCLEOPROTEIN La IN A MOUSE TUMOUR MODEL

### Materials and Methods

### Materials

The suppliers of the materials are identified in brackets after each material. Cell culture media, RPMI-1640, DMEM and Ham's F12, and fetal calf serum (FCS) (JRH Biosciences Inc., Lenexa, KS); Trypsin-EDTA solution, trypan blue, propidium iodide (PI), bovine serum albumin (BSA), BCIP/NBT premixed substrate solution for alkaline phophatase (AP), hydrocortisone, monodansylcadaverine (MDC) and staurosporine (STS) and mouse anti-human β-tubulin mAb (TUB 2.1) (Sigma-Aldrich Co., St. Louis, MO). Hybond-P membrane (PVDF) and protein G purification columns (Amersham Biosciences, Piscataway, NJ). BCA Protein Reagent Assay (Pierce Biotechnology Inc., Rockford, IL). Anti-poly(ADP-ribose) polymerase (PARP) monoclonal antibody (mAb) clone C-2-10 and anti-proliferating cell nuclear antigen (PCNA) mAb clone PC10 (Oncogene Research Products, Cambridge, MA). Trichostatin A (TSA), anti-phospho-histone H2AX (Ser139) clone JBW301 biotin-conjugated mAb and anti-human H2A polyclonal antibody (Millipore Inc., Billerica, MA). Anti-β fodrin mAb (Chemicon International, Temecula, CA). Anti-β actin affinity-purified rabbit polyclonal antibody, Fluorescein isothiocyanate (FITC)-conjugated goat anti-rabbit IgG and AP-conjugated goat anti-rabbit IgG antibodies (Rockland, Gilbertsville, PA). The anti-La/SS-B 3B9 mAb hybridoma is a murine IgG₂ₐ autoantibody, which is crossreactive with human La and which was prepared by Dr M. Bachmann (Oklahoma Medical Research Foundation, OK, USA), was a kind gift from Dr T.P. Gordon (Department of Immunology, Allergy and Arthritis, Flinders Medical Centre, SA, Australia). The isotype control Sal5 (1D4.5) mAb hybridoma, prepared by Dr L.K. Ashman (Medical Science Building, University of Newcastle, NSW, Australia), was kindly supplied by Dr S. McColl (School of Molecular Biosciences, University of Adelaide, SA, Australia). The mAb were affinity-purified using protein G columns and FITC-conjugates were prepared according to the manufacturer's instructions (Sigma-Aldrich Co., St. Louis, MO). Anti-human nucleolin mAb, anti-human nucleophosmin (NPM) mAb, anti-mouse IgG antibody conjugated to Alexa₄₈₈, streptavidin-Alexa₄₈₈, streptavidin-PE, 7-amino-actinomycin D (7-AAD) and biotinylated cadaverine (Invitrogen, Carlsbad, CA). Lymphoprep (Axis-Shield PoC AS, Norton, MA). Etoposide (Pfizer Inc., NY, USA), cyclophosphamide and cisplatin (Bristol-Myers Squibb Company, Princeton, NJ) and gemcitabine (Eli Lilly, Indianapolis, IN) were obtained from the Royal Adelaide Hospital Cytotoxics Pharmacy (Adelaide, SA, Australia).

### Cell culture

Suspension cultures of Jurkat and U-937 leukemia cell lines were maintained in RPMI-1640 containing 5% FCS and passaged by splitting at 1:10 every 72h. Cultures of adherent MDA-MB-231, MCF-7, PC-3, LNCaP and A549 cancer cell lines were maintained in RPMI-1640 containing 5% FCS and passaged every 48-72h at a 1:4 dilution after detachment with trypsin-EDTA solution. Adherent sub-confluent cultures of the pancreatic adenocarcinoma cell line, PANC-1, were routinely cultured in RPMI-1640 containing 10% FCS and passaged as above. The squamous cell carcinoma cell line, SCC-25, was cultured in a 1:1 mixture of DMEM and Ham's F12 medium supplemented with 400ng/mL hydrocortisone and 5% FCS and passaged after detachment using trypsin-EDTA solution. Peripheral blood mononuclear cells (PBMC) were isolated from fresh heparinized blood obtained from normal healthy volunteer donors using Lymphoprep separation and cultured overnight in RPMI-1640 containing 5% FCS to separate adherent cells from those remaining in suspension. Cytofluographic analysis indicated that >70% of both suspension and adherent cells were CD3⁺ and CD14⁺, respectively. Hence, CD3- and CD14-enriched PBMC preparations will be described as peripheral blood lymphocytes and monocytes, respectively. Clonetics conditioned cultures of primary human cells were maintained according to the manufacturer's instructions (Cambrex Corporation, East Rutherford, NJ) and included cultures of Human Mammary Epithelial Cells (HMEC), Prostate Epithelial Cells (PrEC) and Normal Human Bronchial Epithelium (NHBE). Finally, buccal cells were isolated from the gum lining of normal healthy volunteer donors as described.

### Induction of apoptosis or cell death and cell permeabilization

Apoptosis or cell death was induced by adding cytotoxic drugs to culture media at the specified concentrations. In some experiments, cells were starved by serum deprivation. In other experiments, TSA used at the specified concentrations was prepared from 1mg/mL stock solution in absolute ethanol. Control (untreated) cells had ≥90% viability determined by PI or trypan blue staining (data not shown). Viable cells were fixed and permeabilized by incubating cells at 5x10⁶ cells/mL in 2% w/v paraformaldehyde solution in PBS (150mM sodium phosphate and 150 sodium chloride, pH 7.2) for 10min. followed by 1:10 dilution in absolute methanol (at -20°C) for 1-3 min. before a final wash with PBS.

### Flow cytometry

Indirect immunofluorescence staining was performed using purified mouse antibodies at 5µg/mL for 30 min. at room temperature (RT) in PBS followed by Alexa₄₈₈-conjugated anti-mouse IgG at 2µg/mL for 30 min. at RT in PBS. Fluorescence was detected using the FL-1 channel (530nm filter). Cell viability was assessed by the exclusion of PI (0.5µg/mL) and detected using the FL-2 channel (585nm filter) or by the exclusion of 7-AAD (2µg/mL for 15 min. at RT) and detected using the FL-3 channel (>650nm filter). Staining for γH2AX was performed using 0.2µg/mL of anti-γH2AX-biotin for 30 min. at RT followed by 2µg/mL of streptavidin-PE or streptavidin-Alexa₄₈₈. Staining of polyclonal antibodies (anti-actin or anti-H2A) was performed using 5µg/mL of antibody solution for 30 min. at RT. Control incubations were performed using protein G purified rabbit IgG from normal rabbit serum (IMVS, SA, Australia). Cells were washed then incubated (30min. at RT) with 2µg/mL of FITC-conjugated anti-rabbit IgG antibody, which was detected using the FL-1 channel. Samples were acquired immediately by a Becton-Dickinson FACScan™ flow cytometry system (BD Biosciences, San Jose, CA). Acquisition was standardized to 10,000 events or in some cases standardized to a set time for acquisition (in seconds) to allow comparison of cell counts in different incubations. Flow cytometry data were analyzed using WinMDI v2.8 (Scripps Research Institute, La Jolla, CA). Unless otherwise specified, no gating was performed in any of the reported analyses. Specific binding of antibodies was calculated as the difference in mean fluorescence intensities (MFI) between the test antibody and the control isotype antibody and expressed as the Net MFI ± standard error of the mean (SEM), which was calculated from replicate incubations (*n*>2).

### SDS-polyacrylamide gel electrophoresis (SDS-PAGE) and immunoblotting

Cell lysates were prepared in SDS lysis solution (2% w/v SDS, 10% v/v glycerol and 62.5 mM Tris-HCl) and protein concentration was determined using BCA protein reagent assay kit according to the manufacturer's instructions. Bromophenol blue (0.05% w/v) and β-mercaptoethanol (5% v/v) were added to lysates after BCA assay measurement. SDS-PAGE was performed according to the manufacturer's instructions with the Hoefer Mighty Small II SE 250 electrophoresis system (Amersham Biosciences, Piscataway, NJ) under reducing conditions using 12% resolving polyacrylamide gel as per Laemmli's method. The transfer of the polyacrylamide gel to Hybond-P membrane was carried out according to the manufacturer's instructions using the TE 22 Mini Tank Transfer Unit (Amersham Biosciences, Piscataway, NJ). Immunoblotting was done using standard methods in which staining with 3B9 was followed by staining with AP-conjugated anti-mouse IgG mAb (Jackson ImmunoResearch Laboratories Inc., West Grove, PA) or anti-actin (N-20) affinity purified goat polyclonal antibody (Santa Cruz Biotechnology Inc., Santa Cruz, CA) followed by AP-conjugated anti-goat IgG mAb (Jackson ImmunoResearch). Some blots were developed using the BCIP/NBT premixed solution as specified by the manufacturer and analyzed using GelPro Analyzer v3.1 (Media Cybernetics Inc., Silver Spring, MD).

Other blots were developed using the ECF™ substrate (Amersham Biosciences, Piscataway, NJ) and scanned using the FluorImager™ 595 (Molecular Dynamics, Amersham Biosciences, Piscataway, NJ) with a 488nm excitation laser and the emissions were collected using a 570nm filter.

### Assays of transglutaminase-mediated crosslinking

Transglutaminase 2 (TG2)-mediated protein-protein crosslinking in apoptotic cells was investigated using a modification of a previously described method. Briefly, cells were resuspended at 1x10⁶ cells/mL in PBS containing 1% Triton X-100, 0.2µg/mL sulforhodamine 101 and 0.1µM SytoxGreen. Samples were vortexed and incubated for 5min. before analysis by flow cytometry where fluorescence from Sytox Green and sulforhodamine were detected using FL-1 and FL-3, respectively. The degree of crosslinking in apoptotic cells was calculated as the ratio of the MFI of sulforhodamine staining in apoptotic cells to the MFI in control cells (protein crosslinking ratio). In other experiments, cells were incubated with PBS or 1% Triton X-100 in PBS for 10min. at RT with intermittent vortexing.

To test inhibition of protein crosslinking, cultured cells were incubated with increasing concentrations of the competitive TG2inhibitor, monodansylcadaverine (MDC). MDC was dissolved in DMSO at 25mM and then diluted in culture media before it was added to cell cultures at the specified concentrations for the specified duration. Incorporation of the TG2 substrate, biotinylated cadaverine, in cellular proteins was used as an index of TG2 activity. Cells were incubated with increasing concentrations of cisplatin with or without 100µM cadaverine-biotin (from 25mM stock solution in DMSO). Cells were collected after 48h, washed extensively with PBS and incubated with streptavidin-Alexa₄₈₈ for cytofluographic analysis. In similar assays, cadaverine-biotin labelled cells were lysed for SDS-PAGE with or without prior immunoprecipitation using 3B9 mAb. PAGE gels were transferred to PVDF membranes and probed using streptavidin-AP to visualise both the total pool of TG2 substrates and 3B9-reactive TG2 substrates.

### Confocal laser scanning microscopy

Cells were stained using immunofluorescence methods described above and then spotted onto glass slides using the cytospin method. The prepared slides were mounted with coverslips using non-fluorescence mounting medium (Dako, Carpinteria, CA). Slides were analyzed using a BioRad Olympus Confocal microscope with appropriate filters and under constant conditions of laser voltage, iris aperture and photomultiplier tube amplification.

### Chromatin-binding assay

Jurkat cells were incubated in the presence or absence of 20µg/mL cisplatin and pelleted 3h after treatment to prepare soluble nuclear and chromatin fractions as decribed. Samples of these fractions were fractionated using 12% SDS-PAGE for immunoblotting as described above. Membranes were probed with 1µg/mL 3B9, 2µg/mL anti-H2A antibodies or 0.2µg/mL anti-γH2AX-biotin followed by the appropriate AP-conjugated secondary antibodies or strepatividin-AP. The presence of H2A in chromatin and not soluble fractions was used to assess the quality of preparation of the chromatin fractions.

### Fluorescent microscopy of γH2AX and 3B9 co-localisation

Chamber slides were seeded with PANC-1 cells and incubated overnight in RPMI-1640 containing 10% FCS before replacement with medium containing 20µg/mL cisplatin alone or in combination with TSA. After 3h, cells were fixed and permeabilized using paraformaldehyde and methanol as described above. Cells were washed and blocked with 5% BSA solution in PBS then stained with 10µg/mL 3B9 followed by 2µg/mL Alexa₄₈₈-conjugated anti-mouse IgG antibody. Cells were washed and incubated with 0.2µg/mL anti-γH2AX-biotin followed by 2µg/mL streptavidin-PE. Finally, cells were washed and coverslips were mounted using non-fluorescent mounting media (Dako, Glostrup, Denmark). Alexa₄₈₈ and PE were excited using a 488nm laser and fluorescence was detected using filters 1 and 2, respectively, of an Olympus fluorescence microscope. Samples stained separately with 3B9 or γH2AX showed that there was no fluorescence bleeding between the two different filters using the specified fluorophores (data not shown).

### Bioinformatics analysis

Oncomine is a database of microarray data, which holds data from 962 studies of which 209 were analyzed. The database contains 14,177 microarrays from 35 cancer types (information publicly available at the website www.oncomine.org). Several cancer signatures have been deduced from large scale analysis of data in the database (24-28). We analyzed the 209 studies using the Advanced Analysis module limiting results to overexpressed genes only and gene enrichment was selected using the following options: (1) InterPro for analysis of motifs in the overexpresed genes, (2) Gene Ontogeny (GO) molecular function for analysis of functions of overexpressed genes, and (3) GO cellular component for cellular compartmentation of the overexpressed genes. Two paramaters were used to describe the gene sets deduced from the above analysis (a) Odds Ratio and (b) P-value, which were provided by the database.

### Statistical analysis

Statistical comparisons were performed using GraphPad Prism v4 (GraphPad Software, San Diego, CA). Generally, two-way analysis of variance (ANOVA) was used to deduce significant differences among the results. The Bonferroni post-test comparison was used to report *P* values. *P* values are denoted as: *, *P*<0.05; **, *P*<0.01; ***, *P*<0.001.

### Results

### La is overexpressed in human malignant cells and is induced by DNA-damaging drugs

As Figure 10 illustrates, the 3B9 target antigen, La, is overexpressed in malignant cells with respect to the corresponding primary cell type. In addition, data mining of Oncomine, which is a cancer gene expression database, indicates that nucleolar proteins and proteins containing the RNA Recognition Motif (RRM) such as La are overexpressed at mRNA level in many human cancers and in normal cells transfected with oncogenes such *c*-*Myc*. Analysis of La/SS-B mRNA expression indicated that La mRNA was overexpressed in malignancy (Tables 2 and 3). Together, these results support the notion that La protein and mRNA overexpression is a feature of malignancy in common with other components of the transcriptional and translational apparatus. It was also inferred that La expression was cell cycle-dependent. Jurkat cells were synchronized by double-thymidine block and 3B9 binding to Jurkat cells, which were fixed and permeabilized at the different phases of the cell cycle, was maximal during S phase (data not shown).

Synchronisation of Jurkat cells by double-thymidine block demonstrates that maximal binding of Apomab, and by inference maximal expression of La, occurs during S phase of the cell cycle (Fig. 11). In further support of the idea that La expression is cell cycle dependent (Fig. 11), the primary cells obtained from Clonetics®, which are cultured in medium supplied by the manufacturer and which contained the activating and mitogenic compound PMA were examined. It was observed that anti-La antibody binding to these permeabilised primary cells is greater than that found in other primary cells such as buccal mucosal cells, which we had freshly isolated. Consequently, we reasoned that PMA as a mitogenic effector may affect the level of La expression in primary and malignant cells. We tested this hypothesis using three cancer cell lines (Fig. 12), and PMA increases La expression

Cytofluographic analysis of etoposide-treated Jurkat cells showed a time-dependent loss of cell membrane integrity, which was measured by binding of propidium iodide (PI) to intracellular nucleic acids. Despite the loss of cell membrane integrity, dead and/or dying cells did not accumulate Sal5 mAb, which is an isotype control antibody of irrelevant specificity (upper panel, Fig. 13A). In contrast, there was a time-dependent increase in accumulation of 3B9, which indicated its specific binding to an intracellular antigen (lower panel, Fig. 13A). The same pattern of binding to dead tumour cells was observed for the human La-specific SW3 mAb and affinity-purified La-specific polyclonal human autoimmune sera (data not shown). Similar accumulation was also observed using other mAb specific for intracellular antigens such as PARP1, PCNA, actin, tubulin and fodrin (data not shown) or after using other DNA damaging agents such as cyclophosamide, cisplatin and staurosporine (data not shown). These results indicate that dead cells contain a reservoir of intracellular antigens, which may potentially be targeted by mAb for diagnostic and therapeutic purposes.

Nonetheless, significant differences in binding levels of the mAb specific for the different intracellular antigens were observed after induction of Jurkat cell apoptosis with the DNA-damaging drug, cisplatin. Antigen-specific mean fluorescence intensity (MFI) of cisplatin-treated Jurkat cells was compared with the MFI of control untreated Jurkat cells, which were fixed and permeabilized (Fig. 13B). While the comparison with control cells showed that antigens such as NPM, La and H2A were at least retained in Jurkat cells after cisplatin-induced cell death, La antigen and H2A were 'created' or 'induced' after cisplatin-induced cell death (Fig. 13B). The 3B9 mAb bound La in the 7-AAD-stained nucleoplasm of control permeabilized cells whereas 3B9 bound La diffusely in the cytoplasm of cisplatin-treated Jurkat cells in which 7-AAD⁺ nuclear fragmentation was apparent (Fig. 13C), which indicated that cisplatin treatment of Jurkat cells induced an apoptotic mode of cell death. This finding was confirmed by demonstrating loss of mitochondrial membrane potential after cisplatin treatment of Jurkat cells (data not shown).

Further evidence for induction of 3B9 binding by DNA-damaging agents was obtained by comparing serum-deprivation with cisplatin-treatment of various malignant cell types. In MCF-7, MDA-MB-231, A549 and PC-3 cancer cell lines, significantly greater 3B9-specific binding to the dead malignant cells was observed after use of the DNA-damaging agent (Fig. 13D). Finally, the overexpression of La observed in permeabilized malignant cells with respect to permeabilized counterpart primary cells was still observed after both malignant and normal cells were treated with cisplatin (Fig. 13E). As shown in Figure 13E, the specific binding of 3B9 to the various malignant cell types was at least two-fold higher than in the corresponding normal cell types.

### 3B9 binding is indicative of DNA damage during drug-induced apoptosis

As Fig. 14A illustrates, the extent of 3B9-specific binding per dead Jurkat cell after induction of apoptosis using the DNA-damaging agent, cisplatin, was proportional to cisplatin dose. Increasing the dose of cisplatin increased the amount of DNA damage, which was measured by cytofluographic detection of the DNA damage marker, γH2AX (inset, Fig. 14A). The correlation of 3B9-specific binding with DNA damage may result from increased expression and/or redistribution and/or accessibility of the La target antigen. Evidence was obtained for redistribution of the La antigen to double stranded breaks (DSB) after observing an increased association of both La and γH2AX with the chromatin fraction derived from cisplatin-treated Jurkat cells (Fig. 14B). Also examined were cisplatin-treated cells of the PANC-1 pancreatic cancer cell line for evidence of DNA damage. As shown in Figure 14C, γH2AX staining co-localized with 3B9 staining of La antigen in nuclei having apoptotic morphology. Together, these data suggest a role for La in DNA-damage responses.

Further evidence for the involvement of La in the DNA-damage response was obtained using chemotherapy-resistant PANC-1 cells. Neither gemcitabine (Fig. 15A) nor the histone deacetylase (HDAC) inhibitor, trichostatin A (TSA), (data not shown) as single agents induced significant levels of cell death among PANC-1 cells. In contrast, the combination of gemcitabine and TSA produced a significantly greater proportion of 7-AAD⁺ dead PANC-1 cells (Fig. 15A). Moreover, significantly greater 3B9-specific binding to 7-AAD⁺ PANC-1 cells was induced when gemcitabine and TSA were used in combination (Fig. 15B). In comparison with control PANC-1 cells, treatment of PANC-1 cells with TSA alone did not alter 3B9 binding (data not shown). In correlation, γH2AX staining increased significantly when PANC-1 cells were treated with the combination of gemcitabine and TSA compared with either gemcitabine treatment alone or no treatment (Fig. 15C). In comparison with untreated PANC-1 cells (upper panels, Fig. 15D), fluorescence microscopy confirmed increased nuclear γH2AX staining in PANC-1 cells, which were treated with gemcitabine and TSA and which showed apoptotic morphology (lower panels, Fig. 15D)

### Cisplastin-treatment of Jurkat cells causes apoptosis and intranuclear accumulation of La

As illustrated in Fig. 16A, staining with the mitochondrial-permeant dye, rhodamine-123, and the DNA binding dye, 7-AAD, confirms that cisplatin-treated Jurkat cells undergo an apoptotic mode of cell death. After 24h treatment with cisplatin, Jurkat cells are smaller and more internally complex, which is consistent with apoptosis induction (Fig. 16A, left-hand panels). Unlike control cells, cisplatin-treated cells bind 7-AAD and show loss of mitochondrial retention of rhodamine-123 (Fig. 16A, right-hand panels). The mitochondrial loss of rhodamine-123 is characteristic of apoptosis (Fig. 16B).

The same dose of cisplatin is used to treat Jurkat cells that are subsequently permeabilised and fixed and stained with mAb specific for a number of nuclear antigens (Fig. 17). The brightness of staining per cell, which is expressed as the antigen-specific mean fluorescence intensity (MFI) of the cisplatin-treated Jurkat cells is compared with the MFI of control untreated Jurkat cells, which are also permeabilised, fixed and stained. The expression of nucleophosmin (NPM) is as high in permeabilised and fixed cisplatin-treated Jurkat cells as in control cells. On the other hand, detection of highly expressed PCNA is markedly reduced after cisplatin treatment. Similarly, other nuclear proteins such as PARP, hTERT, nucleolin and lamin B together with the cytoplasmic cytoskeletal protein actin are all less detectable in permeabilised and fixed apoptotic Jurkat cells compared with permeabilised and fixed control cells (Fig. 17A , B and C). In contrast, detection of the La antigen is significantly increased in apoptotic compared with control Jurkat cells, particularly 48h after apoptosis induction. Similarly, histone H2A is increased in apoptotic Jurkat cells 72h post-apoptosis induction. These data indicate that while antigens such as NPM, La and H2A are retained in Jurkat cells during cisplatin-induced apoptosis, some antigens such as La in particular, and perhaps H2A, are 'created' or 'induced' during apoptosis induced by DNA-damaging agents (Fig. 17D).

### La becomes "fixed" in dead and dying malignant cells

Resistance of apoptotic cells to the non-ionic detergent, Triton X-100, has been reported and correlates with the activity of tissue transglutaminase or transglutaminase 2 (TG2), which mediates protein-protein crosslinking. It was found that during apoptosis, the proportion of 7-AAD⁺ cells and the level of 3B9 binding to these cells were comparable irrespective of Triton X-100 treatment (Fig 18A), which suggested that crosslinking was an integral component of apoptosis in this *in vitro* system. Immunoblot analysis of Jurkat cell lysates demonstrated that 3B9 recognized a 48kDa band, which is consistent with the known molecular weight of La and which remained unchanged during a 72h culture period both in amount and integrity (Fig. 18B). In contrast, in lysates of cisplatin-treated Jurkat cells, 3B9 identified higher molecular weight and SDS-insoluble La-containing complexes, which accumulated as cisplatin-induced apoptosis progressed, together with apoptosis-related La cleavage products (Fig. 18B). The low molecular weight La-specific bands represent caspase-generated cleavage products, which have been reported following apoptosis. Consistent with the cytofluographic findings (Fig. 18A), the SDS-stable high molecular weight bands may represent La antigen covalently bound in higher order complexes with either itself and/or other proteins.

Next, it was hypothesized that inhibition of TG2 activity reduces 3B9 binding to apoptotic cells because of reduced protein-protein crosslinking. First, the effect of increasing doses of cisplatin on the activity of TG2 in Jurkat cells was investigated using the TG2 substrate cadaverine-biotin. Incorporation of cadaverine-biotin into Jurkat cells showed a dose-dependent relationship to cisplatin concentration (Fig. 18C). Based on these results, cisplatin at 20µg/mL was chosen to induce maximal activity of TG2. Then the level of protein crosslinking in apoptotic cells was investigated. An assay was usedin which apoptotic cells were incubated in Triton X-100 solution containing the fluorescent protein-binding dye, sulforhodamine. Sulforhodamine staining in apoptotic cells was higher than in viable cells thus creating a crosslinking ratio to compare protein crosslinking in cisplatin-treated Jurkat cells with or without use of the TG2 inhibitor, monodansylcadaverine (MDC) (Fig. 18D). As shown in Figure 18D, MDC had a dose-dependent inhibitory effect on protein crosslinking. Similarly, 3B9-specific binding to apoptotic Jurkat cells was inhibited in a dose-dependent manner by MDC (Fig. 18E). The concentration of MDC (± SEM) required to inhibit half of the binding of 3B9 was 260±1, 110±1 and 150±1 µM at 24, 48 and 72h, respectively. Together, these results indicated that the La antigen was retained in apoptotic cells by a generalized protein crosslinking process, which was most likely mediated by TG2.

### Protein crosslinking covalently attaches 3B9 to the interior of leaky dead malignant cells during apoptosis

Using etoposide or cisplatin, Jurkat cells were induced to undergo apoptosis in the presence of 3B9 or its Sal5 isotype control mAb. 3B9 binding to apoptotic Jurkat cells was detectable even after stripping of the Jurkat cells with Triton X-100, which suggests that the protein cross-linking process in apoptotic cells includes the cross-linking of bound antigen-specific mAb (Fig. 19A). Furthermore, and importantly, the detergent resistance of 3B9 binding to apoptotic Jurkat cells (Fig. 19A) was much more evident than it was to apoptotic CD3⁺-enriched lymphocytes (Fig. 19B). Confocal microscopic analysis of apoptotic Jurkat cells and apoptotic CD3⁺-enriched lymphocytes supported the contention that 3B9 preferentially binds malignant cells. Reflecting the per cell measure of fluorescence intensity given by the MFI, apoptotic Jurkat cells (Fig. 19C) stain much more intensely with sulforhodamine (red) and 3B9 (green) than their counterpart primary CD3⁺-enriched lymphocytes (Fig. 19D). Moreover, this intense staining is well preserved after Triton X-100 treatment, which suggests that 3B9 is itself cross-linked to other sulforhodamine-staining proteins in the apoptotic Jurkat cells. The lack of 3B9 staining of control Jurkat cells indicates that 3B9 binding depends on induction of apoptosis and consequent loss of cell membrane integrity (Fig. 19A and C). Similar results were obtained for the matched pair of U937 monocytic leukemia cells and normal CD14-enriched peripheral blood monocytes (data not shown). Altogether, these data suggest that along with other proteins in dying malignant cells, both 3B9 and the target antigen La are covalently crosslinked as the result of a TG2-dependent process.

### EXAMPLE 4

### IN VIVO TARGETTING OF THE RIBONUCLEOPROTEIN La IN A MOUSE TUMOUR MODEL

### Materials and Methods

### Cell culture and mAb production

The EL4 murine T-lymphoblastic lymphoma cell line was obtained from American Type Cell Culture (TIB-39) and murine thymocytes were freshly prepared from 6-8 week old C57BL/6 mice. Cells were routinely cultured in RPMI-1640 containing 5% FCS (JRH Biosciences Inc., Lenexa, KS) and passaged every 48-72h at 1:4 dilution. 3B9 and the relevant control (Sal5) were prepared as described previously (first paper). Fluorescein isothiocyanate (FITC) conjugated of these agents was prepared as described by manufacturer's instructions (Sigma-Aldrich Co., St. Louis, MO).

### Induction of Apoptosis

Apoptosis in cultures was induced by adding 20µg/mL etoposide (Pfizer Inc., NY, USA) and 20µg/mL cyclophosphamide (Bristol-Myers Squibb Company, Princeton, NJ) to the culture media. Data presented here from control (untreated cells) originated from samples with higher than 90% viability as determined by PI or trypan blue (Sigma-Aldrich Co., St. Louis, MO) staining (data not shown). Permeabilization of viable cells was performed by 10 min. incubation in 2% w/v paraformaldehyde solution in PBS (150 mM sodium phosphate and 150 mM sodium chloride, pH 7.2) at 5 x 10⁶ cells/mL, which was diluted 1:10 with -20°C-cold absolute methanol (1-3 min.) before washing with PBS.

### Flow cytometry

Direct immunofluorescence staining was performed using 1-2 x 10⁵ cells at 10⁶ cells/mL for 30 min. at room temperature (RT) in PBS containing 0.1% bovine serum albumin (BSA, Sigma-Aldrich Co., St. Louis, MO) and 5µg/mL of FITC-conjugated 3B9 or FITC-conjugated Sal5 isotype control mAb. Indirect immunofluorescence staining was performed using purified mouse antibodies (5µg/mL for 30 min. at RT in PBS) followed by anti-mouse IgG conjugated to Alexa₄₈₈ (Invitrogen, Carlsbad, CA) (2µg/mL for 30 min. at RT in PBS) and detected using the FL-1 channel (530-nm filter). Cell viability was assessed by the exclusion of PI (0.5µg/mL) and detected using the FL-2 channel (585-nm filter) or by the exclusion of 7-AAD (Invitrogen, Carlsbad, CA) (2µg/mL for 15 min. at RT) and detected using the FL-3 channel (>650 nm filter). Samples were acquired immediately by Becton-Dickinson FACScan™ flow cytometry system (BD Biosciences, San Jose, CA). Acquisition was standardized to 10,000 events. Flow cytometry data was analyzed using WinMDI v 2.8 (Scripps Research Institute, La Jolla, CA). Unless otherwise specified, no gating was performed in any of the reported analyses. Specific binding of antibodies was calculated as the difference in the mean fluorescent intensity (MFI) from the test antibodies and that from control isotype antibody and was expressed as the Net MFI ± standard error of the mean (SEM) calculated from replicate incubations (n > 2).

### SDS-polyacrylamide gel electrophoresis (SDS-PAGE) and Western blotting

Cell lysates were prepared in SDS lysis solution (2% w/v SDS, 10% v/v glycerol and 62.5 mM Tris-HCl) and protein concentration was determined using BCA protein reagent assay kit according to the manufacturer's instructions (Pierce Biotechnology Inc., Rockford, IL). Bromophenol blue (0.05% w/v) and β-mercaptoethanol (5% v/v) (Sigma-Aldrich Co., St. Louis, MO) were added to lysates after BCA assay measurement. An amount of 12µg of lysate was added to each lane for SDS-PAGE. SDS-PAGE was performed according to the manufacturer's instructions with the Hoefer® Mighty Small II SE 250 electrophoresis system (Amersham Biosciences, Piscataway, NJ) under reducing condition using 12% resolving polyacrylamide gel as per Laemmli's method. The transfer of the polyacrylamide gel to Hybond-P membrane was carried out according to the manufacturer's instructions using the TE 22 Mini Tank Transfer Unit (Amersham Biosciences, Piscataway, NJ). Immunoblotting was done using standard methods in which staining with 3B9 was followed by staining with alkaline phosphatase (AP)-conjugated anti-mouse IgG mAb (Jackson ImmunoResearch Laboratories, Inc., West Grove, PA). Blots were developed using the BCIP/NBT premixed solution as specified by manufacturer (Sigma-Aldrich Co., St. Louis, MO) and analyzed using GelPro Analyzer v3.1 (Media Cybernetics Inc., Silver Spring, MD).

### Radioligand binding studies

3B9 and matched control were labelled with ¹⁴C by incubating the hybridoma cells (35 million cells) in 35mL RPMI-1640 containing 5% FCS in the production module of miniPERM bioreactor (Vivascience GmbH, Hannover, Germany) and 400mL of RPMI-1640 containing 5% FCS and 250µCi of D-[U-¹⁴C]glucose and 250µCi of L-[U-¹⁴C]leucine (Amersham Biosciences, Piscataway, NJ) in the nutrient module. The bioreactor was incubated in 5% CO₂ humidified air at 37°C on a bottle-rotating device for 5 days. The medium in the production chamber was collected for purification using Protein G purification columns. Radioactivity of purified antibodies (10µL sample) was counted in UltimaGold™ scintillation liquid (1mL) for 20 min using Packard Tri-Carb 3100 β-counter (PerkinElmer Inc., Wellesley, MA) regularly calibrated using supplied ¹⁴C standards. Protein concentration was determined using BCA protein reagent assay. The specific radioactivity of ¹⁴C-control and ¹⁴C-3B9 was 120.3 and 130.8dpm/µg, respectively.

Saturation binding assays were performed by incubating apoptotic EL4 cells (5 x 10⁵ cells) after 48h treatment with etoposide and cyclophosphamide with increasing concentrations of ¹⁴C-3B9 in the presence (to measure non-specific binding) or absence (to measure total binding) of a 50-fold molar excess of unlabelled 3B9. After 30 min., cells were washed thoroughly using PBS and radioactivity was measured using the □-counter as described above. Specific binding was calculated as the difference between total and non-specific binding and plotted as a function of the concentration of ¹⁴C-3B9. Competition binding assays were carried out by incubating apoptotic EL4 cells with 100 nM ¹⁴C-3B9 in the presence of increasing concentrations of unlabelled 3B9. Radioactivity was measured as described above and plotted as a function of unlabelled 3B9 concentration. Association assays were performed by incubation of apoptotic EL4 cells with 100 nM ¹⁴C-3B9 in the presence or absence of a 50-fold molar excess of unlabelled 3B9 for the specified times. Samples were washed, radioactivity was measured and specific binding was plotted as function of time. Dissociation assays were performed after incubation of apoptotic EL4 cells with 100 nM ¹⁴C-3B9 in the absence or presence of 50-fold molar excess of unlabelled 3B9 for 30 min. at RT. Cells were washed, incubated at 37°C in PBS and samples were removed at the specified times. These samples were washed with PBS, radioactivity was measured and specific binding was plotted as function of time.

### EL4 tumour model in C57BL/6 mice

The EL4 thymic lymphoblastic lymphoma is a robust model of apoptosis, which is induced by the cytotoxic drugs, cyclophosphamide and etoposide (14). Subcutaneous EL4 tumour implants were established in 6-8 week old syngeneic C57BL/6 mice where 10⁶ EL4 cells were injected subcutaneously in the right flank of each mouse. Mice were housed and treated as per protocols approved by the Animal Ethics Committee at The University of Adelaide and the Animal Ethics Committee at the Institute of Medical and Veterinary Sciences (IMVS). Once the tumour reached 1cm diameter, mice were randomly divided into control or treatment groups.

Treatment comprised intraperitoneal injections of cytotoxic chemotherapy given in one of the following five regimens of reducing dose intensity. The respective cyclophosphamide and etoposide doses are indicated and are mg/kg. (i) full dose as published: 100, 76 at 0h and 24h; (ii) half dose 2d: 50, 38 at 0h and 24h; (iii) half dose 1d: 50, 38 at 0h; (iv) quarter dose 2d: 25, 19 at 0h and 24h; (v) quarter dose 1d: 25, 19 at 0h.

### Flow cytometry analysis of control and chemotherapy treated EL4 tumours

Control and chemotherapy-treated mice bearing EL4 tumours were euthanized 48h after treatment and tumours were excised. Excised tumour tissue was disrupted by incubation in RPMI-1640 containing 2mg/mL collagenase (Sigma-Aldrich Co., St. Louis, MO) for 30 min. at 37°C with constant shaking. Single cell suspensions were washed with PBS and used for immunofluorescent staining with 3B9-FITC or Sal-FITC and PI and flow cytometry analysis as described earlier.

### ¹⁴C-3B9 biodistribution in EL4 tumour bearing mice

Control and chemotherapy treated mice received an intravenous injection of ¹⁴C-3B9 or ¹⁴C-Sal5 (control) at time 0. After 48h, mice were euthanized, whole blood was collected by cardiac puncture, and EL4 tumours and other tissues were collected for radioactivity measurement. Serum and body tissues were solubilized using 1mL Solvable™ (PerkinElmer Inc., Wellesley, MA) for 2h at 50°C, and then decolorized using 100µL H₂O₂ (30%) (Sigma-Aldrich Co., St. Louis, MO). UltimaGold™ scintillation liquid (1mL) was added and samples were counted for 10min. using the β-counter.

### ¹¹¹In-DOTA-3B9 biodistribution and pharmacokinetics in EL4 tumour bearing mice

Protein G purified 3B9 or Sal5 mAb were mixed at 2.5mg/mL in 0.1M sodium bicarbonate and 0.1M sodium phosphate buffer (pH8.5) with 50-fold molar excess of DOTA-NHS-Ester (Macrocyclics, Dallas, TX) dissolved in DMSO (Sigma-Aldrich Co., St. Louis, MO). DMSO represented <10% of the final reaction, which was incubated for 2h at 23°C with constant vigorous shaking. DOTA-NHS-Ester was added at a 50-fold molar excess to the antibody as this ratio was found to minimize inactivation of 3B9 and showed favorable *in vivo* biodistribution compared to Sal5 conjugated at the same ratio and to 3B9 conjugated at higher ratios (data not shown). Addition of 1.5M Tris-HCl (pH8.8) at a final dilution of 1:10 was used to stop the reaction, which was loaded onto a 100kDa-cutoff microconcentrator (Millipore, Billerica, MA). Reactions were buffer-exchanged using 5x500µL washes of PBS. The concentration of IgG was measured using a BCA protein assay kit and the concentration of conjugated DOTA was measured using a modification of a published Cu:Arsenazo(III) assay. The ligand/protein (L/P) ratio of 7.5 was calculated as the ratio of the concentrations (µM) of DOTA-immunoconjugates to IgG. The reaction was stored at 4°C before radiolabeling.

Purified DOTA-immunoconjugate solutions were concentrated and buffer-exchanged into metal free 0.2M ammonium acetate buffer (pH5.5) (Sigma-Aldrich Co., St. Louis, MO) containing 6mg/mL ascorbic acid (Sigma-Aldrich Co., St. Louis, MO). Indium-111 chloride (PerkinElmer Inc., Wellesley, MA) was diluted in the same buffer (5mCi/mL; 185MBq/mL) and mixed with DOTA immunoconjugates (10mg/mL). Radiolabeling reactions were incubated at 37°C for 2h then mixed with an equal volume of 0.2M ammonium acetate buffer (pH8) containing 5mM EDTA to quench free and loosely bond radionuclide. Quenched reactions were loaded onto 100kDa-cutoff microconcentrators and purified by three washes of 500µL endotoxin free PBS. The incorporation of ¹¹¹In in the purified reactions was determined using instant thin layer chromatography (ITLC) which was performed using ITLC-SG strips (Pall Corporation, East Hills, NY) in 0.2M ammonium acetate solution (pH 8) containing 5mM EDTA as mobile phase. Briefly, aliquots (2µL) of purified reactions and free ¹¹¹In in 0.2M ammonium acetate and 5mM EDTA were used for ITLC in the mobile phase and the origin and solvent front halves of strips were counted using a Cobra 5010 gamma counter (PerkinElmer Inc., Wellesley, MA) normalized for ¹¹¹In counting using a 100-350keV counting window. While 99% of radioactivity on the ITLC-SG strip was at the solvent front when ITLC was performed using ¹¹¹InCl₃ solution, 99% of radioactivity in the DOTA-conjugates remained at the origin indicating the complete incorporation of ¹¹¹In in the DOTA-3B9 conjugate (data not shown).¹ At 0h, ¹¹¹In-DOTA-3B9 (72µg in 100µL of PBS) was given by intravenous injection to EL4 tumour-bearing mice, which were left untreated or treated with cytotoxic chemotherapy as described above. Mice were euthanized at selected time points, blood was collected by cardiac puncture, and tumours and organs were collected. Blood and organs were weighed and placed in gamma-counter tubes and radioactivity was measured using normalized Cobra5010 gamma counter. Radioactivity in organs was normalized to the weight of the organs and accumulation was calculated as the percentage of radioactivity per gram in the organs to the radioactivity of the injected dose of ¹¹¹In-DOTA-3B9 at time 0 (%ID/g). Time activity curves for blood and tumours were constructed using GraphPad Prism v.4 (GraphPad Software, San Diego, CA) for each treatment group where accumulation %ID/g (±SEM, n=5) plotted as a function of time. Curves were fitted to one-phase exponential decay for blood and one-phase exponential association for tumours and the half-life (t_{1/2}) was provided from the fitted models. In the case of tumour accumulation, the fitted association model also provided a value for maximal accumulation at saturation together with the corresponding standard error of this measurement, which we report as %ID/g + SEM at saturation. Data points did not deviate from the fitted models as judged by runs test performed using the fitting software and the regression value of the fitted model was provided.

### Statistical analysis

Statistical comparisons were preformed using GraphPad Prism. Two-way analysis of variances (2-way ANOVA) was used to deduce significant differences in the results. The Bonferroni post-test in the 2-way ANOVA function in GraphPad prism was used to report P values. P values less than 0.05 were considered significant where one, two and three asterisks denote P values less than 0.05, 0.01 and 0.001, respectively.

### Results

### La is overexpressed in malignant EL4 cells and La-specific mAb binding to dead EL4 cells is induced by cytotoxic drug treatment

Cytofluographic analysis of cultured EL4 lymphoma cells, which were stained with the DNA binding dye 7-AAD as a test of membrane integrity, indicated that although <10% of the cultured cells demonstrated evidence of spontaneous cell death, these dead cells did not bind La-specific mAb 3B9 (Fig. 20A). Nevertheless, after fixation and permeabilization of the EL4 cells, 3B9 binding to 7-AAD⁺ cells was evident, which indicated that La autoantigen could be accessed using a specific mAb (Fig. 20B). Furthermore, after EL4 cells were treated with the cytotoxic and DNA-damaging drugs, cyclophosphamide and etoposide, the intensity of 3B9 binding to the resulting 7-AAD⁺ EL4 cells was greater than to fixed and permeabilized 7-AAD⁺ cells (Fig. 20C). As illustrated in Fig. 20D, per cell binding of La-specific mAb to dead EL4 cells killed by cytotoxic drug treatment was significantly higher than the binding to dead cells killed by fixation and permeabilization, which suggested that cytotoxic drug treatment either induced higher levels of target expression and/or increased the accessibility of the epitope. In addition, the 3B9 binding to the dead EL4 cells killed with cytotoxic drugs was preserved after the dead cells were treated with a non-ionic detergent, which indicated that the La target antigen had been cross-linked in the dead cells. Fig. 20D also illustrates that, after fixation and permeabilization, the per cell binding of 3B9 mAb was significantly higher for the malignant EL4 cell than for its normal murine counterpart cell type of the thymocytes. This cytofluographic evidence of La overexpression in EL4 cells was corroborated by immunoblot analysis of cell lysates prepared from thymocytes and EL4 cells (inset, Fig. 20D). The binding of 3B9 to dead thymocytes was the same irrespective of whether they were killed by cytotoxic drugs or fixation and permeabilization, which contrasts with the significantly increased binding of 3B9 to dead EL4 cells after cytotoxic drug treatment (Fig. 20D).

### ¹⁴C-labelled 3B9 binds specifically to EL4 cells

Next, biosynthetically labelled 3B9 mAb was used to further characterize the binding of La-specific mAb to dead EL4 cells, which were killed with cytotoxic drugs. It was found that ¹⁴C-3B9 bound dead EL4 cells in a specific and saturable manner. The concentration of ¹⁴C-3B9 required to reach half-maximal saturation was 18nM and maximal binding was ~7500 femtomole/million dead cells (Fig. 21A). Half-maximal saturation of ¹⁴C-3B9 binding occurred within 5 minutes at room temperature (Fig. 21B). The concentration of unlabelled 3B9 required to inhibit half-maximal binding of ¹⁴C-3B9 (IC₅₀) was estimated to be 118nM (Fig. 21C). Dissociation of bound 3B9 was minimal when cells were incubated in PBS for 30 minutes at 37°C (Fig. 21D). Altogether, these data describe specific, rapid and high affinity binding of ¹⁴C-3B9 to dead tumour cells. Binding was not detected when EL4 cells were tested in the absence of cytotoxic drugs (data not shown), which indicated that binding depended on the induction of cell death.

### Cytotoxic chemotherapy increases the target for 3B9 in the tumour mass

The *in vitro* findings depicted in Fig. 20 were confirmed *in vivo* using the EL4 lymphoma model. After the use of cytotoxic chemotherapy, the fraction of PI⁺ cells in the tumour explants increased significantly from a mean (±SEM) of 50±2% to 70±1% (*P*<0.001). Similarly, the 3B9⁺ subset of PI⁺ cells increased significantly from 15±1% to 38±2% (*P*<0.01) with use of cytotoxic chemotherapy whereas isotype control staining did not alter with use of chemotherapy (Fig. 22). Only the PI⁺ subpopulation of tumour cells displayed binding with the 3B9-FITC conjugate, which indicated that the La antigen was recognized specifically in dead tumour cells. In order to describe the effect of chemotherapy on 3B9 targeting to La, we analyzed the frequency distributions of PI⁺ cells, which bound FITC conjugates and which were defined in the upper right quadrant of each density plot (Fig 22). As illustrated in the representative histograms in Fig. 22, the specific binding of 3B9-FITC to PI⁺ cells was significantly augmented in tumours exposed *in vivo* to cytotoxic chemotherapy (net MFI ± SEM of 18±3 with chemotherapy and 1±3 without chemotherapy, *P*<0.05).. These results provide *in vivo* evidence of increased per cell binding of 3B9, which may reflect increased expression and/or increased availability of the 3B9 epitope in dead cells obtained from tumours that were exposed to cytotoxic chemotherapy.

### 3B9 targets EL4 tumours in vivo especially after cytotoxic chemotherapy

The biodistribution of La-specific mAb was studied in the EL4 lymphoma model using intrinsically or extrinsically labelled 3B9 mAb. First, biosynthetically labelled ¹⁴C-3B9 mAb was used to demonstrate that tumour accumulation of 3B9 mAb was an inherent property of its antigen-binding activity. EL4 tumour-bearing mice were treated with the full dose schedule of cyclophosphamide and etoposide and simultaneously administered 100µg each of either ¹⁴C-3B9 or ¹⁴C-Sal5 isotype control mAb before analysis of the mice after 48 hours. The ¹⁴C-Sal5 mAb did not accumulate significantly in any organ or tissue including the tumour. In contrast and compared with all other organs, we found that ¹⁴C-3B9 accumulated significantly in serum and in the tumour (*P*<0.001). Moreover, after the mice were treated with cytotoxic chemotherapy, only the tumours accumulated significantly more ¹⁴C-3B9 (*P*<0.001) (data not shown).

In the second series of experiments, 3B9 conjugated to a metal chelator, DOTA, was radiolabelled with ¹¹¹In and used to investigate the biodistribution and pharmacokinetics in EL4 tumour-bearing mice before and after chemotherapy treatment (Fig. 23&24). The clearance of ¹¹¹In-DOTA-3B9 from blood of control mice and mice treated with quarter dose 2d or with half dose 1d was not significantly different (*P>0*.*05*). On the other hand, the clearance of ¹¹¹In-DOTA-3B9 from blood of mice treated with quarter dose 1d or with half dose 2d was significantly faster than that in control mice (*P*<*0*.*001*) (Fig. 23). The accumulation of 3B9 in the tumours of mice treated quarter dose 2d (40±3%ID/g, n=5) and half dose 1d (37±3%ID/g, n=5) was significantly different from that in control mice (20±1%ID/g, n=5) *(P<0.05).* The more rapid clearance of 3B9 from blood of mice treated with quarter dose 1d or half dose 2d was associated with more significant accumulation of the agent in the tumours, (48±8%ID/g, n=5) and (47±6%ID/g, n=5), respectively (*P<0.01*) (Fig. 23). Despite the exponential growth of control tumours (measured as tumour weight, Fig. 23), weight-normalized tumour accumulation (%ID/g) of ¹¹¹In-DOTA-3B9, which has a physical half-life of 2.8 days, was unchanged, suggesting that rates of tumour cell death balanced tumour cell proliferation to allow continued binding of 3B9 to these growing tumours. Finally, the reduction of tumour weights in mice treated with cytotoxic chemotherapy and the accumulation of ¹¹¹In-DOTA-3B9 in these tumours was associated with increased levels of cell death measured by cytofluographic analysis (% of 7-AAD⁺ cells) (Fig 23 - inset).

The specificity of ¹¹¹In-DOTA-3B9 towards the dying tumours was expressed as the ratio of tumour accumulation to that in normal organs (tumour/organ ratio, Fig. 24). At 24h after chemotherapy and ¹¹¹In-DOTA-3B9 administration, only the tumour/muscle ratio was significantly higher compared to control mice (*P*<*0*.*001*, data not shown). Compared to control mice, the tumour/organ ratio at 48h (Fig. 24A) and 72h (Fig. 24B) after treatment was significantly higher for all organs except for blood. Further evidence for tumour specificity was obtained from biodistribution studies of ¹⁴C-3B9 in EL4 tumour-bearing mice which were extended to include lower doses of the radiolabelled agent. As illustrated in Fig. 25, there was no significant accumulation of ¹⁴C-3B9 in any tissue except the tumour and only after administration of cytotoxic chemotherapy. Tumour accumulation of ¹⁴C-3B9 was dose-dependent and the sigmoidal dose-response relationship suggested that the binding of the target was specific and saturable. In comparison to untreated mice, tumour uptake of ¹⁴C-3B9 with chemotherapy was significantly higher and demonstrated fold increases of 1.9, 1.9 and 1.8 at ¹⁴C-3B9 doses of 25 µg, 50 µg and 100 µg, respectively. Irrespective of the use of chemotherapy, no significant difference in the tumour uptake of ¹⁴C-3B9 was observed at a 5µg dose. These data further support the concept that 3B9 selectively targets malignant tissues rather than critical normal tissues. Altogether, 3B9, using two different radiotracer formats, showed specific and preferential accumulation to EL4 tumours which was augmented by chemotherapy treatment of this chemo-responsive tumour model.

### EXAMPLE 5

### The La antigen is a TG2 substrate

A biotinylated form of cadaverine, which is a polyamine TG2 substrate and which consequently becomes covalently bound to other TG2 substrates, is used to identify protein targets of cross-linking during apoptosis. As shown in Fig. 26A, the incorporation of cadaverine-biotin in apoptotic Jurkat cells was confirmed by fluorocytometric analysis using streptavidin-Alexa₄₈₈. Immunoblot analysis of Jurkat cells induced to undergo apoptosis in the presence of cadaverine-biotin results in the detection of additional protein bands labelled with cadaverine-biotin (lane 2 in inset, Fig. 26). These data indicate that cadaverine-biotin is incorporated in an SDS-stable manner into cellular proteins during apoptosis. Immunoprecipitation with Apomab of Jurkat cell lysates after treatment of the cells with cadaverine-biotin during apoptosis induction demonstrates incorporation of cadaverine-biotin into a putative La antigen band as detected by streptavidin-AP on the immunoblot (arrow, lane 2, Fig. 26B).

### EXAMPLE 6

### DNA-DAMAGING CHEMOTHERAPY INDUCES ANTI-LA ANTIBODY BINDING IN PRIMARY HUMAN MALIGNANT CELLS

Two studies were performed on patient material to indicate that Apomab binding was augmented by DNA-damaging cytotoxic chemotherapy. In the first study, primary ALL blasts from a chemonaïve patient were treated *in vitro* with cytotoxic drugs and then analysed (Fig. 27&28). In the second study, circulating tumour cells of a patient receiving this first cycle of chemotherapy for extensive stage small cell lung cancer (SCLC) were analysed *in vitro* (Fig. 29).

### Treatment and analysis of acute lymphoblastic leukemic blasts in vitro

To purify primary ALL blasts for further analyses, Ficoll gradient separation was performed using a 10 mL peripheral blood sample from a newly diagnosed and untreated ALL patient. The buffy coat was collected and washed with culture medium before incubation for 10 min in red cell lysis buffer. Cells were washed, aliquots were removed for immediate analysis while other aliquots were incubated or not in different concentrations of cytotoxic drugs singly or in combination. At 24h, 48h, and 72h after treatment was initiated *in vitro,* ALL blasts were stained with 10 µg/mL Sal5 control or Apomab then with 2 µg/mL Alexa₄₈₈-conjugated anti-mouse IgG. Cells were washed and incubated with 2 µg/mL 7-AAD then analysed by flow cytometry immediately.

The level of Apomab binding to permeabilised primary ALL blasts (inset, Fig. 28) was similar to the level found in blasts dying spontaneously in culture (control, Fig. 28). Apomab binding was highest after ALL blasts were treated with double strand DNA break (DSB)-inducing agents, etoposide and/or cisplatin, and peaked 48h after treatment (Fig. 28).

### Analysis in vitro of circulating small lung cancer cells of a patient treated with cytotoxic drugs

Anti-La antibody binding to circulating tumour cells in peripheral blood of a 73-year-old male patient, GP, who had extensive-stage SCLC was studied. The patient, GP, who had not previously been treated for his cancer, was administered cytotoxic chemotherapy using carboplatin at AUC 5 on day 1 together with etoposide 120 mg/m² on days 1, 2 and 3. Heparinised peripheral blood samples were drawn from the patient before chemotherapy (0h) and 24, 48 and 72 hours after initiation of chemotherapy. To demonstrate that GP's blood contained circulating tumour cells (CTC), his blood was enriched before and after cytotoxic drug treatment for BerEP4-expressing cells, which were present minimally if at all in the peripheral blood of a normal healthy volunteer (control) (Fig. 29, upper row of panels). Forty-eight hours after cytotoxic chemotherapy, there was a large increase in the number of dead (7-AAD⁺) CTC, which clearly and specifically bound Apomab (Fig. 29, third row of panels). At 72h post-treatment, Apomab-specific staining was also detected on peripheral blood 7-AAD⁺ material of GP that has scatter characteristics of apoptotic bodies (Fig. 29, fourth row of panels). The greatest increase of 7-AAD⁺ cells in GP's blood was observed 48 hours after initiation of cytotoxic chemotherapy and the 7-AAD⁺ cells had the highest binding of Apomab (Fig. 29B).

Although the laboratory findings were not correlated with a formal radiological evaluation of overall tumour response to chemotherapy, GP did have a palpable and painful sternal mass before cytotoxic chemotherapy, which became markedly less painful and shrunk significantly in size within two weeks of cytotoxic chemotherapy administration.

In conclusion, these results using patient tumour material indicate that anti-La antibody identifies malignant cells that have died as the result of cytotoxic drug administration and, hence, this antibody may be useful for predicting early chemotherapy responses in cancer patients.

### DNA-damaging antineoplastic induces the intrinsic apoptosis pathway and induces sustained Apomab to apoptotic cells unlike extrinsic pathway activation, culture stress, serum withdrawal or primary necrosis

Apoptotic changes after intrinsic pathway activation took longer than after extrinsic pathway activation. During activation of the intrinsic pathway by cisplatin or γ-radiation, evidence of DNA damage was observed at the early 5h time point in permeabilised cells by γ-phosphorylation of H2AX and, simultaneously, by significantly increased antibody binding, which reflects increased expression of the La target antigen (Fig. 30A & B). DSB and early induction of La expression were most prominent and sustained after γ-radiation (Fig. 30B). These changes preceded apoptosome formation that is marked by mitochondrial membrane permeabilisation (reduced retention of rhodamine 123) and caspase-3 activation. During activation of the extrinsic pathway by Fas ligation, apoptosis of Jurkat cells was induced more rapidly than by the intrinsic pathway inducers of cisplatin and γ-radiation because caspase-3 activation was evident only 5h after treatment. At this time point also were detected marked elevations of γ H2AX and La antigen, which subsided 24h later as Jurkat cells were losing cell membrane integrity manifest as 7-AAD staining (Fig. 30C). After Fas ligation, DSB may occur as the cell undergoes apoptosis and is dismantled into apobodies.

In contrast, cells that were overgrown *in vitro* (Fig. 30D) or starved by serum deprivation (Fig. 30E), did not induce La expression manifest as increased anti-La antibody binding, and in fact antibody binding to the permeabilised cells declined significantly with time after the stressful stimulus. Under these stressful conditions, loss of cell membrane integrity manifest as 7-AAD staining did not occur until 72h after the stress. These conditions often induce ER stress and cells may survive by undergoing autophagy before finally succumbing to a death that is not apparently marked by apoptotic features. These *in vitro* conditions may mirror the endogenous tumour cell death resulting from adverse micronenvironmental conditions *in vivo* such as hypoxia, acidosis, and ischemia. Importantly, these *in vitro* conditions did not result in induction of the La target antigen, and thus did not augment anti-La antibody binding. Finally, a primary necrotic insult such as heat did not induce either apoptosis or La expression and antibody binding despite earlier loss of cell membrane integrity (Fig. 30F).

Further analysis indicated differences in the course of apoptosis after γ-radiation vis à vis cisplatin treatment (Fig. 31). For irradiated cells, activation of caspase-3 occurred in parallel to the conversion of apoptotic cells to apoptotic bodies (apobodies), which were smaller (lower FSC), more internally complex or granular (higher SSC) with intermediate 7-AAD-staining. In response to this apoptotic stimulus, γH2AX and La induction was apparent at 5h before caspase-3 activation and persisted (Fig. 31B). Interestingly, La induction persisted in all cells whereas γH2AX foci were lost in apobodies reflecting loss of H2AX γ-phosphorylation. Another interpretation is that La partitioned to both RNA- and DNA-containing apobodies, which may be mutually exclusive, whereas γH2AX foci associated only with DNA (Fig. 31B).

After Fas ligation, similar considerations applied to activation of caspase-3. As cells turned into apobodies, caspase-3 was activated simultaneously. Caspase activation indicated that cells were being dismantled into apobodies and the caspase-mediated processes likely included DNA degradation. Hence, the creation of simultaneous DSB seemed to be responsible for the striking induction of γH2AX foci and the concomitant induction of La. Activated caspase-3 activation persisted in cells as they converted to apobodies. On the other hand, the γH2AX and La induction occurred initially and transiently in all cells, and so appeared to be a process that was executed in response to the death stimulus applied to all cells and then was complete (Fig. 31C).

In confirmation that cisplatin treatment and γ-radiation of Jurkat cells induces expression of La antigen, immunoblots of cell lysates prepared shortly after apoptosis induction but before loss of cell membrane integrity were probed with anti-La antibodies or anti-actin antibodies as a loading control (Fig. 32A). In contrast to starvation of Jurkat cells by serum withdrawal, densitometric scanning of the immunoblots (Fig. 32B) indicates that relatively more La was expressed in Jurkat cells after both cisplatin treatment and γ-radiation (Fig. 32C).

To show that anti-La antibody bound a variety of other dead human malignant cell lines after cytotoxic treatment, anti-La antibody or its Sal5 isotype control mAb was used to stain malignant cells after treatment with the pan-tyrosine kinase inhibitor (STS), the topoisomerase II inhibitor, etoposide, or the tubule depolymerising agent, vincristine (Fig. 33 and Table 6).

### EXAMPLE 7

### DNA-DAMAGING CHEMOTHERAPY INDUCES Apomab BINDING IN VIVO

### Aims

*In vivo* studies were performed to quantify dead cells in EL4 tumours before and after CE chemotherapy, and to measure anti-La antibody uptake by these tumours. To provide evidence *in vivo* of induction of the La antigen by DNA-damaging chemotherapy, the relationship between tumour anti-La antibody uptake and the percentage of dead cells within tumours were examined. Anti-La antibody uptake by control tumours was compared with tumour anti-La antibody uptake after CE chemotherapy to determine if it exceeded the level expected if it were only proportionate to the tumour content of dead cells.

### Treatment and analysis of cell death in EL4 tumours

C57BL/6 mice bearing EL4 tumours were left untreated (*n*=3) or treated by intraperitoneal injection (IPI) of 19.5mg/kg etoposide and 25mg/kg of cyclophosphamide (*n*=3). Mice were killed at specified time points, tumours were excised, minced into pieces <10mm³ using scissors, and 0.1 g of minced tumour weighed and placed in 10 mL 2 mg/mL solution of collagenase Type 1 in HBSS containing 2.5 mM Ca²⁺. Solutions were incubated at 37°C with constant rotation for 1h. Digested tumour cell suspensions were passed sequentially through syringes with 19 G, 23 G then 25 G needles. Suspensions were centrifuged at 2000 rpm for 5 min and pellets were washed with 10 mL HBSS. Washed pellets were resuspended in 1 mL HBSS and 100 µL aliquots were stained for 10 min at room temperature (RT) in duplicate with 2 µg/mL 7-AAD. Samples were analysed using flow cytometry for the percentage of cells that bound 7-AAD to measure the percentage of dead cells (7-AAD⁺).

### Treatment and analysis of anti-La antibody (Apomab) binding to EL4 tumours in vivo

C57BL/6 mice bearing EL4 tumours were left untreated (*n*=5) or treated with chemotherapy (*n*=5) as described above. All mice were also injected intravenously with ¹¹¹In-Apomab. Mice were killed at specified time points, tumours were excised, weighed, and placed in gamma counting tubes to measure radioactivity. Measured radioactivity was normalised to tumour weight (cpm/g) and accumulation was calculated as the percentage of weight-normalised radioactivity counts to total radioactivity counts of injected dose at time 0h (% cpm/g in tumour / cpm of injected ¹¹¹In-Apomab at time 0h).

### Results

In comparison with untreated control mice (Fig. 34A), treatment of mice with CE chemotherapy increased the EL4 tumour cell death rate (Fig.34B). Similarly, treating EL4 tumour-bearing mice with CE chemotherapy augmented tumour accumulation of ¹¹¹In-Apomab (data not shown). To determine the rate of tumour accumulation of Apomab itself, time-activity data were corrected for ¹¹¹Indium decay because ¹¹¹In-Apomab accumulation represented 97.0, 78.3, 61.3, 48.0, and 37.5% of Apomab accumulation at 24, 48, 72, and 96h post-injection, respectively. It is readily apparent that this correction shows that the rate of Apomab accumulation in control tumours matched the tumour cell death rate (Fig.34A) whereas the rate of Apomab accumulation in treated tumours exceeded the tumour cell death rate (Fig.34B).

To demonstrate this finding more explicitly, Apomab binding to dead tumour cells was related directly to the tumour content of dead cells at each time point (Fig. 35). In control tumours, the ratio of Apomab accumulation (%ID/g) to tumour content of dead cells (%7-AAD⁺ cells) never exceeded 100% and indeed declined as tumours grew whereas, after chemotherapy, the ratio increased beyond 100% with time. It is hypothesised that DNA-damaging antineoplastic treatment induces expression of the La antigen and so augments per cell binding of Apomab, which has been demonstrated previously *in vitro* and as *ex vivo* staining of tumour cell suspensions obtained from mice treated or not with DNA-damaging chemotherapy.

In conjunction with the data showing therapeutic efficacy of La-directed monoRIT, at least two significant inferences may be drawn from these observations:
(i) The first step of DNA-damaging antineoplastic therapy creates additional targets for binding of armed versions of Apomab or other potential dead tumour cell-interacting radioligands, and thus creates a self-amplifying or feed-forward loop for the second step of circulating tumour-binding activity.
(ii) Delivery of armed Apomab to tumour cells dying as the result of DNA-damaging antineoplastic therapy initiates cell death among nearby viable tumour cells and this bystander killing amplifies the effect of the original targeting by creating targets that were not at first manifest within the tumour. According to this method, we hypothesise that bystander killing will be most efficient if the immunoconjugate induces DSB. Such conjugates include ionising radiation and calicheamicin.

Furthermore, cytotoxic radiosensitising drugs such as cisplatin, the antimetabolites gemcitabine and 5-fluorouracil, and the taxanes, will augment the potency of La-directed radioimmunotherapy (RIT) by lowering the threshold for tumour cell death. Therefore, it would be expected that appropriately scheduled administration of a cytotoxic radiosensitising drug and La-directed RIT would lower the effective dose of RIT required for tumour cell kill.

Other than ionising radiation, the radiomimetic drug, cisplatin, topoisomerase inhibitors, DNA-intercalating agents, which are all antineoplastic treatments well known to induce double-stranded DNA breaks (DSB) and some which have been shown to augment Apomab binding to apoptotic tumour cells *in vitro*, newer agents induce DSB *via* novel mechanisms of action. For example, PARP inhibitors induce DSB and sensitise to apoptosis those tumour cells that lack DNA repair mechanisms based homologous recombination (HR) such as BRCA nullizygous tumour cells, or tumour cells with features of 'BRCAness' (N Turner et al. Nat Rev Cancer 4, 1-6, 2004) because HR is required to repair endogenous DNA damage (HE Bryant et al. Nature 434, 913-917, 2005; H Farmer et al. Nature 434, 917-921, 2005). The antimetabolite and masked DNA chain terminator, gemcitabine, induces few DSB unless the S-phase checkpoint is abrogated by a class of drug known as CHK1/2 inhibitors when DSB become greatly increased and apoptosis ensues (G McArthur et al. J Clin Oncol 24, 3045a, 2006; MA Morgan et al. Cancer Res 65, 6835-6842, 2005). Similar effects were exerted by CHK1/2 inhibitors after ionising radiation induced G₂ and S checkpoints (J Falck et al. Nature 410, 842-847, 2001; H Zhao et al. PNAS 99, 14795-14800, 2002).

### EXAMPLE 8

### COMBINATION TREATMENT OF CYTOTOXIC DRUGS AND THE HISTONE DEACETYLASE INHIBITOR (HDACi), TRICHOSTATIN A (TSA) INDUCES CELL DEATH AMONG MALIGNANT CELLS AND PROVIDES GREATER OPPORTUNITY FOR ANTI-LA ANTIBODY (Apomab) BINDING

Further data are presented in support of this hypothesis using combination treatment with cytotoxic drugs and the histone deacetylase inhibitor (HDACi), trichostatin A (TSA), to show that the combination is very effective in inducing cell death among malignant cells and, therefore, provides the opportunity to maximise target creation.

Adding TSA to cisplatin treatment of Jurkat cells *in vitro* produces a TSA dose-dependent increase in Apomab-specific binding to dead Jurkat cells (Fig. 36). As depicted in Figures 36 and 37, it is hypothesised that the observed effect results from TSA augmenting the DNA damage response initiated by cisplatin, which together 'induce' La in dead malignant cells. As illustrated in Figure 38, exposure of Jurkat cells to cisplatin is required at the time that TSA is added to the Jurkat cell cultures for an increase in Apomab-specific binding to be manifest. If increased Apomab binding does occur as the result of enhanced DNA damage, then these data would be consistent with findings in which HDACi increase DNA damage initiated by conventional DNA-damaging agents. DNA damage, which is measured by the phosphorylation of H2A to create γH2AX, was induced by epirubicin in cell lines and potentiated by the HDAC inhibitors, suberoylanilide hydroxamic acid (SAHA) (Marchion DC et al. J Cell Biochem 92,223-237, 2004) and valproic acid (Marchion DC et al. Mol Cancer Ther 4, 1993-2000, 2005). Nonetheless, La is 'induced' in malignant cells after cisplatin treatment with or without the addition of TSA, As Figure 39 shows, the induced La becomes 'fixed' in the dead malignant cells and its detection is clearly shown to be detergent resistant.

Similar synergistic interactions were found between gemcitabine and TSA in cells of the pancreatic cancer cell line, PANC-1, *in vitro.* In these experiments, the TSA doses of 200, 100, 50, 25 and 12.5ng/mL are equivalent to TSA concentrations of 667, 333, 167, 83 and 42nM, respectively. Together with the chosen concentrations of gemcitabine, TSA significantly inhibited proliferation of PANC-1 cells *in vitro* (Piacentini P et al. Virchows Arch 448, 797-804, 2006). Moreover, a moderate dose-dependent effect of TSA was observed on the induction of markers of apoptosis among cultures of PANC-1 cells *in vitro* (Donadelli M et al. Mol Carcinogenesis 38, 59-69, 2003).

Using the MTS proliferation assay, it was observed that TSA alone inhibited proliferation of PANC-1 cells *in vitro.* Addition of gemcitabine completely inhibited PANC-1 proliferation at a TSA concentration of 50nM whereas at a TSA concentration of 200nM, gemcitabine significantly reduced the numbers of PANC-1 cells in the cultures (Fig. 40). As illustrated in the cell death assay (Fig. 41), which was performed in parallel using the same experimental design, it is clear that the reduction in PANC-1 cell numbers at the TSA concentration of 200nM resulted from augmented cell death in the gemcitabine-containing cultures. Moreover, this assay shows that enhancement of Apomab-specific binding to the dead 7-AAD⁺ PANC-1 cells only occurs 48 hours after the induction of cell death and this effect is not observed at the 72 hour time point (Fig. 41). A further such assay was performed at the 48 hour time point and again showed the synergistic effect of combination treatment with TSA and gemcitabine on both cell death and Apomab-specific binding to the dead PANC-1 cells at all concentrations of gemcitabine when the TSA concentrations were either 100ng/mL or 200ng/mL (Fig. 42).

While gemcitabine may not be viewed as a conventional DNA-damaging agent, recent evidence indicates that it produces γH2AX DNA damage foci in a PC-3 tumour xenograft model (McArthur GA et al. J Clin Oncol 24(18S) 3045a, 2006). The synergistic effect of TSA on the induction of cell death among gemcitabine-treated PANC-1 cells together with Apomab-specific binding to the dead cells was evident at the lowest concentration of gemcitabine.

Altogether, it is believed that the data illustrated in Figures 36-42 support the contention that La, as the target antigen for Apomab binding in dead malignant cells, is a prime example of a target that is created by the use of anti-neoplastic treatment. La is 'induced' by the action of anti-neoplastic treatment, particularly DNA-damaging drugs, *via* an unknown mechanism, and its retention in dead malignant cells as a target antigen is enhanced by 'fixation', which probably results from the activity of apoptosis-induced transglutaminase-2.

### EXAMPLE 9

### METHODS FOR OPTIMIZING RADIOIMMUNOCONJUGATES (RIC) AND THEIR EFFECTS ON ACTIVITY

### Materials and Methods

### Cell culture and antibody production

Suspension cultures of Jurkat leukemia cell line and EL4 murine T-lymphoblastic lymphoma cell line were maintained in RPMI-1640 containing 5% FCS (JRH Biosciences Inc., Lenexa, KS) and passaged by splitting at 1:10 every 72h. The anti-La/SS-B 3B9 mAb hybridoma (Tran et al. 2002, Arthritis Rheum. 46(1):202-8) is a murine IgG₂ₐ autoantibody, which is crossreactive with human La and which was prepared by Dr M. Bachmann (Oklahoma Medical Research Foundation, OK), was a kind gift from Dr T.P. Gordon (Department of Immunology, Allergy and Arthritis, Flinders Medical Centre, SA, Australia). The isotype control Sal5 (1D4.5) mAb hybridoma, prepared by Dr L.K. Ashman (Medical Science Building, University of Newcastle, NSW, Australia), was kindly supplied by Dr S. McColl (School of Molecular Biosciences, University of Adelaide, SA, Australia). Hybridoma were cultured in RPMI-1640 containing 5% FCS and the produced antibodies were affinity-purified from culture media using protein G columns. FITC-conjugates were prepared according to the manufacturer instructions (Sigma-Aldrich Co., St. Louis, MO).

### Conjugation of mAb with DOTA-NHS-ESTER

DOTA-NHS-ESTER (Macrocyclics, Dallas, TX) was dissolved in DMSO (Sigma-Aldrich Co., St. Louis, MO) at 25mg/mL. Purified 3B9 and Sal5 in 0.1 M sodium phosphate/0.1 M sodium bicarbonate buffer (pH 8.6) were mixed at 2mg/mL with 50-, 100-, 150- and 200-fold molar excess of DOTA-NHS-ESTER. Control reactions were prepared using equivalent volume of DMSO to replace the NHS-ESTER compound. Reactions were incubated at 23°C for 2 h with constant rotation and were stopped using 1.5M Tris-HCl (pH 8.3) at 10% v/v final concentration. Removal of unconjugated DOTA and buffer exchange of immunoconjugates was achieved by 5x500µL PBS washes in 100kDa cut off microconcentrators as described by manufacturer instructions (Millipore, Billerica, MA). Conjugates were stored at 4°C for further use.

### Determination of DOTA/antibody ratio

Protein concentration was determined using the BCA protein assay kit as described by manufacturer instructions (Pierce Biotechnology Inc., Rockford, IL). Protein concentration was converted from units of mg/mL to µM based on the Mr of IgG (150'000 g/mol). The concentration of DOTA was determined using a modified form of the previously described Arsenazo(III) assay (Pippin et al. 1992, Bioconjug Chem 3(4):342-5; Dadachova et al. 1999, Nucl Med Biol, 26(8):977-82; Brady et al. 2004, Nucl Med Biol, 31(6):795-802). Briefly, assays were performed in 96-wells titre plates to reduce the volume used. Stock solutions of Cu:Arsenazo(III) were prepared using 100µL of 1mg/mL standard Cu atomic absorption solution (Sigma-Aldrich Co., St. Louis, MO), 0.875mg Arsenazo(III) (Sigma-Aldrich Co., St. Louis, MO) and 3mL of metal free 5M ammonium acetate (Sigma-Aldrich Co., St. Louis, MO) in a final 10 mL volume of milliQ water and stored at room temperature (RT) in the dark. Standard concentrations of DOTA were prepared using DOTA-NHS-ESTER dissolved in milliQ water. Aliquots (10µL) of conjugation reactions and standard DOTA solutions were mixed with 190µL of working dilution (see figure legends) of Cu:Arsenazo(III) solution in milliQ water, incubated at 37°C for 30 min and absorbance was measured at 630nm. The concentration of conjugated DOTA was interpolated from the standard curve constructed for the relationship between DOTA standard solutions and the absorbance of Cu:Arsenazo(III) reagent. DOTA/antibody ratio in prepared conjugates was calculated as the ratio of DOTA concentration (µM) to IgG concentration (µM).

### SDS-polyacrylamide gel electrophoresis (SDS-PAGE) and Western blotting

SDS-PAGE was performed as per manufacturer instructions using the Hoefer® Mighty Small II SE 250 electrophoresis system (Amersham Biosciences, Piscataway, NJ) under reducing condition using 12% resolving polyacrylamide gel as per Laemmli (Laemmli U.K., 2005, J. Biol Regul Homeost Agents 19(3-4):105-112). Transfer of polyacrylamide gel onto Hybond-P membrane was carried out as per manufacturer instruction using the TE 22 Mini Tank Transfer Unit (Amersham Biosciences, Piscataway, NJ). After blocking with 5% skim milk, probing was performed using 5µg/mL ¹¹¹In-labelled 3B9 (prepared as described below). Membranes were washed then exposed to x-ray films (3h at RT) which were developed and documented.

### Determination of Rf value in native PAGE

Native polyacrylamide gel electrophoresis (native PAGE) was used to determine the relative fractionation (Rf) value for unmodified and conjugated antibody. Briefly, 7% native gel was prepared using 100µL APS, 20µL TEMED (Sigma-Aldrich Co., St. Louis, MO), 1.75mL of 40% acrylamide (Biorad®) and 1.25mL of 1.5MTris-HCl (pH 8.6) in a final 10mL volume of milliQ water. Native PAGE running buffer was prepared by dissolving 3.4g of glycine and 1.2g of Tris in 500mL of milliQ water (pH adjusted to 8.5). Unmodified and conjugated antibody were mixed with equal volume of native PAGE loading dye (20% v/v glycerol, 1.5M Tris-HCl and 0.05% w/v bromophenol blue in milliQ water) and samples were loaded onto gel. Electrophoresis was performed at 200V for 2.5h and gel was stained using brilliant blue R250 (BBR250) solution (1g of BBR250 [Sigma-Aldrich Co., St. Louis, MO] in 200mL methanol and 100mL H₂O). The Rf values were determined using the gel analysis program, GelPro Analyzer™ v3.1 (Media Cybernetics Inc., Silver Spring, MD).

### Flow cytometry

Jurkat cell were fixed and permeabilized by incubation at 1x107 cells/mL in 2% paraformaldehyde for 10min, diluted 1:10 in -20°C methanol (5 min) then washed extensively using PBS. Direct immunofluorescent staining was performed using 5µg/mL of 3B9-FITC or 5µg/mL Sal5-FITC. Indirect immunofluorescent assays were performed using permeabilized cells incubated with 5µg/mL of unmodified 3B9, 3B9-DOTA or matching irrelevant isotype (Sal5 or Sal5-DOTA) for 30 min at RT. Cells were washed then incubated in 2µg/mL of goat anti-mouse IgG Alexa₄₈₈-conjugated antibody (Invitrogen, Carlsbad, CA) for 30 min at RT. Cells were washed and samples were acquired using Becton-Dickinson FACScan™ flow cytometry system (BD Biosciences, San Jose, CA). Acquisition was standardized to 10'000 events Flow cytometry data was analyzed using WinMDI v 2.8 (Scripps Research Institute, La Jolla, CA).

Activity of 3B9-DOTA conjugates was tested by their ability to inhibit the binding of 3B9-FITC. Briefly, fixed/permeabilized Jurkat cells were incubated for 30 min at RT with 5µg/mL of 3B9-FITC in the absence or presence of 5, 25, 50 and 100µg/mL of 3B9-DOTA conjugates. Cells were washed and analyzed by flow cytometry as described above.

### Recognition of Fc and Fab regions of DOTA-conjugated IgG

Samples (0.1µg) of unmodified or DOTA-conjugated IgG in native form (PBS) were applied onto nitrocellulose membranes (Amersham Biosciences, Piscataway, NJ). Membranes were dried and blocked with 5% skim milk for 30 min at RT. Membranes were incubated (30 min at RT) with 1µg/mL of goat antibodies conjugated with biotin and directed against the mouse IgG or against the Fc or (Fab)₂ regions of mouse IgG (Rockland, Gilbertsville, PA). Membranes were washed and incubated with 0.1µg/mL streptavidin-alkaline phosphatase (AP) (Rockland, Gilbertsville, PA) for 15 min at RT. Membranes were washed and developed using the BCIP/NBT premixed solution as specified by manufacturer (Sigma-Aldrich Co., St. Louis, MO) and analyzed using GelPro Analyzer™.

### Kinetics of labelling of mAb-DOTA with Terbium

Arsenazo(III) reagent was utilized to study the kinetics of labelling of 3B9-DOTA with Terbium (Tb). TbCl₃ (Sigma-Aldrich Co., St. Louis, MO) was diluted in milliQ water at 20mg/ml. Stock of Arsenazo(III):metal complexes was prepared by mixing 18.75µL of TbCl₃ solution with 1.75mg Arsenazo(III) in 3mL of SM ammonium acetate in a final 10mL of milliQ water. Stock was diluted 1:10 and 90 µL was added to 175µg 3B9-DOTA solutions (10 µL). Absorbance at 630nm was measured kinetically at 37°C every 1 min for 1h.

### Radiolabelling of mAb-DOTA with ¹¹¹In

3B9-DOTA or Sal5-DOTA conjugates were buffer exchanged into 0.2M ammonium acetate (pH 5.5) containing 6mg/mL ascorbic acid by four washes in 100kDa cut off microconcentrators. Indium-111 (¹¹¹InCl3, PerkinElmer Inc., Wellesley, MA) and DOTA-conjugates were incubated for 2 h at 37°C at concentrations of 2mCi/mL (74 MBq/mL) of ¹¹¹In and 2mg/mL of conjugates in (i.e. 1:1 mCi:mg or 37MBq:mg ratio) 0.2M ammonium acetate buffer containing 6mg/mL ascorbic acid. In some cases, ascorbic acid which enhances the incorporation of ¹¹¹In into the conjugates was eliminated from the radiolabelling reaction as interferes with the BCA protein assay kit. Incorporation of ¹¹¹In was measured as described in next section.

For purification of ¹¹¹In-labelled conjugates, radiolabelling reactions were washed twice with 400µL volumes of 0.2M ammonium acetate containing 5mM EDTA (pH 8.0) then 3 times with 400µL volumes of PBS in 100kDa cut off microconcentrators as described earlier. Protein concentration of purified ¹¹¹In-labelled conjugates was measured using the BCA assay kit and the radioactivity concentration was measured using a specified volume placed in gamma counting tubes and counted using Cobra5010 gamma counter (PerkinElmer Inc., Wellesley, MA) which was normalized for ¹¹¹In counting at 100-350 keV window. Specific radioactivity was measured as cpm from gamma counter per µg of protein present in the counted volume.

### Instant thin Layer chromatography

Incorporation efficiency was measured using instant thin layer chromatography (ITLC). Briefly, 2µL aliquots of labelling reactions were mixed with 2µL of 0.2M ammonium acetate containing 5mM EDTA (pH 8.0) and applied at 1 cm from the bottom of 1x9 cm ITLC-SG strips (Pall Corporation, East Hills, NY). Chromatography was performed in 0.2M ammonium acetate containing 5mM EDTA (pH 8.0) as mobile phase and was stopped when solvent front reached 1 cm from the top of the strips. Strips were cut in two halves where the bottom half represented the origin (ori) and the top half represented the solvent front (SF). The activity of the two halves was measured using gamma counter. Incorporation efficiency was calculated as the percentage of activity remaining at the origin to the total activity of the strip [% ori/(ori + SF)].

### In vivo biodistribution of DOTA-conjugates

We have previously described an *in vivo* model of apoptosis which we have shown specific and preferential accumulation of 3B9 in tumors of chemotherapy treated mice (Al-Ejeh *et al.,* 2007). Briefly, EL4 thymic lymphoma cells (1x10⁶ cells) were injected subcutaneously in the right flanks of 6-8 weeks old C57BL/6 female mice. Mice were housed and treated as per protocols approved by the Animal Ethics Committee at The University of Adelaide and the Animal Ethics Committee at the Institute of Medical and Veterinary Sciences (IMVS). Once the tumor reached 1cm diameter, mice were treated by intraperitoneal injections of cytotoxic chemotherapy (38mg/kg etoposide and 50mg/kg cyclophosphamide) given at time 0 and 24h. DOTA-conjugates labelled with ¹¹¹In (100µg) was injected intravenously at time 0. Mice were euthanized at 48h, blood was collected by cardiac puncture, and tumors and organs were collected. Blood and organs were weighed and placed in gamma-counter tubes and radioactivity was measured using gamma counter. Radioactivity in organs was normalized to the weight of the organs and accumulation was calculated as the percentage of radioactivity per gram in the organs to the radioactivity of the injected dose at time 0 (%ID/g).

### Statistical analysis

Statistical comparisons were preformed using GraphPad Prism v.4 (GraphPad Software, San Diego, CA). Two-way analysis of variances (2-way ANOVA) was used to deduce significant differences in the results. The Bonferroni post-test in the 2-way ANOVA function in GraphPad prism was used to report P values. P values less than 0.05 were considered significant where one, two and three asterisks denote P values less than 0.05, 0.01 and 0.001, respectively.

### Results

### Modification of Cu:Arsenazo(III) assay

Previously described assays for the measurement of metal chelators attached to antibodies uses a standard curve for the absorbance (deep blue color) of Cu:Arsenazo(III) solutions of different concentrations. The absorbance of this reagent when mixed with immunoconjugates is then used to interpolate (from standard Cu:Arsenazo(III) curve) the concentration of Cu remaining after chelation which allows calculation of chelated Cu thus representing the concentration of metal chelator (Pippin *et al*. 1992 *supra*; Dadachova *et al.* 1999 *supra*; Brady *et al.* 2004 *supra*). We found that while Cu:Arsenazo(III) standard curve represented dilutions of the deep blue color of the reagent (Figure 43A), mixtures of the reagent with increasing concentrations of free DOTA (Figure 43B), DTPA or EDTA (data not shown) does not follow the dilution of the reagent itself. This was clearly evident when using high concentration of the metal chelator where chelation of Cu resulted in the disappearance of the blue color and the appearance of the color related to Arsenazo(III) solution (Figure 43C). Therefore, the assay was modified where Cu:Arsenazo(III) reagent mixed with standard solutions of metal chelators at different concentrations was used to construct standard curves for the relationship between absorbance and the concentration of metal chelator (Figure 43C). The absorbance of immunoconjugates mixed with the reagent was directly used to interpolate the concentration of metal chelator from the standard curve.

### DOTA/antibody ratio is manipulated by conjugation condition

Conjugation of 3B9 with DOTA-NHS-ESTER was performed in three separate occasions. The average DOTA/antibody ratios were 2.8±0.3, 8.2±0.3, 11.6±1.1 and 15.0±0.7 for the conjugation conditions of 50-, 100-, 150- and 200-fold molar excess of DOTA-NHS-ESTER to 3B9 (Figure 44A). In correlation with increased DOTA/antibody ratio with increasing concentration of DOTA-NHS-ETSER, the Rf value of 3B9 increased in native PAGE with the addition of more DOTA molecules. As shown in Figure 44B, the average Rf value for unmodified 3B9 was 0.23±0.01 whereas the average Rf values were 0.65±0.01, 0.82±0.01, 0.92±0.01 and 0.97±0.01 when it was conjugated at 50-, 100-, 150- and 200-fold molar excess of DOTA-NHS-ESTER. This change in the Rf value with increasing the DOTA/antibody ratio is expected due to the replacement of the positive charge on the primary amine residues on the antibody by three negative charges on the carboxyl-groups of attached DOTA.

### Increased conjugation affects antibody avidity

The avidity of DOTA-conjugated 3B9 was investigated using competition binding assays. As shown in Figure 45, 3B9-DOTA conjugates competed the binding of 33.3nM 3B9-FITC. The IC₅₀ for competition was 64±4, 128±8, 155±9 and 223±13nM for conjugates prepared at 50-, 100-, 150- and 200-fold molar excess of DOTA-NHS-ESTER. The IC₅₀ was significantly higher for the 100-fold conjugates (*P<0.01*), 150-fold conjugates (*P*<*0*.*001*) and 200-fold conjugates (*P*<*0*.*001*) compared to that for the 50-fold conjugates. This indicated a significant loss of avidity with increasing the concentration of DOTA-NHS-ESTER in the conjugation reaction. In fact, a linear correlation described the relationship between the IC₅₀ of 3B9-DOTA conjugates and their DOTA/antibody ratios (Figure 45 - inset).

### Modification of Fc and Fab region during conjugation

Detection of 3B9-DOTA conjugates using antibodies against whole IgG molecule or antibodies against the Fc and (Fab)₂ regions decreased significantly compared to that of unmodified 3B9. Samples of 3B9 and 3B9-DOTA conjugates fractioned in non-reducing SDS-PAGE showed comparable amounts of protein in these samples as judged by the intensity of BBR250 staining (Figure 46A). Probing of identical samples of 3B9 and 3B9-DOTA applied onto nitrocellulose using secondary antibodies decreased with increasing concentration of DOTA-NHS-ESTER added to the conjugation reaction (Figure 46B). The optical density of detection using secondary antibodies was normalized to the optical density of BBR250 staining and plotted as a function of the DOTA/antibody ratio in these preparations (Figure 46C). These data indicate modification of the Fab region and to a larger extent the Fc region which were significant enough to affect recognition by secondary antibodies.

Further evidence for the modification of 3B9 during conjugation both in structure and avidity was obtained using indirect immunofluorescent staining of permeabilized Jurkat cells. As shown in Figure 46D, increased DOTA/antibody ratio was associated with a decline in the mean fluorescent intensity (MFI) for target staining in permeabilized Jurkat cells.

### Chelation rate and metal loading capacity is affected by the DOTA/antibody ratio

The absorbance of Terbium:Arsenazo(III) reagent mixed with 3B9-DOTA conjugates decreased during 1h incubation at 37°C (Figure 47A), indicating chelation of terbium as seen in previous Cu:Arsenazo(III) assays. The rate of chelation (min⁻¹) was measured for the one-phase exponential decay model fitted using GraphPad (*r²*=0.90) and showed a linear correlation with the DOTA/antibody ratio (Figure 47A-inset). This indicated that both the rate of chelation and the amount of chelated metal is directly proportional to the amount of DOTA attached to the antibody. Additional evidence for the increased metal loading capacity with increased DOTA/antibody ratio was obtained from the specific radioactivity of ¹¹¹In-labelled 3B9-DOTA (Figure 47B). It is noteworthy that both the heavy and light chains were labelled with ¹¹¹In as judged from the autoradiograph of reducing SDS-PAGE fractionation of ¹¹¹In-DOTA-3B9 samples (Figure 47B-inset), confirming the modification of both chains during conjugation.

### Modulation of antibody properties during conjugation is reflected in vivo

There has been reported the specific and saturable accumulation of 3B9 in EL4 tumors after cytotoxic chemotherapy treatment compared to the irrelevant control of matched isotype, Sal5 (Al-Ejeh *et al*., 2007). This model was used to investigate the significance of the above described modification of antibody properties after conjugation *in vivo.* Biodistribution of 3B9-DOTA in chemotherapy treated mice was affected when prepared at 200-fold molar excess of DOTA-NHS-ESTER compared to that prepared at 50-fold excess (Figure 48). Accumulation was significantly lower in the tumor (*P*<*0*.*001*) and blood (*P<0.05*) and significantly higher (*P*<*0*.*001*) in the liver and spleen of mice injected with 3B9-DOTA prepared at 200-fold molar excess of DOTA-NHS-ESTER compared to that prepared using 50-fold excess (Figure 48).

### EXAMPLE 10

### BIODISTRIBUTION OF Apomab TRACER AND CORRELATION OF ITS UPTAKE WITH CHEMOTHERAPY-INDUCED TUMOUR APOPTOSIS IN VIVO

### Materials and Methods

### Tumour model description

EL4 murine lymphoma model: cultured EL4 cells were collected and 1x10⁶ cells injected subcutaneously in the right flank of 6-8 week-old female mice of the syngeneic C57BL/6J strain. Tumours were left to grow for 7-8 days to reach 100mm³ volume before initiation of any of experiments. (This model yields tumours 100% of the time).

### Results

### Biodistribution of Apomab in EL4-tumour bearing mice under different schedules of chemotherapy and antibody injection

Accumulation of Apomab labelled with ¹¹¹Indium was traced overtime in treated mice where ¹¹¹In-Apomab was injected either concurrently with CE chemotherapy or 24h after chemotherapy (Figure 50). Scheduling of Apomab injection 24h after chemotherapy administration resulted in faster accumulation in the tumour and enhanced tumour/organ ratios.

### Immunohistochemistry of control and chemotherapy-treated EL4-tumour bearing mice

C57BL/6 mice were injected with EL4 cells. Tumours were grown for 1 week and mice were divided into three groups for treatments described below.
1) Control
2) 9.5mg/kg etoposide and 12.5mg/kg cyclophosphamide
3) 19mg/kg etoposide and 25mg/kg cyclophosphamide

Mice were sacrificed at 24, 48, 72 and 96h after chemotherapy administration (3 mice per time point) and tumours fixed in formalin, paraffin-embedded and stored for immunohistochemistry (IHC). Staining was performed by Jim Manavis (IMVS) for the cell death marker (activated caspase-3) (Figure 51).

### Gamma-camera imaging/gamma-counter biodistribution of Apomab in control and chemotherapy-treated EL4-tumour bearing mice

C57BL/6 mice were injected with EL4 cells. Tumours were grown for 1 week and mice then divided into three groups for treatments described below administered.
1) Control
2) 9.5mg/kg etoposide and 12.5mg/kg cyclophosphamide
3) 19mg/kg etoposide and 25mg/kg cyclophosphamide

Apomab labelled with ¹¹¹In was administered in all mice and 3 mice from each group were killed at 3, 24, 72 and 96 after Apomab injection and gamma-camera images were obtained at RAH Department of Nuclear Medicine (Figure 52). Mice were dissected after image acquisition for counting organs using gamma-counter (Figure 53).

Maximum binding of Apomab in control mice and mice treated with 1/8 1d or 1/4 1d CE chemotherapy was calculated from gamma camera image analysis (Figure 52) and from gamma counting biodistribution (Figure 53) and expressed as the maximum number of pixels per unit area (Max. Pixels/Area; Figure 54) and Max. %ID/g (Figure 55), respectively. The time course of apoptosis or necrosis in each tumour was measured using video image analyses of appropriately stained tumour tissue sections (Figure 51) and used to calculate cumulative indices of apoptosis (Figure 56) or necrosis (Figure 57). As shown in Figure 58A, tumour accumulation of radiolabelled Apomab correlated directly with a cumulative apoptotic index in a CE-chemotherapy dose-dependent manner In contrast, control tumours of untreated mice displayed significantly higher levels of necrosis than tumours of CE-treated mice. Consequently, the cumulative necrotic index was inversely related with tumour accumulation of radio labelled Apomab (Figure 58B).

### IHC and radioimmunoimaging of size-matched chemotherapy-treated EL4 tumours and control EL-4 tumours

Previous experiments using the EL4 tumour model indicated relationship between tumour size/mass and response to chemotherapy as well as accumulation of Apomab in correlation to the mode of cell death. This current study was design to investigate the effect of tumour mass/volume on Apomab binding to EL4 tumours in control mice and mice treated with CE-chemotherapy at two different doses; 1/8 1d (half-dose) or 1/4 1d (full-dose) regimens.

C57BL/6 female (6-8 weeks old) mice were inoculated with identical number of EL4 cells subcutaneously at the right hindquarter on day 1, 2, 3, 4 and 5 (18 mice per day). On day 7, mice from each inoculation day were divided into 3 groups of 6 mice each to produce 3 cohorts which contained 5 groups of 6 mice per group with each group being inoculated with EL4 cell on a different day (3 cohorts x 5 groups [inoculated d1, 2, 3, 4, and 5] x 6 mice = 90 mice). One cohort was left untreated (control), one was injected with 1/8 1d CE treatment (1/8 chemo; half-dose) and one with 1/4 1d CE treatment (1/4 chemo; full-dose). On day 8, each of these three cohorts was further divided into two identical cohorts containing 5 groups of 3 mice per group:
A. 2 cohorts x 5 groups [inoculated d1, 2, 3, 4, and 5] x 3 mice = 30 mice for control
B. 2 cohorts x 5 groups [inoculated d1, 2, 3, 4, and 5] x 3 mice = 30 mice for 1/8 chemo (half-dose)
C. 2 cohorts x 5 groups [inoculated d1, 2, 3, 4, and 5] x 3 mice = 30 mice for 1/4 chemo (full-dose)

One cohort from A, B and C was injected with ¹¹¹In-Apomab for radio-imaging and radio-counting studies while the other cohort from A, B and C was left for IHC analysis. On day 10, all IHC mice were killed by cardiac bleeding and cervical dislocation. Tumours were dissected and stored in 10% formalin overnight, embedded in paraffin then subjected to immunohistochemistry staining of activated caspase-3. Tumour volumes were measured using callipers before formalin fixation. On the same day, mice injected with ¹¹¹In-Apomab were killed by inhalation of anaesthetic and imaged using GE millennium clinical gamma camera (10 min acquisition per image using acquisition windows suitable for ¹¹¹Indium; peaks at 171 and 245 keV). Two mice from control, 1/8 chemo (half-dose) and 1/4 chemo (full-dose) cohorts were selected based on matched tumour volume (measured from IHC study) and dissected immediately after image acquisition to collect tumours. All mice were then dissected and organs collected for radioactivity counting using the Cobra 5010 gamma counter. Organs were weight and accumulation was measured as radioactivity counts standardised to the mass of counted sample and calculated as a percentage of the injected dose (%ID/g).

Mice were imaged using GE Millennium clinical gamma camera before dissection. Images of two representative mice from each group at all time points are shown in Figure 60. Gamma counts of ¹¹¹In-Apomab injected 48h before killing (day 8) was determined for tumours from all mice with all the different tumour sizes after dissection using Cobra gamma counter (Figure 61A, C and E). Pixels from gamma camera images was determined for tumours using ImageJ image analysis software (Figure 61B, D and F). Gamma counts and image pixels were plotted in correlation of tumour mass as shown in Figure 61. Furthermore, biodistribution of ¹¹¹In-Apomab expressed as %ID/g was determined for all organs using the gamma counts and the mass of counted organs (Figure 62). Based on Figure 59, size matched tumours from control mice (6 days of growth), half chemo (8 days of growth) and full chemo (10 days of growth) treated mice were selected for comparison in biodistribution (Figure 62D). Tumour accumulation from biodistribution and gamma camera image analysis were correlated to the dose of chemotherapy used (Figure 62).

### Localisation of bound-Apomab in EL4 tumours

C57BL/6 mice were injected with EL4 tumours on day 0, day 2 or day 4. Mice with EL4 cell implanted on day 0 and day 2 were injected with full or half dose chemotherapy on day 7, respectively. All mice received an i.v. injection of Apomab-biotin or Sal5-biotin (3 mice per treatment per antibody) on day 8 and killed on day 10 for tumour dissection. Control tumours and tumours from mice treated with half and full chemo were grown for 6, 8 and 10 days on day of collection, respectively. Tumours were fixed in formalin, embedded in paraffin and sectioned for probing with streptavidin-HRP to detect antibody binding via IHC (Fig. 64).

### Imaging of EL4 tumour response to chemotherapy using Apomab in Fab₂ vs. IgG forms

Mice to be treated with full dose of chemotherapy were injected with EL4 cells (18 mice). Control mice were injected with EL4 cells (18 mice). Chemotherapy was administered on the 4th of June 2007. Groups of 3 mice were injected with ¹¹¹In-labelled antibody as follows:
1) Control mice injected with ¹¹¹In-3B9 (2 groups of 3 mice)
2) Chemo-treated mice injected with ¹¹¹In-3B9 (2 groups of 3 mice)
3) Control mice injected with Fab2 of ¹¹¹In-Sal5 (2 groups of 3 mice)
4) Control mice injected with Fab2 of ¹¹¹In-3B9 (2 groups of 3 mice)
5) Chemo-treated mice injected with Fab2 of ¹¹¹In-Sal5 (2 groups of 3 mice)
6) Chemo-treated mice injected with Fab2 of ¹¹¹In-3B9 (2 groups of 3 mice)

One group of cohorts 1 and 2 was imaged (24h after RIC injection) while the other group was imaged (48h after RIC injection). One group of cohorts 5-8 were injected with D-lysine solution 4 times at 2h intervals starting 30 min before RIC injection (40mg/injection in 200µL). Groups from cohorts 5-8 (without or with D-Lysine injections) were images at 24h after RIC injection. All mice were dissected after imaging and biodistribution (%ID/g) was calculated.

As shown in Figure 65B, injection of D-Lysine decreased accumulation of ¹¹¹In-labelled F(ab)2 fragments in the kidney compared (cf. Fig 65A). This inhibition of renal uptake was associated with high specific accumulation of ¹¹¹In-labelled F(ab)₂ of 3B9 in tumours specially after chemotherapy. Specific detection of chemotherapy response using F(ab)₂ fragment of 3B9 was at better resolution (Figure 65B lower panel) than that using whole IgG of 3B9 (Figure 65C upper panel at 24h and lower panel at 48h). Image analysis was used to quantify signals (pixels per cm²) in order to compare tumour accumulation (Figure 66).

Biodistribution of IgG Apomab in control and chemotherapy treated mice is shown in Figure 67. Tumour accumulation was significantly higher after chemotherapy at 24 and 48h (*P*<0.001). Interestingly, spleen accumulation was significantly higher in chemotherapy-treated mice compared to control mice at 24h than 48h. However this difference may be explained by Fc mediated effect as it was not evident using F(ab)₂ fragments (Figure 68). Based on the data from F(ab)₂ fragments specially using D-lysine to reduce renal uptake, this form or RIC preparation appears to enhance the resolution of Apomab tumour imaging. Furthermore, Fc-mediated binding may play a role in background accumulation in the EL4 tumours and the spleens (or livers as seen in some previous assays). Furthermore, one would suggest dose study of Apomab injection (both IgG and F(ab)₂ forms) to determine the lowest dose which gives appropriate resolution for tumour response measurement (i.e. highest tumour accumulation and lowest body level at the earliest time point after RIC administration).

Those skilled in the art will appreciate that the invention described herein is susceptible to variations and modifications other than those specifically described. It is to be understood that the invention includes all such variations and modifications. The invention also includes all of the steps, features, compositions and compounds referred to or indicated in this specification, individually or collectively, and any and all combinations of any two or more of said steps or features.

### BIBLIOGRAPHY

Brown and Attardi, 2004, Nature Reviews Cancer 5:231-237
Dong G et al. EMBO J 23, 1000-1007, 2004
Duncan (Nature Reviews Drug Discovery, 2:347-360, 2003)
Erlich, 1989 (J Clin Immunol 9(6):437-47
Ferrari (Nature Reviews Cancer 5:161-171, 2005)
Gorer, British Journal of Cancer 4: 372-379, 1950
Laemmli (Nature 227: 680-685, 1970
Maraia J. J Cell Biol 153, F13-18, 2001
Milas L et al. Cancer Res 59, 107-114, 1999
Moore et al., BBA, 1402:239-249, 1988
Mullis et al., 1987; (Methods Enzymol 155:335-50)
Tran *et al*., 2002
Wedemeyer et al., Clinical Chemistry 48:9 1398-1405, 2002
Weissleder et al., Nature Medicine, 6:351-355, 2000
Wolin SL and Tommy Cedervall T. Annu Rev Biochem 71, 375-403, 2002
Phillips HS, Kharbanda S, Chen R, et al. Molecular subclasses of high-grade glioma predict prognosis, delineate a pattern of disease progression, and resemble stages in neurogenesis. Cancer Cell 2006;9: 157-73
Shai R, Shi T, Kremen TJ, et al. Gene expression profiling identifies molecular subtypes of gliomas. Oncogene 2003;22: 4918-23.
Wang Y, Klijn JG, Zhang Y, et al. Gene-expression profiles to predict distant metastasis of lymph-node-negative primary breast cancer. Lancet 2005;365: 671-9 Bild AH, Yao G, Chang JT, et al. Oncogenic pathway signatures in human cancers as a guide to targeted therapies. Nature 2006;439: 353-7.
Notterman DA, Alon U, Sierk AJ, Levine AJ. Transcriptional gene expression profiles of colorectal adenoma, adenocarcinoma, and normal tissue examined by oligonucleotide arrays. Cancer Res 2001;61: 3124-30
Chen X, Cheung ST, So S, et al. Gene expression patterns in human liver cancers. Mol Biol Cell 2002;13: 1929-39.
Bhattacharjee A, Richards WG, Staunton J, et al. Classification of human lung carcinomas by mRNA expression profiling reveals distinct adenocarcinoma subclasses. Proc Natl Acad Sci U S A 2001;98: 13790-5.
Hummel M, Bentink S, Berger H, et al. A biologic definition of Burkitt's lymphoma from transcriptional and genomic profiling. N Engl J Med 2006;354: 2419-30
Singh D, Febbo PG, Ross K, et al. Gene expression correlates of clinical prostate cancer behavior. Cancer Cell 2002;1: 203-9.
Korkola JE, Houldsworth J, Chadalavada RS, et al. Down-regulation of stem cell genes, including those in a 200-kb gene cluster at 12p13.31, is associated with in vivo differentiation of human male germ cell tumors. Cancer Res 2006;66: 820-7.
Ramaswamy S, Tamayo P, Rifkin R, et al. Multiclass cancer diagnosis using tumor gene expression signatures. Proc Natl Acad Sci U S A 2001;98: 15149-54.
Garber ME, Troyanskaya OG, Schluens K, et al. Diversity of gene expression in adenocarcinoma of the lung. Proc Natl Acad Sci U S A 2001;98: 13784-9.
albot SG, Estilo C, Maghami E, et al. Gene expression profiling allows distinction between primary and metastatic squamous cell carcinomas in the lung. Cancer Res 2005;65: 3063-71.
Toruner GA, Ulger C, Alkan M, et al. Association between gene expression profile and tumor invasion in oral squamous cell carcinoma. Cancer Genet Cytogenet 2004;154: 27-35.
Freije WA, Castro-Vargas FE, Fang Z, et al. Gene expression profiling of gliomas strongly predicts survival. Cancer Res 2004;64: 6503-10.
Ginos MA, Page GP, Michalowicz BS, et al. Identification of a gene expression signature associated with recurrent disease in squamous cell carcinoma of the head and neck. Cancer Res 2004;64: 55-63.
Welsh JB, Zarrinkar PP, Sapinoso LM, et al. Analysis of gene expression profiles in normal and neoplastic ovarian tissue samples identifies candidate molecular markers of epithelial ovarian cancer. Proc Natl Acad Sci U S A 2001;98: 1176-81.
Lu KH, Patterson AP, Wang L, et al. Selection of potential markers for epithelial ovarian cancer with gene expression arrays and recursive descent partition analysis. Clin Cancer Res 2004;10: 3291-300.
Carson JP, Zhang N, Frampton GM, Gerry NP, Lenburg ME, Christman MF. Pharmacogenomic identification of targets for adjuvant therapy with the topoisomerase poison camptothecin. Cancer Res 2004;64: 2096-104.
Pawitan Y, Bjohle J, Amler L, et al. Gene expression profiling spares early breast cancer patients from adjuvant therapy: derived and validated in two population-based cohorts. Breast Cancer Res 2005;7: R953-64.
Sorlie T, Perou CM, Tibshirani R, et al. Gene expression patterns of breast carcinomas distinguish tumor subclasses with clinical implications. Proc Natl Acad Sci U S A 2001;98: 10869-74.
Lapointe J, Li C, Higgins JP, et al. Gene expression profiling identifies clinically relevant subtypes of prostate cancer. Proc Natl Acad Sci U S A 2004;101: 811-6.
Tran, H. B.; Ohlsson, M.; Beroukas, D.; Hiscock, J.; Bradley, J.; Buyon, J. P.; Gordon, T. P., Subcellular redistribution of la/SSB autoantigen during physiologic apoptosis in the fetal mouse heart and conduction system: a clue to the pathogenesis of congenital heart block. Arthritis Rheum 2002, 46,(1), 202-8.
Pippin, C. G.; Parker, T. A.; McMurry, T. J.; Brechbiel, M. W., Spectrophotometric method for the determination of a bifunctional DTPA ligand in DTPA-monoclonal antibody conjugates. Bioconjug Chem 1992, 3, (4), 342-5.
Dadachova, E.; Chappell, L. L.; Brechbiel, M. W., Spectrophotometric method for determination of bifunctional macrocyclic ligands in macrocyclic ligand-protein conjugates. Nucl Med Biol 1999, 26, (8), 977-82.
Brady, E. D.; Chong, H. S.; Milenic, D. E.; Brechbiel, M. W., Development of a spectroscopic assay for bifunctional ligand-protein conjugates based on copper. Nucl Med Biol 2004, 31, (6), 795-802.
Laemmli, U. K., Cleavage of structural proteins during the assembly of the head of bacteriophage T4. Nature 1970, 227, 680-685.

## Claims

1. An *in vitro* method for detecting a neoplastic cell in a subject, assessing or monitoring a neoplastic condition in a subject, or assessing and/or monitoring the effectiveness of a neoplastic therapeutic treatment regime in a subject, said method comprising screening for a change in the level of La protein and/or gene expression in cells in a biological sample derived from said subject, wherein
(a) to detect a neoplastic cell or to assess or monitor a neoplastic condition said method comprises correlating the levels of La protein and/or gene expression in cells in said sample to a normal level of La protein and/or gene expression in a biological sample from a subject who has not developed a neoplastic condition, wherein an increase in the level of said La expression in cells of the subject relative to normal La expression levels is indicative of a neoplastic cell,
(b) to detect a dead neoplastic cell, which cell death has been induced by a DNA damaging agent, or
(c) to assess and/or monitor the effectiveness of a neoplastic therapeutic treatment regime in a subject,
said method comprises screening for the level of La protein and/or gene expression in dead cells in said biological sample and correlating said level to the level of La expression in viable neoplastic cells, wherein an increase in the level of La expression in said dead cells relative to viable neoplastic cell levels is indicative of DNA damage induced neoplastic cell death, and hence of the presence of a neoplastic cell rendered dead by a DNA-damaging agent or the effectiveness of said therapeutic treatment regime.

2. The method according to claim 1 wherein said DNA damaging agent is:
(a) a cytotoxic agent; or
(b) selected from:
(i) a radioisotope;
(ii) gemcitabine together with a CHK1/2 inhibitor;
(iii) irinotecan together with a CHK1/2 inhibitor;
(iv) a histone deacetylase inhibitor;
(v) tumour necrosis factor related apoptosis-inducing ligand.

3. The method according to claim 2 wherein:
(a) said cytotoxic agent is selected from Actinomycin D, Arsenic Trioxide, Bleomycin, Busulfan, Carboplatin, Carmustine, Chlorambucil, Cisplatin, Cyclophosphamide, Daunorubicin, Doxorubicin, Epirubicin, Etoposide, Hydroxyurea, Idarubicin, Ifosfamide, Irinotecan, Lomustine, Melphalan, Mitomycin, Mitoxantrone, Oxaliplatin, Procarbizine, Streptozocin, Thiotepa, Topotecan, Treosulfan;
(b) said CHK1/2 inhibitor is CBP-501 or AZD7762; or
(c) said histone deacetylase inhibitor is vorinostat or a BH3 mimetic such as ABT737.

4. The method according to any one of claims 1 to 3 wherein said neoplasm is a central nervous system tumour, retinoblastoma, neuroblastoma, paediatric tumour, a head and neck cancer such as squamous cell cancer, breast cancer, prostate cancer, lung cancer, kidney cancer such as renal cell adenocarcinoma, oesophagogastric cancer, hepatocellular carcinoma, pancreaticobiliary neoplasia such as adenocarcinoma and islet cell tumour, colorectal cancer, cervical cancer, anal cancer, uterine or other reproductive tract cancer, urinary tract cancer such as of the ureter or bladder, a germ cell tumour such as a testicular germ cell tumour or an ovarian germ cell tumour, ovarian cancer such as ovarian epithelial cancer, carcinoma of unknown primary human immunodeficiency associated malignancy such as Kaposi's sarcoma, a lymphoma, leukemia, a malignant melanoma, a sarcoma, an endocrine tumour such as of the thyroid gland, mesothelioma or other pleural or peritoneal tumour, a neuroendocrine tumour or a carcinoid tumour.

5. The method according to any one of claims 1 to 4 wherein said neoplasm is malignant.

6. The method according to any one of claims 1 to 5 wherein La is detected by an immunointeractive molecule directed to the La protein or fragment thereof.

7. The method according to claim 6 wherein said immunointeractive molecule is an antibody or fragment thereof or a T-cell associated antigen-binding molecule.

8. The method according to claim 7 wherein said antibody is a monoclonal antibody or a fragment thereof or a polyclonal antibody or a fragment thereof, optionally wherein said antibody or antibody fragment is associated with a reporter molecule.

9. The method according to any one of claims 7 to 8 wherein said antibody or fragment thereof is selected from a single chain antibody, a deimmunised antibody, an Fv, Fab, Fab', or F(ab')₂ fragment, a single chain Fv fragment, an antibody, a disulphide-stabilised Fv fragment, or a single variable region domain (dAb).

10. The method of claim 9 wherein the deimmunised antibody is a humanised antibody.

11. The method according to claim 8 wherein said reporter molecule is selected from a radioisotope, a chromogen, a catalyst, an enzyme, a fluorochrome, a chemiluminescent molecule, a paramagnetic ion, a lanthanide ion, a colloidal metallic or non-metallic particle, a dye particle, an organic polymer, a latex particle, a liposome or a vesicle containing a signal-producing substance.

12. The method according to claim 11 wherein said radioisotope is Carbon-11, Copper-64, Fluorine 18, Gallium-68, Indium-111, Lutetium-177, Nitrogen-13, Iodine-131, Iodine-124, Oxygen-15, Rhenium-186/188, or Technetium-99.

13. The method according to any one of claims 1 to 12 wherein said La is localised to the cytoplasm.

14. The method according to any one of claims 6 to 13 wherein said immunointeractive molecule becomes fixed.

15. The method according to any one of claims 1 to 14 wherein said subject is a human.

16. An immunointeractive molecule capable of disclosing altered expression levels of La protein expression product for *in vivo* use in a diagnostic or prognostic method for
(a) the diagnosis or prognosis of a neoplastic condition in a subject, wherein said diagnosis or prognosis comprises
(i) detecting a neoplastic cell in said subject, by screening for the level of La protein expression in dead cells in said subject and correlating this to a normal level of La protein expression in a subject who has not developed a neoplastic condition wherein an increase in the level of said La expression relative to normal La expression levels is indicative of a neoplastic cell; or
(ii) detecting a dead neoplastic cell in said subject, which cell death has been induced by a DNA damaging agent, by screening for the level of La protein expression in dead cells in said subject and correlating said level to the level of La expression in viable neoplastic cells, wherein an increase in the level of La expression relative to viable neoplastic cell La expression levels is indicative of DNA damage induced neoplastic cell death; or
(b) a method for assessing and/or monitoring a neoplastic condition or the effectiveness of a neoplastic therapeutic treatment regime in a subject, said method comprising screening for the level of La protein expression in dead cells in said subject, and correlating said level to the level of La expression to a normal level of La expression or to the level of La expression in viable neoplastic cells, wherein an increase in the level of La in said cells relative to normal levels is indicative of a neoplastic cell and an increase in the level of La in dead cells relative to viable neoplastic cell levels is indicative of the presence of DNA damage induced neoplastic cell non-viability;
wherein said immunointeractive molecule is an antibody or a fragment thereof, or a T-cell associated antigen-binding molecule, and
wherein said immunointeractive molecule is coupled to an imaging agent to enable detection.

17. The immunointeractive molecule for use according to claim 16 wherein said DNA damaging agent is:
a) a cytotoxic agent; or
b) selected from:
(i) a radioisotope;
(ii) gemcitabine together with a CHK1/2 inhibitor;
(iii) irinotecan together with a CHK1/2 inhibitor;
(iv) a histone deacetylase inhibitor;
(v) tumour necrosis factor related apoptosis-inducing ligand.

18. The immunointeractive molecule for use according to claim 17 wherein:
(a) said cytotoxic agent is selected from Actinomycin D, Arsenic Trioxide, , Bleomycin, Busulfan, Carboplatin, Carmustine, Chlorambucil, Cisplatin, Cyclophosphamide, Daunorubicin, Doxorubicin, Epirubicin, Etoposide, Hydroxyurea, Idarubicin, Ifosfamide, Irinotecan, Lomustine, Melphalan, Mitomycin, Mitoxantrone, Oxaliplatin, Procarbizine, Streptozocin, Thiotepa, Topotecan, Treosulfan;
(b) said CHK1/2 inhibitor is CBP-501 or AZD7762; or
(c) said histone deacetylase inhibitor is vorinostat or a BH3 mimetic such as ABT737.

19. The immunointeractive molecule for use according to any of claims 16 to 18 wherein said neoplasm is a central nervous system tumour, retinoblastoma, neuroblastoma, paediatric tumour, a head and neck cancer such as squamous cell cancer, breast cancer, prostate cancer, lung cancer, kidney cancer such as renal cell adenocarcinoma, oesophagogastric cancer, hepatocellular carcinoma, pancreaticobiliary neoplasia such as adenocarcinoma and islet cell tumour, colorectal cancer, cervical cancer, anal cancer, uterine or other reproductive tract cancer, urinary tract cancer such as of the ureter or bladder, a germ cell tumour such as a testicular germ cell tumour or an ovarian germ cell tumour, ovarian cancer such as ovarian epithelial cancer, carcinoma of unknown primary human immunodeficiency associated malignancy such as Kaposi's sarcoma, a lymphoma, leukemia, a malignant melanoma, a sarcoma, an endocrine tumour such as of the thyroid gland, mesothelioma or other pleural or peritoneal tumour, a neuroendocrine tumour or a carcinoid tumour.

20. The immunointeractive molecule for use according to any one of claims 16 to 19 wherein said neoplasm is malignant.

21. The immunointeractive molecule for use according to any one of claims 16 to 20 wherein said antibody is a monoclonal antibody or a fragment thereof or is a polyclonal antibody or a fragment thereof.

22. The immunointeractive molecule for use according to claim 21 wherein said antibody or fragment thereof is selected from a single chain antibody, a deimmunised antibody, an Fv, Fab, Fab', or F(ab')₂ fragment, a single chain Fv fragment, an antibody, a disulphide-stabilised Fv fragment, or a single variable region domain (dAb).

23. The immunointeractive molecule for use according to claim 22 wherein the deimmunised antibody is a humanised antibody.

24. The immunointeractive molecule for use according to claim 16 wherein said imaging agent is selected from a radioisotope, a PET imaging agent, a chromogen, a catalyst, an enzyme, a fluorochrome, a chemiluminescent molecule, a paramagnetic ion, a lanthanide ion, a colloidal metallic or non-metallic particle, a dye particle, an organic polymer, a latex particle, a liposome or an advanced functional nanoparticle.

25. The immunointeractive molecule for use according to claim 24 wherein said radioisotope is Carbon-11, Copper-64, Fluorine 18, Gallium-68, Indium-111, Lutetium-177, Nitrogen-13, Iodine-131, Iodine-124, Oxygen-15, Rhenium-186/188, or Technetium-99.

26. The immunointeractive molecule for use according to any one of claims 16 to 25 wherein said La is localised to the cytoplasm.

27. The immunointeractive molecule for use according to any one of claims 16 to 26 wherein said immunointeractive molecule becomes fixed.

28. The immunointeractive molecule for use according to any one of claims 16 to 27 wherein said subject is a human.

## Patentansprüche

1. *In*-*vitro-*Verfahren zum Detektieren einer neoplastischen Zelle in einem Individuum, zum Überprüfen oder Überwachen eines neoplastischen Zustands in einem Individuum oder zum Überprüfen und/oder Überwachen der Effektivität eines neoplastischen therapeutischen Behandlungsschemas in einem Individuum, wobei das Verfahren das Screenen hinsichtlich einer Veränderung des La-Protein- und/oder -Genexpressionsniveaus in Zellen in einer von dem Individuum abgeleiteten biologischen Probe umfasst, wobei
(a) das Verfahren, um eine neoplastische Zelle zu detektieren oder einen neoplastischen Zustand zu überprüfen oder zu überwachen, das In-Relation-setzen der La-Protein- und/oder -Genexpressionsniveaus in Zellen in der Probe mit einem normalen La-Protein- und/oder -Genexpressionsniveau in einer biologischen Probe von einem Individuum, das keinen neoplastischen Zustand entwickelt hat, umfasst, wobei eine Erhöhung des La-Expressionsniveaus in Zellen des Individuums relativ zu normalen La-Expressionsniveaus eine neoplastische Zelle anzeigt, wobei,
(b) um eine tote neoplastische Zelle zu detektieren, deren Zelltod durch ein DNA-schädigendes Agens induziert worden ist, oder
(c) um die Effektivität eines neoplastischen therapeutischen Behandlungsschemas in einem Individuum zu überprüfen und/oder zu überwachen,
das Verfahren das Screenen hinsichtlich des La-Protein- und/oder -Genexpressionsniveaus in toten Zellen in der biologischen Probe und das In-Relation-setzen des Niveaus mit dem La-Expressionsniveau in lebensfähigen neoplastischen Zellen umfasst, wobei eine Erhöhung des La-Expressionsniveaus in den toten Zellen relativ zu Niveaus in lebensfähigen neoplastischen Zellen durch DNA-Schädigung induzierten neoplastischen Zelltod und damit das Vorhandensein einer neoplastischen Zelle, die durch ein DNA-schädigendes Agens getötet wurde, oder die Effektivität des therapeutischen Behandlungsregimes anzeigt.

2. Verfahren nach Anspruch 1, wobei das DNA-schädigende Agens:
(a) ein cytotoxisches Agens ist; oder
(b) ausgewählt ist aus:
(i) einem Radioisotop;
(ii) Gemcitabine zusammen mit einem CHK1/2-Inbibitor;
(iii) Irinotecan zusammen mit einem CHK1/2-Inhibitor;
(iv) einem Histondeacetylase-Inhibitor;
(v) einem mit dem Tumornekrosefaktor verwandten, Apoptoseinduzierenden Liganden.

3. Verfahren nach Anspruch 2, wobei:
(a) das cytotoxische Agens aus Actinomycin D, Arsentrioxid, Bleomycin, Busulfan, Carboplatin, Carmustin, Chlorambucil, Cisplatin, Cyclophosphamid, Daunorubicin, Doxorubicin, Epirubicin, Etoposid, Hydroxyharnstoff, Idarubicin, Ifosfamid, Irinotecan, Lomustin, Melphalan, Mitomycin, Mitoxantron, Oxaliplatin, Procarbizin, Streptozocin, Thiotepa, Topotecan, Treosulfan ausgewählt ist;
(b) es sich bei dem CHK1/2-Inbibitor um CBP-501 oder AZD7762 handelt; oder
(c) es sich bei dem Histondeacetylase-Inhibitor um Vorinostat oder ein BH3-Mimetikum wie ABT737 handelt.

4. Verfahren nach einem der Ansprüche 1 bis 3, wobei es sich bei der Neoplasie um einen Tumor des zentralen Nervensystems, ein Retinoblastom, ein Neuroblastom, einen pädiatrischen Tumor, einen Kopf- und Halskrebs, z. B. einen Plattenepithelkrebs, Brustkrebs, Prostatakrebs, Lungenkrebs, Nierenkrebs, z. B. ein Nierenzelladenokarzinom, einen Krebs der Speiseröhre oder des Magens, ein hepatozelluläres Karzinom, eine pankreatikobiliäre Neoplasie, z. B. ein Adenokarzinom und einen Inselzelltumor, Kolorektalkrebs, Gebärmutterhalskrebs, Anuskrebs, Uteruskrebs oder einen anderen Krebs des Fortpflanzungstrakts, Harnwegskrebs, z. B. des Harnleiters oder der Blase, einen Keimzelltumor, z. B. einen testikulären Keimzelltumor oder einen ovarialen Keimzelltumor, Eierstockkrebs, wie z. B. Eierstockepithelkrebs, ein Karzinom infolge einer unbekannten, mit primärer humaner Immundefizienz assoziierten Malignität wie z. B. Kaposi-Sarkom, ein Lymphom, Leukämie, ein malignes Melanom, ein Sarkom, einen endokrinen Tumor, z. B. der Schilddrüse, ein Mesotheliom oder einen anderen Pleura- oder Peritonealtumor, einen neuroendokrinen Tumor oder einen carcinoiden Tumor handelt.

5. Verfahren nach einem der Ansprüche 1 bis 4, wobei die Neoplasie maligne ist.

6. Verfahren nach einem der Ansprüche 1 bis 5, wobei La durch ein immuninteraktives Molekül detektiert wird, welches auf das La-Protein oder ein Fragment davon abzielt.

7. Verfahren nach Anspruch 6, wobei es sich bei dem immuninteraktiven Molekül um einen Antikörper oder ein Fragment davon oder um ein T-Zell-assoziiertes antigenbindendes Molekül handelt.

8. Verfahren nach Anspruch 7, wobei es sich bei dem Antikörper um einen monoklonalen Antikörper oder um ein Fragment davon oder um einen polyklonalen Antikörper oder um ein Fragment davon handelt, wobei der Antikörper bzw. das Antikörperfragment optional mit einem Reporter-Molekül assoziiert ist.

9. Verfahren nach einem der Ansprüche 7 bis 8, wobei der Antikörper oder das Fragment davon aus einem einzelkettigen Antikörper, einem deimmunisierten Antikörper, einem Fv-, Fab-, Fab'- oder F(ab')₂-Fragment, einem einzelkettigen Fv-Fragment, einem Antikörper, einem Disulfid-stabilisierten Fv-Fragment oder einer Variable-Region-Einzeldomäne (dAb) ausgewählt ist.

10. Verfahren nach Anspruch 9, wobei es sich bei dem deimmunisierten Antikörper um einen humanisierten Antikörper handelt.

11. Verfahren nach Anspruch 8, wobei das Reportermolekül aus einem Radioisotop, einem Chromogen, einem Katalysator, einem Enzym, einem Fluorochrom, einem chemilumineszierenden Molekül, einem paramagnetischen Ion, einem Lanthanidnion, einem kolloidalen metallischen oder nicht-metallischen Partikel, einem Farbstoffpartikel, einem organischen Polymer, einem Latexpartikel, einem Liposom oder einem Vesikel, das eine Signal erzeugende Substanz enthält, ausgewählt ist.

12. Verfahren nach Anspruch 11, wobei es sich bei dem Radioisotop um Kohlenstoff-11, Kupfer-64, Fluor-18, Gallium-68, Indium-111, Lutetium-177, Stickstoff-13, Iod-131, Iod-124, Sauerstoff-15, Rhenium-186/188 oder Technetium-99 handelt.

13. Verfahren nach einem der Ansprüche 1 bis 12, wobei das La im Cytoplasma lokalisiert ist.

14. Verfahren nach einem der Ansprüche 6 bis 13, wobei das immuninteraktive Molekül fixiert wird.

15. Verfahren nach einem der Ansprüche 1 bis 14, wobei es sich bei dem Individuum um einen Menschen handelt.

16. Immuninteraktives Molekül, das zur Offenbarung veränderter Expressionsniveaus eines La-Proteinexpressionsprodukts in der Lage ist, zur *In*-*vivo*-Verwendung in einem diagnostischen oder prognostischen Verfahren für
(a) die Diagnose oder Prognose eines neoplastischen Zustands in einem Individuum, wobei die Diagnose bzw. Prognose Folgendes umfasst:
(i) Detektieren einer neoplastischen Zelle in dem Individuum durch Screenen hinsichtlich des La-Proteinexpressionsniveaus in toten Zellen in dem Individuum und In-Relation-setzen dieses Niveaus zu einem normalen La-Proteinexpressionsniveau in einem Individuum, das keinen neoplastischen Zustand entwickelt hat, wobei eine Erhöhung des La-Expressionsniveaus relativ zu normalen La-Expressionsniveaus eine neoplastische Zelle anzeigt; oder
(ii) Detektieren einer toten neoplastischen Zelle in dem Individuum, deren Zelltod durch ein DNA-schädigendes Agens induziert worden ist, durch Screenen hinsichtlich des La-Proteinexpressionsniveaus in toten Zellen in dem Individuum und In-Relation-setzen dieses Niveaus zu dem La-Expressionsniveau in lebensfähigen neoplastischen Zellen, wobei eine Erhöhung des La-Expressionsniveaus relativ zu La-Expressionsniveaus in lebensfähigen neoplastischen Zellen durch DNA-Schädigung induzierten neoplastischen Zelltod anzeigt; oder
(b) ein Verfahren zum Überprüfen und/oder Überwachen eines neoplastischen Zustands oder der Effektivität eines neoplastischen therapeutischen Behandlungsschemas in einem Individuum, wobei das Verfahren das Screenen hinsichtlich des La-Proteinexpressionsniveaus in toten Zellen in dem Individuum und In-Relation-setzen dieses Niveaus zu dem La-Expressionsniveau zu einen normalen La-Expressionsniveau oder zu dem La-Expressionsniveau in lebensfähigen neoplastischen Zellen umfasst, wobei eine Erhöhung des La-Niveaus in den Zellen relativ zu normalen Niveaus eine neoplastische Zelle anzeigt und eine Erhöhung des La-Niveaus in toten Zellen relativ zu Niveaus in lebensfähigen neoplastischen Zellen das Vorhandensein einer durch DNA-Schädigung induzierten Nichtlebensfähigkeit neoplastischer Zellen anzeigt;
wobei es sich bei dem immuninteraktiven Molekül um einen Antikörper oder ein Fragment davon oder um ein T-Zell-assoziiertes, Antigen-bindendes Molekül handelt und
wobei das immuninteraktive Molekül mit einem Bildgebungsagens gekoppelt ist, um eine Detektion zu ermöglichen.

17. Immuninteraktives Molekül zur Verwendung nach Anspruch 16, wobei das DNA-schädigende Agens:
a) ein cytotoxisches Agens ist; oder
b) ausgewählt ist aus:
(i) einem Radioisotop;
(ii) Gemcitabine zusammen mit einem CHK1/2-Inbibitor;
(iii) Irinotecan zusammen mit einem CHK1/2-Inhibitor;
(iv) einem Histondeacetylase-Inhibitor;
(v) einem mit dem Tumornekrosefaktor verwandten, Apoptoseinduzierenden Liganden.

18. Immuninteraktives Molekül zur Verwendung nach Anspruch 17, wobei:
(a) das cytotoxische Agens aus Actinomycin D, Arsentrioxid, Bleomycin, Busulfan, Carboplatin, Carmustin, Chlorambucil, Cisplatin, Cyclophosphamid, Daunorubicin, Doxorubicin, Epirubicin, Etoposid, Hydroxyharnstoff, Idarubicin, Ifosfamid, Irinotecan, Lomustin, Melphalan, Mitomycin, Mitoxantron, Oxaliplatin, Procarbizin, Streptozocin, Thiotepa, Topotecan, Treosulfan ausgewählt ist;
(b) es sich bei dem CHK1/2-Inbibitor um CBP-501 oder AZD7762 handelt; oder
(c) es sich bei dem Histondeacetylase-Inhibitor um Vorinostat oder ein BH3-Mimetikum wie ABT737 handelt.

19. Immuninteraktives Molekül zur Verwendung nach einem der Ansprüche 16 bis 18, wobei es sich bei der Neoplasie um einen Tumor des zentralen Nervensystems, ein Retinoblastom, ein Neuroblastom, einen pädiatrischen Tumor, einen Kopf- und Halskrebs, z. B. einen Plattenepithelkrebs, Brustkrebs, Prostatakrebs, Lungenkrebs, Nierenkrebs, z. B. ein Nierenzelladenokarzinom, einen Krebs der Speiseröhre oder des Magens, ein hepatozelluläres Karzinom, eine pankreatikobiliäre Neoplasie, z. B. ein Adenokarzinom und einen Inselzelltumor, Kolorektalkrebs, Gebärmutterhalskrebs, Anuskrebs, Uteruskrebs oder einen anderen Krebs des Fortpflanzungstrakts, Harnwegskrebs, z. B. des Harnleiters oder der Blase, einen Keimzelltumor, z. B. einen testikulären Keimzelltumor oder einen ovarialen Keimzelltumor, Eierstockkrebs, wie z. B. Eierstockepithelkrebs, ein Karzinom infolge einer unbekannten, mit primärer humaner Immundefizienz assoziierten Malignität wie z. B. Kaposi-Sarkom, ein Lymphom, Leukämie, ein malignes Melanom, ein Sarkom, einen endokrinen Tumor, z. B. der Schilddrüse, ein Mesotheliom oder einen anderen Pleura- oder Peritonealtumor, einen neuroendokrinen Tumor oder einen carcinoiden Tumor handelt.

20. Immuninteraktives Molekül zur Verwendung nach einem der Ansprüche 16 bis 19, wobei die Neoplasie maligne ist.

21. Immuninteraktives Molekül zur Verwendung nach einem der Ansprüche 16 bis 20, wobei es sich bei dem Antikörper um einen monoklonalen Antikörper oder um ein Fragment davon oder um einen polyklonalen Antikörper oder um ein Fragment davon handelt.

22. Immuninteraktives Molekül zur Verwendung nach Anspruch 21, wobei der Antikörper oder das Fragment davon aus einem einzelkettigen Antikörper, einem deimmunisierten Antikörper, einem Fv-, Fab-, Fab'- oder F(ab')2-Fragment, einem einzelkettigen Fv-Fragment, einem Antikörper, einem Disulfid-stabilisierten Fv-Fragment oder einer Variable-Region-Einzeldomäne (dAb) ausgewählt ist.

23. Immuninteraktives Molekül zur Verwendung nach Anspruch 22, wobei es sich bei dem deimmunisierten Antikörper um einen humanisierten Antikörper handelt.

24. Immuninteraktives Molekül zur Verwendung nach Anspruch 16, wobei das Bildgebungsagens aus einem Radioisotop, einem PET-Bildgebungsagens, einem Chromogen, einem Katalysator, einem Enzym, einem Fluorochrom, einem chemilumineszierenden Molekül, einem paramagnetischen Ion, einem Lanthanidnion, einem kolloidalen metallischen oder nicht-metallischen Partikel, einem Farbstoffpartikel, einem organischen Polymer, einem Latexpartikel, einem Liposom oder einem Nanopartikel mit erweiterter Funktion ausgewählt ist.

25. Immuninteraktives Molekül zur Verwendung nach Anspruch 24, wobei es sich bei dem Radioisotop um Kohlenstoff-11, Kupfer-64, Fluor-18, Gallium-68, Indium-111, Lutetium-177, Stickstoff-13, Iod-131, Iod-124, Sauerstoff-15, Rhenium-186/188 oder Technetium-99 handelt.

26. Immuninteraktives Molekül zur Verwendung nach einem der Ansprüche 16 bis 25, wobei das La im Cytoplasma lokalisiert ist.

27. Immuninteraktives Molekül zur Verwendung nach einem der Ansprüche 16 bis 26, wobei das immuninteraktive Molekül fixiert wird.

28. Immuninteraktives Molekül zur Verwendung nach einem der Ansprüche 16 bis 27, wobei es sich bei dem Individuum um einen Menschen handelt.

## Revendications

1. Procédé *in vitro* de détection d'une cellule néoplasique chez un sujet, d'évaluation ou de surveillance d'une affection néoplasique chez un sujet, ou d'évaluation et/ou de surveillance de l'efficacité d'un régime de traitement thérapeutique antinéoplasique chez un sujet, ledit procédé comprenant le dépistage d'un changement du niveau de protéine et/ou d'expression génétique La dans des cellules dans un échantillon biologique dérivé dudit sujet, dans lequel
(a) pour détecter une cellule néoplasique ou pour évaluer ou surveiller une affection néoplasique ledit procédé comprend la mise en corrélation des niveaux de protéine et/ou d'expression génétique La dans des cellules dans ledit échantillon avec un niveau normal de protéine et/ou d'expression génétique La d'un échantillon biologique provenant d'un sujet qui n'a pas développé d'affection néoplasique, dans lequel une augmentation du niveau de ladite expression La dans des cellules du sujet par rapport à des niveaux normaux d'expression La indique une cellule néoplasique,
(b) pour détecter une cellule néoplasique morte, laquelle mort cellulaire a été induite par un agent d'endommagement d'ADN, ou
(c) pour évaluer et/ou surveiller l'efficacité d'un régime de traitement thérapeutique antinéoplasique chez un sujet,
ledit procédé comprend le dépistage du niveau de protéine et/ou d'expression génétique La dans des cellules mortes dans ledit échantillon biologique et la mise en corrélation dudit niveau avec le niveau d'expression La dans des cellules néoplasiques viables, dans lequel une augmentation du niveau d'expression La dans lesdites cellules mortes par rapport aux niveaux dans des cellules néoplasiques viables indique une mort de cellules néoplasiques induite par un endommagement d'ADN, et par conséquent la présence d'une cellule néoplasique rendue morte par un agent d'endommagement d'ADN ou l'efficacité dudit régime de traitement thérapeutique.

2. Procédé selon la revendication 1 dans lequel ledit agent d'endommagement d'ADN est :
(a) un agent cytotoxique ; ou
(b) sélectionné parmi :
(i) un radio-isotope ;
(ii) la gemcitabine conjointement avec un inhibiteur de CHK1/2 ;
(iii) l'irinotécan conjointement avec un inhibiteur de CHK1/2 ;
(iv) un inhibiteur d'histone désacétylase ;
(v) un ligand induisant une apoptose associée au facteur de nécrose tumorale.

3. Procédé selon la revendication 2 dans lequel :
(a) ledit agent cytotoxique est sélectionné parmi l'actinomycine D, le trioxyde d'arsénique, la bléomycine, le busulfan, le carboplatine, la carmustine, le chlorambucil, le cisplatine, le cyclophosphamide, la daunorubicine, la doxorubicine, l'épirubicine, l'étoposide, l'hydroxyurée, l'idarubicine, l'ifosfamide, l'irinotécan, la lomustine, le melphalan, la mitomycine, la mitoxantrone, l'oxaliplatine, la procarbizine, la streptozocine, le thiotépa, le topotécan, le tréosulfan ;
(b) ledit inhibiteur de CHK1/2 est le CBP-501 ou l'AZD7762 ; ou
(c) ledit inhibiteur d'histone désacétylase est le vorinostat ou un mimétique BH3 tel que ABT737.

4. Procédé selon l'une quelconque des revendications 1 à 3 dans lequel ledit néoplasme est une tumeur du système nerveux central, un rétinoblastome, un neuroblastome, une tumeur pédiatrique, un cancer de la tête et du cou comme un cancer squameux, un cancer du sein, un cancer de la prostate, un cancer du poumon, un cancer du rein comme un adénocarcinome des cellules rénales, un cancer oesophago-gastrique, un carcinome hépatocellulaire, une néoplasie pancréaticobiliaire comme un adénocarcinome et une tumeur des cellules des îlots, un cancer colorectal, un cancer du col de l'utérus, un cancer anal, un cancer de l'utérus ou un autre cancer de l'appareil génital, un cancer du tractus urinaire comme de l'urètre ou de la vessie, une tumeur des cellules germinales comme une tumeur des cellules germinales testiculaires ou une tumeur des cellules germinales ovariennes, un cancer des ovaires comme un cancer de l'épithélium ovarien, un carcinome d'une malignité associée à une immunodéficience humaine primaire inconnue comme le carcinome de Kaposi, un lymphome, une leucémie, un mélanome malin, un sarcome, une tumeur endocrine comme de la glande thyroïde, un mésothéliome ou une autre tumeur pleurale ou péritonéale, une tumeur neuroendocrinienne ou une tumeur carcinoïde.

5. Procédé selon l'une quelconque des revendications 1 à 4 dans lequel ledit néoplasme est malin.

6. Procédé selon l'une quelconque des revendications 1 à 5 dans lequel La est détectée par une molécule immuno-interactive dirigée vers la protéine La ou un fragment de celle-ci.

7. Procédé selon la revendication 6 dans lequel ladite molécule immuno-interactive est un anticorps ou un fragment de celui-ci ou une molécule se liant à un antigène associée aux lymphocytes T.

8. Procédé selon la revendication 7 dans lequel ledit anticorps est un anticorps monoclonal ou un fragment de celui-ci ou un anticorps polyclonal ou un fragment de celui-ci, facultativement dans lequel ledit anticorps ou fragment d'anticorps est associé à une molécule rapporteur.

9. Procédé selon l'une quelconque des revendications 7 et 8 dans lequel ledit anticorps ou fragment de celui-ci est sélectionné parmi un anticorps à chaîne unique, un anticorps désimmunisé, un fragment Fv, Fab, Fab', ou F(ab')₂, un fragment Fv à chaîne unique, un anticorps, un fragment Fv stabilisé par disulfure, ou un domaine de région variable unique (dAb).

10. Procédé selon la revendication 9 dans lequel l'anticorps désimmunisé est un anticorps humanisé.

11. Procédé selon la revendication 8 dans lequel ladite molécule rapporteur est sélectionnée parmi un radio-isotope, un chromogène, un catalyseur, une enzyme, un fluorochrome, une molécule chimioluminescente, un ion paramagnétique, un ion de lanthanide, une particule métallique ou non métallique colloïdale, une particule de colorant, un polymère organique, une particule de latex, un liposome ou une vésicule contenant une substance produisant un signal.

12. Procédé selon la revendication 11 dans lequel ledit radio-isotope est le carbone-11, le cuivre-64, le fluor 18, le gallium-68, l'indium-111, le lutétium-177, l'azote-13, l'iode-131, l'iode-124, l'oxygène-15, le rhénium-186/188, ou le technétium-99.

13. Procédé selon l'une quelconque des revendications 1 à 12 dans lequel ladite La est localisée dans le cytoplasme.

14. Procédé selon l'une quelconque des revendications 6 à 13 dans lequel ladite molécule immuno-interactive devient fixée.

15. Procédé selon l'une quelconque des revendications 1 à 14 dans lequel ledit sujet est un humain.

16. Molécule immuno-interactive capable de révéler des niveaux d'expression altérés d'un produit d'expression de protéine La pour une utilisation *in vivo* dans un procédé de diagnostic ou de pronostic pour
(a) le diagnostic ou le pronostic d'une affection néoplasique chez un sujet, dans laquelle ledit diagnostic ou pronostic comprend
(i) la détection d'une cellule néoplasique chez ledit sujet, par le dépistage du niveau d'expression de protéine La dans des cellules mortes chez ledit sujet et la mise en corrélation de celui-ci avec un niveau normal d'expression de protéine La chez un sujet qui n'a pas développé d'affection néoplasique dans laquelle une augmentation du niveau de ladite expression La par rapport à des niveaux normaux d'expression La indique une cellule néoplasique ; ou
(ii) la détection d'une cellule néoplasique morte chez ledit sujet, laquelle mort cellulaire a été induite par un agent d'endommagement d'ADN, par le dépistage du niveau d'expression de protéine La dans des cellules mortes chez ledit sujet et la mise en corrélation dudit niveau avec le niveau d'expression La dans des cellules néoplasiques viables, dans laquelle une augmentation du niveau d'expression La par rapport à des niveaux d'expression La de cellules néoplasiques viables indique une mort de cellules néoplasiques induites par un endommagement d'ADN ; ou
(b) un procédé d'évaluation et/ou de surveillance d'une affection néoplasique ou de l'efficacité d'un régime de traitement thérapeutique antinéoplasique chez un sujet, ledit procédé comprenant le dépistage du niveau d'expression de protéine La dans des cellules mortes chez ledit sujet, et la mise en corrélation dudit niveau avec le niveau d'expression La avec un niveau normal d'expression La ou avec le niveau d'expression La dans des cellules néoplasiques viables, dans laquelle une augmentation du niveau de La dans lesdites cellules par rapport aux niveaux normaux indique une cellule néoplasique et une augmentation du niveau de La dans des cellules mortes par rapport à des niveaux de cellules néoplasiques viables indique la présence d'une non-viabilité de cellule néoplasique induite par un endommagement d'ADN ;
dans laquelle ladite molécule immuno-interactive est un anticorps ou un fragment d'anticorps, ou une molécule se liant à un antigène associée aux lymphocytes T, et
dans laquelle ladite molécule immuno-interactive est couplée à un agent d'imagerie pour permettre une détection.

17. Molécule immuno-réactive pour son utilisation selon la revendication 16 dans laquelle ledit agent d'endommagement d'ADN est :
(a) un agent cytotoxique ; ou
(b) sélectionné parmi :
(i) un radio-isotope ;
(ii) la gemcitabine conjointement avec un inhibiteur de CHK1/2 ;
(iii) l'irinotécan conjointement avec un inhibiteur de CHK1/2 ;
(iv) un inhibiteur d'histone désacétylase ;
(v) un ligand induisant une apoptose associée au facteur de nécrose tumorale.

18. Molécule immuno-réactive pour son utilisation selon la revendication 17 dans laquelle :
(a) ledit agent cytotoxique est sélectionné parmi l'actinomycine D, le trioxyde d'arsénique, la bléomycine, le busulfan, le carboplatine, la carmustine, le chlorambucil, le cisplatine, le cyclophosphamide, la daunorubicine, la doxorubicine, l'épirubicine, l'étoposide, l'hydroxyurée, l'idarubicine, l'ifosfamide, l'irinotécan, la lomustine, le melphalan, la mitomycine, la mitoxantrone, l'oxaliplatine, la procarbizine, la streptozocine, le thiotépa, le topotécan, le tréosulfan ;
(b) ledit inhibiteur de CHK1/2 est le CBP-501 ou l'AZD7762 ; ou
(c) ledit inhibiteur d'histone désacétylase est le vorinostat ou un mimétique BH3 tel que ABT737.

19. Molécule immuno-réactive pour son utilisation selon l'une quelconque des revendications 16 à 18 dans laquelle ledit néoplasme est une tumeur du système nerveux central, un rétinoblastome, un neuroblastome, une tumeur pédiatrique, un cancer de la tête et du cou comme un cancer squameux, un cancer du sein, un cancer de la prostate, un cancer du poumon, un cancer du rein comme un adénocarcinome des cellules rénales, un cancer oesophago-gastrique, un carcinome hépatocellulaire, une néoplasie pancréaticobiliaire comme un adénocarcinome et une tumeur des cellules des îlots, un cancer colorectal, un cancer du col de l'utérus, un cancer anal, un cancer de l'utérus ou un autre cancer de l'appareil génital, un cancer du tractus urinaire comme de l'urètre ou de la vessie, une tumeur des cellules germinales comme une tumeur des cellules germinales testiculaires ou une tumeur des cellules germinales ovariennes, un cancer des ovaires comme un cancer de l'épithélium ovarien, un carcinome d'une malignité associée à une immunodéficience humaine primaire inconnue comme le carcinome de Kaposi, un lymphome, une leucémie, un mélanome malin, un sarcome, une tumeur endocrine comme de la glande thyroïde, un mésothéliome ou une autre tumeur pleurale ou péritonéale, une tumeur neuroendocrinienne ou une tumeur carcinoïde.

20. Molécule immuno-réactive pour son utilisation selon l'une quelconque des revendications 16 à 19 dans laquelle ledit néoplasme est malin.

21. Molécule immuno-réactive pour son utilisation selon l'une quelconque des revendications 16 à 20 dans laquelle ledit anticorps est un anticorps monoclonal ou un fragment de celui-ci ou est un anticorps polyclonal ou un fragment de celui-ci.

22. Molécule immuno-réactive pour son utilisation selon la revendication 21 dans laquelle ledit anticorps ou fragment de celui-ci est sélectionné parmi un anticorps à chaîne unique, un anticorps désimmunisé, un fragment Fv, Fab, Fab', ou F(ab')₂, un fragment Fv à chaîne unique, un anticorps, un fragment Fv stabilisé par disulfure, ou un domaine de région variable unique (dAb).

23. Molécule immuno-réactive pour son utilisation selon la revendication 22 dans laquelle l'anticorps désimmunisé est un anticorps humanisé.

24. Molécule immuno-réactive pour son utilisation selon la revendication 16 dans laquelle ledit agent d'imagerie est sélectionné parmi un radio-isotope, un agent pour imagerie PET, un chromogène, un catalyseur, une enzyme, un fluorochrome, une molécule chimioluminescente, un ion paramagnétique, un ion de lanthanide, une particule métallique ou non métallique colloïdale, une particule de colorant, un polymère organique, une particule de latex, un liposome ou une nanoparticule fonctionnelle avancée.

25. Molécule immuno-réactive pour son utilisation selon la revendication 24 dans laquelle ledit radio-isotope est le carbone-11, le cuivre-64, le fluor 18, le gallium-68, l'indium-111, le lutétium-177, l'azote-13, l'iode-131, l'iode-124, l'oxygène-15, le rhénium-186/188, ou le technétium-99.

26. Molécule immuno-réactive pour son utilisation selon l'une quelconque des revendications 16 à 25 dans laquelle ladite La est localisée dans le cytoplasme.

27. Molécule immuno-réactive pour son utilisation selon l'une quelconque des revendications 16 à 26 dans laquelle ladite molécule immuno-interactive devient fixée.

28. Molécule immuno-réactive pour son utilisation selon l'une quelconque des revendications 16 à 27 dans laquelle ledit sujet est un humain.
